# EUROPEAN PATENT APPLICATION

(11) **EP 3 825 313 A1**
(43) Date of publication of application: **26.05.2021**
(21) Application number: 20201882.6
(22) Date of filing: 14.10.2016
(51) Int. Cl.: C07D 403/12, C07D 401/14, C07D 401/12, C07D 405/12, C07D 413/12, C07D 417/12, A61K 31/4412, A61P 35/00

(54) **PYRIDIN-2(1H)-ONE DERIVATIVES AS BROMODOMAIN INHIBITORS**

(62) Divisional of application: 16918654.1
(71) Applicant: AbbVie Inc., North Chicago, IL 60064 (US)
(72) Inventor: COWART, Marlon, Round Lake Beach, IL 60073 (US); FIDANZE, Steven, Grayslake, IL 60030 (US); HASVOLD, Lisa, Grayslake, IL 60030 (US); LIU, Dachun, Vernon Hills, IL 60061 (US); MCDANIEL, Keith, Wauconda, IL 60084 (US); PRATT, John, Kenosha, WI 53142 (US); WANG, Le, Vernon Hills, IL 60061 (US)
(74) Representative: Schlich, George

(57) **Abstract**

The present invention provides for compounds of formula (I) 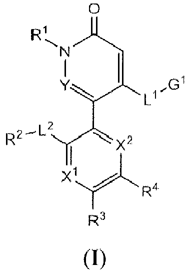 wherein R¹, Y, L¹, G¹, X¹, X², L², R², R³, and R⁴ have any of the values defined in the specification, and pharmaceutically acceptable salts thereof, that are useful as agents in the treatment of diseases and conditions, including inflammatory diseases, cancer, and AIDS. Also provided are pharmaceutical compositions comprising compounds of formula (I).

## Description

### BACKGROUND

Bromodomains refer to conserved protein structural folds which bind to *N*-acetylated lysine residues that are found in some proteins. The BET family of bromodomain containing proteins comprises four members (BRD2, BRD3, BRD4 and BRDt). Each member of the BET family employs two bromodomains to recognize *N*-acetylated lysine residues typically, but not exclusively those found on transcription factors (Shi, J., et al. Cancer Cell 25(2): 210-225 (2014)) or on the amino-terminal tails of histone proteins. Numbering from the N-terminal end of each BET protein the tandem bromodomains are typically labelled Binding Domain I (BDI) and Binding Domain II (BDII). These interactions modulate gene expression by recruiting transcription factors to specific genome locations within chromatin. For example, histone-bound BRD4 recruits the transcription factor P-TEFb to promoters, resulting in the expression of a subset of genes involved in cell cycle progression (Yang et al., Mol. Cell. Biol. 28: 967-976 (2008)). BRD2 and BRD3 also function as transcriptional regulators of growth promoting genes (LeRoy et al., Mol. Cell 30: 51-60 (2008)). BET family members were recently established as being important for the maintenance of several cancer types (Zuber et al., Nature 478: 524-528 (2011); Mertz et al; Proc. Nat'l. Acad. Sci. 108: 16669-16674 (2011); Delmore et al., Cell 146: 1-14, (2011); Dawson et al., Nature 478: 529-533 (2011)). BET family members have also been implicated in mediating acute inflammatory responses through the canonical NF-KB pathway (Huang et al., Mol. Cell. Biol. 29: 1375-1387 (2009)) resulting in the upregulation of genes associated with the production of cytokines (Nicodeme et al., Nature 468: 1119-1123, (2010)). Suppression of cytokine induction by BET bromodomain inhibitors has been shown to be an effective approach to treat inflammation-mediated kidney disease in an animal model (Zhang, et al., J. Biol. Chem. 287: 28840-28851 (2012)). BRD2 function has been linked to pre-disposition for dyslipidemia or improper regulation of adipogenesis, elevated inflammatory profiles and increased susceptibility to autoimmune diseases (Denis, Discovery Medicine 10: 489-499 (2010)). The human immunodeficiency virus utilizes BRD4 to initiate transcription of viral RNA from stably integrated viral DNA (Jang et al., Mol. Cell, 19: 523-534 (2005)). BET bromodomain inhibitors have also been shown to reactivate HIV transcription in models of latent T cell infection and latent monocyte infection (Banerjee, et al, J. Leukocyte Biol. doi:10.1189/jlb.0312165). BRDt has an important role in spermatogenesis that is blocked by BET bromodomain inhibitors (Matzuk, et al., Cell 150: 673-684 (2012)). Thus, compounds that inhibit the binding of BET family bromodomains to their cognate acetylated lysine proteins are being pursued for the treatment of cancer, inflammatory diseases, kidney diseases, diseases involving metabolism or fat accumulation, and some viral infections, as well as for providing a method for male contraception. Accordingly, there is an ongoing medical need to develop new drugs to treat these indications.

### SUMMARY

In one aspect the present invention provides for compounds of formula (I) or a pharmaceutically acceptable salt thereof, 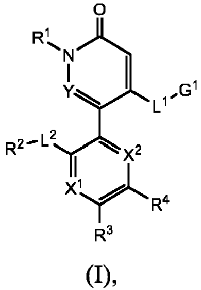 wherein
R¹ is C₁-C₃ alkyl;
Y is N or C(R^{Y}) wherein R^{Y} is hydrogen or C₁-C₃ alkyl;
L¹ is O or N(R^{x}) wherein R^{x} is hydrogen or C₁-C₃ alkyl;
G¹ is a 4-11 membered monocyclic, bicyclic, or polycyclic hydrocarbon ring with zero, one, or two double bonds, wherein one or two carbon ring atoms of G¹ are optionally replaced by heteroatoms selected from the group consisting of N, O, and S; the rings within the polycyclic and bicyclic are in a bridged, fused, or spiro orientation, or combinations thereof; each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2G})₂, -C(O)R^{2g}, cyclopropyl, and cyclobutyl; wherein each R^{2g} is independently hydrogen, C₁-C₃ alkyl, or C₁-C₃ haloalkyl;
R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂k^{b} -N(R^{a})C(O)R^{b}, -N(R^{a})C(O)C(O)R^{b}, -N(R^{d})N(R^{c})C(O)R^{b}, -N(R^{a})C(O)OR^{b}, -N(R^{d})N(R^{c})C(O)OR^{b}, -N(R^{a})C(O)N(R^{b})₂, -N(R^{a})(C₁-C₃ alkylenyl)-C(O)R^{b}, -N(R^{a})(C₁-C₃ alkylenyl)-S(O)₂R^{b}, or C₁-C₆ alkyl substituted with an substituent selected from the group consisting of -OR^{b}, -C(O)R^{b}, -C(O)OR^{c}, -C(O)N(R^{b})₂, -S(O)₂R^{b}, -N(R^{a})S(O)2R^{b}, -N(R^{a})C(O)R^{b}, -N(R^{a})C(O)OR^{b}, and -N(R^{a})C(O)N(R^{b})₂;
R^{a}, at each occurrence, is independently hydrogen, C₁-C₆ alkyl, C₁-C₆ haloalkyl, C₂-C₆ alkenyl, -N(R^{j})₂, -(C₂-C₆ alkylenyl)-OR^{j}, or -(C₁-C₆ alkylenyl)-C(O)OR^{j};
R^{b}, at each occurrence, is independently hydrogen, C₁-C₆ alkyl, C₂-C₆ alkenyl, C₁-C₆ haloalkyl, G^{1B}, -(C₁-C₆ alkylenyl)-OR^{j}, -(C₁-C₆ alkylenyl)-N(R^{j})₂, or -(C₁-C₆ alkylenyl)-C(O)OR^{j};
R^{c}, at each occurrence, is independently hydrogen, C₁-C₆ alkyl, C₂-C₆ alkenyl, or C₁-C₆ haloalkyl;
R^{d}, at each occurrence, is independently hydrogen, C₁-C₆ alkyl, C₂-C₆ alkenyl, C₁-C₆ haloalkyl, or -C(O)R^{b};
G^{1A} is phenyl, C₃-C₁₁ cycloalkyl, 4-11 membered heterocycle, or 5-11 membered heteroaryl; wherein each G^{1A} is optionally substituted with 1, 2, 3, 4, or 5 independently selected R^{s} groups;
G^{1B} is phenyl, C₃-C₁₁ cycloalkyl, 4-11 membered heterocycle, or 5-11 membered heteroaryl; wherein each G^{1B} is optionally substituted with 1, 2, 3, or 4 independently R^{t} groups;
L² is O or N(R^{e}) wherein R^{e} is hydrogen or C₁-C₃ alkyl;
R² is phenyl or monocyclic heteroaryl; each R² is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH;
R³ is hydrogen, halogen, -CN, C₁-C₆ haloalkyl, or C₁-C₆ alkyl;
R⁴ is 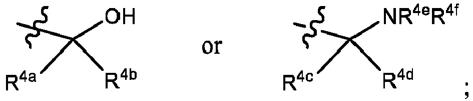 wherein
   R^{4a} is C₁-C₆ alkyl or C₁-C₆ haloalkyl, wherein the C₁-C₆ alkyl and the C₁-C₆ haloalkyl are each optionally substituted with one substituent selected from the group consisting of -OH and -CN;
   R^{4b} is C₁-C₆ alkyl or C₁-C₆ haloalkyl;
   R^{4c} and R^{4d} are each independently hydrogen, C₁-C₆ alkyl, or C₁-C₆ haloalkyl;
   R^{4e} is hydrogen, C₁-C₃ alkyl, C₂-C₄ alkenyl, C₁-C₃ haloalkyl, or -(C₁-C₃ alkylenyl)-G^{1C}; wherein G^{1C} is phenyl, monocyclic heteroaryl, monocyclic C₃-C₆ cycloalkyl, or 4-6 membered monocyclic heterocycle; wherein each G^{1C} is optionally substituted with 1, 2, 3, or 4 independently selected R^{u} groups;
   R^{4f} is C₁-C₃ alkyl, C₂-C₄ alkenyl, C₁-C₃ haloalkyl, -C(O)R^{4cc}, or -C(O)N(R^{4cd})(R^{4ce}); wherein R^{4cc} is C₁-C₃ alkyl, C₂-C₄ alkenyl, or C₁-C₃ haloalkyl; and R^{4cd} and R^{4ce} are each independently hydrogen, C₁-C₃ alkyl, C₂-C₄ alkenyl, or C₁-C₃ haloalkyl;
X¹ and X² are C(R⁵) or
one of X¹ and X² is N and the other is C(R⁵);
R⁵, at each occurrence, is independently hydrogen or halogen;
R^{S}, R^{t}, and R^{u}, at each occurrence, are each independently C₁-C₆ alkyl, C₂-C₆ alkenyl, C₂-C₆ alkynyl, halogen, C₁-C₆ haloalkyl, -CN, oxo, NO₂, -OR^{j}, -OC(O)R^{k}, -OC(O)N(R^{j})₂, -SR^{j}, -S(O)₂R^{j}, -S(O)₂N(R^{j})₂, -C(O)R^{j}, -C(O)OR^{j}, -C(O)N(R^{j})₂, -C(O)N(R^{j})S(O)₂R^{k}, -N(R^{j})₂, -N(R^{j})C(O)R^{k}, -N(R^{j})S(O)₂R^{k}, -N(R^{j})C(O)O(R^{k}), -N(R^{j})C(O)N(R^{j})₂, -(C₁-C₆ alkylenyl)-OR^{j}, -(C₁-C₆ alkylenyl)-OC(O)R^{k}, -(C₁-C₆ alkylenyl)-OC(O)N(R^{j})₂, -(C₁-C₆ alkylenyl)-SR^{j}, -(C₁-C₆ alkylenyl)-S(O)₂R^{j}, -(C₁-C₆ alkylenyl)-S(O)₂N(R^{j})₂, -(C₁-C₆ alkylenyl)-C(O)R^{j}, -(C₁-C₆ alkylenyl)-C(O)OR^{j}, -(C₁-C₆ alkylenyl)-C(O)N(R^{j})₂, -(C₁-C₆ alkylenyl)-C(O)N(R^{j})S(O)₂R^{k}, -(C₁-C₆ alkylenyl)-N(R^{j})₂, -(C₁-C₆ alkylenyl)-N(R^{j})C(O)R^{k}, -(C₁-C₆ alkylenyl)-N(R^{j})S(O)₂R^{k}, -(C₁-C₆ alkylenyl)-N(R^{j})C(O)O(R^{k}), -(C₁-C₆ alkylenyl)-N(R^{j})C(O)N(R^{j})₂, or -(C₁-C₆ alkylenyl)-CN;
R^{j}, at each occurrence, is independently hydrogen, C₁-C₆ alkyl, or C₁-C₆ haloalkyl; and
R^{k}, at each occurrence, is independently C₁-C₆ alkyl or C₁-C₆ haloalkyl.

In another aspect, the present invention provides for methods for treating or preventing disorders that are ameliorated by inhibition of BET. Such methods comprise of administering to the subject a therapeutically effective amount of a compound of formula (I), (I-a), (I-b), or (I-c), alone, or in combination with a pharmaceutically acceptable carrier.

Some of the methods are directed to treating or preventing an inflammatory disease or cancer or AIDS.

In another aspect, the present invention relates to methods of treating cancer in a subject comprising administering a therapeutically effective amount of a compound of formula (I), (I-a), (I-b), or (I-c), or a pharmaceutically acceptable salt thereof, to a subject in need thereof. In certain embodiments, the cancer is selected from the group consisting of: acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenström's macroglobulinemia, testicular tumors, uterine cancer, and Wilms' tumor. In certain embodiments, the methods further comprise administering a therapeutically effective amount of at least one additional therapeutic agent. In certain embodiments, the additional therapeutic agent is selected from the group consisting of cytarabine, bortezomib, and 5-azacitidine.

In another aspect, the present invention relates to methods of treating a disease or condition in a subject comprising administering a therapeutically effective amount of a compound of formula (I), (I-a), (I-b), or (I-c), or a pharmaceutically acceptable salt thereof, to a subject in need thereof, wherein said disease or condition is selected from the group consisting of: Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis. In certain embodiments, the methods further comprise administering a therapeutically effective amount of at least one additional therapeutic agent.

In another aspect, the present invention relates to methods of treating a chronic kidney disease or condition in a subject comprising administering a therapeutically effective amount of a compound of formula (I), (I-a), (I-b), or (I-c), or a pharmaceutically acceptable salt thereof, to a subject in need thereof, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, and tubular interstitial nephritis. In certain embodiments, the methods further comprise administering a therapeutically effective amount of at least one additional therapeutic agent.

In another aspect, the present invention relates to methods of treating an acute kidney injury or disease or condition in a subject comprising administering a therapeutically effective amount of a compound of formula (I), (I-a), (I-b), or (I-c), or a pharmaceutically acceptable salt thereof, to a subject in need thereof, wherein said acute kidney injury or disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease. In certain embodiments, the methods further comprise administering a therapeutically effective amount of at least one additional therapeutic agent.

In another aspect, the present invention relates to methods of treating AIDS in a subject comprising administering a therapeutically effective amount of a compound of formula (I), (I-a), (I-b), or (I-c), or a pharmaceutically acceptable salt thereof, to a subject in need thereof. In certain embodiments, the methods further comprise administering a therapeutically effective amount of at least one additional therapeutic agent.

In another aspect, the present invention relates to methods of treating obesity, dyslipidemia, hypercholesterolemia, Alzheimer's disease, metabolic syndrome, hepatic steatosis, type II diabetes, insulin resistance, diabetic retinopathy, or diabetic neuropathy in a subject comprising administering a therapeutically effective amount of a compound of formula (I), (I-a), (I-b), or (I-c), or a pharmaceutically acceptable salt thereof, to a subject in need thereof. In certain embodiments, the methods further comprise administering a therapeutically effective amount of at least one additional therapeutic agent.

In another aspect, the present invention relates to methods of preventing conception by inhibiting spermatogenesis in a subject comprising administering a therapeutically effective amount of a compound of formula (I), (I-a), (I-b), or (I-c), or a pharmaceutically acceptable salt thereof, to a subject in need thereof. In certain embodiments, the methods further comprise administering a therapeutically effective amount of at least one additional therapeutic agent.

A further aspect of the invention provides the use of a compound of formula (I), (I-a), (I-b), or (I-c), alone or in combination with at least one additional therapeutic agent, in the manufacture of a medicament for treating or preventing conditions and disorders disclosed herein, with or without a pharmaceutically acceptable carrier.

Pharmaceutical compositions comprising a compound of formula (I), (I-a), (I-b), or (I-c), or a pharmaceutically acceptable salt, alone or in combination with at lease one additional therapeutic agent, are also provided.

### DETAILED DESCRIPTION

Disclosed herein are compounds of formula (I) 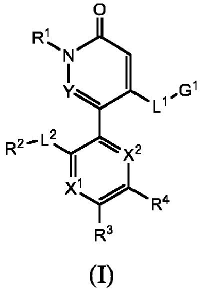 wherein R¹, Y, L¹, G¹, X¹, X², L², R², R³, and R⁴ are defined above in the Summary of the Invention and below in the Detailed Description. Further, compositions comprising such compounds and methods for treating conditions and disorders using such compounds and compositions are also disclosed.

Compounds disclosed herein may contain one or more variable(s) that occur more than one time in any substituent or in the formulae herein. Definition of a variable on each occurrence is independent of its definition at another occurrence. Further, combinations of substituents are permissible only if such combinations result in stable compounds. Stable compounds are compounds, which can be isolated from a reaction mixture.

### a. Definitions

It is noted that, as used in this specification and the intended claims, the singular form "a," "an," and "the" include plural referents unless the context clearly dictates otherwise. Thus, for example, reference to "a compound" includes a single compound as well as one or more of the same or different compounds, reference to "a pharmaceutically acceptable carrier" means a single pharmaceutically acceptable carrier as well as one or more pharmaceutically acceptable carriers, and the like.

As used in the specification and the appended claims, unless specified to the contrary, the following terms have the meaning indicated:

The term "alkenyl" as used herein, means a straight or branched hydrocarbon chain containing from 2 to 10 carbons and containing at least one carbon-carbon double bond. The term "C₂-C₆ alkenyl" means an alkenyl group containing 2-6 carbon atoms. Non-limiting examples of C₂-C₆ alkenyl include buta-1,3-dienyl, ethenyl, 2-propenyl, 2-methyl-2-propenyl, 3-butenyl, 4-pentenyl, and 5-hexenyl.

The term "alkyl" as used herein, means a saturated, straight or branched hydrocarbon chain radical. In some instances, the number of carbon atoms in an alkyl moiety is indicated by the prefix "Cₓ-C_{y}", wherein x is the minimum and y is the maximum number of carbon atoms in the substituent. Thus, for example, "C₁-C₆ alkyl" means an alkyl substituent containing from 1 to 6 carbon atoms and "C₁-C₃ alkyl" means an alkyl substituent containing from 1 to 3 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 1-methylbutyl, 2-methylbutyl, 3-methylbutyl, 3,3-dimethylbutyl, 1,1-dimethylpropyl, 1,2-dimethylpropyl, 2,2-dimethylpropyl, 1-methylpropyl, 2-methylpropyl, 1-ethylpropyl, and 1,2,2-trimethylpropyl. The terms "alkyl," "C₁-C₆ alkyl," "C₁-C₄ alkyl," and "C₁-C₃ alkyl" used herein are unsubstituted, unless otherwise indicated.

The term "alkylene" or "alkylenyl" means a divalent radical derived from a straight or branched, saturated hydrocarbon chain, for example, of 1 to 10 carbon atoms or of 1 to 6 carbon atoms (C₁-C₆ alkylenyl) or of 1 to 4 carbon atoms or of 1 to 3 carbon atoms (C₁-C₃ alkylenyl) or of 2 to 6 carbon atoms (C₂-C₆ alkylenyl). Examples of C₁-C₆ alkylenyl include, but are not limited to, -CH₂-, -CH₂CH₂-, -C((CH₃)₂)-CH₂CH₂CH₂-, -C((CH₃)₂)-CH₂CH₂, -CH₂CH₂CH₂CH₂-, and -CH₂CH(CH₃)CH₂-.

The term "C₂-C₆ alkynyl" as used herein, means a straight or branched chain hydrocarbon radical containing from 2 to 6 carbon atoms and containing at least one carbon-carbon triple bond. Representative examples of C₂-C₆ alkynyl include, but are not limited, to acetylenyl, 1-propynyl, 2-propynyl, 3-butynyl, 2-pentynyl, and 1-butynyl.

The term "C₃-C₁₁ cycloalkyl" as used herein, means a hydrocarbon ring radical containing 3-11 carbon atoms, zero heteroatom, and zero double bond. The C₃-C₁₁ cycloalkyl group may be a single-ring (monocyclic) or have two or more rings (polycyclic or bicyclic). Monocyclic cycloalkyl typically contains 3-8 carbon ring atoms (monocyclic C₃-C₈ cycloalkyl) or more typically contains 3-6 carbon ring atoms (monocyclic C₃-C₆ cycloalkyl). Monocyclic C₃-C₆ cycloalkyl groups means cyclopropyl, cyclobutyl, cyclopentyl, or cyclohexyl. Polycyclic cycloalkyl groups contain two or more rings, and bicyclic cycloalkyls contain two rings. In certain embodiments, the polycyclic cycloalkyl groups contain 2 or 3 rings. The rings within the polycyclic and the bicyclic cycloalkyl groups are in a bridged, fused, or spiro orientation, or combinations thereof. In a spirocyclic cycloalkyl, one atom is common to two different rings. An example of a spirocyclic cycloalkyl is spiro[3.3]heptanyl. In a bridged cycloalkyl, the rings share at least two non-adjacent atoms. Non limiting examples of bridged cycloalkyl include bicyclo[1.1.1]pentanyl, bicyclo[2.1.1]hexanyl, and bicyclo[2.2.2]octanyl. In a fused ring cycloalkyl, the rings share one common bond.

The term "C₄-C₆ monocyclic cycloalkenyl" as used herein, means cyclobutenyl, cyclopentenyl, and cyclohexenyl.

The term "halo" or "halogen" as used herein, means Cl, Br, I, and F.

The term "haloalkyl" as used herein, means an alkyl group, as defined herein, in which one, two, three, four, five, or six hydrogen atoms are replaced by halogen. The term "C₁-C₆ haloalkyl" means a C₁-C₆ alkyl group, as defined herein, in which one, two, three, four, five, or six hydrogen atoms are replaced by halogen. The term "C₁-C₃ haloalkyl" means a C₁-C₃ alkyl group, as defined herein, in which one, two, three, four, or five hydrogen atoms are replaced by halogen. Representative examples of haloalkyl include, but are not limited to, chloromethyl, 2-fluoroethyl, 2,2-difluoroethyl, fluoromethyl, 2,2,2-trifluoroethyl, trifluoromethyl, difluoromethyl, pentafluoroethyl, 2-chloro-3-fluoropentyl, trifluorobutyl, and trifluoropropyl. The terms "haloalkyl," "C₁-C₆ haloalkyl," and "C₁-C₃ haloalkyl," as used herein are unsubstituted, unless otherwise indicated.

The term "5-11 membered heteroaryl" as used herein, means a monocyclic heteroaryl and a bicyclic heteroaryl. The monocyclic heteroaryl is a five- or six-membered hydrocarbon ring wherein at least one carbon ring atom is replaced by heteroatom independently selected from the group consisting of O, N, and S. The five-membered ring contains two double bonds. The five membered ring may have one heteroatom selected from O or S; or one, two, three, or four nitrogen atoms and optionally one oxygen or one sulfur atom. The six-membered ring contains three double bonds and one, two, three or four nitrogen atoms. Examples of monocyclic heteroaryl include, but are not limited to, furanyl, imidazolyl, isoxazolyl, isothiazolyl, oxadiazolyl, 1,3-oxazolyl, pyridinyl, pyridazinyl, pyrimidinyl, pyrazinyl, pyrazolyl, pyrrolyl, tetrazolyl, thiadiazolyl, 1,3-thiazolyl, thienyl, triazolyl, and triazinyl. The bicyclic heteroaryl consists of a monocyclic heteroaryl fused to a phenyl, or a monocyclic heteroaryl fused to a monocyclic C₃-C₆ cycloalkyl, or a monocyclic heteroaryl fused to C₄-C₆ monocyclic cycloalkenyl, or a monocyclic heteroaryl fused to a monocyclic heteroaryl, or a monocyclic heteroaryl fused to a 4-6 membered monocyclic heterocycle. Representative examples of bicyclic heteroaryl groups include, but are not limited to, benzofuranyl, benzothienyl, benzoxazolyl, benzimidazolyl, benzoxadiazolyl, phthalazinyl, 2,6-dihydropyrrolo[3,4-c]pyrazol-5(4H)-yl, 6,7-dihydro-pyrazolo[1,5-a]pyrazin-5(4H)-yl, 6,7-dihydro-1,3-benzothiazolyl, imidazo[1,2-a]pyridinyl, indazolyl, indolyl, isoindolyl, isoquinolinyl, naphthyridinyl, pyridoimidazolyl, quinolinyl, 2,4,6,7-tetrahydro-5H-pyrazolo[4,3-c]pyridin-5-yl, thiazolo[5,4-b]pyridin-2-yl, thiazolo[5,4-d]pyrimidin-2-yl, and 5,6,7,8-tetrahydroquinolin-5-yl. The nitrogen atom in the heteroaryl rings may optionally be oxidized and may optionally be quaternized.

The term "4-11 membered heterocycle" as used herein, means a hydrocarbon ring radical of 4-11 carbon ring atoms wherein at least one carbon ring atom is replaced by heteroatom independently selected from the group consisting of O, N, and S. The 4-11 membered heterocycle ring may be a single ring (monocyclic) or have two or more rings (bicyclic or polycyclic). In certain embodiments, the monocyclic heterocycle is a four-, five-, six-, seven-, or eight-membered hydrocarbon ring wherein at least one carbon ring atom is replaced by heteroatom independently selected from the group consisting of O, N, and S. In certain embodiments, the monocyclic heterocycle is a 4-6 membered hydrocarbon ring wherein at least one carbon ring atom is replaced by heteroatom. A four-membered monocyclic heterocycle contains zero or one double bond, and one carbon ring atom replaced by a heteroatom selected from the group consisting of O, N, and S. A five-membered monocyclic heterocycle contains zero or one double bond and one, two, or three carbon ring atoms replaced by heteroatoms selected from the group consisting of O, N, and S. Examples of five-membered monocyclic heterocycles include those containing in the ring: 1 O; 1 S; 1 N; 2 N; 3 N; 1 S and 1 N; 1 S, and 2 N; 1 O and 1 N; or 1 O and 2 N. Non limiting examples of 5-membered monocyclic heterocyclic groups include 1,3-dioxolanyl, tetrahydrofuranyl, dihydrofuranyl, tetrahydrothienyl, dihydrothienyl, imidazolidinyl, oxazolidinyl, imidazolinyl, isoxazolidinyl, isothiazolidinyl, pyrazolidinyl, pyrazolinyl, pyrrolidinyl, 2-pyrrolinyl, 3-pyrrolinyl, thiazolinyl, and thiazolidinyl. A six-membered monocyclic heterocycle contains zero, one, or two double bonds and one, two, or three carbon ring atoms replaced by heteroatoms selected from the group consisting of O, N, and S. Examples of six-membered monocyclic heterocycles include those containing in the ring: 1 O; 2 O; 1 S; 2 S; 1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N. Examples of six-membered monocyclic heterocycles include 1,3-oxazinanyl, tetrahydropyranyl, dihydropyranyl, 1,6-dihydropyridazinyl, 1,2-dihydropyrimidinyl, 1,6-dihydropyrimidinyl, dioxanyl, 1,4-dithianyl, hexahydropyrimidinyl, morpholinyl, piperazinyl, piperidinyl, 1,2,3,6-tetrahydropyridinyl, tetrahydrothiopyranyl, thiomorpholinyl, thioxanyl, and trithianyl. Seven- and eight-membered monocyclic heterocycles contains zero, one, two, or three double bonds and one, two, or three carbon ring atoms replaced by heteroatoms selected from the group consisting of O, N, and S. Examples of monocyclic heterocycles include, but are not limited to, azetidinyl, azepanyl, aziridinyl, diazepanyl, 1,3-dioxanyl, 1,3-dioxolanyl, 1,3-dithiolanyl, 1,3-dithianyl, 1,6-dihydropyridazinyl, 1,2-dihydropyrimidinyl, 1,6-dihydropyrimidinyl, hexahydropyrimidinyl, imidazolinyl, imidazolidinyl, isoindolinyl, isothiazolinyl, isothiazolidinyl, isoxazolinyl, isoxazolidinyl, morpholinyl, oxadiazolinyl, oxadiazolidinyl, 1,3-oxazinanyl, oxazolinyl, 1,3-oxazolidinyl, oxetanyl, piperazinyl, piperidinyl, pyranyl, pyrazolinyl, pyrazolidinyl, pyrrolinyl, pyrrolidinyl, 1,2-dihydropyridinyl, tetrahydrofuranyl, tetrahydropyridinyl, tetrahydropyrimidinyl, tetrahydropyranyl, tetrahydrothienyl, thiadiazolinyl, thiadiazolidinyl, thiazolinyl, thiazolidinyl, thiomorpholinyl, thiopyranyl, and trithianyl. Polycyclic heterocycle groups contain two or more rings, and bicyclic heterocycles contain two rings. In certain embodiments, the polycyclic heterocycle groups contain 2 or 3 rings. The rings within the polycyclic and the bicyclic heterocycle groups are in a bridged, fused, or spiro orientation, or combinations thereof. In a spirocyclic heterocycle, one atom is common to two different rings. Non limiting examples of spirocyclic heterocycles include 4,6-diazaspiro[2.4]heptanyl, 6-azaspiro[3.4]octane, 2-oxa-6-azaspiro[3.4]octan-6-yl, and 2,7-diazaspiro[4.4]nonane. In a fused ring heterocycle, the rings share one common bond. Examples of fused bicyclic heterocycles are a 4-6 membered monocyclic heterocycle fused to a phenyl group, or a 4-6 membered monocyclic heterocycle fused to a monocyclic C₃-C₆ cycloalkyl, or a 4-6 membered monocyclic heterocycle fused to a C₄-C₆ monocyclic cycloalkenyl, or a 4-6 membered monocyclic heterocycle fused to a 4-6 membered monocyclic heterocycle. Examples of fused bicyclic heterocycles include, but are not limited to hexahydropyrano[3,4-*b*][1,4]oxazin-1(5*H*)-yl, hexahydropyrrolo[3,4-*c*]pyrrol-2(1*H*)-yl, hexahydro-1*H*-imidazo[5,1-*c*][1,4]oxazinyl, hexahydro-1*H*pyrrolo[1,2-*c*]imidazolyl, hexahydrocyclopenta[c]pyrrol-3a(1*H*)-yl, and 3-azabicyclo[3.1.0]hexanyl. In a bridged heterocycle, the rings share at least two non-adjacent atoms. Examples of such bridged heterocycles include, but are not limited to, azabicyclo[2.2.1]heptyl (including 2-azabicyclo[2.2.1]hept-2-yl), 8-azabicyclo[3.2.1]oct-8-yl, octahydro-2,5-epoxypentalene, hexahydro-1*H*-1,4-methanocyclopenta[c]furan, aza-admantane (1-azatricyclo[3.3.1.1^{3,7}]decane), and oxa-adamantane (2-oxatricyclo[3.3.1.1^{3,7}]decane). The nitrogen and sulfur heteroatoms in the heterocycle rings may optionally be oxidized (e.g. 1,1 - dioxidotetrahydrothienyl, 1,1-dioxido-1,2-thiazolidinyl, 1,1-dioxidothiomorpholinyl)) and the nitrogen atoms may optionally be quaternized.

The phenyl, the cycloalkyls, the cycloalkenyls, the heteroaryls, and the heterocycles, including the exemplary rings, are optionally substituted unless otherwise indicated; and are attached to the parent molecular moiety through any substitutable atom contained within the ring system.

The term "heteroatom" as used herein, means a nitrogen, oxygen, and sulfur.

The term "oxo" as used herein, means a =O group.

The term "radiolabel" means a compound of the invention in which at least one of the atoms is a radioactive atom or a radioactive isotope, wherein the radioactive atom or isotope spontaneously emits gamma rays or energetic particles, for example alpha particles or beta particles, or positrons. Examples of such radioactive atoms include, but are not limited to, ³H (tritium), ¹⁴C, ¹¹C, ¹⁵O, ¹⁸F, ³⁵S, ¹²³I, and ¹²⁵I.

A moiety is described as "substituted" when a non-hydrogen radical is in the place of hydrogen radical of any substitutable atom of the moiety. Thus, for example, a substituted heterocycle moiety is a heterocycle moiety in which at least one non-hydrogen radical is in the place of a hydrogen radical on the heterocycle. It should be recognized that if there are more than one substitution on a moiety, each non-hydrogen radical may be identical or different (unless otherwise stated).

If a moiety is described as being "optionally substituted," the moiety may be either (1) not substituted or (2) substituted. If a moiety is described as being optionally substituted with up to a particular number of non-hydrogen radicals, that moiety may be either (1) not substituted; or (2) substituted by up to that particular number of non-hydrogen radicals or by up to the maximum number of substitutable positions on the moiety, whichever is less. Thus, for example, if a moiety is described as a heteroaryl optionally substituted with up to 3 non-hydrogen radicals, then any heteroaryl with less than 3 substitutable positions would be optionally substituted by up to only as many non-hydrogen radicals as the heteroaryl has substitutable positions. To illustrate, tetrazolyl (which has only one substitutable position) would be optionally substituted with up to one non-hydrogen radical. To illustrate further, if an amino nitrogen is described as being optionally substituted with up to 2 non-hydrogen radicals, then a primary amino nitrogen will be optionally substituted with up to 2 non-hydrogen radicals, whereas a secondary amino nitrogen will be optionally substituted with up to only 1 non-hydrogen radical.

The terms "treat", "treating", and "treatment" refer to a method of alleviating or abrogating a disease and/or its attendant symptoms. In certain embodiments, "treat," "treating," and "treatment" refer to ameliorating at least one physical parameter, which may not be discernible by the subject. In yet another embodiment, "treat", "treating", and "treatment" refer to modulating the disease or disorder, either physically (for example, stabilization of a discernible symptom), physiologically (for example, , stabilization of a physical parameter), or both. In a further embodiment, "treat", "treating", and "treatment" refer to slowing the progression of the disease or disorder.

The terms "prevent", "preventing", and "prevention" refer to a method of preventing the onset of a disease and/or its attendant symptoms or barring a subject from acquiring a disease. As used herein, "prevent", "preventing" and "prevention" also include delaying the onset of a disease and/or its attendant symptoms and reducing a subject's risk of acquiring or developing a disease or disorder.

The phrase "therapeutically effective amount" means an amount of a compound, or a pharmaceutically acceptable salt thereof, sufficient to prevent the development of or to alleviate to some extent one or more of the symptoms of the condition or disorder being treated when administered alone or in conjunction with another therapeutic agent for treatment in a particular subject or subject population. The "therapeutically effective amount" may vary depending on the compound, the disease and its severity, and the age, weight, health, etc., of the subject to be treated. For example in a human or other mammal, a therapeutically effective amount may be determined experimentally in a laboratory or clinical setting, or may be the amount required by the guidelines of the United States Food and Drug Administration, or equivalent foreign agency, for the particular disease and subject being treated.

The term "subject" is defined herein to refer to animals such as mammals, including, but not limited to, primates (e.g., humans), cows, sheep, goats, pigs, horses, dogs, cats, rabbits, rats, mice and the like. In one embodiment, the subject is a human. The terms "human," "patient," and "subject" are used interchangeably herein.

The term 'at least one additional therapeutic agent' means one to four therapeutic agents other than the compounds of the invention. In one embodiment it means one to three additional therapeutic agents. In further embodiments it means one or two additional therapeutic agents. In a yet further embodiment it means one additional therapeutic agent. In a yet further embodiment it means two additional therapeutic agents. In a yet further embodiment it means three additional therapeutic agents.

### b. Compounds

Compounds of the invention have the general formula (I) as described above.

Particular values of variable groups in compounds of formula (I), (I-a), (I-b), or (I-c) are as follows. Such values may be used where appropriate with any of the other values, definitions, claims or embodiments defined hereinbefore or hereinafter.

In certain embodiments, R¹ is CH₃.

In certain embodiments, Y is N.

In certain embodiments, Y is C(R^{Y}) wherein R^{Y} is hydrogen or C₁-C₃ alkyl. In some such embodiments, R^{Y} is hydrogen. In some such embodiments, R^{Y} is C₁-C₃ alkyl. In some such embodiments, R^{Y} is ethyl.

In certain embodiments, L¹ is O.

In certain embodiments, L¹ is N(R^{x}) wherein R^{x} is hydrogen or C₁-C₃ alkyl. In some such embodiments, R^{x} is hydrogen or CH₃. In some such embodiments, R^{x} is hydrogen. In some such embodiments, R^{x} is C₁-C₃ alkyl.

In certain embodiments, G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.

In certain embodiments, G¹ is cyclobutyl, cyclopentyl, cyclohexyl, spiro[3.3]heptanyl, pyrrolidinyl, or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen.

In certain embodiments, G¹ is cyclohexyl or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen.

In certain embodiments, G¹ is cyclohexyl which is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen.

In certain embodiments, G¹ is piperidinyl which is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen.

In certain embodiments, G¹ is 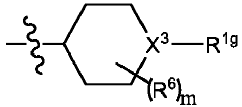 wherein
X³ is N, C(H), or C(R⁶);
each R⁶ is independently C₁-C₃ alkyl, C₁-C₃ haloalkyl, or halogen; and
m is 0, 1, or 2.

In some such embodiments, m is 0. In some such embodiments, m is 1.

In some such embodiments, R⁶ is C₁-C₃ alkyl. In some such embodiments, R⁶ is methyl.

In one embodiment, the invention is directed to compounds of formula (I-a), 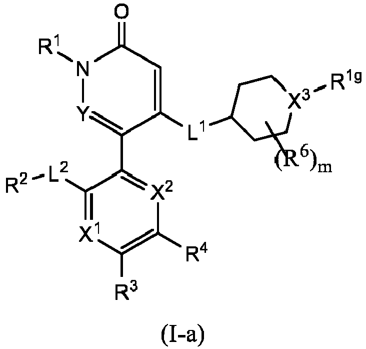 wherein X³ is N, C(H), or C(R⁶); each R⁶ is independently is C₁-C₃ alkyl, C₁-C₃ haloalkyl, or halogen; m is 0, 1, or 2; and R¹, Y, L¹, R^{1g}, X¹, X², L², R², R³, and R⁴ have values as defined in the Summary and embodiments herein above and below.

In some such embodiments, m is 0. In some such embodiments, m is 1.

In some such embodiments, R⁶ is C₁-C₃ alkyl. In some such embodiments, R⁶ is methyl.

In some such embodiments, Y is C(R^{Y}), and X¹ and X² are C(R⁵). In some such embodiments, Y ia C(R^{Y}), X¹ is N, and X² is C(R⁵).

In certain embodiments, G¹ is 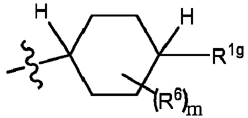 wherein
each R⁶ is independently C₁-C₃ alkyl, C₁-C₃ haloalkyl, or halogen; and
m is 0, 1, or 2.

In some such embodiments, m is 0.

In one embodiment, the invention is directed to compounds of formula (I-b), 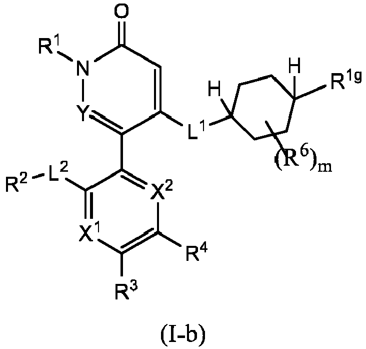 wherein each R⁶ is independently C₁-C₃ alkyl, C₁-C₃ haloalkyl, or halogen; m is 0, 1, or 2; and R¹, Y, L¹, R^{1g}, X¹, X², L², R², R³, and R⁴ have values as defined in the Summary and embodiments herein above and below.

In some such embodiments, m is 0.

In some such embodiments, Y is C(R^{Y}); and X¹ and X² are C(R⁵).

In some such embodiments, Y is C(R^{Y}); X¹ is N; and X² is C(R⁵).

In one embodiment, the invention is directed to compounds of formula (I-c), 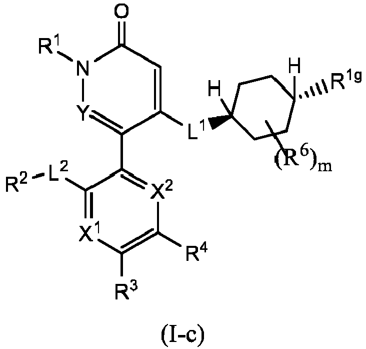 wherein each R⁶ is independently C₁-C₃ alkyl, C₁-C₃ haloalkyl, or halogen; m is 0, 1, or 2; and R¹, Y, L¹, R^{1g}, X¹, X², L², R², R³, and R⁴ have values as defined in the Summary and embodiments herein above and below.

In some such embodiments, m is 0.

In some such embodiments, Y is C(R^{Y}); and X¹ and X² are C(R⁵).

In some such embodiments, Y is C(R^{Y}); X¹ is N; and X² is C(R⁵).

In certain embodiments, R^{1g} is -CN, -OR^{b}, G^{1A}, -C(O)R^{b}, -C(O)OR^{c}, -C(O)N(R^{b})₂, -S(O)2R^{b}, -N(R^{a})S(O)2R^{b}, -N(R^{a})C(O)R^{b}, -N(R^{a})C(O)C(O)R^{b}, -N(R^{a})C(O)OR^{b}, or C₁-C₆ alkyl substituted with an substituent selected from the group consisting of -OR^{b}, -N(R^{a})C(O)R^{b}, and -N(R^{a})C(O)OR^{b}.

In certain embodiments, R^{1g} is -CN, G^{1A}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)OR^{b}, or C₁-C₆ alkyl substituted with an -OR^{b}.

In some such embodiments, Y is C(R^{Y}); and X¹ and X² are C(R⁵).

In some such embodiments, Y is C(R^{Y}); X¹ is N; and X² is C(R⁵).

In certain embodiments, R^{1g} is G^{1A}, -N(R^{a})C(O)R^{b}, or -N(R^{a})C(O)OR^{b}.

In some such embodiments, Y is C(R^{Y}); and X¹ and X² are C(R⁵).

In some such embodiments, Y is C(R^{Y}); X¹ is N; and X² is C(R⁵).

In certain embodiments, R^{1g} is G^{1A}.

In certain embodiments, R^{1g} is G^{1A} wherein G^{1A} is a 4-11 membered heterocycle.

In certain embodiments, R^{1g} is G^{1A} wherein G^{1A} is azetidinyl, pyrrolidinyl, 1,3-oxazolidinyl, imidazolidinyl, isothiazolidinyl, pyrazolidinyl, piperidinyl, hexahydropyrimidinyl, morpholinyl, 1,3-oxazinanyl, azepanyl, isoindolinyl, 1,6-dihydropyridazinyl, 1,2-dihydropyrimidinyl, 1,6-dihydropyrimidinyl, hexahydro-1*H-*pyrrolo[1,2-c]imidazolyl, hexahydro- 1*H*-imidazo[5,1-*c*][1,4] oxazinyl, 3-azabicyclo[3.1.0]hexanyl, or 4,6-diazaspiro[2.4]heptanyl.

In certain embodiments, R^{1g} is G^{1A} wherein G^{1A} is a 5-11 membered heteroaryl. In some such embodiments, the 5-11 membered heteroaryl is pyrazolyl.

In certain embodiments, G^{1A}, including the exemplary rings, are each optionally substituted with 1, 2, 3, 4, or 5 independently selected R^{s} groups.

In certain embodiments, G^{1A}, including the exemplary rings, are substituted with 1, 2, 3, 4, or 5 independently selected R^{s} groups.

In certain embodiments, each R^{s}, when present, is independently C₁-C₆ alkyl, C₂-C₆ alkenyl, oxo, -OR^{j}, -C(O)R^{j}, or -(C₁-C₆ alkylenyl)-OR^{j}.

In certain embodiments, each R^{s}, when present, is independently C₁-C₆ alkyl or oxo.

In certain embodiments, R^{1g} is -N(R^{a})C(O)R^{b}.

In certain embodiments, R^{1g} is -N(R^{a})C(O)R^{b} wherein R^{a} is hydrogen, C₁-C₃ alkyl, -N(R^{j})₂, -(C₂-C₆ alkylenyl)-OR^{j}, or -(C₂-C₆ alkylenyl)-C(O)OR^{j}, and R^{b} is C₁-C₆ alkyl, C₂-C₆ alkenyl, C₁-C₆ haloalkyl, G^{1B}, -(C₁-C₆ alkylenyl)-OR^{j}, -(C₁-C₆ alkylenyl)-C(O)ORⁱ, or -(C₁-C₆ alkylenyl)-N(R^{j})₂.

In certain embodiments, G^{1B} is phenyl, C₃-C₁₁ cycloalkyl, or 4-11 membered heterocycle.

In certain embodiments, G^{1B} is cyclopropyl, cyclobutyl, oxetanyl, pyrrolidinyl, imidazolidinyl, morpholinyl, bicyclo[1.1.1]pentanyl, bicyclo[2.1.1]hexanyl, or bicyclo[2.2.2] octanyl.

In certain embodiments, G^{1B}, including the exemplary rings, are each optionally substituted with 1, 2, 3, or 4 independently selected R^{t} groups.

In certain embodiments, R^{1g} is -N(R^{a})C(O)OR^{b}.

In certain embodiments, R^{1g} is -N(R^{a})C(O)OR^{b} wherein R^{a} is hydrogen or C₁-C₃ alkyl, and R^{b} is C₁-C₆ alkyl.

In certain embodiments, X¹ is N or C(R⁵); and X² is C(R⁵).

In certain embodiments, X¹ and X² are C(R⁵). In some such embodiments, R⁵ is hydrogen.

In certain embodiments, X¹ is N and X² is C(R⁵). In some such embodiments, R⁵ is hydrogen.

In certain embodiments, L² is O.

In certain embodiments, L² is N(R^{e}).

In certain embodiments, R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.

In certain embodiments, R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH.

In certain embodiments, R² is monocyclic heteroaryl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.

In certain embodiments, R² is pyridinyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.

In certain embodiments, R² is pyridinyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH.

In certain embodiments, R³ is hydrogen.

In certain embodiments, R⁴ is

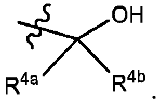

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is 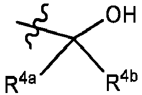 wherein
R^{4a} is C₁-C₆ alkyl or C₁-C₆ haloalkyl, wherein the C₁-C₆ alkyl and the C₁-C₆ haloalkyl are each optionally substituted with one-OH; and
R^{4b} is C₁-C₆ alkyl or C₁-C₆ haloalkyl. In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is 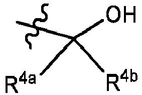 wherein R^{4a} and R^{4b} are each independently C₁-C₆ alkyl or C₁-C₆ haloalkyl.

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is 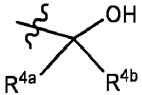 wherein R^{4a} and R^{4b} are each independently C₁-C₆ alkyl.

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is

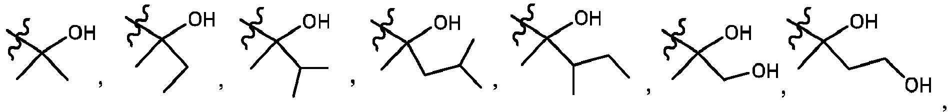

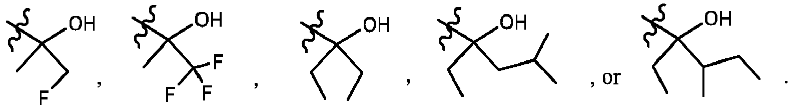

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is

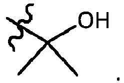

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is

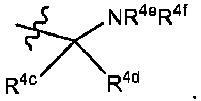

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is 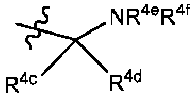 wherein R^{4e} is hydrogen, C₁-C₃ alkyl, or -(C₁-C₃ alkylenyl)-G^{1C}wherein G^{1C} is optionally substituted phenyl; and R^{4f} is -C(O)R^{4cc} or -C(O)N(R^{4cd})(R^{4ce}). In some such embodiments, Y is C(R^{Y}); and X¹ and X² are C(R⁵).

In some such embodiments, Y is C(R^{Y}); X¹ is N; and X² is C(R⁵). In certain embodiments, R⁴ is 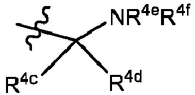 wher
ein R^{4e} is hydrogen, C₁-C₃ alkyl, or -(C₁-C₃ alkylenyl)-G^{1C}wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

In some such embodiments, Y is C(R^{Y}); and X¹ and X² are C(R⁵).

In some such embodiments, Y is C(R^{Y}); X¹ is N; and X² is C(R⁵).

In certain embodiments, R⁴ is 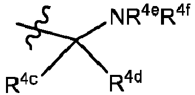
wherein R^{4c} and R^{4d} are each independently hydrogen or C₁-C₆ alkyl;
R^{4e} is hydrogen, C₁-C₃ alkyl, or -(C₁-C₃ alkylenyl)-G^{1C}wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

In some such embodiments, Y is C(R^{Y}); and X¹ and X² are C(R⁵).

In some such embodiments, Y is C(R^{Y}); X¹ is N; and X² is C(R⁵).

In some such embodiments, R^{4c} and R^{4d} are each independently hydrogen or methyl.

Various embodiments of substituents R¹, Y, L¹, G¹, R^{1g}, R^{a}, R^{b}, R^{c}, R^{d} , G^{1A}, G^{1B}, L², R², R³, R⁴, R^{4a} , R^{4b}, R^{4c}, R^{4d}, R^{4e}, R^{4f} , R^{4cc}, R^{4cd}, R^{4ce}, X¹, X², X³ , and R⁵ have been discussed above. These substituents embodiments may be combined to form various embodiments of present compounds. All embodiments of compounds of the invention, formed by combining the substituent embodiments discussed above are within the scope of Applicant's invention, and some illustrative embodiments of the compounds of the invention are provided below.

In one embodiment, the invention is directed to compounds of formula (I), (I-a), (I-b), or (I-c) wherein Y is C(R^{Y}); X¹ is N or C(R⁵); and X² is C(R⁵).

In one embodiment, the invention is directed to compounds of formula (I), (I-a), (I-b), or (I-c), wherein Y is C(R^{Y}); and X¹ and X² are C(R⁵).

In one embodiment, the invention is directed to compounds of formula (I), (I-a), (I-b), or (I-c), wherein Y is C(R^{Y}); X¹ is N; and X² is C(R⁵).

In one embodiment, the invention is directed to compounds of formula (I), (I-a), (I-b), or (I-c), wherein X¹ is N or C(R⁵); X² is C(R⁵); and L² is O.

In some such embodiments, Y is C(R^{Y}); and X¹ and X² are C(R⁵). In some such embodiments, Y is C(R^{Y}); X¹ is N; and X² is C(R⁵).

In one embodiment, the invention is directed to compounds of formula (I), (I-a), (I-b), or (I-c), wherein X¹ is N or C(R⁵); X² is C(R⁵); L² is O; and L¹ is O or N(R^{x}) wherein R^{x} is hydrogen.

In some such embodiments, Y is C(R^{Y}); and X¹ and X² are C(R⁵). In some such embodiments, Y is C(R^{Y}); X¹ is N; and X² is C(R⁵).

In one embodiment, the invention is directed to compounds of formula (I), (I-a), (I-b), or (I-c), wherein X¹ is N or C(R⁵); X² is C(R⁵); L² is O; and L¹ is O.

In some such embodiments, Y is C(R^{Y}); and X¹ and X² are C(R⁵). In some such embodiments, Y is C(R^{Y}); X¹ is N; and X² is C(R⁵).

In one embodiment, the invention is directed to compounds of formula (I), (I-a), (I-b), or (I-c), wherein X¹ is N or C(R⁵); X² is C(R⁵); L² is O; L¹ is N(R^{x}) wherein R^{x} is hydrogen.

In some such embodiments, Y is C(R^{Y}); and X¹ and X² are C(R⁵). In some such embodiments, Y is C(R^{Y}); X¹ is N; and X² is C(R⁵).

In one embodiment, the invention is directed to compounds of formula (I), wherein Y is C(R^{Y}); X¹ is N or C(R⁵); X² is C(R⁵); and G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.

In some such embodiments, Y is C(R^{Y}), and X¹ and X² are C(R⁵). In some such embodiments, Y is C(R^{Y}), X¹ is N, and X² is C(R⁵).

In one embodiment, the invention is directed to compounds of formula (I), wherein
Y is C(R^{Y}),
X¹ and X² are C(R⁵); and
G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.

In one embodiment, the invention is directed to compounds of formula (I), wherein
Y is C(R^{Y}),
X¹ is N,
X² is C(R⁵); and
G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.

In one embodiment, the invention is directed to compounds of formula (I), wherein
G¹ is cyclobutyl, cyclopentyl, cyclohexyl, spiro[3.3]heptanyl, pyrrolidinyl, or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen; and
R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)C(O)R^{b}, -N(R^{a})C(O)OR^{b}, or C₁-C₆ alkyl substituted with an substituent selected from the group consisting of -OR^{b}, -N(R^{a})C(O)R^{b}, and -N(R^{a})C(O)OR^{b}.

In some such embodiments, Y is C(R^{Y}), and X¹ and X² are C(R⁵). In some such embodiments, Y is C(R^{Y}), X¹ is N, and X² is C(R⁵).

In one embodiment, the invention is directed to compounds of formula (I), wherein
G¹ is cyclobutyl, cyclopentyl, cyclohexyl, spiro[3.3]heptanyl, pyrrolidinyl, or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen; and
R^{1g} is G^{1A}, -N(R^{a})C(O)R^{b}, or -N(R^{a})C(O)OR^{b}.

In some such embodiments, Y is C(R^{Y}), and X¹ and X² are C(R⁵). In some such embodiments, Y is C(R^{Y}), X¹ is N, and X² is C(R⁵).

In one embodiment, the invention is directed to compounds of formula (I), (I-a), (I-b), or (I-c), wherein
L² is O and
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.

In some such embodiments, R² is phenyl which is substituted with 2, 3, or 4 substituents wherein two of the substituents are independently selected from the group consisting of halogen, C₁-C₆ alkyl, and C₁-C₆ haloalkyl, and the optional substituents are halogen and -(C₂-C₆ alkylenyl)-OH.

In some such embodiments, Y is C(R^{Y}), and X¹ and X² are C(R⁵). In some such embodiments, Y is C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵); and
L¹ is O or N(R^{x}) wherein R^{x} is hydrogen.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), wherein
L² is O;
X¹ is N or C(R⁵);
X² is C(R⁵);
L¹ is O or N(R^{x}) wherein R^{x} is hydrogen;
G¹ is cyclohexyl or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen; and
R^{1g} is G^{1A}, -N(R^{a})C(O)R^{b}, or -N(R^{a})C(O)OR^{b}.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), wherein
L² is O;
X¹ is N or C(R⁵);
X² is C(R⁵);
L¹ is O or N(R^{x}) wherein R^{x} is hydrogen;
G¹ is cyclohexyl or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen;
R^{1g} is G^{1A}, -N(R^{a})C(O)R^{b}, or -N(R^{a})C(O)OR^{b}; and
R⁴ is

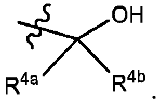

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), wherein
L² is O;
X¹ is N or C(R⁵);
X² is C(R⁵);
L¹ is O or N(R^{x}) wherein R^{x} is hydrogen;
G¹ is cyclohexyl or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen;
R^{1g} is G^{1A}, -N(R^{a})C(O)R^{b}, or -N(R^{a})C(O)OR^{b}; and
R⁴ is

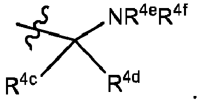

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), wherein
L² is O;
X¹ is N or C(R⁵);
X² is C(R⁵);
L¹ is O or N(R^{x}) wherein R^{x} is hydrogen;
G¹ is cyclohexyl or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen;
R^{1g} is G^{1A}, -N(R^{a})C(O)R^{b}, or -N(R^{a})C(O)OR^{b}; and
R⁴ is

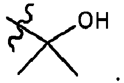

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen; and
R^{1g} is G^{1A}, -N(R^{a})C(O)R^{b}, or -N(R^{a})C(O)OR^{b}.

In some such embodiments, R^{1g} is G^{1A} wherein G^{1A} is a 4-11 membered heterocycle optionally substituted with 1, 2, 3, 4, or 5 independently selected R^{s} groups.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen;
R^{1g} is G^{1A}, -N(R^{a})C(O)R^{b}, or -N(R^{a})C(O)OR^{b}; and
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen;
R^{1g} is G^{1A}, -N(R^{a})C(O)R^{b}, or -N(R^{a})C(O)OR^{b};
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH;
R⁴ is 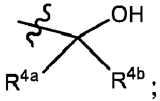 and
R^{4a} and R^{4b} are each independently C₁-C₆ alkyl or C₁-C₆ haloalkyl.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen;
R^{1g} is G^{1A}, -N(R^{a})C(O)R^{b}, or -N(R^{a})C(O)OR^{b};
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH;
R⁴ is 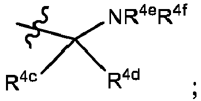 R^{4e} is hydrogen, C₁-C₃ alkyl, or -(C₁-C₃ alkylenyl)-G^{1C} wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen;
R^{1g} is G^{1A}, -N(R^{a})C(O)R^{b}, or -N(R^{a})C(O)OR^{b}; and
R⁴ is

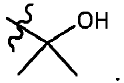

In some such embodiments, R^{1g} is G^{1A} wherein G^{1A} is a 4-11 membered heterocycle optionally substituted with 1, 2, 3, 4, or 5 independently selected R^{s} groups.

In some such embodiments, Y is C(R^{Y}), and X¹ and X² are C(R⁵). In some such embodiments, Y ia C(R^{Y}), X¹ is N, and X² is C(R⁵).

In one embodiment, the invention is directed to compounds of formula (I), (I-a), (I-b), or (I-c), wherein
L² is O;
X¹ is N or C(R⁵);
X² is C(R⁵);
L¹ is O or N(R^{x}) wherein R^{x} is hydrogen;
R^{1g} is G^{1A}, -N(R^{a})C(O)R^{b}, or -N(R^{a})C(O)OR^{b};
R⁴ is 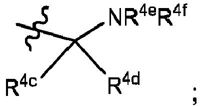 R^{4c} and R^{4d} are each independently hydrogen or C₁-C₆ alkyl;
R^{4e} is hydrogen, C₁-C₃ alkyl, or -(C₁-C₃ alkylenyl)-G^{1C} wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

In some such embodiments, R^{1g} is G^{1A} wherein G^{1A} is a 4-11 membered heterocycle optionally substituted with 1, 2, 3, 4, or 5 independently selected R^{s} groups.

In some such embodiments, Y is C(R^{Y}), and X¹ and X² are C(R⁵). In some such embodiments, Y ia C(R^{Y}), X¹ is N, and X² is C(R⁵).

Compound of the invention are named by using Name 2015 naming algorithm by Advanced Chemical Development or Struct=Name naming algorithm as part of CHEMDRAW® ULTRA v. 12.0.2.1076.

Compounds of the invention may exist as stereoisomers wherein asymmetric or chiral centers are present. These stereoisomers are "R" or "*S*" depending on the configuration of substituents around the chiral carbon atom. The terms "R" and "*S*" used herein are configurations as defined in IUPAC 1974 Recommendations for Section E, Fundamental Stereochemistry, in Pure Appl. Chem., 1976, 45: 13-30. The invention contemplates various stereoisomers and mixtures thereof and these are specifically included within the scope of this invention. Stereoisomers include enantiomers and diastereomers, and mixtures of enantiomers or diastereomers. Individual stereoisomers of compounds of the invention may be prepared synthetically from commercially available starting materials which contain asymmetric or chiral centers or by preparation of racemic mixtures followed by methods of resolution well-known to those of ordinary skill in the art. These methods of resolution are exemplified by (1) attachment of a mixture of enantiomers to a chiral auxiliary, separation of the resulting mixture of diastereomers by recrystallization or chromatography and optional liberation of the optically pure product from the auxiliary as described in Furniss, Hannaford, Smith, and Tatchell, "Vogel's Textbook of Practical Organic Chemistry", 5th edition (1989), Longman Scientific & Technical, Essex CM20 2JE, England, or (2) direct separation of the mixture of optical enantiomers on chiral chromatographic columns or (3) fractional recrystallization methods.

Compounds of the invention may exist as *cis* or *trans* isomers, wherein substituents on a ring may attached in such a manner that they are on the same side of the ring (*cis*) relative to each other, or on opposite sides of the ring relative to each other (*trans*). For example, cyclobutane may be present in the *cis* or *trans* configuration, and may be present as a single isomer or a mixture of the *cis* and *trans* isomers. Individual *cis* or *trans* isomers of compounds of the invention may be prepared synthetically from commercially available starting materials using selective organic transformations, or prepared in single isomeric form by purification of mixtures of the *cis* and *trans* isomers. Such methods are well-known to those of ordinary skill in the art, and may include separation of isomers by recrystallization or chromatography.

It should be understood that the compounds of the invention may possess tautomeric forms, as well as geometric isomers, and that these also constitute an aspect of the invention.

The present disclosure includes all pharmaceutically acceptable isotopically-labelled compounds of formula (I), (I-a), (I-b), or (I-c) wherein one or more atoms are replaced by atoms having the same atomic number, but an atomic mass or mass number different from the atomic mass or mass number which predominates in nature. Examples of isotopes suitable for inclusion in the compounds of the disclosure include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds of formula (I), (I-a), (I-b), or (I-c), for example, those incorporating a radioactive isotope, are useful in drug and/or substrate tissue distribution studies. The radioactive isotopes tritium, *i.e.* ³H, and carbon-14, i.e. ¹⁴C, are particularly useful for this purpose in view of their ease of incorporation and ready means of detection. Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances. Substitution with positron emitting isotopes, such as ¹¹C, ¹⁸F, ¹⁵O and ¹³N, may be useful in Positron Emission Topography (PET) studies for examining substrate receptor occupancy. Isotopically-labeled compounds of formula (I), (I-a), (I-b), or (I-c) may generally be prepared by conventional techniques known to those skilled in the art or by processes analogous to those described in the accompanying Examples using an appropriate isotopically-labeled reagents in place of the non-labeled reagent previously employed.

The formula drawings within this specification may represent only one of the possible tautomeric, geometric, or stereoisomeric forms. It is to be understood that the invention encompasses any tautomeric, geometric, or stereoisomeric form, and mixtures thereof, and is not to be limited merely to any one tautomeric, geometric, or stereoisomeric form utilized within the formula drawings.

Exemplary compounds include, but are not limited to:
*N*-(*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)acetamide;
5-[2-(2,6-dimethylphenoxy)-5-(2-hydroxypropan-2-yl)phenyl]-4-{[1-(methanesulfonyl)piperidin-4-yl]amino}-1-methylpyridin-2(1*H*)-one;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide;
methyl[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamate;
methyl(*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)carbamate;
*N*-{[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}acetamide;
methyl{[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
*tert*-butyl{[cis-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
5-[2-(4-fluoro-2,6-dimethylphenoxy)-5-(2-hydroxypropan-2-yl)phenyl]-4-{[1-(methanesulfonyl)piperidin-4-yl]amino}-1-methylpyridin-2(1*H*)-one;
*N*-[6-({5-[2-(2,6-dimethylphenoxy)-5-(2-hydroxypropan-2-yl)phenyl]-1-methyl-2-oxo-1,2-dihydropyridin-4-yl}amino)spiro[3.3]heptan-2-yl]acetamide;
*tert*-butyl3-({5-[2-(2,6-dimethylphenoxy)-5-(2-hydroxypropan-2-yl)phenyl]-1-methyl-2-oxo-1,2-dihydropyridin-4-yl}amino)pyrrolidine-1-carboxylate;
5-[2-(2,6-dimethylphenoxy)-5-(2-hydroxypropan-2-yl)phenyl]-4-{[1-(methanesulfonyl)pyrrolidin-3-yl]amino}-1-methylpyridin-2(1*H*)-one;
*N*-{*trans*-4-[{5-[2-(4-fluoro-2,6-dimethylphenoxy)-5-(2-hydroxypropan-2-yl)phenyl]-1-methyl-2-oxo-1,2-dihydropyridin-4-yl}(methyl)amino]cyclohexyl}acetamide;
4-[(1-acetylpyrrolidin-3-yl)amino]-5-[2-(2,6-dimethylphenoxy)-5-(2-hydroxypropan-2-yl)phenyl]-1-methylpyridin-2(1*H*)-one;
*tert*-butyl[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-methylacetamide;
*N*-[*trans*-4-(15'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]acetamide;
*N*-{*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl}acetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}amino)cyclohexyl]acetamide;
methyl{*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl}carbamate;
methyl[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate;
*N*-([(1*R*,2*S*,3*S*)-3-({5-[2-(4-fluoro-2,6-dimethylphenoxy)-5-(2-hydroxypropan-2-yl)phenyl]-1-methyl-2-oxo-1,2-dihydropyridin-4-yl}oxy)-2-methylcyclopentyl]methyl}acetamide;
5-[2-(4-fluoro-2,6-dimethylphenoxy)-5-(2-hydroxypropan-2-yl)phenyl]-4-[(*trans*-4-hydroxy-4-methylcyclohexyl)amino]-1-methylpyridin-2(1*H*)-one;
5-[2-(4-fluoro-2,6-dimethylphenoxy)-5-(2-hydroxypropan-2-yl)phenyl]-1-methyl-4-([*trans*-4-(2-oxopyrrolidin-1-yl)cyclohexyl]oxy}pyridin-2(1*H*)-one;
methyl [*trans*-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]carbamate;
2'-(4-fluoro-2,6-dimethylphenoxy)-5'-(2-hydroxypropan-2-yl)-4-{[1-(methoxyacetyl)piperidin-4-yl]oxy}-1-methyl[3,3'-bipyridin]-6(1*H*)-one;
5-[2-(2,6-dimethylphenoxy)-5-(2-hydroxypropan-2-yl)phenyl]-1-methyl-4-{[*trans*-4-(2-oxopyrrolidin-1-yl)cyclohexyl]oxylpyridin-2(1H)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]prop-2-enamide;
5-[2-(4-fluoro-2,6-dimethylphenoxy)-5-(2-hydroxypropan-2-yl)phenyl]-1-methyl-4-[trans-4-(2-oxopiperidin-1-yl)cyclohexyl]oxylpyridin-2(1*H*)-one;
4-{[*trans*-4-(3,3-dimethyl-2-oxoazetidin-1-yl)cyclohexyl]oxyl-5-[2-(4-fluoro-2,6-dimethylphenoxy)-5-(2-hydroxypropan-2-yl)phenyl]-1-methylpyridin-2(1*H*)-one;
1-[*trans*-4-({5-[2-(4-fluoro-2,6-dimethylphenoxy)-5-(2-hydroxypropan-2-yl)pheny1]-1-methyl-2-oxo-1,2-dihydropyridin-4-yl}oxy)cyclohexyl]tetrahydropyrimidin-2(1*H*)-one;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)-1-methylcyclohexyl]acetamide;
6-ethyl-5-[2-(4-fluoro-2,6-dimethylphenoxy)-5-(2-hydroxypropan-2-yl)phenyl]-1-methyl-4-{[*trans*-4-(2-oxopyrrolidin-1-yl)cyclohexyl]oxy}pyridin-2(1H)-one;
5-[2-(4-fluoro-2,6-dimethylphenoxy)-5-(2-hydroxypropan-2-yl)phenyl]-1-methyl-4-[*trans*-4-methyl-4-(2-oxopyrrolidin-1-yl)cyclohexyl]oxy}pyridin-2(1*H*)-one;
5-[2-(4-fluoro-2,6-dimethylphenoxy)-5-(2-hydroxypropan-2-yl)phenyl]-1-methyl-4-{[*trans*-4-(2-oxoimidazolidin-1-yl)cyclohexyl]oxy}pyridin-2(1*H*)-one;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hex-5-enamide;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]azepan-2-one;
5-[2-(4-fluoro-2,6-dimethylphenoxy)-5-(2-hydroxypropan-2-yl)phenyl]-1-methyl-4-{[*trans*-4-(2-oxo-1,3-oxazolidin-3-yl)cyclohexyl]oxy}pyridin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,3-oxazinan-2-one;
4-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]morpholin-3-one;
1-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyltetrahydropyrimidin-2(1*H*)-one;
5-[2-(4-fluoro-2,6-dimethylphenoxy)-5-(2-hydroxypropan-2-yl)phenyl]-1-methyl-4-{[*trans*-4-(2-methyl-5-oxopyrrolidin-1-yl)cyclohexyl]oxy}pyridin-2(1H)-one;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1*H*-isoindole-1,3(2*H*)-dione;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrrolidine-2,5-dione;
2-{[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}benzoic acid;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methanesulfonamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-2,2-dimethylbutanamide;
4-{[*trans*-4-(3,3-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-hydroxypropanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropanecarboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methylcyclopropane-1-carboxamide;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1lambda^{6,2}-thiazolidine-1,1-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methoxyacetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-hydroxyethyl)acetamide;
ethyl 3-{[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]amino}-3-oxopropanoate;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-oxopropanamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2,2-dimethylpropanamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N,1-dimethylcyclopropane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*,2,2-trimethylpropanamide;
2'-(4-fluoro-2,6-dimethylphenoxy)-5'-(2-hydroxypropan-2-yl)-1-methyl-4-{[*trans*-4-(2-oxopyrrolidin-1-yl)cyclohexyl]oxy}[3,3'-bipyridin]-6(1*H*)-one;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxylic acid;
5-[2-(4-fluoro-2,6-dimethylphenoxy)-5-(2-hydroxypropan-2-yl)phenyl]-1-methyl-4-([*trans*-4-(pyrrolidine-1-carbonyl)cyclohexyl]oxy}pyridin-2(1*H*)-one;
*trans*-*N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-methoxyethyl)-1-methylcyclopropane-1-carboxamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(3-methoxypropyl)-1-methylcyclopropane-1-carboxamide;
ethyl *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(1-methylcyclopropane-1-carbonyl)glycinate;
5-[2-(4-fluoro-2,6-dimethylphenoxy)-5-(2-hydroxypropan-2-yl)phenyl]-4-{[*trans*-4-(2-hydroxypropan-2-yl)cyclohexyl]oxy}-1-methylpyridin-2(1*H*)-one;
*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-(methoxymethyl)cyclopropane-1-carboxamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methoxy-2,2-dimethylpropanamide;
*tert*-butyl (3-{[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[1.1.1]pentan-1-yl)carbamate;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyloxetane-3-carboxamide;
5-[2-(4-fluoro-2,6-dimethylphenoxy)-5-(2-hydroxypropan-2-yl)phenyl]-4-{[*cis*-4-(2-hydroxypropan-2-yl)cyclohexyl]oxy}-1-methylpyridin-2(1*H*)-one;
*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxylic acid;
*cis*-*N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
5-[2-(4-fluoro-2,6-dimethylphenoxy)-5-(2-hydroxypropan-2-yl)phenyl]-1-methyl-4-{[*cis-*4-(pyrrolidine-1-carbonyl)cyclohexyl]oxy}pyridin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methylimidazolidine-2,4-dione;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-hydroxy-2,2-dimethylpentanamide;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]piperidine-2,6-dione;
(5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,3-oxazolidine-2,4-dione;
(5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)-4-methylcyclohexyl]acetamide;
1-cyano-*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methyl-3-oxocyclobutane-1-carboxamide;
1-cyano-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclobutane-1-carboxamide;
1-[*trans*-4-({5-[2-(4-fluoro-2,6-dimethylphenoxy)-5-(2-hydroxypropan-2-yl)pheny1]-1-methyl-2-oxo-1,2-dihydropyridin-4-yl}oxy)cyclohexyl]-3,3-dimethylpiperidine-2,6-dione;
1-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpyrrolidine-2,5-dione;
(7a*S*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-c]imidazole-1,3(2*H*)-dione;
(5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methyl-1,3-oxazolidine-2,4-dione;
3-amino-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide;
5-[2-(4-fluoro-2,6-dimethylphenoxy)-5-(2-hydroxypropan-2-yl)phenyl]-4-{[*cis-*3-(2-hydroxypropan-2-yl)cyclobutyl]oxy}-1-methylpyridin-2(1*H*)-one;
*cis-N*-ethyl-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxamide;
5-[2-(4-fluoro-2,6-dimethylphenoxy)-5-(2-hydroxypropan-2-yl)phenyl]-1-methyl-4-{[*cis*-3-(pyrrolidine-1-carbonyl)cyclobutyl]oxy}pyridin-2(1*H*)-one;
*cis*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxylic acid;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
*N*'-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
*N*-[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]acetamide;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N*-methylcyclohexane-1-carboxamide;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
*tert*-butyl(4-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[2.1.1]hexan-1-yl)carbamate;
*tert*-butyl [(1-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl} cyclopropyl)methyl]carbamate;
*N*'-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
*tert*-butyl 2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
*tert*-butyl 2-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N,N-*dimethylcyclohexane-1-carboxamide;
5-[2-(4-fluoro-2,6-dimethylphenoxy)-5-(2-hydroxypropan-2-yl)phenyl]-1-methyl-4-{[*trans*-4-(5 -oxopyrazolidin-1-yl)cyclohexyl] oxy}pyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-(hydroxymethyl)bicyclo[2.2.2]octane-1-carboxamide;
*N*¹-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropane-1,1-dicarboxamide;
4-{[*trans*-4-(2-acetyl-5-oxopyrazolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
*N*-[*trans*-4-({5-[2-(2,6-dimethylphenoxy)-5-{[methyl(methylcarbamoyl)amino]methyl}phenyl]-1-methyl-2-oxo-1,2-dihydropyridin-4-yl}oxy)cyclohexyl]-1-methylcyclopropane-1-carboxamide;
5-[2-(4-fluoro-2,6-dimethylphenoxy)-5-(2-hydroxypropan-2-yl)phenyl]-4-{[*trans*-4-(5-hydroxy-1*H*-pyrazol-1-yl)cyclohexyl]oxy}-1-methylpyridin-2(1*H*)-one;
*N*-[*trans*-4-({5-[5-{[carbamoyl(methyl)amino]methyl}-2-(2,6-dimethylphenoxy)phenyl]-1-methyl-2-oxo-1,2-dihydropyridin-4-yl}oxy)cyclohexyl]-1-methylcyclopropane-1-carboxamide;
*N*-{*trans*-4-[(5-{5-[(1*R*)-1-{acetyl[(1*S*)-1-phenylethyl]amino}ethyl]-2-(2,6-dimethylphenoxy)phenyl}-1-methyl-2-oxo-1,2-dihydropyridin-4-yl)oxy]cyclohexyl}-1-methylcyclopropane-1-carboxamide;
2,2,2-trifluoro-*N*-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]acetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-3,3-dimethylbutanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-hydroxy-4-methylpentanamide;
4-{[*trans*-4-(4,4-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
1-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrazolidine-3,5-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]morpholine-4-carboxamide;
*N*-[*trans*-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-oxoimidazolidine-1-carboxamide;
(5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methylimidazolidine-2,4-dione;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H*-imidazo[5,1-c][1,4]oxazine-1,3(2*H*)-dione;
1-ethyl-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N'-methylurea;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-azabicyclo[3.1.0]hexane-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-6-methylpyridazin-3(2*H*)-one;
(5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl)oxy)cyclohexyl]-5-(propan-2-yl)imidazolidine-2,4-dione;
(5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methylimidazolidine-2,4-dione;
5-ethylidene-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
5-[2-(4-fluoro-2,6-dimethylphenoxy)-5-(2-hydroxypropan-2-yl)phenyl]-1-methyl-4-{[*trans*-4-(2,3,4,4-tetramethyl-5-oxoimidazolidin-1-yl)cyclohexyl]oxy}pyridin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-(2-methoxyethyl)imidazolidine-2,4-dione;
6-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4,6-diazaspiro[2.4]heptane-5,7-dione;
4-acetamido-*N*-[*trans-*4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[2.1.1]hexane-1-carboxamide;
(7*aR*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-c]imidazole-1,3(2*H*)-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methylalaninamide;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5,5-dimethylimidazolidine-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyridazin-3(2*H*)-one;
3-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5,5-trimethylimidazolidine-2,4-dione;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-4(3*H*)-one; and
6-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-methyl-4,6-diazaspiro[2.4]heptane-5,7-dione.

Compounds of formula (I), (I-a), (I-b), or (I-c) may be used in the form of pharmaceutically acceptable salts. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts have been described in S. M. Berge et al. J. Pharmaceutical Sciences, 1977, 66: 1-19.

Compounds of formula (I), (I-a), (I-b), or (I-c) may contain either a basic or an acidic functionality, or both, and may be converted to a pharmaceutically acceptable salt, when desired, by using a suitable acid or base. The salts may be prepared in situ during the final isolation and purification of the compounds of the invention.

Examples of acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, malate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups may be quaternized with such agents as lower alkyl halides such as, but not limited to, methyl, ethyl, propyl, and butyl chlorides, bromides and iodides; dialkyl sulfates like dimethyl, diethyl, dibutyl and diamyl sulfates; long chain halides such as, but not limited to, decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts may be prepared in situ during the final isolation and purification of compounds of this invention by reacting a carboxylic acid-containing moiety with a suitable base such as, but not limited to, the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as, but not limited to, lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other examples of organic amines useful for the formation of base addition salts include ethylenediamine, ethanolamine, diethanolamine, piperidine, piperazine and the like.

The term "pharmaceutically acceptable prodrug" or "prodrug"as used herein, represents those prodrugs of the compounds of the present invention which are, within the scope of sound medical judgement, suitable for use in contact with the tissues of humans and lower animals without undue toxicity, irritation, allergic response, and the like, commensurate with a reasonable benefit/risk ratio, and effective for their intended use.

The present invention contemplates compounds of formula (I), (I-a), (I-b), or (I-c) formed by synthetic means or formed by in vivo biotransformation of a prodrug.

Compounds described herein may exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

### c. General Synthesis

The compounds described herein, including compounds of general formula (I), (I-a), (I-b), or (I-c) and specific examples, may be prepared, for example, through the reaction routes depicted in schemes 1-5. The variables X¹, X², L¹, L², R¹, R², R³, R⁴, R^{4a}, R^{4b}, R^{4c}, R^{4cc}, R4^{cd}, R^{4ce}, R^{x}, R^{b}, R^{e}, G¹, G^{1A}, G^{1C}, and Y used in the following schemes have the meanings as set forth in the summary and detailed description sections unless otherwise noted.

Abbreviations used in the descriptions of the schemes and the specific examples have the following meanings: DMF for *N,N*-dimethylformamide, DMSO for dimethyl sulfoxide, psi for pounds per square inch, HPLC for high performance liquid chromatography, LCMS for liquid chromatography mass spectrometry, and SFC for Supercritical Fluid Chromatography.

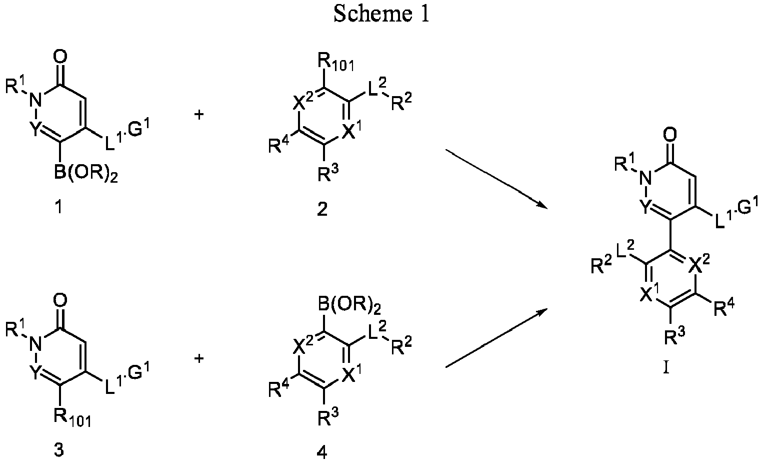

Compounds of general formula (I) may be prepared as shown in Scheme 1 by reaction of boronic acids or a derivative thereof (e.g., a pinacol ester) of formula (1) with compounds of formula (2), wherein R₁₀₁ is Cl, Br, I, or triflate, under Suzuki coupling conditions (N. Miyama and A. Suzuki, Chem. Rev. 1995, 95:2457-2483, J. Organomet. Chem. 1999, 576:147-148). For example, the coupling reaction may be conducted in the presence of a palladium catalyst and a base, and optionally in the presence of a ligand, and in a suitable solvent at elevated temperature (about 60 °C to about 150 °C). The reaction may be facilitated by microwave irradiation. Examples of the palladium catalyst include, but are not limited to, tetrakis(triphenylphosphine)palladium(0), tris(dibenzylideneacetone)dipalladium(0), bis(triphenylphosphine)palladium(II) dichloride, and palladium(II)acetate. Examples of suitable bases that may be employed include, but not limited to, carbonates, acetates, or phosphates of sodium, potassium, and cesium, and cesium fluoride. Examples of suitable ligands include, but are not limited to, 1,3,5,7-tetramethyl-8-phenyl-2,4,6-trioxa-8-phosphaadamante, 2-dicyclohexylphosphino-2',4',6'-triisopropylbiphenyl (X-phos), and 1,1'-bis(diphenylphosphanyl) ferrocene. Non-limiting examples of suitable solvent include methanol, ethanol, dimethoxyethane, N,N-dimethylformamide, dimethylsulfoxide, dioxane, tetrahydrofuran, toluene, and water, or a mixture thereof.

Compounds of general formula (I) may also be prepared as shown in Scheme 1 by reaction of boronic acids or a derivative thereof (e.g., a pinacol ester) of formula (4) with compounds of formula (3), wherein R₁₀₁ is Cl, Br, I, or triflate, under Suzuki coupling conditions as described above.

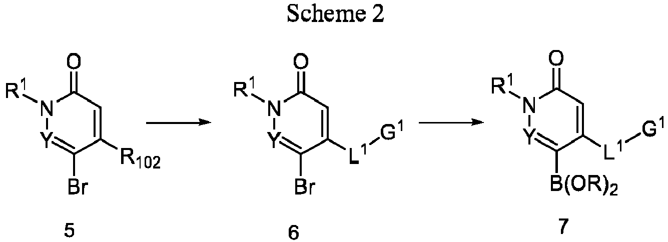

Compounds of formula (1) and formula (3) wherein R₁₀₁ is Br may be prepared using general synthetic route as shown in Scheme 2. Displacement of the halogen of compounds of formula (5), wherein R₁₀₂ is Cl or F, with alcohols (L¹ = O) or amines (L¹=N(R^{x})) may be accomplished in a solvent such as, but not limited to, dimethylsulfoxide, dimethylformamide, dioxane, or tetrahydrofuran and in the presence of a base such as, but not limited to, potassium *tert*-butoxide, carbonate of cesium, potassium, or sodium, or sodium hydride, and at a temperature from about 40 °C to about 120 °C. Treatment of the compounds of formula (6) with 4,4,4',4',5,5,5',5'-octamethyl-2,2'-bi(1,3,2-dioxaborolane) generally affords compounds of formula (7). In general, the conversion may be facilitated by a palladium catalyst such as, but not limited to, tetrakis(triphenylphosphine)palladium(0), tris(dibenzylideneacetone)dipalladium(0), or palladium(II)acetate, an optional ligand such as, but not limited to, 2-dicyclohexylphosphino-2',4',6'-triisopropylbiphenyl, 2-dicyclohexylphosphino-2',4',6'-triisopropylbiphenyl (X-phos), or 1,1'-bis( diphenylphosphanyl) ferrocene, and a base such as, but not limited to, carbonates, acetates, or phosphates.of sodium, potassium, and cesium; and cesium fluoride. Non-limiting examples of suitable solvents include methanol, dimethoxyethane, N,N-dimethylformamide, dimethylsulfoxide, dioxane, tetrahydrofuran, and water, or a mixture thereof.

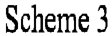

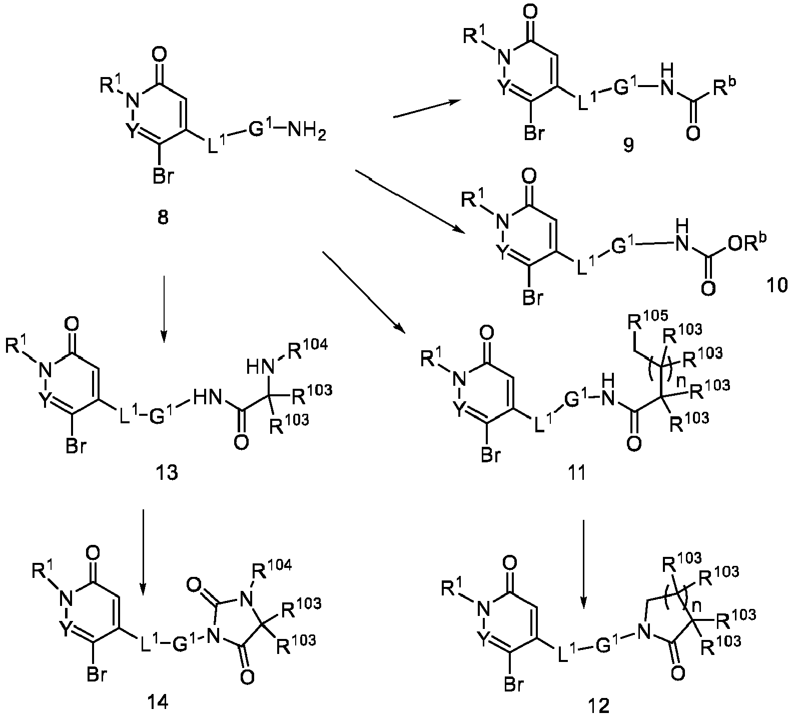

Representative examples of compound of formula (3) wherein R₁₀₁ is bromo, and G¹ is substituted with G^{1A}, N(H)C(O)R^{b}, or N(H)C(O)OR^{b} may be prepared as shown as illustrated in Scheme 3.

Transformation of (8) to the corresponding amides (9) and carbamates (10) are known to one skilled in the art, for example, by treatment with suitable anhydrides or suitable chloroformates respectively, in the presence of a base such as, but not limited to, trimethylamine.

Treatment of (8) with dihydrofuran-2(3*H*)-one which is optionally substituted with C₁-C₆ alkyl in the presence of a base such as, but not limited to, diisopropylethylamine, provides compounds of formula (11) wherein n is 1, each R¹⁰³ is independently hydrogen or C₁-C₆ alkyl, and R¹⁰⁵ is -OH. Treatment of compounds of formula (11) wherein R¹⁰⁵ is -OH in the presence of triphenylphosphine and diisopropyl azodicarboxylate provides compounds of formula (12) wherein n is 1 and each R¹⁰³ is independently hydrogen or C₁-C₆ alkyl.

Treatment of (8) with compounds of formula ClC(O)C(R¹⁰³)₂(C(R¹⁰³)₂)ₙCl wherein each R¹⁰³ is independently hydrogen or C₁-C₆ alkyl and n is 1 or 2, in the presence of a base such as, but not limited to, trimethylamine, provides compounds of formula (11) wherein R¹⁰⁵ is Cl. Transformation of (11) to (12) may be achieved in the presence of sodium hydride at elevated temperature.

Treatment of (8) with compounds of formula N(H)R¹⁰⁴C(R¹⁰³)₂C(O)OH wherein each R¹⁰³ is independently hydrogen or C₁-C₆ alkyl, and R¹⁰⁴ is hydrogen, C₁-C₆ alkyl, or a nitrogen protecting group such as, but not limited to *tert*-butoxycabonyl, under amide bond forming conditions, followed by the removal of the protecting group affords compounds of formula (13) wherein R¹⁰⁴ is hydrogen or C₁-C₆ alkyl. Examples of conditions known to generate amides from a mixture of a carboxylic acid and an amine include but are not limited to adding a coupling reagent such as but not limited to *N*-(3-dimethylaminopropyl)-*N'* ethylcarbodiimide or 1-(3-dimethylaminopropyl)-3-ethylcarbodiimide (EDC, EDAC or EDCI) or the corresponding hydrochloride salt, 1,3-dicyclohexylcarbodiimide (DCC), bis(2-oxo-3-oxazolidinyl)phosphinic chloride (BOPCl), *N*-[(dimethylamino)-1*H*-1,2,3-triazolo-[4,5-b]pyridin-1-ylmethylene]-*N*-methylmethanaminium hexafluorophosphate *N*-oxide or 2-(7-azabenzotriazol-1-yl)-*N,N,N',N*'-tetramethyluronium hexafluorophosphate or 1-[bis(dimethylamino)methylene]-1*H*-1,2,3-triazolo[4,5-*b*]pyridinium 3-oxid hexafluorophosphate (HATU), *O*-(benzotriazol-1-yl)-*N;N;N',N*-tetramethyluronium tetrafluoroborate (TBTU), 2-(1*H*benzo[*d*][1,2,3]triazol-1-yl)-1,1,3,3-tetramethylisouronium hexafluorophosphate(V) (HBTU), and 2,4,6-tripropyl-1,3,5,2,4,6-trioxatriphosphinane 2,4,6-trioxide (T3P®). The coupling reagents may be added as a solid, a solution, or as the reagent bound to a solid support resin. In addition to the coupling reagents, auxiliary-coupling reagents may facilitate the coupling reaction. Auxiliary coupling reagents that are often used in the coupling reactions include but are not limited to (dimethylamino)pyridine (DMAP), 1-hydroxy-7-azabenzotriazole (HOAT) and 1-hydroxybenzotriazole (HOBT). The reaction may be carried out optionally in the presence of a base such as triethylamine, *N,N-*diisopropylethylamine or pyridine. The coupling reaction may be carried out in solvents such as but not limited to tetrahydrofuran, *N,N*-dimethylformamide, *N,N*-dimethylacetamide, dimethyl sulfoxide, dichloromethane, and ethyl acetate. The reactions may be carried out at ambient temperature or heated. The heating can be accomplished either conventionally or with microwave irradiation. Treatment of (13) with di(1H-imidazol-1-yl)methanone in the presence of a base such as, but limited to, trimethylamine, at elevated temperature (e.g. about 50 °C to about 100 °C) provides compounds of formula (14).

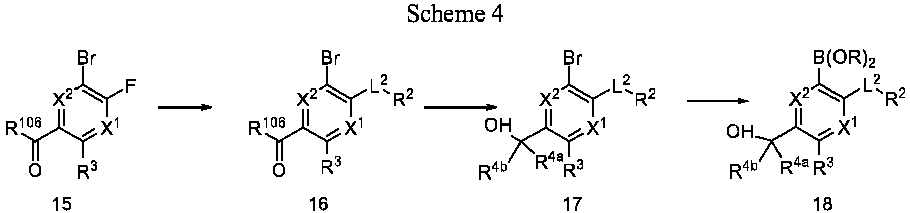

Compounds of formula (2) and (4) wherein R⁴ is an alcohol may be prepared using general synthetic route as shown in Scheme 4.

Compounds of formula (16) wherein L² is O or N(R^{e}) may be prepared by displacement of the fluorine atom of the compounds (15) with an appropriate alcohol or an amine. Displacement of the fluorine atom may be accomplished in a solvent such as, but not limited to, dimethylsulfoxide, dimethylformamide, dioxane, or tetrahydrofuran and in the presence of a base such as, but not limited to, carbonate of cesium, potassium, or sodium, or sodium hydride, and at a temperature from about 40 °C to about 120 °C. Compounds of formula (17) wherein R^{4a} and R^{4b} are different may be prepared by reaction of compounds of formula (16) wherein R¹⁰⁶ = R^{4a} with a Grignard reagent of formula R^{4b}MgBr or R^{4b}MgCl in a solvent such as tetrahydrofuran, diethyl ether, or dioxane at about ambient temperatures. Compounds of formula (17) wherein R^{4a} and R^{4b} are the same may be prepared by reaction of compounds of formula (16) wherein R¹⁰⁶ = Oalkyl with greater than two equivalents of a Grignard reagent of formula R^{4b}MgBr or R^{4b}MgCl in a solvent such as tetrahydrofuran, diethyl ether, or dioxane at about ambient temperatures.

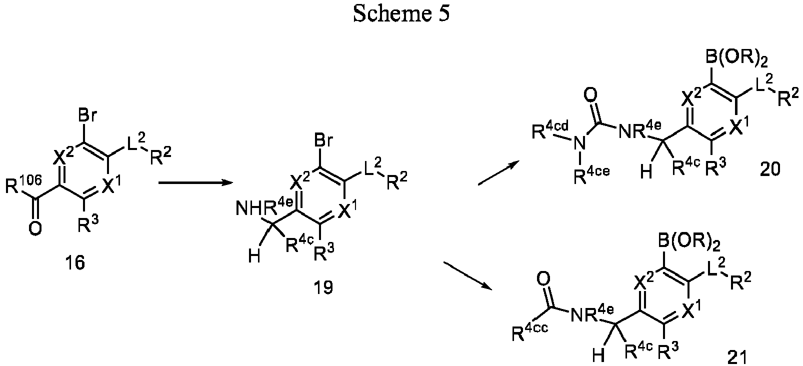

Compounds of formula (2) and (4) wherein R⁴ is an amine or substituted amine may be prepared using general synthetic route as shown in Scheme 5.

Compounds of formula (16) wherein R¹⁰⁶ is R^{4c} and R^{4c} is hydrogen or C₁-C₆ alkyl may be treated with amines of formula N(R^{4e})H₂ wherein R^{4e} is hydrogen, C₁-C₃ alkyl, or -(CH₂)-G^{1c}, in the presence of zinc (II) chloride or titanium (IV) chloride, to provide amines of formula (19).

Amines of formula (19) may be treated with 2,5-dioxopyrrolidin-1-yl methylcabamate or isocyanatotrimethylsilane, in the presence of a base such as, but not limited to, trimethylamine, to provide compounds of formula (20) wherein R^{4cd} is hydrogen, and R^{4ce} is methyl or hydrogen respectively.

Transformation of amines (19) to compounds of formula (21) may be achieved by treatment with an acyl chloride of formula R^{4cc}C(O)Cl in the presence of a base such as, but not limited to, trimethylamine.

It can be appreciated that the synthetic schemes and specific examples as illustrated in the synthetic examples section are illustrative and are not to be read as limiting the scope of the invention as it is defined in the appended claims. All alternatives, modifications, and equivalents of the synthetic methods and specific examples are included within the scope of the claims.

Optimum reaction conditions and reaction times for each individual step can vary depending on the particular reactants employed and substituents present in the reactants used. Unless otherwise specified, solvents, temperatures and other reaction conditions may be readily selected by one of ordinary skill in the art. Specific procedures are provided in the Synthetic Examples section. Reactions may be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or may be prepared by one skilled in the art from commercially available materials using methods described in the chemical literature.

Routine experimentations, including appropriate manipulation of the reaction conditions, reagents and sequence of the synthetic route, protection of any chemical functionality that can not be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which can be found in T. Greene and P. Wuts, Protecting Groups in Organic Synthesis (3rd ed.), John Wiley & Sons, NY (1999), which is incorporated herein by reference in its entirety. Synthesis of the compounds of the invention can be accomplished by methods analogous to those described in the synthetic schemes described hereinabove and in specific examples.

Starting materials, if not commercially available, may be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound is required, it may be obtained by carrying out one of the procedures described herein using an optically active starting material (prepared, for example, by asymmetric induction of a suitable reaction step), or by resolution of a mixture of the stereoisomers of the compound or intermediates using a standard procedure (such as chromatographic separation, recrystallization or enzymatic resolution).

Similarly, when a pure geometric isomer of a compound is required, it may be prepared by carrying out one of the above procedures using a pure geometric isomer as a starting material, or by resolution of a mixture of the geometric isomers of the compound or intermediates using a standard procedure such as chromatographic separation.

### d. Pharmaceutical Compositions

When employed as a pharmaceutical, a compound of the invention is typically administered in the form of a pharmaceutical composition. Such composition may be prepared in a manner well known in the pharmaceutical art and comprise a therapeutically effective amount of a compound of formula (I), (I-a), (I-b), or (I-c), or a pharmaceutically acceptable salt thereof, alone or in combination with at least one additional therapeutic agent, together with a pharmaceutically acceptable carrier. The phrase "pharmaceutical composition" refers to a composition suitable for administration in medical or veterinary use.

The pharmaceutical compositions that comprise a compound of formula (I), (I-a), (I-b), or (I-c), alone or in combination with at least one additional therapeutic agent, may be administered to the subjects orally, rectally, parenterally, intracisternally, intravaginally, intraperitoneally, topically (as by powders, ointments or drops), bucally or as an oral or nasal spray. The term "parenterally" as used herein, refers to modes of administration which include intravenous, intramuscular, intraperitoneal, intrasternal, subcutaneous, and intraarticular injection and infusion.

The term "pharmaceutically acceptable carrier" as used herein, means a non-toxic, inert, solid, semi-solid or liquid filler, diluent, encapsulating material or formulation auxiliary of any type. Some examples of materials which may serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols such as, but not limited to, propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants may also be present in the composition, according to the judgment of the formulator.

Pharmaceutical compositions for parenteral injection comprise pharmaceutically acceptable sterile aqueous or nonaqueous solutions, dispersions, suspensions, or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity may be maintained, for example, by the use of coating materials such as lecithin, by the maintenance of the required particle size in the case of dispersions and by the use of surfactants.

These compositions may also contain adjuvants such as preservatives, wetting agents, emulsifying agents, and dispersing agents. Prevention of the action of micro organisms may be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, which delay absorption such as aluminum monostearate and gelatin.

In some cases, in order to prolong the effect of the drug, it may be desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally-administered drug form may be accomplished by dissolving or suspending the drug in an oil vehicle.

Injectable depot forms may be made by forming microencapsule matrices of the drug in biodegradable polymers such as polylactide-polyglycolide. Depending upon the ratio of drug to polymer and the nature of the particular polymer employed, the rate of drug release may be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations may be sterilized, for example, by filtration through a bacterial-retaining filter or by incorporating sterilizing agents in the form of sterile solid compositions which may be dissolved or dispersed in sterile water or other sterile injectable medium just prior to use.

Solid dosage forms for oral administration include capsules, tablets, pills, powders and granules. In certain embodiments, solid dosage forms may contain from 1% to 95% (w/w) of a compound of formula (I), (I-a), (I-b), or (I-c). In certain embodiments, the compound of formula (I), (I-a), (I-b), or (I-c) may be present in the solid dosage form in a range of from 5% to 70% (w/w). In such solid dosage forms, the active compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

The pharmaceutical composition may be a unit dosage form. In such form the preparation is subdivided into unit doses containing appropriate quantities of the active component. The unit dosage form may be a packaged preparation, the package containing discrete quantities of preparation, such as packeted tablets, capsules, and powders in vials or ampules. Also, the unit dosage form may be a capsule, tablet, cachet, or lozenge itself, or it may be the appropriate number of any of these in packaged form. The quantity of active component in a unit dose preparation may be varied or adjusted from 0.1 mg to 1000 mg, from 1 mg to 100 mg, or from 1% to 95% (w/w) of a unit dose, according to the particular application and the potency of the active component. The composition can, if desired, also contain other compatible therapeutic agents.

The dose to be administered to a subject may be determined by the efficacy of the particular compound employed and the condition of the subject, as well as the body weight or surface area of the subject to be treated. The size of the dose also will be determined by the existence, nature, and extent of any adverse side-effects that accompany the administration of a particular compound in a particular subject. In determining the effective amount of the compound to be administered in the treatment or prophylaxis of the disorder being treated, the physician can evaluate factors such as the circulating plasma levels of the compound, compound toxicities, and/or the progression of the disease, etc. In general, the dose equivalent of a compound is from about 1 µg/kg to 100 mg/kg for a typical subject.

For administration, compounds of the formula (I), (I-a), (I-b), or (I-c) may be administered at a rate determined by factors that may include, but are not limited to, the LD₅₀ of the compound, the pharmacokinetic profile of the compound, contraindicated drugs, and the side-effects of the compound at various concentrations, as applied to the mass and overall health of the subject. Administration may be accomplished via single or divided doses.

The compounds utilized in the pharmaceutical method of the invention may be administered at the initial dosage of about 0.001 mg/kg to about 100 mg/kg daily. In certain embodiments, the daily dose range is from about 0.1 mg/kg to about 10 mg/kg. The dosages, however, may be varied depending upon the requirements of the subject, the severity of the condition being treated, and the compound being employed. Determination of the proper dosage for a particular situation is within the skill of the practitioner. Treatment may be initiated with smaller dosages, which are less than the optimum dose of the compound. Thereafter, the dosage is increased by small increments until the optimum effect under circumstances is reached. For convenience, the total daily dosage may be divided and administered in portions during the day, if desired.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules may be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which may be used include polymeric substances and waxes.

The active compounds may also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which may be prepared by mixing the compounds of this invention with suitable non-irritating carriers or carriers such as cocoa butter, polyethylene glycol or a suppository wax which are solid at room temperature but liquid at body temperature and therefore melt in the rectum or vaginal cavity and release the active compound.

Compounds of formula (I), (I-a), (I-b), or (I-c) may also be administered in the form of liposomes. Liposomes generally may be derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes may be used. The present compositions in liposome form may contain, in addition to a compound of formula (I), (I-a), (I-b), or (I-c), stabilizers, preservatives, excipients and the like. Examples of lipids include, but are not limited to, natural and synthetic phospholipids and phosphatidyl cholines (lecithins), used separately or together.

Methods to form liposomes have been described, see example, Prescott, Ed., Methods in Cell Biology, Volume XIV, Academic Press, New York, N.Y. (1976), p. 33 et seq.

Dosage forms for topical administration of a compound described herein include powders, sprays, ointments and inhalants. The active compound may be mixed under sterile conditions with a pharmaceutically acceptable carrier and any needed preservatives, buffers or propellants which may be required. Opthalmic formulations, eye ointments, powders and solutions are also contemplated as being within the scope of this invention.

### e. Methods of Use

The compounds of formula (I), (I-a), (I-b), or (I-c), or pharmaceutically acceptable salts thereof, and pharmaceutical compositions comprising a compound of formula (I), (I-a), (I-b), or (I-c), or a pharmaceutically acceptable salt thereof, may be administered to a subject suffering from a bromodomain-mediated disorder or condition. The term "administering" refers to the method of contacting a compound with a subject. Thus, the compounds of formula (I), (I-a), (I-b), or (I-c) may be administered by injection, that is, intravenously, intramuscularly, intracutaneously, subcutaneously, intraduodenally, parentally, or intraperitoneally. Also, the compounds described herein may be administered by inhalation, for example, intranasally. Additionally, the compounds of formula (I), (I-a), (I-b), or (I-c) may be administered transdermally, topically, and via implantation. In certain embodiments, the compounds of the formula (I), (I-a), (I-b), or (I-c) may be delivered orally. The compounds may also be delivered rectally, bucally, intravaginally, ocularly, andially, or by insufflation. Bromodomain-mediated disorders and conditions may be treated prophylactically, acutely, and chronically using compounds of formula (I), (I-a), (I-b), or (I-c), depending on the nature of the disorder or condition. Typically, the host or subject in each of these methods is human, although other mammals may also benefit from the administration of a compound of formula (I), (I-a), (I-b), or (I-c).

A "bromodomain-mediated disorder or condition" is characterized by the participation of one or more bromodomains (e.g., BRD4) in the inception, manifestation of one or more symptoms or disease markers, severity, or progression of a disorder or condition. Accordingly, the invention provides a method for treating cancer, including, but not limited to acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenström's macroglobulinemia, testicular tumors, uterine cancer and Wilms' tumor. The method comprises the step of administering to a subject in need thereof a therapeutically effective amount of a compound of formula (I), (I-a), (I-b), or (I-c) or a preferred embodiment thereof, with or without a pharmaceutically acceptable carrier.

The invention further provides a method for treating inflammatory diseases, inflammatory conditions, and autoimmune diseases, including, but not limited to: Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis. The method comprises the step of administering to a subject in need thereof a therapeutically effective amount of a compound of formula (I), (I-a), (I-b), or (I-c) or a preferred embodiment thereof, with or without a pharmaceutically acceptable carrier.

The invention further provides a method for treating diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, or tubular interstitial nephritis. The method comprises the step of administering to a subject in need thereof a therapeutically effective amount of a compound of formula (I), (I-a), (I-b), or (I-c) or a preferred embodiment thereof, with or without a pharmaceutically acceptable carrier.

The invention further provides a method for treating acute kidney injury or disease or condition, wherein said acute kidney injury or disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease. The method comprises the step of administering to a subject in need thereof a therapeutically effective amount of a compound of formula (I), (I-a), (I-b), or (I-c) or a preferred embodiment thereof, with or without a pharmaceutically acceptable carrier.

The invention further provides a method for treating chronic kidney disease or condition, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease, and tubular interstitial nephritis. The method comprises the step of administering to a subject in need thereof a therapeutically effective amount of a compound of formula (I), (I-a), (I-b), or (I-c), or a preferred embodiment thereof, with or without a pharmaceutically acceptable carrier.

The invention further provides a method for treating AIDS. The method comprises the step of administering to a subject in need thereof a therapeutically effective amount of a compound of formula (I), (I-a), (I-b), or (I-c), or a preferred embodiment thereof, with or without a pharmaceutically acceptable carrier.

In another embodiment, the present invention provides compounds of the invention, or pharmaceutical compositions comprising a compound of the invention, for use in medicine. In a particular embodiment, the present invention provides compounds of the invention, or pharmaceutical compositions comprising a compound of the invention, for use in the treatment of diseases or disorders as described herein above.

One embodiment is directed to the use of a compound according to formula (I), (I-a), (I-b), or (I-c), or a pharmaceutically acceptable salt thereof in the preparation of a medicament. The medicament optionally may comprise at least one additional therapeutic agent. In some embodiments the medicament is for use in the treatment of diseases and disorders as described herein above.

This invention is also directed to the use of a compound according to formula (I), (I-a), (I-b), or (I-c), or a pharmaceutically acceptable salt thereof in the manufacture of a medicament for the treatment of the diseases and disorders as described herein above. The medicament optionally may comprise at least one additional therapeutic agent.

The compounds of formula (I), (I-a), (I-b), or (I-c) may be administered as the sole active agent or it may be co-administered with other therapeutic agents, including other compounds that demonstrate the same or a similar therapeutic activity and that are determined to be safe and efficacious for such combined administration. The term "co-administered" means the administration of two or more different therapeutic agents or treatments (e.g., radiation treatment) that are administered to a subject in a single pharmaceutical composition or in separate pharmaceutical compositions. Thus coadministration involves administration at the same time of a single pharmaceutical composition comprising two or more different therapeutic agents or administration of two or more different compositions to the same subject at the same or different times.

The compounds of the invention may be co-administered with a therapeutically effective amount of at least one additional therapeutic agent to treat cancer, where examples of the therapeutic agents include, such as radiation, alkylating agents, angiogenesis inhibitors, antibodies, antimetabolites, antimitotics, antiproliferatives, antivirals, aurora kinase inhibitors, apoptosis promoters (for example, Bcl-xL, Bcl-w and Bfl-1) inhibitors, activators of death receptor pathway, Bcr-Abl kinase inhibitors, BiTE (Bi-Specific T cell Engager) antibodies, antibody drug conjugates, biologic response modifiers, cyclin-dependent kinase inhibitors, cell cycle inhibitors, cyclooxygenase-2 inhibitors, DVDs (dual variable domain antibodies), leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNA's, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (bromodomain) inhibitors, proteosome inhibitors, purine analogs, pyrimidine analogs, receptor tyrosine kinase inhibitors, etinoids/deltoids plant alkaloids, small inhibitory ribonucleic acids (siRNAs), topoisomerase inhibitors, ubiquitin ligase inhibitors, and the like, and in combination with at least one of these agents .

BiTE antibodies are bi-specific antibodies that direct T-cells to attack cancer cells by simultaneously binding the two cells. The T-cell then attacks the target cancer cell. Examples of BiTE antibodies include adecatumumab (Micromet MT201), blinatumomab (Micromet MT103) and the like. Without being limited by theory, one of the mechanisms by which T-cells elicit apoptosis of the target cancer cell is by exocytosis of cytolytic granule components, which include perforin and granzyme B. In this regard, Bcl-2 has been shown to attenuate the induction of apoptosis by both perforin and granzyme B. These data suggest that inhibition of Bcl-2 could enhance the cytotoxic effects elicited by T-cells when targeted to cancer cells (V.R. Sutton, D.L. Vaux and J.A. Trapani, J. of Immunology 1997, 158 (12), 5783).

SiRNAs are molecules having endogenous RNA bases or chemically modified nucleotides. The modifications do not abolish cellular activity, but rather impart increased stability and/or increased cellular potency. Examples of chemical modifications include phosphorothioate groups, 2'-deoxynucleotide, 2'-OCH₃-containing ribonucleotides, 2'-F-ribonucleotides, 2'-methoxyethyl ribonucleotides, combinations thereof and the like. The siRNA can have varying lengths (e.g., 10-200 bps) and structures (e.g., hairpins, single/double strands, bulges, nicks/gaps, mismatches) and are processed in cells to provide active gene silencing. A double-stranded siRNA (dsRNA) can have the same number of nucleotides on each strand (blunt ends) or asymmetric ends (overhangs). The overhang of 1-2 nucleotides may be present on the sense and/or the antisense strand, as well as present on the 5'- and/ or the 3'-ends of a given strand.

Multivalent binding proteins are binding proteins comprising two or more antigen binding sites. Multivalent binding proteins are engineered to have the three or more antigen binding sites and are generally not naturally occurring antibodies. The term "multispecific binding protein" means a binding protein capable of binding two or more related or unrelated targets. Dual variable domain (DVD) binding proteins are tetravalent or multivalent binding proteins binding proteins comprising two or more antigen binding sites. Such DVDs may be monospecific (i.e., capable of binding one antigen) or multispecific (i.e., capable of binding two or more antigens). DVD binding proteins comprising two heavy chain DVD polypeptides and two light chain DVD polypeptides are referred to as DVD Ig's. Each half of a DVD Ig comprises a heavy chain DVD polypeptide, a light chain DVD polypeptide, and two antigen binding sites. Each binding site comprises a heavy chain variable domain and a light chain variable domain with a total of 6 CDRs involved in antigen binding per antigen binding site. Multispecific DVDs include DVD binding proteins that bind DLL4 and VEGF, or C-met and EFGR or ErbB3 and EGFR.

Alkylating agents include altretamine, AMD-473, AP-5280, apaziquone, bendamustine, brostallicin, busulfan, carboquone, carmustine (BCNU), chlorambucil, CLORETAZINE® (laromustine, VNP 40101M), cyclophosphamide, decarbazine, estramustine, fotemustine, glufosfamide, ifosfamide, KW-2170, lomustine (CCNU), mafosfamide, melphalan, mitobronitol, mitolactol, nimustine, nitrogen mustard N-oxide, ranimustine, temozolomide, thiotepa, TREANDA® (bendamustine), treosulfan, rofosfamide and the like.

Angiogenesis inhibitors include endothelial-specific receptor tyrosine kinase (Tie-2) inhibitors, epidermal growth factor receptor (EGFR) inhibitors, insulin growth factor-2 receptor (IGFR-2) inhibitors, matrix metalloproteinase-2 (MMP-2) inhibitors, matrix metalloproteinase-9 (MMP-9) inhibitors, platelet-derived growth factor receptor (PDGFR) inhibitors, thrombospondin analogs, vascular endothelial growth factor receptor tyrosine kinase (VEGFR) inhibitors and the like.

Antimetabolites include ALIMTA® (pemetrexed disodium, LY231514, MTA), 5-azacitidine, XELODA® (capecitabine), carmofur, LEUSTAT® (cladribine), clofarabine, cytarabine, cytarabine ocfosfate, cytosine arabinoside, decitabine, deferoxamine, doxifluridine, eflornithine, EICAR (5-ethynyl-1-β-D-ribofuranosylimidazole-4-carboxamide), enocitabine, ethnylcytidine, fludarabine, 5-fluorouracil alone or in combination with leucovorin, GEMZAR® (gemcitabine), hydroxyurea, ALKERAN®(melphalan), mercaptopurine, 6-mercaptopurine riboside, methotrexate, mycophenolic acid, nelarabine, nolatrexed, ocfosfate, pelitrexol, pentostatin, raltitrexed, Ribavirin, triapine, trimetrexate, S-1, tiazofurin, tegafur, TS-1, vidarabine, UFT and the like.

Antivirals include ritonavir, hydroxychloroquine and the like.

Aurora kinase inhibitors include ABT-348, AZD-1152, MLN-8054, VX-680, Aurora A-specific kinase inhibitors, Aurora B-specific kinase inhibitors and pan-Aurora kinase inhibitors and the like.

Bcl-2 protein inhibitors include AT-101 ((-)gossypol), GENASENSE® (G3139 or oblimersen (Bcl-2-targeting antisense oligonucleotide)), IPI-194, IPI-565, N-(4-(4-((4'-chloro(1,1'-biphenyl)-2-yl)methyl)piperazin-1-yl)benzoyl)-4-(((1R)-3-(dimethylamino)-1-((phenylsulfanyl)methyl)propyl)amino)-3-nitrobenzenesulfonamide) (ABT-737), N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide (ABT-263), GX-070 (obatoclax) and the like.

Bcr-Abl kinase inhibitors include DASATINIB® (BMS-354825), GLEEVEC® (imatinib) and the like.

CDK inhibitors include AZD-5438, BMI-1040, BMS-032, BMS-387, CVT-2584, flavopyridol, GPC-286199, MCS-5A, PD0332991, PHA-690509, seliciclib (CYC-202, R-roscovitine), ZK-304709 and the like.

COX-2 inhibitors include ABT-963, ARCOXIA® (etoricoxib), BEXTRA® (valdecoxib), BMS347070, CELEBREX® (celecoxib), COX-189 (lumiracoxib), CT-3, DERAMAXX® (deracoxib), JTE-522, 4-methyl-2-(3,4-dimethylphenyl)-1-(4-sulfamoylphenyl-1*H*-pyrrole), MK-663 (etoricoxib), NS-398, parecoxib, RS-57067, SC-58125, SD-8381, SVT-2016, S-2474, T-614, VIOXX® (rofecoxib) and the like.

EGFR inhibitors include EGFR antibodies, ABX-EGF, anti-EGFR immunoliposomes, EGF-vaccine, EMD-7200, ERBITUX® (cetuximab), HR3, IgA antibodies, IRESSA® (gefitinib), TARCEVA® (erlotinib or OSI-774), TP-38, EGFR fusion protein, TYKERB® (lapatinib) and the like.

ErbB2 receptor inhibitors include CP-724-714, CI-1033 (canertinib), HERCEPTIN® (trastuzumab), TYKERB® (lapatinib), OMNITARG® (2C4, petuzumab), TAK-165, GW-572016 (ionafarnib), GW-282974, EKB-569, PI-166, dHER2 (HER2 vaccine), APC-8024 (HER-2 vaccine), anti-HER/2neu bispecific antibody, B7.her2IgG3, AS HER2 trifunctional bispecfic antibodies, mAB AR-209, mAB 2B-1 and the like.

Histone deacetylase inhibitors include depsipeptide, LAQ-824, MS-275, trapoxin, suberoylanilide hydroxamic acid (SAHA), TSA, valproic acid and the like.

HSP-90 inhibitors include 17-AAG-nab, 17-AAG, CNF-101, CNF-1010, CNF-2024, 17-DMAG, geldanamycin, IPI-504, KOS-953, MYCOGRAB® (human recombinant antibody to HSP-90), NCS-683664, PU24FCl, PU-3, radicicol, SNX-2112, STA-9090 VER49009 and the like.

Inhibitors of inhibitors of apoptosis proteins include HGS1029, GDC-0145, GDC-0152, LCL-161, LBW-242 and the like.

Antibody drug conjugates include anti-CD22-MC-MMAF, anti-CD22-MC-MMAE, anti-CD22-MCC-DM1, CR-011-vcMMAE, PSMA-ADC, MEDI-547, SGN-19Am SGN-35, SGN-75 and the like

Activators of death receptor pathway include TRAIL, antibodies or other agents that target TRAIL or death receptors (e.g., DR4 and DR5) such as Apomab, conatumumab, ETR2-ST01, GDC0145, (lexatumumab), HGS-1029, LBY-135, PRO-1762 and trastuzumab.

Kinesin inhibitors include Eg5 inhibitors such as AZD4877, ARRY-520; CENPE inhibitors such as GSK923295A and the like.

JAK-2 inhibitors include CEP-701 (lesaurtinib), XL019 and INCB018424 and the like.

MEK inhibitors include ARRY-142886, ARRY-438162 PD-325901, PD-98059 and the like.

mTOR inhibitors include AP-23573, CCI-779, everolimus, RAD-001, rapamycin, temsirolimus, ATP-competitive TORC1/TORC2 inhibitors, including PI-103, PP242, PP30, Torin 1 and the like.

Non-steroidal anti-inflammatory drugs include AMIGESIC® (salsalate), DOLOBID® (diflunisal), MOTRIN® (ibuprofen), ORUDIS® (ketoprofen), RELAFEN® (nabumetone), FELDENE® (piroxicam), ibuprofen cream, ALEVE® (naproxen) and NAPROSYN® (naproxen), VOLTAREN® (diclofenac), INDOCIN® (indomethacin), CLINORIL® (sulindac), TOLECTIN® (tolmetin), LODINE® (etodolac), TORADOL® (ketorolac), DAYPRO® (oxaprozin) and the like.

PDGFR inhibitors include C-451, CP-673, CP-868596 and the like.

Platinum chemotherapeutics include cisplatin, ELOXATIN® (oxaliplatin) eptaplatin, lobaplatin, nedaplatin, PARAPLATIN® (carboplatin), satraplatin, picoplatin and the like.

Polo-like kinase inhibitors include BI-2536 and the like.

Phosphoinositide-3 kinase (PI3K) inhibitors include wortmannin, LY294002, XL-147, CAL-120, ONC-21, AEZS-127, ETP-45658, PX-866, GDC-0941, BGT226, BEZ235, XL765 and the like.

Thrombospondin analogs include ABT-510, ABT-567, ABT-898, TSP-1 and the like.

VEGFR inhibitors include AVASTIN® (bevacizumab), ABT-869, AEE-788, ANGIOZYME™ (a ribozyme that inhibits angiogenesis (Ribozyme Pharmaceuticals (Boulder, CO.) and Chiron, (Emeryville, CA)), axitinib (AG-13736), AZD-2171, CP-547,632, IM-862, MACUGEN (pegaptamib), NEXAVAR® (sorafenib, BAY43-9006), pazopanib (GW-786034), vatalanib (PTK-787, ZK-222584), SUTENT® (sunitinib, SU-11248), VEGF trap, ZACTIMA™ (vandetanib, ZD-6474), GA101, ofatumumab, ABT-806 (mAb-806), ErbB3 specific antibodies, BSG2 specific antibodies, DLL4 specific antibodies and C-met specific antibodies, and the like.

Antibiotics include intercalating antibiotics aclarubicin, actinomycin D, amrubicin, annamycin, adriamycin, BLENOXANE® (bleomycin), daunorubicin, CAELYX® or MYOCET® (liposomal doxorubicin), elsamitrucin, epirbucin, glarbuicin, ZAVEDOS® (idarubicin), mitomycin C, nemorubicin, neocarzinostatin, peplomycin, pirarubicin, rebeccamycin, stimalamer, streptozocin, VALSTAR® (valrubicin), zinostatin and the like.

Topoisomerase inhibitors include aclarubicin, 9-aminocamptothecin, amonafide, amsacrine, becatecarin, belotecan, BN-80915, CAMPTOSAR® (irinotecan hydrochloride), camptothecin, CARDIOXANE® (dexrazoxine), diflomotecan, edotecarin, ELLENCE® or PHARMORUBICIN® (epirubicin), etoposide, exatecan, 10-hydroxycamptothecin, gimatecan, lurtotecan, mitoxantrone, orathecin, pirarbucin, pixantrone, rubitecan, sobuzoxane, SN-38, tafluposide, topotecan and the like.

Antibodies include AVASTIN® (bevacizumab), CD40-specific antibodies, chTNT-1/B, denosumab, ERBITUX® (cetuximab), HUMAX-CD4® (zanolimumab), IGFlR-specific antibodies, lintuzumab, PANOREX® (edrecolomab), RENCAREX® (WX G250), RITUXAN® (rituximab), ticilimumab, trastuzimab, CD20 antibodies types I and II and the like.

Hormonal therapies include ARIMIDEX® (anastrozole), AROMASIN® (exemestane), arzoxifene, CASODEX® (bicalutamide), CETROTIDE® (cetrorelix), degarelix, deslorelin, DESOPAN® (trilostane), dexamethasone, DROGENIL® (flutamide), EVISTA® (raloxifene), AFEMA™ (fadrozole), FARESTON® (toremifene), FASLODEX® (fulvestrant), FEMARA® (letrozole), formestane, glucocorticoids, HECTOROL® (doxercalciferol), RENAGEL® (sevelamer carbonate), lasofoxifene, leuprolide acetate, MEGACE® (megesterol), MIFEPREX® (mifepristone), NILANDRON™ (nilutamide), NOLVADEX® (tamoxifen citrate), PLENAXIS™ (abarelix), prednisone, PROPECIA® (finasteride), rilostane, SUPREFACT® (buserelin), TRELSTAR® (luteinizing hormone releasing hormone (LHRH)), VANTAS® (Histrelin implant), VETORYL® (trilostane or modrastane), ZOLADEX® (fosrelin, goserelin) and the like.

Deltoids and retinoids include seocalcitol (EB1089, CB 1093), lexacalcitrol (KH1060), fenretinide, PANRETIN® (aliretinoin), ATRAGEN® (liposomal tretinoin), TARGRETIN® (bexarotene), LGD-1550 and the like.

PARP inhibitors include ABT-888 (veliparib), olaparib, KU-59436, AZD-2281, AG-014699, BSI-201, BGP-15, INO-1001, ONO-2231 and the like.

Plant alkaloids include, but are not limited to, vincristine, vinblastine, vindesine, vinorelbine and the like.

Proteasome inhibitors include VELCADE® (bortezomib), MG132, NPI-0052, PR-171 and the like.

Examples of immunologicals include interferons and other immune-enhancing agents. Interferons include interferon alpha, interferon alpha-2a, interferon alpha-2b, interferon beta, interferon gamma-la, ACTIMMUNE® (interferon gamma-lb) or interferon gamma-nl, combinations thereof and the like. Other agents include ALFAFERONE®,(IFN-α), BAM-002 (oxidized glutathione), BEROMUN® (tasonermin), BEXXAR® (tositumomab), CAMPATH® (alemtuzumab), CTLA4 (cytotoxic lymphocyte antigen 4), decarbazine, denileukin, epratuzumab, GRANOCYTE® (lenograstim), lentinan, leukocyte alpha interferon, imiquimod, MDX-010 (anti-CTLA-4), melanoma vaccine, mitumomab, molgramostim, MYLOTARG™ (gemtuzumab ozogamicin), NEUPOGEN® (filgrastim), OncoVAC-CL, OVAREX® (oregovomab), pemtumomab (Y-muHMFG1), PROVENGE® (sipuleucel-T), sargaramostim, sizofilan, teceleukin, THERACYS® (Bacillus Calmette-Guerin), ubenimex, VIRULIZIN® (immunotherapeutic, Lorus Pharmaceuticals), Z-100 (Specific Substance of Maruyama (SSM)), WF-10 (Tetrachlorodecaoxide (TCDO)), PROLEUKIN® (aldesleukin), ZADAXIN® (thymalfasin), ZENAPAX® (daclizumab), ZEVALIN® (90Y-Ibritumomab tiuxetan) and the like.

Biological response modifiers are agents that modify defense mechanisms of living organisms or biological responses, such as survival, growth or differentiation of tissue cells to direct them to have anti-tumor activity and include krestin, lentinan, sizofiran, picibanil PF-3512676 (CpG-8954), ubenimex and the like.

Pyrimidine analogs include cytarabine (ara C or Arabinoside C), cytosine arabinoside, doxifluridine, FLUDARA® (fludarabine), 5-FU (5-fluorouracil), floxuridine, GEMZAR® (gemcitabine), TOMUDEX® (ratitrexed), TROXATYL™ (triacetyluridine troxacitabine) and the like.

Purine analogs include LANYIS® (thioguanine) and PURI-NETHOL® (mercaptopurine).

Antimitotic agents include batabulin, epothilone D (KOS-862), N-(2-((4-hydroxyphenyl)amino)pyridin-3-yl)-4-methoxybenzenesulfonamide, ixabepilone (BMS 247550), paclitaxel, TAXOTERE® (docetaxel), PNU100940 (109881), patupilone, XRP-9881 (larotaxel), vinflunine, ZK-EPO (synthetic epothilone) and the like.

Ubiquitin ligase inhibitors include MDM2 inhibitors, such as nutlins, NEDD8 inhibitors such as MLN4924 and the like.

Compounds of this invention may also be used as radiosensitizers that enhance the efficacy of radiotherapy. Examples of radiotherapy include external beam radiotherapy, teletherapy, brachytherapy and sealed, unsealed source radiotherapy and the like.

Additionally, compounds of formula (I), (I-a), (I-b), or (I-c) may be combined with other chemotherapeutic agents such as ABRAXANE™ (ABI-007), ABT-100 (farnesyl transferase inhibitor), ADVEXIN® (Ad5CMV-p53 vaccine), ALTOCOR® or MEVACOR® (lovastatin), AMPLIGEN® (poly I:poly C12U, a synthetic RNA), APTOSYN® (exisulind), AREDIA® (pamidronic acid), arglabin, L-asparaginase, atamestane (1-methyl-3,17-dione-androsta-1,4-diene), AVAGE® (tazarotene), AVE-8062 (combreastatin derivative) BEC2 (mitumomab), cachectin or cachexin (tumor necrosis factor), canvaxin (vaccine), CEAVAC® (cancer vaccine), CELEUK® (celmoleukin), CEPLENE® (histamine dihydrochloride), CERVARIX® (human papillomavirus vaccine), CHOP® (C: CYTOXAN® (cyclophosphamide); H: ADRIAMYCIN® (hydroxydoxorubicin); O: Vincristine (ONCOVIN®); P: prednisone), CYPAT™ (cyproterone acetate), combrestatin A4P, DAB(389)EGF (catalytic and translocation domains of diphtheria toxin fused via a His-Ala linker to human epidermal growth factor) or TransMID-107R™ (diphtheria toxins), dacarbazine, dactinomycin, 5,6-dimethylxanthenone-4-acetic acid (DMXAA), eniluracil, EVIZON™ (squalamine lactate), DIMERICINE® (T4N5 liposome lotion), discodermolide, DX-8951f (exatecan mesylate), enzastaurin, EPO906 (epithilone B), GARDASIL® (quadrivalent human papillomavirus (Types 6, 11, 16, 18) recombinant vaccine), GASTRIMMUNE®, GENASENSE®, GMK (ganglioside conjugate vaccine), GVAX® (prostate cancer vaccine), halofuginone, histerelin, hydroxycarbamide, ibandronic acid, IGN-101, IL-13-PE38, IL-13-PE38QQR (cintredekin besudotox), IL-13-pseudomonas exotoxin, interferon-a, interferon-y, JUNOVAN™ or MEPACT™ (mifamurtide), lonafarnib, 5,10-methylenetetrahydrofolate, miltefosine (hexadecylphosphocholine), NEOVASTAT®(AE-941), NEUTREXIN® (trimetrexate glucuronate), NIPENT® (pentostatin), ONCONASE® (a ribonuclease enzyme), ONCOPHAGE®(melanoma vaccine treatment), ONCOVAX® (IL-2 Vaccine), ORATHECIN™ (rubitecan), OSIDEM® (antibody-based cell drug), OVAREX® MAb (murine monoclonal antibody), paclitaxel, PANDIMEX™ (aglycone saponins from ginseng comprising 20(S)protopanaxadiol (aPPD) and 20(S)protopanaxatriol (aPPT)), panitumumab, PANVAC®-VF (investigational cancer vaccine), pegaspargase, PEG Interferon A, phenoxodiol, procarbazine, rebimastat, REMOVAB® (catumaxomab), REVLIMID® (lenalidomide), RSR13 (efaproxiral), SOMATULINE® LA (lanreotide), SORIATANE® (acitretin), staurosporine (Streptomyces staurospores), talabostat (PT100), TARGRETIN® (bexarotene), TAXOPREXIN® (DHA-paclitaxel), TELCYTA® (canfosfamide, TLK286), temilifene, TEMODAR® (temozolomide), tesmilifene, thalidomide, THERATOPE® (STn-KLH), thymitaq (2-amino-3,4-dihydro-6-methyl-4-oxo-5-(4-pyridylthio)quinazoline dihydrochloride), TNFERADE™ (adenovector: DNA carrier containing the gene for tumor necrosis factor-a), TRACLEER® or ZAVESCA® (bosentan), tretinoin (Retin-A), tetrandrine, TRISENOX® (arsenic trioxide), VIRULIZIN®, ukrain (derivative of alkaloids from the greater celandine plant), vitaxin (anti-alphavbeta3 antibody), XCYTRIN® (motexafin gadolinium), XINLAY™ (atrasentan), XYOTAX™ (paclitaxel poliglumex), YONDELIS® (trabectedin), ZD-6126, ZINECARD® (dexrazoxane), ZOMETA® (zolendronic acid), zorubicin and the like.

The compounds of the invention may also be co-administered with a therapeutically effective amount of at least one additional therapeutic agents to treat an inflammatory disease or condition, or autoimmune disease, where examples of the agents include, such as methotrexate, 6-mercaptopurine, azathioprine sulphasalazine, mesalazine, olsalazine chloroquinine/ hydroxychloroquine, pencillamine, aurothiomalate (intramuscular and oral), azathioprine, cochicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g., NIK, IKK, p38 or MAP kinase inhibitors), IL-1β converting enzyme inhibitors, T-cell signalling inhibitors such as kinase inhibitors, metalloproteinase inhibitors, sulfasalazine, 6-mercaptopurines, angiotensin converting enzyme inhibitors, soluble cytokine receptors and derivatives thereof (e.g. soluble p55 or p75 TNF receptors and the derivatives p75TNFRIgG (etanercept) and p55TNFRIgG (Lenercept), sIL-1RI, sIL-1RII, sIL-6R), antiinflammatory cytokines (e.g. IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone HCl, hydrocodone bitartrate/apap, diclofenac sodium/misoprostol, fentanyl, anakinra, tramadol HCl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline HCl, sulfadiazine, oxycodone HCl/acetaminophen, olopatadine HCl misoprostol, naproxen sodium, omeprazole, cyclophosphamide, rituximab, IL-1 TRAP, MRA, CTLA4-IG, IL-18 BP, anti-IL-12, Anti-IL15, BIRB-796, SCIO-469, VX-702, AMG-548, VX-740, Roflumilast, IC-485, CDC-801, S1P1 agonists (such as FTY720), PKC family inhibitors (such as Ruboxistaurin or AEB-071) and Mesopram. In certain embodiments, combinations include methotrexate or leflunomide and in moderate or severe rheumatoid arthritis cases, cyclosporine and anti-TNF antibodies as noted above.

Non-limiting examples of therapeutic agents for inflammatory bowel disease with which a compound of formula (I), (I-a), (I-b), or (I-c) may be co-administered include the following: budenoside; epidermal growth factor; corticosteroids; cyclosporin, sulfasalazine; aminosalicylates; 6-mercaptopurine; azathioprine; metronidazole; lipoxygenase inhibitors; mesalamine; olsalazine; balsalazide; antioxidants; thromboxane inhibitors; IL-1 receptor antagonists; anti-IL-1β monoclonal antibodies; anti-IL-6 monoclonal antibodies; growth factors; elastase inhibitors; pyridinyl-imidazole compounds; antibodies to or antagonists of other human cytokines or growth factors, for example, TNF, LT, IL-1, IL-2, IL-6, IL-7, IL-8, IL-12, IL-15, IL-16, IL-23, EMAP-II, GM-CSF, FGF, and PDGF; cell surface molecules such as CD2, CD3, CD4, CD8, CD25, CD28, CD30, CD40, CD45, CD69, CD90 or their ligands; methotrexate; cyclosporine; FK506; rapamycin; mycophenolate mofetil; leflunomide; NSAIDs, for example, ibuprofen; corticosteroids such as prednisolone; phosphodiesterase inhibitors; adenosine agonists; antithrombotic agents; complement inhibitors; adrenergic agents; agents which interfere with signalling by proinflammatory cytokines such as TNFα or IL-1 (e.g. NIK, IKK, or MAP kinase inhibitors); IL-1β converting enzyme inhibitors; TNFα converting enzyme inhibitors; T-cell signalling inhibitors such as kinase inhibitors; metalloproteinase inhibitors; sulfasalazine; azathioprine; 6-mercaptopurines; angiotensin converting enzyme inhibitors; soluble cytokine receptors and derivatives thereof (e.g. soluble p55 or p75 TNF receptors, sIL-1RI, sIL-1RII, sIL-6R) and antiinflammatory cytokines (e.g. IL-4, IL-10, IL-11, IL-13 and TGFβ). Preferred examples of therapeutic agents for Crohn's disease with which a compound of formula (I), (I-a), (I-b), or (I-c) may be combined include the following: TNF antagonists, for example, anti-TNF antibodies, D2E7 (adalimumab), CA2 (infliximab), CDP 571, TNFR-Ig constructs, (p75TNFRIgG (etanercept) and p55TNFRIgG (LENERCEPT™) inhibitors and PDE4 inhibitors. A compound of formula (I), (I-a), (I-b), or (I-c) may be combined with corticosteroids, for example, budenoside and dexamethasone; sulfasalazine, 5-aminosalicylic acid; olsalazine; and agents which interfere with synthesis or action of proinflammatory cytokines such as IL-1, for example, IL-1β converting enzyme inhibitors and IL-1ra; T cell signaling inhibitors, for example, tyrosine kinase inhibitors; 6-mercaptopurine; IL-11; mesalamine; prednisone; azathioprine; mercaptopurine; infliximab; methylprednisolone sodium succinate; diphenoxylate/atrop sulfate; loperamide hydrochloride; methotrexate; omeprazole; folate; ciprofloxacin/dextrose-water; hydrocodone bitartrate/apap; tetracycline hydrochloride; fluocinonide; metronidazole; thimerosal/boric acid; cholestyramine/sucrose; ciprofloxacin hydrochloride; hyoscyamine sulfate; meperidine hydrochloride; midazolam hydrochloride; oxycodone HCl/acetaminophen; promethazine hydrochloride; sodium phosphate; sulfamethoxazole/trimethoprim; celecoxib; polycarbophil; propoxyphene napsylate; hydrocortisone; multivitamins; balsalazide disodium; codeine phosphate/apap; colesevelam HCl; cyanocobalamin; folic acid; levofloxacin; methylprednisolone; natalizumab and interferon-gamma.

Non-limiting examples of therapeutic agents for multiple sclerosis with which a compound of formula (I), (I-a), (I-b), or (I-c) may be co-administered include the following: corticosteroids; prednisolone; methylprednisolone; azathioprine; cyclophosphamide; cyclosporine; methotrexate; 4-aminopyridine; tizanidine; interferon-β1a (AVONEX®; Biogen); interferon-β1b (BETASERON®; Chiron/Berlex); interferon α-n3) (Interferon Sciences/Fujimoto), interferon-α (Alfa Wassermann/J&J), interferon β1A-IF (Serono/Inhale Therapeutics), Peginterferon α 2b (Enzon/Schering-Plough), Copolymer 1 (Cop-1; COPAXONE®; Teva Pharmaceutical Industries, Inc.); hyperbaric oxygen; intravenous immunoglobulin; cladribine; antibodies to or antagonists of other human cytokines or growth factors and their receptors, for example, TNF, LT, IL-1, IL-2, IL-6, IL-7, IL-8, IL-12, IL-23, IL-15, IL-16, EMAP-II, GM-CSF, FGF, and PDGF. A compound of formula (I), (I-a), (I-b), or (I-c) may be combined with antibodies to cell surface molecules such as CD2, CD3, CD4, CD8, CD19, CD20, CD25, CD28, CD30, CD40, CD45, CD69, CD80, CD86, CD90 or their ligands. A compound of formula (I), (I-a), (I-b), or (I-c) may also be combined with agents such as methotrexate, cyclosporine, FK506, rapamycin, mycophenolate mofetil, leflunomide, an S1P1 agonist, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory cytokines such as TNFα or IL-1 (e.g., NIK, IKK, p38 or MAP kinase inhibitors), IL-1β converting enzyme inhibitors, TACE inhibitors, T-cell signaling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors, sIL-1RI, sIL-1RII, sIL-6R) and antiinflammatory cytokines (e.g. IL-4, IL-10, IL-13 and TGFβ).

A compound of formula (I), (I-a), (I-b), or (I-c) may also be co-administered with agents, such as alemtuzumab, dronabinol, daclizumab, mitoxantrone, xaliproden hydrochloride, fampridine, glatiramer acetate, natalizumab, sinnabidol, α-immunokine NNSO3, ABR-215062, AnergiX.MS, chemokine receptor antagonists, BBR-2778, calagualine, CPI-1189, LEM (liposome encapsulated mitoxantrone), THC.CBD (cannabinoid agonist), MBP-8298, mesopram (PDE4 inhibitor), MNA-715, anti-IL-6 receptor antibody, neurovax, pirfenidone allotrap 1258 (RDP-1258), sTNF-R1, talampanel, teriflunomide, TGF-beta2, tiplimotide, VLA-4 antagonists (for example, TR-14035, VLA4 Ultrahaler, Antegran-ELAN/Biogen), interferon gamma antagonists and IL-4 agonists.

Non-limiting examples of therapeutic agents for ankylosing spondylitis with which a compound of formula (I), (I-a), (I-b), or (I-c) may be co-administered include the following: ibuprofen, diclofenac, misoprostol, naproxen, meloxicam, indomethacin, diclofenac, celecoxib, rofecoxib, sulfasalazine, methotrexate, azathioprine, minocyclin, prednisone, and anti-TNF antibodies, D2E7 (HUMIRA®), CA2 (infliximab), CDP 571, TNFR-Ig constructs, (p75TNFRIgG (ENBREL®) and p55TNFRIgG (LENERCEPT®).

Non-limiting examples of therapeutic agents for asthma with which a compound of formula (I), (I-a), (I-b), or (I-c) may be co-administered include the following: albuterol, salmeterol/fluticasone, montelukast sodium, fluticasone propionate, budesonide, prednisone, salmeterol xinafoate, levalbuterol HCl, albuterol sulfate/ipratropium, prednisolone sodium phosphate, triamcinolone acetonide, beclomethasone dipropionate, ipratropium bromide, azithromycin, pirbuterol acetate, prednisolone, theophylline anhydrous, methylprednisolone sodium succinate, clarithromycin, zafirlukast, formoterol fumarate, influenza virus vaccine, amoxicillin trihydrate, flunisolide, allergy injection, cromolyn sodium, fexofenadine hydrochloride, flunisolide/menthol, amoxicillin/clavulanate, levofloxacin, inhaler assist device, guaifenesin, dexamethasone sodium phosphate, moxifloxacin HCl, doxycycline hyclate, guaifenesin/d-methorphan, p-ephedrine/cod/chlorphenir, gatifloxacin, cetirizine hydrochloride, mometasone furoate, salmeterol xinafoate, benzonatate, cephalexin, pe/hydrocodone/chlorphenir, cetirizine HCl/pseudoephed, phenylephrine/cod/promethazine, codeine/promethazine, cefprozil, dexamethasone, guaifenesin/pseudoephedrine, chlorpheniramine/hydrocodone, nedocromil sodium, terbutaline sulfate, epinephrine, methylprednisolone, anti-IL-13 antibody, and metaproterenol sulfate.

Non-limiting examples of therapeutic agents for COPD with which a compound of formula (I), (I-a), (I-b), or (I-c) may be co-administered include the following: albuterol sulfate/ipratropium, ipratropium bromide, salmeterol/fluticasone, albuterol, salmeterol xinafoate, fluticasone propionate, prednisone, theophylline anhydrous, methylprednisolone sodium succinate, montelukast sodium, budesonide, formoterol fumarate, triamcinolone acetonide, levofloxacin, guaifenesin, azithromycin, beclomethasone dipropionate, levalbuterol HCl, flunisolide, ceftriaxone sodium, amoxicillin trihydrate, gatifloxacin, zafirlukast, amoxicillin/clavulanate, flunisolide/menthol, chlorpheniramine/hydrocodone, metaproterenol sulfate, methylprednisolone, mometasone furoate, p-ephedrine/cod/chlorphenir, pirbuterol acetate, p-ephedrine/loratadine, terbutaline sulfate, tiotropium bromide, (R,R)-formoterol, TgAAT, cilomilast and roflumilast.

Non-limiting examples of therapeutic agents for psoriasis with which a compound of formula (I), (I-a), (I-b), or (I-c) may be co-administered include the following: calcipotriene, clobetasol propionate, triamcinolone acetonide, halobetasol propionate, tazarotene, methotrexate, fluocinonide, betamethasone diprop augmented, fluocinolone acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate/emoll, fluticasone propionate, azithromycin, hydrocortisone, moisturizing formula, folic acid, desonide, pimecrolimus, coal tar, diflorasone diacetate, etanercept folate, lactic acid, methoxsalen, hc/bismuth subgal/znox/resor, methylprednisolone acetate, prednisone, sunscreen, halcinonide, salicylic acid, anthralin, clocortolone pivalate, coal extract, coal tar/salicylic acid, coal tar/salicylic acid/sulfur, desoximetasone, diazepam, emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, cyclosporine, alefacept, efalizumab, tacrolimus, pimecrolimus, PUVA, UVB, sulfasalazine, ABT-874 and ustekinamab.

Non-limiting examples of therapeutic agents for psoriatic arthritis with which a compound of formula (I), (I-a), (I-b), or (I-c) may be co-administered include the following: methotrexate, etanercept, rofecoxib, celecoxib, folic acid, sulfasalazine, naproxen, leflunomide, methylprednisolone acetate, indomethacin, hydroxychloroquine sulfate, prednisone, sulindac, betamethasone diprop augmented, infliximab, methotrexate, folate, triamcinolone acetonide, diclofenac, dimethylsulfoxide, piroxicam, diclofenac sodium, ketoprofen, meloxicam, methylprednisolone, nabumetone, tolmetin sodium, calcipotriene, cyclosporine, diclofenac sodium/misoprostol, fluocinonide, glucosamine sulfate, gold sodium thiomalate, hydrocodone bitartrate/apap, ibuprofen, risedronate sodium, sulfadiazine, thioguanine, valdecoxib, alefacept, D2E7 (adalimumab), and efalizumab.

Examples of therapeutic agents for SLE (Lupus) with which a compound of formula (I), (I-a), (I-b), or (I-c) may be co-administered include the following: NSAIDS, for example, diclofenac, naproxen, ibuprofen, piroxicam, indomethacin; COX2 inhibitors, for example, celecoxib, rofecoxib, valdecoxib; anti-malarials, for example, hydroxychloroquine; steroids, for example, prednisone, prednisolone, budenoside, dexamethasone; cytotoxics, for example, azathioprine, cyclophosphamide, mycophenolate mofetil, methotrexate; inhibitors of PDE4 or purine synthesis inhibitor, for example Cellcept®. A compound of formula (I), (I-a), (I-b), or (I-c) may also be combined with agents such as sulfasalazine, 5-aminosalicylic acid, olsalazine, Imuran® and agents which interfere with synthesis, production or action of proinflammatory cytokines such as IL-1, for example, caspase inhibitors like IL-1β converting enzyme inhibitors and IL-1ra. A compound of formula (I), (I-a), (I-b), or (I-c) may also be used with T cell signaling inhibitors, for example, tyrosine kinase inhibitors; or molecules that target T cell activation molecules, for example, CTLA-4-IgG or anti-B7 family antibodies, anti-PD-1 family antibodies. A compound of formula (I), (I-a), (I-b), or (I-c) may be combined with IL-11 or anti-cytokine antibodies, for example, fonotolizumab (anti-IFNg antibody), or anti-receptor receptor antibodies, for example, anti-IL-6 receptor antibody and antibodies to B-cell surface molecules. A compound of formula (I), (I-a), (I-b), or (I-c) may also be used with LJP 394 (abetimus), agents that deplete or inactivate B-cells, for example, Rituximab (anti-CD20 antibody), lymphostat-B (anti-BlyS antibody), TNF antagonists, for example, anti-TNF antibodies, D2E7 (adalimumab), CA2 (infliximab), CDP 571, TNFR-Ig constructs, (p75TNFRIgG (etanercept) and p55TNFRIgG (LENERCEPT™).

The compounds of the invention can also be co-administered with a therapeutically effective amount of at least one additional therapeutic agents used in the prevention or treatment of AIDS, where examples of the agents include, HIV reverse transcriptase inhibitors, HIV protease inhibitors, immunomodulators, and other retroviral drugs. Examples of reverse transcriptase inhibitors include, but are not limited to, abacavir, adefovir, didanosine, dipivoxil delavirdine, efavirenz, lamivudine, nevirapine, stavudine zalcitabine, and zidovudine. Examples of protease inhibitors include, but are not limited to, amprenavir, indinavir, lopinavir, nelfinavir, ritonavir, and saquinavir.

The following Examples may be used for illustrative purposes and should not be deemed to narrow the scope of the invention.

### f. Examples

All reagents were of commercial grade and were used as received without further purification, unless otherwise stated. Commercially available anhydrous solvents were used for reactions conducted under inert atmosphere. Reagent grade solvents were used in all other cases, unless otherwise specified. Chemical shifts (δ) for ¹H NMR spectra were reported in parts per million (ppm) relative to tetramethylsilane (δ 0.00) or the appropriate residual solvent peak, i.e. CHCl₃ (δ 7.27), as internal reference. Multiplicities were given as singlet (s), doublet (d), triplet (t), quartet (q), quintuplet (quin), multiplet (m) and broad (br).

### Chiral Analytical Supercritical Fluid Chromatography (SFC)

Analytical SFC was performed on an Aurora A5 SFC Fusion and Agilent 1100 system running under Agilent Chemstation software control. The SFC system included a 10-way column switcher, CO₂ pump, modifier pump, oven, and backpressure regulator. The mobile phase comprised of supercritical CO₂ supplied by a beverage-grade CO₂ cylinder with a modifier mixture of methanol at a flow rate of 3 mL/minutes. Oven temperature was at 35 °C and the outlet pressure at 150 bar. The mobile phase gradient started with 5% modifier and held it for 0.1 minutes at a flow rate of 1 mL/min, then the flow rate was ramped up to 3 mL/min and held for 0.4 min. The modifier was ramped from 5% to 50% over the next 8 minutes at 3 mL/min then held for 1 minute at 50% modifier (3 mL/min). The gradient was ramped down from 50% to 5% modifier over 0.5 min (3 mL/min). The instrument was fitted with a Whelk-O1 (S,S) column with dimensions of 4.6 mm i.d. x 150 mm length with 5 µm particles.

### Example 1

### N-(trans-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)acetamide

### Example 1a

### 5-bromo-4-fluoropyridin-2-amine

To a solution of 2-amino-4-fluoropyridine (CAS 944401-77-8, 17.56 g, 157 mmol) in acetonitrile (234 mL) at 5 °C under nitrogen in a foil wrapped round bottom flask was added portionwise N-bromosuccinimide (28.4 g, 160 mmol). Upon completion of the addition the mixture was stirred in darkness warming to ambient temperature over 1 hour. The reaction mixture was concentrated on the rotary evaporator and the residue was washed repeatedly with water (3 x 300 mL) to remove the succinimide. The resulting solid was collected by filtration and dried to constant mass affording the title compound as a light tan powder (23.1 g, 76% yield)

### Example 1b

### 5-bromo-4-fluoropyridin-2-ol

To a solution of sulfuric acid (21.81 mL, 393 mmol) and water (25 mL) at ambient temperature was added portionwise the product from Example 1a (15 g, 79 mmol). The resulting solution was cooled to 5 °C and treated dropwise with a solution of sodium nitrite (17.88 g, 259 mmol) in water (50 mL). The resulting mixture was stirred at 5-10 °C for four hours and treated dropwise with ammonium hydroxide (43.7 mL, 314 mmol) to a constant pH of 9. The mixture was stirred for 20 minutes and the solid was collected by filtration, rinsed with cold water, and dried to a constant mass to afford the title compound as a tan solid (14.5 g, 92% yield).

### Example 1c

### 5-bromo-4-fluoro-1-methylpyridin-2(1H)-one

To a mixture of Example 1b (29 g, 151 mmol) and cesium carbonate (59.1 g, 181 mmol) in dimethylformamide (150 mL) at ambient temperature under nitrogen was added dropwise iodomethane (11.3 mL, 181 mmol), maintaining the temperature below 30 °C. The addition was done over a period of approximately 30 minutes. Upon completion of the addition the mixture was stirred at ambient temperature for 2 hours and then diluted into 800 mL of water. The aqueous mixture was extracted 5 x 150 mL with ethyl acetate. The organics were combined and washed 3 x 50 mL with saturated aqueous sodium chloride, dried over anhydrous sodium sulfate, treated with decolorizing charcoal for 30 minutes at ambient temperature, filtered, and concentrated. The crude solid was triturated with a minimal volume of 9:1 heptane/ethyl acetate and filtered to afford the title compound as an off white powder (17.1 g). The filtrate was concentrated and purified by chromatography (silica gel, 20-50% of 3:1 ethyl acetate/ethanol in heptanes) to afford an additional 8.67 g. Total yield of the title compound was 25.77 g (81% yield).

### Example 1d

### tert-butyl {trans-4-[(5-bromo-1-methyl-2-oxo-1,2-dihydropyridin-4-yl)oxy] cyclohexyl} carbamate

A solution of Example 1c (9.0 g, 43.7 mmol) and *tert*-butyl (*trans-4-*hydroxycyclohexyl)carbamate (9.88 g, 45.9 mmol) in tetrahydrofuran (300 mL) at 0 °C under nitrogen was treated dropwise with potassium *tert*-butoxide,(50.2 mL, 50.2 mmol, 1 M in tetrahydrofuran). The mixture was stirred for 60 minutes under nitrogen at 0-5 °C and then partitioned between ethyl acetate and water. The aqueous layer was extracted once more with ethyl acetate. The organic extracts were combined and washed with saturated aqueous sodium chloride, dried over anhydrous sodium sulfate, filtered, and concentrated to give an off-white solid. Purification of the residue by trituration in a minimal volume of 95:5 heptane/ethyl acetate afforded the title compound as a white powder (17.27 g, 43.0 mmol, 99 % yield).

### Example 1e

### 4-[(trans-4-aminocyclohexyl)oxy]-5-bromo-1-methylpyridin-2(1H)-one hydrochloride

Example 1d (17.27 g, 43.0 mmol) was treated with hydrochloric acid (215 mL, 861 mmol, 4M in dioxane), stirred for three hours at 40 °C and then at ambient temperature overnight. The reaction mixture was diluted with heptane (200 mL) and the resulting solid was collected by vacuum filtration, washed with additional heptane, and dried overnight in a vacuum oven at 50 °C to provide the title compound as the HCl salt (15.54 g, 46.0 mmol, quantitative yield).

### Example 1f

### N-{trans-4-[(5-bromo-1-methyl-2-oxo-1,2-dihydropyridin-4-yl)oxy]cyclohexyl}acetamide

Example 1e (0.359 g, 1.06 mmol) in dichloromethane (10.6 mL) was treated with triethylamine (0.592 mL, 4.25 mmol) and acetic anhydride (0.150 mL, 1.59 mmol). The reaction mixture was stirred at ambient temperature for about 1 hour. The reaction mixture was quenched with water and extracted with ethyl acetate. The organic layer was washed with saturated aqueous sodium chloride, dried over anhydrous magnesium sulfate, filtered, and concentrated. The residue was purified by flash chromatography (silica gel, 50% (3:1 ethyl acetate:ethanol):heptanes to 100% (3:1 ethyl acetate:ethanol)) to provide the title compound as a white solid (0.0663g, 0.193 mmol, 18% yield).

### Example 1g

### 4- fluoro-2,6-dimethylphenol

A 1L three-necked round-bottomed flask equipped with a magnetic stir bar was charged with di-*tert-*butyl(2',4',6'-triisopropyl-[1,1'-biphenyl]-2-yl)phosphine (8.37 g, 19.70 mmol), tris(dibenzylideneacetone)dipalladium (4.51 g, 4.92 mmol) and potassium hydroxide (41.4 g, 739 mmol). The flask was evacuated and backfilled with nitrogen. Separately, dioxane (150 mL), 2-bromo-5-fluoro-1,3 dimethylbenzene (50 g, 246 mmol) and water (150 mL) were flow purged with nitrogen for about 30 minutes and were transferred to the reaction flask via a cannula. The reaction vessel was heated to about 100 °C and stirred overnight. The reaction mixture was cooled to ambient temperature. The reaction mixture was acidified to pH 2 by adding 6N HCl and the product was extracted with dichloromethane (3 x 250 mL). The combined organic layers were stirred with mercaptopropyl silica gel for about 30 minutes, dried over anhydrous magnesium sulfate, filtered, and concentrated to afford the title compound as a white solid. (31.2 g, 223 mmol, 90% yield)

### Example 1h

### methyl 5-bromo-6-(4-fluoro-2,6-dimethylphenoxy)nicotinate

A mixture of methyl 5-bromo-6-chloronicotinate (0.4965 g, 1.982 mmol), Example 1g (0.278 g, 1.98 mmol), and cesium carbonate (0.969 g, 2.97 mmol) in dimethylsulfoxide (DMSO) (6.61 mL) was stirred at 110 °C overnight. The reaction mixture was partitioned between water and ethyl acetate. The aqueous layer was extracted with ethyl acetate. The combined organic layers were washed with saturated aqueous sodium chloride, dried over anhydrous magnesium sulfate, filtered, and concentrated. The residue was purified by flash chromatography (silica gel, 30% (3:1 ethyl acetate:ethanol):heptanes to 100% (3:1 ethyl acetate:ethanol)) to provide the title compound as a colorless oil (0.4038 g, 1.14 mmol, 57% yield).

### Example 1i

### 2-(5-bromo-6-(4-fluoro-2,6-dimethylphenoxy)pyridin-3-yl)propan-2-ol

A solution of Example 1h (0.5706 g, 1.611 mmol) in tetrahydrofuran (8.95 mL) was treated with methylmagnesium bromide (3.0M in ether) (1.611 mL, 4.83 mmol). The reaction mixture was stirred at ambient temperature for 1 hour after complete addition. The reaction mixture was quenched with saturated aqueous ammonium chloride, and the layers were separated. The aqueous layer was extracted with ether. The combined organic layers were washed with water and saturated aqueous sodium chloride, dried over anhydrous magnesium sulfate, filtered, and concentrated. The residue was purified by flash chromatography (silica gel, 100% heptanes to 70% ethyl acetate:heptanes) to provide the title compound as a white solid (0.498 g, 1.41 mmol, 87% yield).

### Example 1j

### 2-(6-(4-fluoro-2,6-dimethylphenoxy)-5-(4,4,5,5-tetramethyl-1,3,2-dioxaborolan-2-yl)pyridin-3-yl)propan-2-ol

In a 250 mL round-bottomed flask fitted with a reflux condenser was added Example 1i (0.4983 g, 1.407 mmol), 4,4,4',4',5,5,5',5'-octamethyl-2,2'-bi(1,3,2-dioxaborolane) (0.714 g, 2.81 mmol), tris(dibenzylideneacetone)dipalladium (0.032 g, 0.035 mmol), 1,3,5,7-tetramethyl-8-phenyl-2,4,6-trioxa-8-phosphaadamantane (0.041 g, 0.141 mmol), and potassium acetate (0.276 g, 2.81 mmol). The solids were sparged with nitrogen for about 1 hour. Degassed 2-methyltetrahydrofuran (11.25 mL) was added. The reaction mixture was heated at 80 °C for about 48 hours. The reaction mixture was cooled to ambient temperature and water was added. The reaction mixture was extracted 2x with ethyl acetate. The combined organic layers were washed with saturated aqueous sodium chloride, dried over anhydrous magnesium sulfate, filtered, and concentrated. The residue was purified by flash chromatography (silica gel, 20% ethyl acetate:heptanes to 100% ethyl acetate) to provide the title compound as a yellow solid (0.499 g, 1.24 mmol, 88% yield).

### Example 1k

### N-(trans-4-{[2'-(4-fluoro-2,6-dimethylphenoxy)-5'-(2-hydroxypropan-2-yl)-1-methyl-6-oxo[1,6-dihydro[3,3'-bipyridine]] -4-yl]oxy} cyclohexyl)acetamide

A mixture of Example 1j (0.052 g, 0.130 mmol), Example 1f (0.0319 g, 0.093 mmol), sodium carbonate (0.034 g, 0.325 mmol), tris(dibenzylideneacetone)dipalladium (4.26 mg, 4.65 µmol), and 1,3,5,7-tetramethyl-8-phenyl-2,4,6-trioxa-8-phosphaadamantane (4.62 mg, 0.016 mmol) was sparged with nitrogen for about 30 minutes. Degassed tetrahydrofuran (0.744 mL) and water (0.186 mL) were added. The reaction mixture was stirred at 50 °C overnight. The reaction mixture was cooled to ambient temperature and partitioned between ethyl acetate and water. The organic layer was washed with saturated aqueous sodium chloride, dried over anhydrous magnesium sulfate, filtered, and concentrated. The residue was purified by flash chromatography (50% (3:1 ethyl acetate:ethanol):heptanes to 100% (3:1 ethyl acetate:ethanol):heptanes) to provide the title compound as a white solid (0.0343 g, 0.064 mmol, 68% yield). ¹H NMR (400 MHz, DMSO-*d*₆) δ 8.01 (d, J = 2.4 Hz, 1H), 7.75 - 7.68 (m, 2H), 7.67 (s, 1H), 6.89 (d, J = 9.2 Hz, 2H), 5.99 (s, 1H), 4.38 (s, 1H), 3.42 (s, 1H), 3.37 (s, 4H), 1.95 (s, 8H), 1.73 (s, 3H), 1.66 (s, 2H), 1.40 (s, 6H), 1.34 - 1.18 (m, 4H). MS (ESI+) m/z 538.0 (M+H)⁺.

### Example 2

### 5-[2-(2,6-dimethylphenoxy)-5-(2-hydroxypropan-2-yl)phenyl]-4-{[1-(methanesulfonyl)piperidin-4-yl]amino}-1-methylpyridin-2(1H)-one

### Example 2a

### 5-bromo-4-chloropyridin-2-amine

A solution of 4-chloropyridin-2-amine (10 g, 78 mmol) in acetonitrile (400 mL) was treated dropwise with a solution of N-bromosuccinimide (14.09 g, 79 mmol) in acetonitrile (200 mL). The mixture was stirred at ambient temperature for 16 hours and concentrated in vacuo. The crude product was purified by chromatography (silica gel, 3:2 hexane / ethyl acetate) to afford the title compound (16 g, 77 mmol, 99 % yield).

### Example 2b

### 5-bromo-4-chloropyridin-2-ol

Example 2a (35 g, 169 mmol) was dissolved in 75 % (v/v) sulfuric acid (700 mL) and then chilled in an ice bath. To this solution was added dropwise a solution of sodium nitrite (38.4 g, 557 mmol) dissolved in water (3.5 L). The mixture was then stirred for 3 hours and treated dropwise with concentrated aqueous ammonia (300 mL, 14.8M). The resulting white precipitate was collected by vacuum filtration, washed with water and dried to constant mass affording the title compound (40.62 g, quantitative yield).

### Example 2c

### 5-bromo-4-chloro-1-methylpyridin-2(1H)-one

A mixture of Example 2b (10 g, 48.0 mmol) and cesium carbonate (19.18 g, 58.9 mmol) in *N*,*N*-dimethylformamide (60 mL) at ambient temperature was treated dropwise with iodomethane (3.54 mL, 56.6 mmol) and stirred at ambient temperature for 1 hour. The mixture was diluted with saturated aqueous sodium chloride (400 mL) and extracted 4 x 200 mL with ethyl acetate. The combined organics were washed with saturated aqueous sodium chloride, dried over anhydrous magnesium sulfate, filtered, and concentrated. The residue was triturated with 10% ethyl acetate in hexanes to afford the title compound (9.6 g, 35.0 mmol, 72.9 % yield).

### Example 2d

### 5-bromo-1-methyl-4-((1-(methylsulfonyl)piperidin-4-yl)amino)pyridin-2(1H)-one

A mixture of Example 2c (1 g, 4.5 mmol), 1-(methylsulfonyl)piperidin-4-amine (1.6 g, 9.0 mmol) and 1,4-dioxane (10 mL) was heated at 100 °C for 3 days. Cesium carbonate (1.465 g, 4.5 mmol) and dimethyl sulfoxide (5 mL) were added and heating was continued for 19 hours at 100 °C. The reaction mixture was concentrated to remove 1,4-dioxane and then additional dimethyl sulfoxide (10 mL) was added. Heating was continued at 120 °C for 6 days. The reaction mixture was partitioned between ethyl acetate and water, and washed with saturated aqueous sodium chloride. The aqueous layers were combined and extracted with ethyl acetate (4 x 50 mL). The organic layers were combined, dried over anhydrous magnesium sulfate, filtered, and concentrated. The residue was purified by flash chromatography (silica gel, 0 to 100% of a 3:1 mixture of ethyl acetate/ethanol in heptanes, then 4% methanol was added to the mixture) to provide the title compound as a mixture with 1-(methylsulfonyl)piperidin-4-amine. The material was further purified by reverse phase HPLC (C18, 5-90% acetonitrile/water (0.1% trifluoroacetic acid)) to provide 0.176 g of pure title compound.

### Example 2e

### methyl 3-bromo-4-(2,6-dimethylphenoxy)benzoate

To a solution of 2,6-dimethylphenol (11.53 g, 94 mmol) and methyl 3-bromo-4-fluorobenzoate (20 g, 86 mmol) in dimethyl sulfoxide(80 mL) was added cesium carbonate (41.9 g, 129 mmol). The mixture was stirred at 80 °C under nitrogen for 2 hours, cooled to ambient temperature, diluted with 200 mL of water, and stirred for 10 minutes. The mixture was transferred to a separatory funnel and extracted 4 x 200 mL with methyl tert-butyl ether. The methyl tert-butyl ether extracts were combined, dried over anhydrous sodium sulfate, filtered, and concentrated. Purification of the residue by chromatography (silica gel, 0-10% ethyl acetate in heptanes) afforded the title compound as an oil that solidified upon standing (25.7 g, 82% yield).

### Example 2f

### 2-(3-bromo-4-(2,6-dimethylphenoxy)phenyl)propan-2-ol

To a solution of Example 2e (25.7 g, 77 mmol) in tetrahydrofuran (383 mL) under nitrogen at 23 °C was added dropwise quickly methylmagnesium bromide (77 mL, 230 mmol) 3.0M in diethyl ether. The mixture was stirred for 1 hour, poured into cold 5% aqueous ammonium chloride and partitioned with 400 mL diethyl ether. The organic layer was washed with saturated aqueous sodium chloride, dried over anhydrous sodium sulfate, filtered, and concentrated. Purification of the residue by chromatography (330 g silica gel, 0-30% ethyl acetate in heptanes) afforded 90% pure product by HPLC. The material was dissolved in 5 mL ethyl acetate/50 mL of hexanes and seeded with a crystal of pure title compound. After 6 hours the precipitates was collected by filtration, rinsed with a minimal volume of cold hexanes, and dried. The mother liquor was concentrated, re-dissolved in a minimal volume of hexanes and seeded again. After filtration and drying, the solids were combined to give 19.8 g (75% yield) of the title compound.

### Example 2g

### 2-(4-(2,6-dimethylphenoxy)-3-(4,4,5,5-tetramethyl-1,3,2-dioxaborolan-2-yl)phenyl)propan-2-ol

Example 2f (10.06 g, 30 mmol), 4,4,4',4',5,5,5',5'-octamethyl-2,2'-bi(1,3,2-dioxaborolane) (15.24 g, 60 mmol), tris(dibenzylideneacetone)dipalladium(0) (0.687 g, 0.75 mmol), 1,3,5,7-tetramethyl-6-phenyl-2,4,8-trioxa-6-phosphaadamantane (0.877 g, 3 mmol) and potassium acetate (5.89 g, 60 mmol) were combined in a 250-mL round bottomed flask and sparged with nitrogen for 30 minutes. Nitrogen-sparged 2-methyl tetrahydrofuran (75 mL) was transferred to the flask. The reaction mixture was stirred at 80 °C for 46 hours, cooled to ambient temperature, and then partitioned between ethyl acetate and water. The organic layer was washed with saturated aqueous sodium chloride, dried over anhydrous sodium sulfate, filtered, and concentrated. The residue was purified by flash chromatography (silica gel, 0-10% ethyl acetate in heptanes) to give 10.23 g (89% yield) of the title compound.

### Example 2h

### 5-[2-(2,6-dimethylphenoxy)-5-(2-hydroxypropan-2-yl)phenyl]-4-{[1-(methanesulfonyl)piperidin-4-yl]amino}-1-methylpyridin-2(1H)-one

Example 2d (0.035 g, 0.096 mmol), Example 2g (0.039 g, 0.101 mmol), tris(dibenzylideneacetone)dipalladium(0) (4.4 mg, 4.8 µmol), 1,3,5,7-tetramethyl-8-phenyl-2,4,6-trioxa-8-phosphaadamantane (4.21 mg, 0.014 mmol) and sodium carbonate (0.044 g, 0.413 mmol) were combined and sparged with nitrogen for 30 minutes. To this mixture were added nitrogen-sparged tetrahydrofuran (1 mL) and water (0.25 mL) via syringe. The reaction mixture was stirred at 60 °C for 5 hours. The reaction mixture was partitioned between ethyl acetate and water. The organic layer was washed with saturated aqueous sodium chloride, treated with 3-mercaptopropyl-functionalized silica gel for 20 minutes, dried over anhydrous magnesium sulfate, filtered through a plug of diatomaceous earth and concentrated. The residue was purified by flash chromatography (silica gel, 25 to 100% of a 3:1 mixture of ethyl acetate/ethanol in heptanes) to provide 0.0239 g (46% yield) of the title compound. ¹H NMR (400 MHz, Pyridine-*d₅*) δ 7.90 (d, J = 2.4 Hz, 1H), 7.68 (dd, J = 8.6, 2.4 Hz, 1H), 7.49 (s, 1H), 7.14 (m, 2H), 6.65 (s, 1H), 6.57 (d, J = 8.6 Hz, 1H), 6.07 (s, 1H), 4.54 (d, J = 7.8 Hz, 1H), 3.76 (d, J = 11.7 Hz, 2H), 3.56 (s, 3H), 3.48 (tdt, J = 11.6, 8.5, 4.5 Hz, 1H), 2.97 (s, 3H), 2.76 (td, J = 12.0, 2.5 Hz, 2H), 2.10 (s, 6H), 1.94 (m, 2H), 1.78 (s, 6H), 1.32 (m, 2H). MS (ESI+) m/z 540.3 (M+H)⁺.

### Example 3

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide

### Example 3a

### methyl 3-bromo-4-(4-fluoro-2,6-dimethylphenoxy)benzoate

A solution of methyl 3-bromo-4-fluorobenzoate (4.22 g, 18.10 mmol) and Example 1g (2.727 g, 19.46 mmol) in dimethyl sulfoxide (18.10 mL) was treated with cesium carbonate (9.28 g, 28.5 mmol). The reaction mixture was heated at about 80 °C for about 2 hours and cooled to ambient temperature. Water (50 mL) was added, and the reaction mixture was stirred for 10 minutes. The reaction mixture was extracted with methyl tert-butyl ether (1x 100 mL, then 2x50 mL). The combined organic layers were dried over anhydrous sodium sulfate, filtered, and concentrated. The residue was purified by flash chromatography (silica gel, 100% heptanes to 30% ethyl acetate:heptanes) to provide the title compound as a white solid (5.29 g, 15.0 mmol, 83% yield).

### Example 3b

### 2-(3-bromo-4-(4-fluoro-2,6-dimethylphenoxy)phenyl)propan-2-ol

A solution of Example 3a (6.25 g, 17.70 mmol) in tetrahydrofuran (100 mL) was treated with methylmagnesium bromide (3.0M in ether) (17.70 mL, 53.1 mmol) in a rapid dropwise manner over 5 minutes. The reaction mixture was stirred for about 1 hour after complete addition. The reaction mixture was quenched with saturated aqueous ammonium chloride, and the layers were separated. The aqueous layer was extracted with ether. The combined organic layers were washed with water and saturated aqueous sodium chloride, dried over anhydrous magnesium sulfate, filtered, and concentrated. The residue was purified by flash chromatography (silica gel, 100% heptanes to 70% ethyl acetate:heptanes) to provide the title compound as a white solid (6.11 g, 17.3 mmol, 98% yield).

### Example 3c

### 2-(4-(4-fluoro-2,6-dimethylphenoxy)-3-(4,4,5,5-tetramethyl-1,3,2-dioxaborolan-2-yl)phenyl)propan-2-ol

Example 3c was prepared according to the procedure used for the preparation of Example 1j, substituting Example 3b for Example 1i, to provide the title compound as a yellow solid (1.53 g, 3.82 mmol, 45% yield).

### Example 3d

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide

Example 3d was prepared according to the procedure used for the preparation of Example 1k, substituting Example 3c for Example 1j, to provide the title compound as a white solid. (0.032 g, 0.060 mmol, 66% yield). ¹H NMR (400 MHz, DMSO-*d*₆) δ 7.71 (d, J = 7.4 Hz, 1H), 7.56 (s, 1H), 7.28 - 7.20 (m, 2H), 6.95 (d, J = 9.1 Hz, 2H), 6.17 (d, J = 8.4 Hz, 1H), 5.95 (s, 1H), 4.89 (s, 1H), 3.43 (s, 1H), 3.36 (s, 3H), 1.98 (s, 6H), 1.93 (s, 2H), 1.73 (s, 3H), 1.66 (s, 2H), 1.37 (s, 6H), 1.26 (p, J = 10.4, 9.9 Hz, 2H). MS (ESI+) m/z 537.0 (M+H)⁺.

### Example 4

### methyl [trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamate

### Example 4a

### methyl{trans-4-[(5-bromo-1-methyl-2-oxo-1,2-dihydropyridin-4-yl)oxy] cyclohexyl} carbamate

A solution of Example 1e (0.3463 g, 1.026 mmol) in dichloromethane (10.26 mL) was treated with triethylamine (0.572 mL, 4.10 mmol) and methyl chloroformate (0.119 mL, 1.538 mmol). The reaction mixture was stirred at ambient temperature for about 4 hours. The reaction mixture was quenched with water and extracted with ethyl acetate. The organic layer was washed with saturated aqueous sodium chloride, dried over anhydrous magnesium sulfate, filtered, and concentrated. The residue was purified by flash chromatography (silica gel, 50% (3:1 ethyl acetate:ethanol):heptanes to 100% (3:1 ethyl acetate:ethanol)) to provide the title compound as a white solid (0.0722 g, 0.201 mmol, 20% yield).

### Example 4b

### methyl [trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamate

Example 4b was prepared according to the procedure used for the preparation of Example 1k, substituting Example 4a for Example If, and Example 3c for Example 1j to provide the title compound as a white solid. (0.039 g, 0.070 mmol, 66% yield). ¹H NMR (400 MHz, DMSO-*d*₆) δ 7.56 (s, 1H), 7.28 - 7.20 (m, 2H), 7.08 (d, J = 7.0 Hz, 1H), 6.95 (d, J = 9.1 Hz, 2H), 6.20 - 6.15 (m, 1H), 5.92 (s, 1H), 4.89 (s, 1H), 4.32 (s, 1H), 3.46 (s, 3H), 3.36 (s, 3H), 3.19 (d, J = 7.8 Hz, 1H), 1.98 (s, 6H), 1.93 (d, J = 10.2 Hz, 2H), 1.68 (d, J = 10.3 Hz, 2H), 1.37 (s, 6H), 1.36 - 1.18 (m, 4H). MS (ESI+) m/z 553.1 (M+H)⁺.

### Example 5

### methyl (trans-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)carbamate

Example 5 was prepared according to the procedure used for the preparation of Example 1k, substituting Example 4a for Example If, to provide the title compound as a white solid. (0.039 g, 0.070 mmol, 71% yield). ¹H NMR (501 MHz, DMSO-*d*₆) δ 8.03 (d, J = 2.5 Hz, 1H), 7.71 (d, J = 2.4 Hz, 1H), 7.69 (s, 1H), 7.11 (d, J = 7.3 Hz, 1H), 6.93 - 6.87 (m, 2H), 5.98 (s, 1H), 5.10 (s, 1H), 4.39 - 4.33 (m, 1H), 3.48 (s, 3H), 3.38 (s, 3H), 3.31 (s, 1H), 1.98 - 1.91 (m, 8H), 1.69 (d, J = 11.0 Hz, 2H), 1.41 (s, 6H), 1.36 - 1.19 (m, 4H). MS (ESI+) m/z 554.1 (M+H)⁺.

### Example 6

### N-{[trans-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}acetamide

### Example 6a

### tert-butyl ({trans-3-[(5-bromo-1-methyl-2-oxo-1,2-dihydropyridin-4-yl)oxy]cyclobutyl}methyl)carbamate

*Tert*-butyl ((*trans*-3-hydroxycyclobutyl)methyl)carbamate (0.588 g, 2.92 mmol) in *N*,*N*-dimethylformamide (11.24 mL) was treated with sodium hydride (0.270 g, 6.74 mmol). The reaction mixture was stirred at ambient temperature for about 30 minutes. Example 2c (0.500 g, 2.248 mmol) was added. The reaction mixture was stirred at 50 °C overnight. The reaction mixture was cooled to ambient temperature, quenched with water and extracted 3 times with ethyl acetate. The combined organic layers were washed 2x with saturated aqueous sodium chloride, dried over anhydrous magnesium sulfate, filtered, and concentrated. The residue was purified by flash chromatography (silica gel, 20% (3:1 ethyl acetate:ethanol):heptanes to 100% (3:1 ethyl acetate:ethanol)) to provide the title compound as a white solid (0.2093 g, 0.540 mmol, 24% yield).

### Example 6b

### 4-{[trans-3-(aminomethyl)cyclobutyl]oxy}-5-bromo-1-methylpyridin-2(1H)-one hydrochloride

Example 6b was prepared according to the procedure used for the preparation of Example 1e, substituting Example 6a for Example 1d, to provide the title compound as a white solid (0.197g, 0.525 mmol, 97% yield).

### Example 6c

### N-({trans-3-[(5-bromo-1-methyl-2-oxo-1,2-dihydropyridin-4-yl)oxy]cyclobutyl}methyl)acetamide

Example 6c was prepared according to the procedure used for the preparation of Example 1f, substituting Example 6b for Example 1e, to provide the title compound as a white solid (0.0446g, 0.135 mmol, 51% yield).

### Example 6d

### N-{[trans-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}acetamide

Example 6d was prepared according to the procedure used for the preparation of Example 1k, substituting Example 6c for Example 1f, and substituting Example 3c for Example 1j, to provide the title compound as a white solid. (0.0418 g, 0.080 mmol, 63% yield). ¹H NMR (501 MHz, DMSO-*d*₆) δ 7.89 (t, J = 5.7 Hz, 1H), 7.58 (s, 1H), 7.30 (d, J = 2.3 Hz, 1H), 7.25 (dd, J = 8.6, 2.4 Hz, 1H), 7.00 - 6.94 (m, 2H), 6.16 (d, J = 8.6 Hz, 1H), 5.65 (s, 1H), 4.93 (s, 1H), 4.78 (p, J = 6.8 Hz, 1H), 3.38 (s, 3H), 3.35 - 3.29 (m, 2H), 3.13 (t, J = 6.4 Hz, 2H), 2.18 (ddd, J = 10.5, 7.2, 3.3 Hz, 1H), 1.99 (s, 8H), 1.79 (s, 3H), 1.40 (s, 6H). MS (ESI+) m/z 523.0 (M+H)⁺.

### Example 7

### methyl {[trans-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate

### Example 7a

### methyl ({trans-3-[(5-bromo-1-methyl-2-oxo-1,2-dihydropyridin-4-yl)oxy]cyclobutyl}methyl)carbamate

Example 7a was prepared according to the procedure used for the preparation of Example 4a, substituting Example 6b for Example 1e, to provide the title compound as a white solid (0.0301g, 0.087 mmol, 34% yield).

### Example 7b

### methyl {[trans-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate

Example 7b was prepared according to the procedure used for the preparation of Example 1k, substituting Example 7a for Example If, and substituting Example 3c for Example 1j to provide the title compound as a white solid. (0.0332 g, 0.062 mmol, 72% yield). ¹H NMR (400 MHz, DMSO-*d*₆) δ 7.56 (s, 1H), 7.29 (d, J = 2.4 Hz, 1H), 7.27 - 7.19 (m, 2H), 6.96 (d, J = 9.1 Hz, 2H), 6.15 (d, J = 8.5 Hz, 1H), 5.63 (s, 1H), 4.91 (s, 1H), 4.75 (p, J = 6.7 Hz, 1H), 3.48 (s, 3H), 3.36 (s, 3H), 3.06 (t, J = 6.5 Hz, 2H), 2.26 - 2.11 (m, 1H), 1.97 (s, 8H), 1.38 (s, 6H). MS (ESI+) m/z 539.1 (M+H)⁺.

### Example 8

### tert-butyl {[cis-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate

### Example 8a

### tert-butyl (((cis)-3-((5-bromo-1-methyl-2-oxo-1,2-dihydropyridin-4-yl)oxy)cyclobutyl)methyl)carbamate

Example 8a was prepared according to the procedure used for the preparation of Example 1d, substituting tert-butyl (((cis)-3-hydroxycyclobutyl)methyl)carbamate for *tert-*butyl (*trans*-4-hydroxycyclohexyl)carbamate, sodium hydride for potassium tert-butoxide and substituting Example 2c for Example 1c, to provide the title compound.

### Example 8b

### tert-butyl {[cis-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate

Example 8b was prepared according to the procedure used for the preparation Example 1k, substituting Example 8a for Example 1f and Example 3c for Example 1j, followed by HPLC purification (C18, 20-50 % acetonitrile in 0.01 N NH₄CO₃/water) to provide to provide the title compound. ¹H NMR (400 MHz, DMSO-*d*₆) δ 7.57 (s, 1H), 7.32 - 7.22 (m, 2H), 6.97 (d, J = 9.1 Hz, 2H), 6.81 (t, J = 5.9 Hz, 1H), 6.15 (d, J = 8.5 Hz, 1H), 5.69 (s, 1H), 4.92 (s, 1H), 4.52 (p, J = 7.3 Hz, 1H), 3.38 (s, 3H), 2.88 (t, J = 6.3 Hz, 2H), 2.39 (m, 2H), 2.00 (s, 6H), 1.96 (m, 1H), 1.56 (d, J = 9.7 Hz, 2H), 1.40 (s, 6H), 1.32 (s, 9H). MS (ESI) m/z 581.0 (M+H)⁺.

### Example 9

### 5-[2-(4-fluoro-2,6-dimethylphenoxy)-5-(2-hydroxypropan-2-yl)phenyl]-4- {[1-(methanesulfonyl)piperidin-4-yl] amino}-1-methylpyridin-2(1H)-one

Example 9 was prepared according to the procedure used for the preparation of Example 2h, substituting Example 3c for Example 2g. The compound was purified by flash chromatography (amine-functionalized silica gel, 0 to 50% of a 3:1 mixture of ethyl acetate/ethanol in heptanes) to provide the title compound. ¹H NMR (400 MHz, Pyridine-*d₅*) δ 7.91 (d, J = 2.4 Hz, 1H), 7.75 (dd, J = 8.6, 2.4 Hz, 1H), 7.49 (s, 1H), 6.94 (d, J = 9.0 Hz, 2H), 6.70 (s, 1H), 6.57 (d, J = 8.6 Hz, 1H), 6.09 (s, 1H), 4.56 (d, J = 7.8 Hz, 1H), 3.78 (d, J = 12.2 Hz, 2H), 3.58 (s, 3H), 3.50 (m, 1H), 2.98 (s, 3H), 2.78 (td, J = 12.0, 2.8 Hz, 2H), 2.06 (s, 6H), 1.92 (m, 1H), 1.80 (s, 6H), 1.32 (m, 2H). MS (ESI+) m/z 558.3 (M+H)⁺.

### Example 10

### N-[6-({5-[2-(2,6-dimethylphenoxy)-5-(2-hydroxypropan-2-yl)phenyl]-1-methyl-2-oxo-1,2-dihydropyridin-4-yl} amino)spiro[3.3]heptan-2-yl]acetamide

### Example 10a

### tert-butyl (6-((5-bromo-1-methyl-2-oxo-1,2-dihydropyridin-4-yl)amino)spiro[3.3]heptan-2-yl)carbamate

A mixture of Example 1c (0.015 g, 0.073 mmol) and *tert*-butyl (6-aminospiro[3.3]heptan-2-yl)carbamate (0.02 g, 0.087 mmol) in dimethyl sulfoxide (0.4 mL) was treated with cesium carbonate (0.047 g, 0.146 mmol) and heated at 50 °C overnight. The reaction mixture was partitioned between ethyl acetate and water, and washed with saturated aqueous sodium chloride. The aqueous layers were combined and extracted with ethyl acetate (2 x 10 mL). The combined organic layers were dried over anhydrous magnesium sulfate, filtered, and concentrated. The residue was purified by flash chromatography (silica gel, 0 to 70% of a 3:1 mixture of ethyl acetate/ethanol in heptanes) to provide 0.0237 g (79% yield) of the title compound.

### Example 10b

### 4-((6-aminospiro[3.3]heptan-2-yl)amino)-5-bromo-1-methylpyridin-2(1H)-one trifluoroacetate salt

Example 10a (0.078 g, 0.189 mmol) was dissolved in dichloromethane (1.5 mL), treated with 2,2,2-trifluoroacetic acid (0.5 mL, 6.49 mmol), and stirred at ambient temperature for 90 minutes. The reaction mixture was concentrated and the title compound was carried forward without purification.

### Example 10c

### N-(6-((5-bromo-1-methyl-2-oxo-1,2-dihydropyridin-4-yl)amino)spiro[3.3]heptan-2-yl)acetamide

Example 10b (0.081 g, 0.189 mmol) was combined with dichloromethane (3 mL), treated sequentially with triethylamine (0.15 mL, 1.076 mmol) and acetyl chloride solution (1 M in dichloromethane) (0.189 mL, 0.189 mmol) and stirred at ambient temperature for 3 hours. The reaction mixture was concentrated to dryness. The residue was purified by flash chromatography (amine-functionalized silica gel, 0 to 55% of a 3:1 mixture of ethyl acetate/ethanol in heptanes) to provide 0.053 g (79% yield) of the title compound.

### Example 10d

### N-[6-({5-[2-(2,6-dimethylphenoxy)-5-(2-hydroxypropan-2-yl)phenyl]-1-methyl-2-oxo-1,2-dihydropyridin-4-yl}amino)spiro[3.3]heptan-2-yl]acetamide

Example 10d was prepared according to the procedure used for the preparation of Example 2h, substituting Example 10c for Example 2d. The compound was purified by flash chromatography (amine-functionalized silica gel, 0 to 70% of a 3:1 mixture of ethyl acetate/ethanol in heptanes). This material was then subjected to a second purification by flash chromatography (amine-functionalized silica gel, 0 to 50% of a 3:1 mixture of ethyl acetate/ethanol in heptanes) to provide the title compound. ¹H NMR (400 MHz, Pyridine-*d₅*) δ 8.63 (d, J = 7.5 Hz, 1H), 7.90 (d, J = 2.5 Hz, 1H), 7.68 (dd, J = 8.6, 2.5 Hz, 1H), 7.45 (s, 1H), 7.16 (m, 3H), 6.62 (s, 1H), 6.55 (d, J = 8.6 Hz, 1H), 5.90 (s, 1H), 5.02 (d, J = 5.6 Hz, 1H), 4.57 (h, J = 8.1 Hz, 1H), 3.83 (q, J = 7.3 Hz, 1H), 3.53 (s, 3H), 2.53 - 2.42 (m, 1H), 2.38 (s, 1H), 2.20 (m, 6H), 2.10 (m, 3H), 2.05 (s, 3H), 1.95 (m, 1H), 1.74 (s, 6H), 1.70 (m, 2H). MS (ESI+) m/z 530.3 (M+H)⁺.

### Example 11

### tert-butyl 3-({5-[2-(2,6-dimethylphenoxy)-5-(2-hydroxypropan-2-yl)phenyl]-1-methyl-2-oxo-1,2-dihydropyridin-4-yl}amino)pyrrolidine-1-carboxylate

### Example 11a

### tert-butyl 3-((5-bromo-1-methyl-2-oxo-1,2-dihydropyridin-4-yl)amino)pyrrolidine-1-carboxylate

To a solution of Example 1c (0.5 g, 2.427 mmol) and *tert*-butyl 3-aminopyrrolidine-1-carboxylate (0.542 g, 2.91 mmol) in dimethylsulfoxide (6 mL) under argon was added cesium carbonate (1.977 g, 6.07 mmol). The mixture was stirred for 18 hours under argon at 80 °C, cooled to ambient temperature, and partitioned between ethyl acetate and water. The organic layer was washed with saturated aqueous sodium chloride, dried over anhydrous sodium sulfate, filtered, and concentrated. Purification of the residue by chromatography (silica gel, 20-65% of 3:1 ethyl acetate/ethanol in heptanes) afforded the title compound as a white foam (0.28 g, 31% yield).

### Example 11b

### tert-butyl 3-({5-[2-(2,6-dimethylphenoxy)-5-(2-hydroxypropan-2-yl)phenyl]-1-methyl-2-oxo-1,2-dihydropyridin-4-yl}amino)pyrrolidine-1-carboxylate

Example 2g (0.045 g, 0.118 mmol), Example 11a (0.04 g, 0.107 mmol), tris(dibenzylideneacetone)dipalladium(0) (2.95 mg, 3.22 µmol), 1,3,5,7-tetramethyl-6-phenyl-2,4,8-trioxa-6-phosphaadamantane (3.14 mg, 10.75 µmol) and sodium carbonate (0.046 g, 0.430 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-7 % methanol in dichloromethane) afforded the title compound as a white powder (0.037 g, 60% yield). ¹H NMR (500 MHz, DMSO-*d*₆) δ 7.42 - 7.26 (m, 3H), 7.18 - 7.03 (m, 3H), 6.23 (d, J = 8.5 Hz, 1H), 5.45 (s, 1H), 4.94 (s, 1H), 4.57 (m, 1H), 4.05 (m, 1H), 3.50 (m, 1H), 3.33 (s, 3H), 3.27 - 3.15 (m, 2H), 3.06 (m, 1H), 2.10 (m, 1H), 2.00 (s, 6H), 1.73 (m, 1H), 1.41 (s, 6H), 1.35 (d, J = 9.1 Hz, 9H). MS (ESI+) m/z 548 (M+H)⁺.

### Example 12

### 5 -[2-(2,6-dimethylphenoxy)-5-(2-hydroxypropan-2-yl)phenyl]-4-{[1-(methanesulfonyl)pyrrolidin-3-yl]amino}-1-methylpyridin-2(1H)-one

### Example 12a

### 5-bromo-1 -methyl-4-((1 -(methylsulfonyl)pyrrolidin-3-yl)amino)pyridin-2(1H)-one

To a solution of Example 1c (0.05 g, 0.243 mmol) and 1-(methylsulfonyl)pyrrolidin-3-amine, hydrochloric acid (0.058 g, 0.291 mmol) in dimethylsulfoxide (1.348 mL) was added cesium carbonate (0.198 g, 0.607 mmol). The mixture was stirred for 18 hours under argon at 80 °C, cooled to ambient temperature, and partitioned between ethyl acetate and water. The organic layer was washed with saturated aqueous sodium chloride, dried over anhydrous sodium sulfate, filtered, and concentrated to afford the title compound that was used without purification (0.04 g, 47% yield).

### Example 12b

### 5 -[2-(2,6-dimethylphenoxy)-5-(2-hydroxypropan-2-yl)phenyl]-4-{[1-(methanesulfonyl)pyrrolidin-3-yl]amino}-1-methylpyridin-2(1H)-one

Example 12a (0.04 g, 0.114 mmol), Example 2g (0.048 g, 0.126 mmol), 1,3,5,7-tetramethyl-6-phenyl-2,4,8-trioxa-6-phosphaadamantane (3.34 mg, 0.011 mmol), tris(dibenzylideneacetone)dipalladium(0) (3.14 mg, 3.43 µmol) and sodium carbonate (0.048 g, 0.457 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-7% methanol in dichloromethane) afforded the title compound as a white powder (0.023 g, 36% yield). ¹H NMR (500 MHz, DMSO-*d*₆) δ 7.45 -7.24 (m, 3H), 7.19 - 7.00 (m, 3H), 6.29 - 6.17 (m, 1H), 5.50 (s, 1H), 4.94 (s, 1H), 4.74 (d, J = 7.0 Hz, 1H), 4.13 (s, 1H), 3.55 (dd, J = 10.2, 6.6 Hz, 1H), 3.33 (s, 3H), 3.24 (t, J = 7.0 Hz, 2H), 3.01 (bs, J = 24.1 Hz, 1H), 2.85 (s, 3H), 2.22 (dq, J = 12.9, 6.5 Hz, 1H), 2.01 (s, 6H), 1.75 (m, 1H), 1.41 (s, 6H). MS (ESI+) m/z 526 (M+H)⁺.

### Example 13

### N-{trans-4-[{5-[2-(4-fluoro-2,6-dimethylphenoxy)-5-(2-hydroxypropan-2-yl)phenyl]-1-methyl-2-oxo-1,2-dihydropyridin-4-yl}(methyl)amino]cyclohexyl}acetamide

### Example 13a

### N-(trans-4-((5-bromo-1-methyl-2-oxo-1,2-dihydropyridin-4-yl)(methyl)amino)cyclohexyl)acetamide

Example 13a was prepared according to the procedure used for the preparation of Example 2d, substituting *N*-(*trans*-4-(methylamino)cyclohexyl)acetamide for 1-(methylsulfonyl)piperidin-4-amine and Example 1c for Example 2c to provide the title compound.

### Example 13b

### N-{trans-4-[{5-[2-(4-fluoro-2,6-dimethylphenoxy)-5-(2-hydroxypropan-2-yl)phenyl]-1-methyl-2-oxo-1,2-dihydropyridin-4-yl}(methyl)amino]cyclohexyl}acetamide

Example 13b was prepared according to the procedure used for the preparation of Example 1k, substituting Example 13a for Example 1f and substituting Example 3c for Example 1j. The crude mixture was purified by HPLC purification (C18, 20-50 % acetonitrile in 0.01 N NH₄CO₃/water) to provide the title compound. ¹H NMR (501 MHz, DMSO-*d*₆) δ 7.54 (d, J = 7.4 Hz, 1H), 7.41 (d, J = 2.4 Hz, 1H), 7.36 (s, 1H), 7.22 (dd, J = 8.6, 2.4 Hz, 1H), 7.00 (d, J = 9.1 Hz, 2H), 6.21 (d, J = 8.6 Hz, 1H), 5.66 (s, 1H), 4.91 (s, 1H), 3.33 (s, 3H), 3.07 (tt, J = 11.4, 3.9 Hz, 1H), 2.55 (m, 1H), 2.53 (s, 3H), 2.04 (s, 6H), 1.69 (s, 3H), 1.61 (d, J = 12.0 Hz, 2H), 1.51 - 1.39 (m, 2H), 1.39 (s, 6H), 1.20 (m, 2H), 0.73 (m, 2H). MS (ESI+) m/z 550.2 (M+H)⁺.

### Example 14

### 4-[(1-acetylpyrrolidin-3-yl)amino]-5-[2-(2,6-dimethylphenoxy)-5-(2-hydroxypropan-2-yl)phenyl]-1-methylpyridin-2(1H)-one

### Example 14a

### 5 -bromo-1-methyl-4-(pyrrolidin-3-ylamino)pyridin-2(1H)-one hydrochloride

Example 11a (0.24 g, 0.645 mmol) was treated with hydrochloric acid (5 mL, 20.00 mmol, 4M in dioxane), stirred for three hours at ambient temperature, and concentrated. The residue was azeotroped twice with toluene and dried to constant mass affording the title compound (0.2 g, quantitative yield).

### Example 14b

### 4-((1-acetylpyrrolidin-3-yl)amino)-5-bromo-1-methylpyridin-2(1H)-one

To a suspension of Example 14a (0.2 g, 0.648 mmol) in dichloromethane (6.48 mL) was added triethylamine (0.361 mL, 2.59 mmol) followed by dropwise addition of acetyl chloride (0.048 mL, 0.680 mmol).The solution was stirred for 2 hours at ambient temperature and partitioned between dichloromethane and a minimal amount of water adjusting the pH to 7-8. The aqueous layer was extracted three more times with dichloromethane. The organics were combined, dried over anhydrous sodium sulfate, filtered, and concentrated to afford the title compound as a sticky foam that was used without purification (0.2 g, 98% yield).

### Example 14c

### 4-[(1-acetylpyrrolidin-3-yl)amino]-5-[2-(2,6-dimethylphenoxy)-5-(2-hydroxypropan-2-yl)phenyl]-1-methylpyridin-2(1H)-one

Example 2g (0.06 g, 0.157 mmol), Example 14b (0.059 g, 0.188 mmol), tris(dibenzylideneacetone)dipalladium(0) (4.31 mg, 4.71 µmol), 1,3,5,7-tetramethyl-6-phenyl-2,4,8-trioxa-6-phosphaadamantane (4.59 mg, 0.016 mmol) and sodium carbonate (0.067 g, 0.628 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-8 % methanol in dichloromethane) afforded the title compound as a white powder (0.046 g, 58% yield). ¹H NMR (400 MHz, DMSO-*d*₆) δ 7.39 - 7.28 (m, 3H), 7.14 - 7.02 (m, 3H), 6.22 (d, J = 8.9 Hz, 1H), 5.47 (d, J = 18.8 Hz, 1H), 4.93 (d, J = 5.4 Hz, 1H), 4.59 (m, 1H), 4.01 (q, J = 7.0 Hz, 1H), 3.60 - 3.34 (m, 2H), 3.32 (s, 3H), 3.26 (m, 1H), 3.15 (ddd, J = 22.5, 11.2,4.9 Hz, 1H), 2.14 (ddq, J = 32.2, 13.1, 6.8 Hz, 1H), 1.98 (s, 6H), 1.86 (s, 3H), 1.40 (s, 6H). MS (ESI-) m/z 488 (M-H)⁺.

### Example 15

### tert-butyl [trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate

### Example 15a

### tert-butyl {trans-4-[(5-bromo-1-methyl-2-oxo-1,2-dihydropyridin-4-yl)oxy]cyclohexyl}methylcarbamate

*Trans*-(4-hydroxy-cyclohexyl)-methyl-carbamic acid tert-butyl ester (0.935 g, 4.08 mmol) in dimethylformamide (19.42 mL) at ambient temperature under nitrogen was treated with sodium hydride (0.349 g, 8.74 mmol, 60% in mineral oil) and stirred for 30 minutes and then treated with Example 1c (0.6 g, 2.91 mmol). The mixture was stirred for 2 hours under nitrogen at 50 °C, cooled to ambient temperature, and partitioned between ethyl acetate and water. The aqueous layer was extracted 3 x 100 mL with ethyl acetate. The organics were combined and washed 3 x 25 mL with saturated aqueous sodium chloride, dried over anhydrous sodium sulfate, filtered, and concentrated. Purification of the residue by trituration in 95/5 heptane/ethyl acetate afforded the title compound as a white powder (1.034 g, 85% yield).

### Example 15b

### tert-butyl [trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate

Example 3c (0.04 g, 0.100 mmol), Example 15a (0.044 g, 0.105 mmol), tris(dibenzylideneacetone)dipalladium(0) (2.75 mg, 3.00 µmol), 1,3,5,7-tetramethyl-6-phenyl-2,4,8-trioxa-6-phosphaadamantane (2.92 mg, 9.99 µmol) and sodium carbonate (0.042 g, 0.400 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 25-75% of 3:1 ethyl acetate/ethanol in heptanes) afforded the title compound as a white powder (0.055 g, 85% yield). ¹H NMR (400 MHz, DMSO-*d*₆) δ 7.57 (s, 1H), 7.31 - 7.22 (m, 2H), 6.96 (d, J = 9.1 Hz, 2H), 6.18 (d, J = 8.3 Hz, 1H), 5.96 (s, 1H), 4.91 (s, 1H), 4.32 (m, 1H), 3.72 (m, 1H), 3.38 (s, 3H), 2.63 (s, 3H), 2.05 (m, 2H), 2.00 (s, 6H), 1.65 (q, J = 12.0 Hz, 2H), 1.53 (s, 2H), 1.39 (s, 6H), 1.36 (s, 9H), 1.23 (q, J = 12.5 Hz, 2H). MS (ESI+) m/z 609 (M+H)⁺.

### Example 16

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N-methylacetamide

### Example 16a

### 5-bromo-1-methyl-4-{[trans-4-(methylamino)cyclohexyl]oxy}pyridin-2(1H)-one hydrochloride

Example 15a (1.0 g, 2.408 mmol) was treated with hydrochloric acid (12.04 mL, 48.2 mmol) 4M in dioxane, stirred for three hours at ambient temperature and concentrated. The residue was azeotroped twice with toluene and dried to constant mass affording the title compound (1.14 g, quantitative yield).

### Example 16b

### N-{trans-4-[(5-bromo-1-methyl-2-oxo-1,2-dihydropyridin-4-yl)oxy]cyclohexyl}-N-methylacetamide

To a solution of Example 16a (0.3 g, 0.853 mmol) and triethylamine (0.357 mL, 2.56 mmol) in dichloromethane (8.53 mL) was added dropwise acetyl chloride (0.079 mL, 1.109 mmol). The mixture was stirred at ambient temperature under nitrogen for 4 hours, diluted with water, and stirred for 10 minutes. The organic layer was separated, dried over anhydrous sodium sulfate, filtered, and concentrated. Purification of the residue by chromatography (silica gel, 20-90% of 3:1ethyl acetate/ethanol in heptanes) afforded the title compound as a white foam (0.185 g, 57% yield).

### Example 16c

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N-methylacetamide

Example 3c (0.04 g, 0.100 mmol), Example 16b (0.036 g, 0.100 mmol), tris(dibenzylideneacetone)dipalladium(0) (2.75 mg, 3.00 µmol), 1,3,5,7-tetramethyl-6-phenyl-2,4,8-trioxa-6-phosphaadamantane (2.92 mg, 9.99 µmol) and sodium carbonate (0.042 g, 0.400 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-7 % methanol in dichloromethane) afforded the title compound as a viscous oil (0.047 g, 80% yield). ¹H NMR (400 MHz, DMSO-*d*₆) δ 7.44 (s, 1H), 7.30 (d, J = 2.4 Hz, 1H), 7.25 (ddd, J = 8.6, 2.5, 0.8 Hz, 1H), 6.87 (d, J = 9.1 Hz, 2H), 6. 22 (d, J = 9.1 Hz, 1H), 5.91 (s, 1H), 5.60 (s, 1H), 4.35 (t, J = 2.7 Hz, 1H), 4.28 (td, J = 10.8, 5.2 Hz, 1H), 3.39 (s, 3H), 2.74 (s, 3H), 2.05 (d, J = 13.9 Hz, 2H), 2.02 (s, 6H), 1.77 - 1.62 (m, 2H), 1.57 (d, J = 12.9 Hz, 2H), 1.88 (s, 3H), 1.43 (s, 6H), 1.38 - 1.26 (m, 2H). MS (ESI-) m/z 549 (M-H)⁺.

### Example 17

### N-[trans-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]acetamide

### Example 17a

### methyl 6-(4-acetyl-2,6-dimethylphenoxy)-5-bromonicotinate

Methyl 5-bromo-6-chloronicotinate (751 mg, 3.00 mmol), 1-(4-hydroxy-3,5-dimethylphenyl)ethanone (493 mg, 3.00 mmol) and cesium carbonate (1.47 g, 4.50 mmol) were combined in dimethyl sulfoxide (3 mL). The reaction mixture was heated at 100 °C for 2 hours, cooled to ambient temperature, and partitioned with ethyl acetate and water. The organic layer was washed with saturated aqueous sodium chloride, dried with anhydrous sodium sulfate, filtered, and concentrated to provide the title compound (980 mg, 86% yield).

### Example 17b

### 2-(4-((3-bromo-5-(2-hydroxypropan-2-yl)pyridin-2-yl)oxy)-3,5-dimethylphenyl)propan-2-ol

To a solution of Example 17a (970 mg, 2.56 mmol) in tetrahydrofuran (15 mL) was added 3M methylmagnesium chloride in tetrahydrofuran (5.13 mL, 15.4 mmol) dropwise at - 78 °C. The reaction mixture was stirred at ambient temperature for 2 hours, 5% aqueous ammonium chloride added, and the mixture partitioned with ethyl acetate and water. The organic layer was washed with saturated aqueous sodium chloride, dried with anhydrous sodium sulfate, filtered, and concentrated. The residue was purified by flash chromatography (silica gel, 20-40% ethyl acetate in heptanes) to provide the title compound (854 mg, 84% yield).

### Example 17c

### 2-(6-(4-(2-hydroxypropan-2-yl)-2,6-dimethylphenoxy)-5-(4,4,5,5-tetramethyl-1,3,2-dioxaborolan-2-yl)pyridin-3-yl)propan-2-ol

Example 17b (733 mg, 1.86 mmol), 4,4,4',4',5,5,5',5'-octamethyl-2,2'-bi(1,3,2-dioxaborolane) (945 mg, 3.72 mmol), tris(dibenzylideneacetone)dipalladium (42.6 mg, 0.0470 mmol), 1,3,5,7-tetramethyl-6-phenyl-2,4,8-trioxa-6-phosphaadamantane (54.4 mg, 0.186 mmol) and potassium acetate (365 mg, 3.72 mmol) were combined in a microwave tube. The reaction mixture was purged with nitrogen for 30 minutes. Degased 2-methyl tetrahydrofuran (8 mL) was transferred to the reaction vessel. The reaction mixture was heated at 80 °C for 46 hours, cooled to ambient temperature, and partitioned with ethyl acetate and water. The organic layer was washed with saturated aqueous sodium chloride, dried with anhydrous sodium sulfate, filtered, and concentrated. The residue was purified by flash chromatography (silica gel, 20-60% ethyl acetate in heptanes) to provide the title compound (450 mg, 55% yield).

### Example 17d

### N-[trans-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]acetamide

Example 1f (27.5 mg, 0.0800 mmol), Example 17c (35.3 mg, 0.0800 mmol), sodium carbonate (29.7 mg, 0.280 mmol), tris(dibenzylideneacetone)dipalladium (2.2 mg, 2.4 µmol) and 1,3,5,7-tetramethyl-6-phenyl-2,4,8-trioxa-6-phosphaadamantane (2.1 mg, 7.2 µmol) were combined in a microwave tube. The reaction mixture was purged with nitrogen for 15 minutes. The mixture of tetrahydrofuran (2.4 mL)/water (0.6 mL) was purged with nitrogen for 15 minutes and transferred to the reaction vessel. The reaction mixture was heated at 60 °C for 3 hours, cooled to ambient temperature, and partitioned with ethyl acetate and water. The organic layer was washed with saturated aqueous sodium chloride, dried with anhydrous sodium sulfate, treated with 3-mercaptopropyl functionalized silica gel, filtered, and concentrated. The residue was purified by flash chromatography (silica gel, 20-80% 3:1 ethyl acetate/ethanol in heptanes) to provide the title compound (35 mg, 76% yield). ¹H NMR (400 MHz, DMSO-*d*₆) δ 8.02 (d, *J* = 2.5 Hz, 1H), 7.77 - 7.66 (m, 3H), 7.12 (s, 2H), 6.01 (s, 1H), 5.09 (s, 1H), 4.89 (s, 1H), 4.44 - 4.35 (m, 1H), 3.47 - 3.38 (m, 4H), 2.01 - 1.92 (m, 8H), 1.75 (s, 3H), 1.73 - 1.64 (m, 2H), 1.42 (s, 6H), 1.39 (s, 6H), 1.34 - 1.20 (m, 4H). (ESI-) m/z 576 (M-H)⁺.

### Example 18

### N-{trans-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl}acetamide

### Example 18a

### methyl 4-(4-acetyl-2,6-dimethylphenoxy)-3-bromobenzoate

To a solution of 3,5-dimethyl-4-hydroxyacetophenone (2.54 g, 15.45 mmol) and methyl 3-bromo-4-fluorobenzoate (3.0 g, 12.87 mmol) in dimethyl sulfoxide (12.87 mL) was added cesium carbonate (6.29 g, 19.31 mmol). The mixture was stirred at 110 °C under nitrogen for 18 hours, cooled to ambient temperature, diluted with 300 mL of water, and stirred for 10 minutes. The mixture was transferred to a separatory funnel and extracted 4 x 100 mL with ethyl acetate. The organic extracts were combined, dried over anhydrous sodium sulfate, filtered, and concentrated. Purification of the residue by chromatography (silica gel, 0-30% ethyl acetate in heptanes) afforded the title compound as light yellow solid (2.8 g, 53% yield).

### Example 18b

### 2-(4-(2-bromo-4-(2-hydroxypropan-2-yl)phenoxy)-3,5-dimethylphenyl)propan-2-ol

To a solution of Example 18a (1.0 g, 2.65 mmol) in tetrahydrofuran (20 mL) at 5 °C under nitrogen was added drop wise 3M methyl magnesium bromide in diethyl ether (5.30 mL, 15.91 mmol). The solution was stirred at ambient temperature for 5 hours, quenched with 5% aqueous ammonium chloride and partitioned with ethyl acetate and water. The organic layer was washed with saturated aqueous sodium chloride, dried over anhydrous sodium sulfate, filtered, and concentrated. The residue was purified by flash chromatography (silica gel, 10-50% ethyl acetate in heptanes) to afford the title compound as a white foam (0.83 g, 76% yield).

### Example 18c

### 2-(4-(4-(2-hydroxypropan-2-yl)-2,6-dimethylphenoxy)-3-(4,4,5,5 -tetramethyl-1,3,2-dioxaborolan-2-yl)phenyl)propan-2-ol

A mixture of Example 18b (0.83 g, 2.110 mmol), 4,4,4',4',5,5,5',5'-octamethyl-2,2'-bi(1,3,2-dioxaborolane) (1.072 g, 4.22 mmol), tris(dibenzylideneacetone)dipalladium(0) (0.048 g, 0.053 mmol) and potassium acetate (0.414 g, 4.22 mmol) were combined in a microwave tube, sealed and sparged with argon for 10 minutes. To this mixture was added argon sparged 2-methyl tetrahydrofuran (8.79 mL) and the mixture was heated at 80 °C for 48 hours, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 0-50% % of ethyl acetate in heptanes) afforded the title compound as a white foam (0.85 g, 69% yield).

### Example 18d

### N-{trans-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy] cyclohexyl} acetamide

Example 1f (0.035 g, 0.102 mmol), Example 18c (0.063 g, 0.143 mmol), tris(dibenzylideneacetone)dipalladium(0) (2.80 mg, 3.06 µmol), 1,3,5,7-tetramethyl-6-phenyl-2,4,8-trioxa-6-phosphaadamantane (2.98 mg, 10.20 µmol) and sodium carbonate (0.043 g, 0.408 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-8% methanol in dichloromethane) afforded the title compound as a white powder (0.046 g, 75% yield). ¹H NMR (400 MHz, DMSO-*d*₆) δ 7.72 (d, J = 7.5 Hz, 1H), 7.56 (s, 1H), 7.27 - 7.20 (m, 2H), 7.18 - 7.10 (m, 2H), 6.15 (d, J = 8.5 Hz, 1H), 5.95 (s, 1H), 4.89 (s, 1H), 4.88 (s, 1H), 4.38 (m, 1H), 3.42 (m, 1H), 3.37 (s, 3H), 1.98 (s, 6H), 1.95 - 1.88 (m, 2H), 1.73 (s, 3H), 1.71 - 1.59 (m, 2H), 1.38 (s, 6H), 1.37 (s, 6H), 1.27 (q, J = 10.5, 8.1 Hz, 4H). MS (ESI-) m/z 575 (M-H)⁺.

### Example 19

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} amino)cyclohexyl]acetamide

### Example 19a

### tert-butyl {trans-4-[(5-bromo-1-methyl-2-oxo-1,2-dihydropyridin-4-yl)amino]cyclohexyl}carbamate

Example 19a was prepared according to the procedure used for the preparation of Example 10a, substituting *tert*-butyl (*trans*-4-aminocyclohexyl)carbamate for *tert-*butyl (6-aminospiro[3.3]heptan-2-yl)carbamate to afford the title compound.

### Example 19b

### 4-[(trans-4-aminocyclohexyl)amino]-5-bromo-1-methylpyridin-2(1H)-one, trifluoroacetate salt

Example 19b was prepared according to the procedure used for the preparation of Example 10b, substituting Example 19a for Example 10a, to provide the title compound.

### Example 19c

### N-{trans-4-[(5-bromo-1-methyl-2-oxo-1,2-dihydropyridin-4-yl)amino]cyclohexyl}acetamide

Example 19c was prepared according to the procedure used for the preparation of Example 10c, substituting Example 19b for Example 10b, to provide the title compound.

### Example 19d

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}amino)cyclohexyl]acetamide

Example 19d was prepared according to the procedure used for the preparation of Example 2h, substituting Example 19c for Example 2d and substituting Example 3c for Example 2g. The crude material was purified by flash chromatography (amine-functionalized silica gel, 0 to 100% of a 3:1 mixture of ethyl acetate/ethanol in heptanes) to provide the title compound. ¹H NMR (501 MHz, DMSO-*d*₆) δ 7.73 (d, J = 7.7 Hz, 1H), 7.35 (m, 2H), 7.31 (d, J = 2.4 Hz, 1H), 7.00 (d, J = 9.1 Hz, 2H), 6.28 (d, J = 8.6 Hz, 1H), 5.43 (s, 1H), 4.97 (s, 1H), 4.17 (d, J = 8.0 Hz, 1H), 3.42 (dddd, J = 15.6, 11.7, 8.3, 4.0 Hz, 1H), 3.32 (s, 3H), 3.26 (m, 1H), 2.02 (s, 6H), 1.91 (br s, 2H), 1.76 (s, 3H), 1.73 (br s, 2H), 1.42 (s, 6H), 1.26 (m, 2H), 1.10 (br s, 2H). MS (ESI+) m/z 536.2 (M+H)⁺.

### Example 20

### methyl {trans-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl}carbamate

Example 4a (0.035 g, 0.097 mmol), Example 18c (0.060 g, 0.136 mmol), tris(dibenzylideneacetone)dipalladium(0) (2.68 mg, 2.92 µmol), 1,3,5,7-tetramethyl-6-phenyl-2,4,8-trioxa-6-phosphaadamantane (2.85 mg, 9.74 µmol) and sodium carbonate (0.041 g, 0.390 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 25-75% of 3:1 ethyl acetate/ethanol in heptanes) afforded the title compound (0.039 g, 64% yield). ¹H NMR (400 MHz, DMSO-*d*₆) δ 7.56 (s, 1H), 7.25 (d, J = 2.3 Hz, 1H), 7.22 (dd, J = 8.5, 2.4 Hz, 1H), 7.16 (t, J = 0.7 Hz, 2H), 7.09 (d, J = 7.4 Hz, 1H), 6.15 (d, J = 8.5 Hz, 1H), 5.92 (s, 1H), 4.90 (s, 1H), 4.88 (s, 1H), 4.30 (m, 1H), 3.46 (s, 3H), 3.36 (s, 3H), 3.19 (m, 1H), 1.97 (s, 6H), 1.95 (s, 2H), 1.69 (s, 2H), 1.38 (s, 6H), 1.37 (s, 6H), 1.28 (p, J = 7.3, 6.3 Hz, 4H).). MS (ESI-) m/z 591 (M-H)⁺.

### Example 21

### methyl [trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate

### Example 21a

### methyl {trans-4-[(5-bromo-1-methyl-2-oxo-1,2-dihydropyridin-4-yl)oxy]cyclohexyl}methylcarbamate

To a solution of Example 16a (0.3 g, 0.853 mmol) and triethylamine (0.357 mL, 2.56 mmol) in dichloromethane (8.53 mL) was added dropwise methyl chloroformate (0.086 mL, 1.109 mmol). The mixture was stirred at ambient temperature under nitrogen for 2 hours, diluted with water, and stirred for 10 minutes. The organic layer was dried over anhydrous sodium sulfate, filtered, and concentrated. Purification of the residue by chromatography (silica gel, 20-90% of 3:1ethyl acetate/ethanol in heptanes) afforded the title compound as a white powder (0.2 g, 62% yield).

### Example 21b

### methyl [trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]methylcarbamate

Example 3c (0.04 g, 0.100 mmol), Example 21a (0.037 g, 0.100 mmol), tris(dibenzylideneacetone)dipalladium(0) (2.75 mg, 3.00 µmol), 1,3,5,7-tetramethyl-6-phenyl-2,4,8-trioxa-6-phosphaadamantane (2.92 mg, 9.99 µmol) and sodium carbonate (0.042 g, 0.400 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 25-75% of 3:1 ethyl acetate/ethanol in heptanes) afforded the title compound as an off white powder (0.049 g, 80% yield). ¹H NMR (400 MHz, DMSO-*d*₆) δ 7.58 (s, 1H), 7.31 - 7.23 (m, 2H), 6.97 (d, J = 9.1 Hz, 2H), 6.19 (d, J = 8.4 Hz, 1H), 5.97 (s, 1H), 4.91 (s, 1H), 4.33 (dt, J = 11.1, 6.9 Hz, 1H), 3.75 (m, 1H), 3.55 (s, 3H), 3.38 (s, 3H), 2.68 (s, 3H), 2.05 (s, 2H), 2.00 (s, 6H), 1.68 (q, J = 12.5 Hz, 2H), 1.53 (d, J = 12.4 Hz, 2H), 1.39 (s, 6H), 1.33 - 1.17 (m, 2H). MS (ESI+) m/z 567 (M+H)⁺.

### Example 22

### N-{[(1R,2S,3S)-3-({5-[2-(4-fluoro-2,6-dimethylphenoxy)-5-(2-hydroxypropan-2-yl)phenyl]-1-methyl-2-oxo-1,2-dihydropyridin-4-yl}oxy)-2-methylcyclopentyl]methyl}acetamide

### Example 22a

### N-(((1R,2S,3S)-3-hydroxy-2-methylcyclopentyl)methyl)acetamide

A 20 mL vial with stirbar was charged with a solution of (1*S*,2*S*,3*R)-*3-(aminomethyl)-2-methylcyclopentanol, hydrochloric acid salt (218 mg, 1.316 mmol), acetic anhydride (0.137 mL, 1.448 mmol) and triethylamine (0.385 mL, 2.76 mmol) in dichloromethane (10 mL). The mixture was stirred at ambient temperature for 16 hours, concentrated and dried in *vacuo.* The crude material was carried forward without purification.

### Example 22b

### N-(((1R,2S,3S)-3-((5-bromo-1-methyl-2-oxo-1,2-dihydropyridin-4-yl)oxy)-2-methylcyclopentyl)methyl)acetamide

Example 22b was prepared according to the procedure used for the preparation of Example 1d, substituting Example 22a for *tert*-butyl (*trans*-4-hydroxycyclohexyl)carbamate and substituting sodium hydride for potassium *tert*-butoxide to provide the title compound.

### Example 22c

### N-{[(1R,2S,3S)-3-({5-[2-(4-fluoro-2,6-dimethylphenoxy)-5-(2-hydroxypropan-2-yl)phenyl]-1-methyl-2-oxo-1,2-dihydropyridin-4-yl}oxy)-2-methylcyclopentyl]methyl}acetamide

Example 22c was prepared according to the procedure used for the preparation of Example 1k, substituting Example 22b for Example 1f and ubstituting Example 3c for Example 1j. The crude material was purified by HPLC purification (C18, 20-50 % acetonitrile in 0.01 N NH₄CO₃/water) to provide the title compound. ¹H NMR (400 MHz, DMSO-*d*₆) δ 7.77 (t, J = 5.9 Hz, 2H), 7.58 (d, J = 3.0 Hz, 2H), 7.36 - 7.21 (m, 5H), 6.96 (d, J = 9.0 Hz, 5H), 6.18 (d, J = 8.4 Hz, 2H), 5.79 (s, 2H), 4.30 - 4.23 (m, 2H), 3.37 (s, 6H), 3.29 (s, 1H), 3.07 (s, 1H), 2.96 (dt, J = 13.2, 5.3 Hz, 2H), 2.82 (dt, J = 13.0, 6.2 Hz, 1H), 2.08 - 1.96 (m, 20H), 1.82 (s, 13H), 1.74 (d, J = 1.9 Hz, 8H), 1.52 (d, J = 8.7 Hz, 1H), 1.49 (s, 4H), 1.45 (dt, J = 9.4, 4.6 Hz, 1H), 1.38 (d, J = 11.0 Hz, 3H), 1.38 (s, 11H), 1.21 (s, 2H), 0.90 (d, J = 5.9 Hz, 6H), 0.54 (d, J = 7.1 Hz, 1H). MS (ESI+) m/z 551.3 (M+H)⁺.

### Example 23

### 5-[2-(4-fluoro-2,6-dimethylphenoxy)-5-(2-hydroxypropan-2-yl)phenyl]-4-[(trans-4-hydroxy-4-methylcyclohexyl)amino]-1-methylpyridin-2(1H)-one

### Example 23a

### methyl (trans-4-hydroxy-4-methylcyclohexyl)carbamate

A 20 mL vial with stirbar was charged with a solution of *trans*-4-amino-1-methylcyclohexanol (181.5 mg, 1.405 mmol), methyl carbonochloridate (0.120 mL, 1.545 mmol) and triethylamine (0.215 mL, 1.545 mmol) in dichloromethane (10 mL). The mixture was stirred at ambient temperature for 16 hours, concentrated, and dried in *vacuo.* The crude material was carried forward without purification.

### Example 23b

### 5-bromo-4-[(trans-4-hydroxy-4-methylcyclohexyl)amino]-1-methylpyridin-2(1H)-one

Example 23b was prepared according to the procedure used for the preparation of Example 1d, substituting Example 23a for *tert*-butyl (*trans*-4-hydroxycyclohexyl)carbamate and substituting sodium hydride for potassium tert-butoxide to provide the title compound.

### Example 23c

### 5-[2-(4-fluoro-2,6-dimethylphenoxy)-5-(2-hydroxypropan-2-yl)phenyl]-4-[(trans-4-hydroxy-4-methylcyclohexyl)amino]-1-methylpyridin-2(1H)-one

Example 23c was prepared according to the procedure used for the preparation of Example 1k, substituting Example 23b for Example 1f and substituting Example 3c for Example 1j. The crude material was purified by HPLC purification (C18, 20-50 % acetonitrile in 0.01 N NH₄CO₃/water) to provide the title compound. ¹H NMR (400 MHz, DMSO-*d*₆) δ 7.36 - 7.27 (m, 3H), 6.96 (d, J = 9.1 Hz, 2H), 6.26 (d, J = 8.5 Hz, 1H), 5.36 (s, 1H), 3.36 (q, J = 4.8 Hz, 1H), 3.28 (s, 3H), 1.98 (s, 6H), 1.79 (m, 6H), 1.37 (s, 6H), 1.34 (m, 2H), 0.98 (s, 3H). MS (ESI+) m/z 509.3(M+H)⁺.

### Example 24

### 5-[2-(4-fluoro-2,6-dimethylphenoxy)-5-(2-hydroxypropan-2-yl)phenyl]-1-methyl-4{(trans-4-(2-oxopyrrolidin-1-yl)cyclohexyl]oxy}pyridin-2(1H)-one

### Example 24a

### 5-bromo-1-methyl-4-{[trans-4-(2-oxopyrrolidin-1-yl)cyclohexyl]oxy}pyridin-2(1H)-one

To a solution of Example 1e (0.2 g, 0.592 mmol) and triethylamine (0.248 mL, 1.777 mmol) in dichloromethane (5.92 mL) was added dropwise 4-chlorobutyryl chloride (0.092 g, 0.652 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (5.92 mL), treated with sodium hydride (0.090 g, 3.55 mmol) and heated at 60 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by chromatography (silica gel, 1-8% methanol in dichloromethane) afforded the title compound as a white solid (0.17 g, 76% yield).

### Example 24b

### 5-[2-(4-fluoro-2,6-dimethylphenoxy)-5-(2-hydroxypropan-2-yl)phenyl]-1-methyl-4-{[trans-4-(2-oxopyrrolidin-1-yl)cyclohexyl]oxy}pyridin-2(1H)-one

Example 3c (0.04 g, 0.100 mmol), Example 24a (0.041 g, 0.110 mmol), tris(dibenzylideneacetone)dipalladium(0) (2.75 mg, 3.00 µmol), 1,3,5,7-tetramethyl-6-phenyl-2,4,8-trioxa-6-phosphaadamantane (2.92 mg, 9.99 µmol) and sodium carbonate (0.042 g, 0.400 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-5% methanol in dichloromethane) afforded the title compound as a foam (0.04 g, 67% yield). ¹H NMR (400 MHz, DMSO-*d*₆) δ 7.58 (s, 1H), 7.31 - 7.19 (m, 2H), 6.97 (d, J = 9.1 Hz, 1H), 6.19 (d, J = 8.4 Hz, 1H), 5.96 (s, 1H), 5.73 (s, 1H), 4.91 (s, 1H), 4.33 (m, 1H), 3.67 (m, 1H), 3.38 (s, 3H), 3.25 (t, J = 7.0 Hz, 2H), 2.17 (t, J = 8.0 Hz, 2H), 2.05 (m, 2H), 2.00 (s, 6H), 1.92 - 1.78 (m, 2H), 1.60 (m, 4H), 1.39 (s, 6H), 1.25 (q, J = 11.2, 10.3 Hz, 2H). MS (ESI-) m/z 561 (M-H)⁺.

### Example 25

### methyl[trans-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]carbamate

Example 25 was prepared according to the procedure used for the preparation of Example 17d, substituting Example 4a for Example If, to provide the title compound (31 mg, 65% yield). ¹H NMR (400 MHz, DMSO-*d*₆) δ 8.00 (d, *J* = 2.5 Hz, 1H), 7.68 (d, *J* = 2.5 Hz, 1H), 7.66 (s, 1H), 7.13 - 7.06 (m, 3H), 5.96 (s, 1H), 5.08 (s, 1H), 4.87 (s, 1H), 4.40 - 4.31 (m, 1H), 3.46 (s, 3H), 3.37 (s, 3H), 3.24 - 3.17 (m, 1H), 2.00 - 1.92 (m, 8H), 1.74 - 1.65 (m, 2H), 1.40 (s, 6H), 1.38 (s, 6H), 1.35 - 1.21 (m, 4H). (ESI-) m/z 592 (M-H)⁺.

### Example 26

### 2'-(4-fluoro-2,6-dimethylphenoxy)-5'-(2-hydroxypropan-2-yl)-4-{[1-(methoxyacetyl)piperidin-4-yl]oxy}-1-methyl[3,3'-bipyridin]-6(1H)-one

### Example 26a

### tert-butyl 4-((5-bromo-1 -methyl-2-oxo-1,2-dihydropyridin-4-yl)oxy)piperidine-1 -carboxylate

Example 26a was prepared according to the procedure used for the preparation of Example 6a, substituting *tert*-butyl 4-hydroxypiperidine-1-carboxylate for tert-butyl ((*trans-*3-hydroxycyclobutyl)methyl)carbamate, to provide the title compound as a white solid (0.830 g, 2.14 mmol, 48% yield).

### Example 26b

### 5-bromo-1-methyl-4-(piperidin-4-yloxy)pyridin-2(1H)-one hydrochloride

Example 26b was prepared according to the procedure used for the preparation of Example 1e, substituting Example 26a for Example 1d, to provide the title compound as a white solid (0.3563 g, 1.10 mmol, 100% yield).

### Example 26c

### 5-bromo-4-((1-(2-methoxyacetyl)piperidin-4-yl)oxy)-1-methylpyridin-2(1H)-one

Example 26b (0.3563 g, 1.101 mmol) in tetrahydrofuran (11.01 mL) was treated with triethylamine (0.460 mL, 3.30 mmol) and 2-methoxyacetyl chloride (0.121 mL, 1.321 mmol). The reaction mixture was stirred at ambient temperature for about 1 hour. The reaction mixture was quenched with water and extracted with ethyl acetate. The organic layer was washed with saturated aqueous sodium chloride, dried over anhydrous magnesium sulfate, filtered, and concentrated to provide the title compound as a light yellow solid (0.2344 g, 0.653 mmol, 59% yield).

### Example 26d

### 2'-(4-fluoro-2,6-dimethylphenoxy)-5'-(2-hydroxypropan-2-yl)-4-{[1-(methoxyacetyl)piperidin-4-yl]oxy}-1-methyl[3,3'-bipyridin]-6(1H)-one

Example 26d was prepared according to the procedure used for the preparation of Example 1k, substituting Example 26c for Example 1f to provide the title compound as a white solid. (0.0332 g, 0.062 mmol, 72% yield). ¹H NMR (400 MHz, DMSO-*d*₆) δ 8.02 (d, J = 2.5 Hz, 1H), 7.73 (d, J = 2.4 Hz, 1H), 7.71 (s, 1H), 6.89 (d, J = 9.2 Hz, 2H), 6.04 (s, 1H), 5.08 (s, 1H), 4.71 (dq, J = 7.2, 3.4 Hz, 1H), 3.98 (s, 2H), 3.38 (s, 3H), 3.37 - 3.30 (m, 2H), 3.27 - 3.22 (m, 2H), 3.20 (s, 4H), 1.93 (s, 6H), 1.81 (s, 2H), 1.51 - 1.41 (m, 2H), 1.38 (s, 6H). MS (ESI-) m/z 551.9 (M-H)⁺.

### Example 27

### 5-[2-(2,6-dimethylphenoxy)-5-(2-hydroxypropan-2-yl)phenyl]-1-methyl-4-{[trans-4-(2-oxopyrrolidin-1-yl)cyclohexyl]oxy}pyridin-2(1H)-one

Example 2g (0.057 g, 0.149 mmol), Example 24a (0.05 g, 0.135 mmol), tris(dibenzylideneacetone)dipalladium(0) (3.72 mg, 4.06 µmol), 1,3,5,7-tetramethyl-6-phenyl-2,4,8-trioxa-6-phosphaadamantane (3.96 mg, 0.014 mmol) and sodium carbonate (0.057 g, 0.542 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-6% methanol in dichloromethane) afforded the title compound as a foam (0.05 g, 64% yield). ¹H NMR (400 MHz, DMSO-*d*₆) δ 7.58 (s, 1H), 7.27 (d, J = 2.4 Hz, 1H), 7.24 (dd, J = 8.5, 2.4 Hz, 1H), 7.10 (d, J = 7.4 Hz, 2H), 7.04 (dd, J = 8.5, 6.3 Hz, 1H), 6.16 (d, J = 8.5 Hz, 1H), 5.97 (s, 1H), 4.90 (s, 1H), 4.40 - 4.26 (m, 1H), 3.74-3.62 (m, 1H), 3.39 (s, 3H), 3.25 (t, J = 7.0 Hz, 2H), 2.17 (t, J = 8.1 Hz, 2H), 2.09 - 2.02 (m, 2H), 2.01 (s, 6H), 1.88 (q, J = 7.5 Hz, 2H), 1.60 (m, 4H), 1.39 (s, 6H), 1.27 (q, J = 10.7, 10.0 Hz, 2H). MS (ESI-) m/z 543 (M-H)⁺.

### Example 28

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]prop-2-enamide

### Example 28a

### N-{trans-4-[(5-bromo-1-methyl-2-oxo-1,2-dihydropyridin-4-yl)oxy]cyclohexyl}prop-2-enamide

To a solution of Example 1e (0.25 g, 0.740 mmol) and triethylamine (0.310 mL, 2.221 mmol) in dichloromethane (7.40 mL) was added dropwise 3-chloropropionyl chloride (0.099 g, 0.777 mmol). The mixture was stirred at ambient temperature under nitrogen for 2 hours and partitioned with water. The organic layer was dried over anhydrous sodium sulfate, filtered, and concentrated. Purification of the residue by chromatography (silica gel, 1-5% methanol in dichloromethane) afforded the title compound as an off-white powder (0.18 g, 64% yield).

### Example28b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]prop-2-enamide

Example 3c (0.050 g, 0.124 mmol), Example 28a (0.04 g, 0.113 mmol), tris(dibenzylideneacetone)dipalladium(0) (3.09 mg, 3.38 µmol), 1,3,5,7-tetramethyl-6-phenyl-2,4,8-trioxa-6-phosphaadamantane (3.29 mg, 0.011 mmol) and sodium carbonate (0.048 g, 0.450 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-8% methanol in dichloromethane) afforded the title compound as a white powder (0.027 g, 40% yield). ¹H NMR (400 MHz, DMSO-*d*₆) δ 7.98 (d, J = 7.5 Hz, 1H), 7.59 (s, 1H), 7.31 - 7.23 (m, 2H), 6.97 (d, J = 9.1 Hz, 2H), 6.24 - 6.17 (m, 2H), 6.03 (dd, J = 17.1, 2.3 Hz, 1H), 5.99 (s, 1H), 5.53 (dd, J = 10.1, 2.3 Hz, 1H), 4.91 (s, 1H), 4.45 - 4.36 (m, 1H), 3.55 (m, 1H), 3.38 (s, 3H), 2.01 (s, 6H), 1.96 (m, 2H), 1.73 (m, 2H), 1.39 (s, 6H), 1.36 - 1.23 (m, 4H). MS (ESI-) m/z 547 (M-H)⁺.

### Example 29

### 5-[2-(4-fluoro-2,6-dimethylphenoxy)-5-(2-hydroxypropan-2-yl)phenyl]-1-methyl-4-{[trans-4-(2-oxopiperidin-1-yl)cyclohexyl]oxy}pyridin-2(1H)-one

### Example 29a

### 5-bromo-1-methyl-4-{[trans-4-(2-oxopiperidin-1-yl)cyclohexyl]oxy}pyridin-2(1H)-one

To a solution of Example 1e (0.26 g, 0.770 mmol) and triethylamine (0.268 mL, 1.925 mmol) in dichloromethane (7.70 mL) was added dropwise 5-chlorovaleryl chloride (0.125 g, 0.809 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (7.70 mL), treated with sodium hydride (0.117 g, 4.62 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by chromatography (silica gel, 1.5-6% methanol in dichloromethane) afforded the title compound as a white solid (0.2 g, 68% yield).

### Example29b

### 5-[2-(4-fluoro-2,6-dimethylphenoxy)-5-(2-hydroxypropan-2-yl)phenyl]-1-methyl-4-{[trans-4-(2-oxopiperidin-1-yl)cyclohexyl]oxy}pyridin-2(1H)-one

Example 3c (0.063 g, 0.157 mmol), Example 29a (0.05 g, 0.130 mmol), tris(dibenzylideneacetone)dipalladium(0) (3.58 mg, 3.91 µmol), 1,3,5,7-tetramethyl-6-phenyl-2,4,8-trioxa-6-phosphaadamantane (3.81 mg, 0.013 mmol) and sodium carbonate (0.055 g, 0.522 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-6% methanol in dichloromethane) afforded the title compound as a foam (0.06 g, 678% yield). ¹H NMR (400 MHz, DMSO-*d*₆) δ 7.56 (s, 1H), 7.28 7.20 (m, 2H), 6.95 (d, J = 9.1 Hz, 2H), 6.17 (d, J = 8.4 Hz, 1H), 5.94 (s, 1H), 4.89 (s, 1H), 4.31 (dq, J = 10.9, 6.4, 5.2 Hz, 1H), 4.20 (ddt, J = 12.0, 7.6, 3.8 Hz, 1H), 3.37 (s, 3H), 3.10 (t, J = 5.6 Hz, 2H), 2.16 (t, J = 6.4 Hz, 2H), 2.02 (d, J = 10.9 Hz, 1H), 1.99 (s, 5H), 1.74 1.54 (m, 6H), 1.52 1.42 (m, 2H), 1.37 (s, 5H), 1.31 1.17 (m, 2H). MS (ESI-) m/z 575 (M-H)⁺.

### Example 30

### 4-{[trans-4-(3,3-dimethyl-2-oxoazetidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

### Example 30a

### 5-bromo-4-{[trans-4-(3,3-dimethyl-2-oxoazetidin-1-yl)cyclohexyl]oxy}-1-methylpyridin-2(1H)-one

To a solution of Example 1e (0.25 g, 0.740 mmol) and triethylamine (0.258 mL, 1.851 mmol) in dichloromethane (7.40 mL) was added dropwise 3-chloropivaloyl chloride (0.115 g, 0.740 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (7.40 mL), treated with sodium hydride (0.112 g, 4.44 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by chromatography (silica gel, 2-7% methanol in dichloromethane) afforded the title compound as a white solid (0.14 g, 48% yield).

### Example30b

### 4-{[trans-4-(3,3-dimethyl-2-oxoazetidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

Example 3c (0.063 g, 0.157 mmol), Example 30a (0.05 g, 0.130 mmol), tris(dibenzylideneacetone)dipalladium(0) (3.58 mg, 3.91 µmol), 1,3,5,7-tetramethyl-6-phenyl-2,4,8-trioxa-6-phosphaadamantane (3.81 mg, 0.013 mmol) and sodium carbonate (0.055 g, 0.522 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-6% methanol in dichloromethane) afforded the title compound as a white powder (0.053 g, 68% yield). ¹H NMR (400 MHz, DMSO-*d*₆) δ 7.57 (s, 1H), 7.24 (d, J = 12.1 Hz, 2H), 6.95 (d, J = 9.0 Hz, 2H), 6.17 (d, J = 8.4 Hz, 1H), 5.93 (s, 1H), 4.89 (s, 1H), 4.36 (s, 1H), 3.36 (s, 3H), 2.95 (s, 2H), 1.98 (s, 6H), 1.93 (m, 2H), 1.69 (d, J = 12.6 Hz, 2H), 1.53 (q, J = 12.2 Hz, 2H), 1.37 (s, 6H), 1.25 (m, 4H), 1.11 (s, 6H). MS (ESI+) m/z 577 (M+H)⁺.

### Example 31

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydropyrimidin-2(1H)-one

### Example 31a

### 1-{trans-4-[(5-bromo-1-methyl-2-oxo-1,2-dihydropyridin-4-yl)oxy]cyclohexyl}tetrahydropyrimidin-2(1H)-one

To a solution of Example 1e (0.3 g, 0.889 mmol) and triethylamine (0.310 mL, 2.221 mmol) in dichloromethane (8.89 mL) was added dropwise 3-chloropropyl isocyanate (0.106 g, 0.889 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (8.89 mL), treated with sodium hydride (0.135 g, 5.33 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated to afford the title compound (0.015 g, 42% yield).

### Example 31b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydropyrimidin-2(1H)-one

Example 3c (0.058 g, 0.144 mmol), Example 31a (0.046 g, 0.120 mmol), tris(dibenzylideneacetone)dipalladium(0) (3.29 mg, 3.59 µmol), 1,3,5,7-tetramethyl-6-phenyl-2,4,8-trioxa-6-phosphaadamantane (3.50 mg, 0.012 mmol) and sodium carbonate (0.051 g, 0.479 mmol) were combined and sparged with argon for 15 minutes. Meanwhile a solution of 4:1 terahydrofuran/water (1.2 mL) was sparged with nitrogen for 15 minutes and transferred by syringe into the reaction vessel under argon. The mixture was stirred for 6 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-8% methanol in dichloromethane) followed by trituration in a minimal volume of 9:1 heptane/ethyl acetate afforded the title compound as a white powder (0.042 g, 58% yield). ¹H NMR (400 MHz, DMSO-*d*₆) δ 7.58 (s, 1H), 7.31 - 7.23 (m, 2H), 6.97 (d, J = 9.1 Hz, 2H), 6.18 (d, J = 8.3 Hz, 1H), 6.10 (d, J = 2.5 Hz, 1H), 5.95 (s, 1H), 4.91 (s, 1H), 4.30 (s, 1H), 4.08 - 4.00 (m, 1H), 3.38 (s, 3H), 3.11 - 2.97 (m, 4H), 2.02 (m, 2H), 2.00 (s, 6H), 1.71 (p, J = 5.9 Hz, 2H), 1.67 - 1.57 (m, 2H), 1.46 (d, J = 12.1 Hz, 2H), 1.39 (s, 6H), 1.24 (t, J = 12.2 Hz, 2H). MS (ESI-) m/z 576 (M-H)⁺.

### Example 32

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-1-methylcyclohexyl]acetamide

### Example 32a

### tert-butyl {trans-4-[(5-bromo-1-methyl-2-oxo-1,2-dihydropyridin-4-yl)oxy]-1-methylcyclohexyl} carbamate

A solution of tert-butyl (trans-4-hydroxy-1-methylcyclohexyl)carbamate (1.002 g, 4.37 mmol) and Example 1c (0.9 g, 4.37 mmol) in tetrahydrofuran (29.1 mL) at 0 °C under nitrogen was treated dropwise with 1M potassium *tert*-butoxide in tetrahydrofuran (5.02 mL, 5.02 mmol). The mixture was stirred for 60 minutes under nitrogen at 0-5 °C and then partitioned between ethyl acetate and water. The aqueous layer was extracted once more with ethyl acetate. The organic extracts were combined and washed with saturated aqueous sodium chloride, dried over anhydrous sodium sulfate, filtered, and concentrated to give an off-white solid (1.76 g, 94% yield) that was used without purification.

### Example 32b

### 4-[(trans-4-amino-4-methylcyclohexyl)oxy] -5 -bromo-1 -methylpyridin-2(1H)-one hydrochloride

Example 32a (1.76 g, 4.24 mmol) was treated with 4M hydrochloric acid (21.19 mL, 85 mmol) in dioxane, stirred for three hours at 35 °C and concentrated. The residue was azeotroped twice with toluene and dried to constant mass affording the title compound (1.7 g, 107% yield).

### Example 32c

### N- {trans-4-[(5-bromo-1-methyl-2-oxo-1,2-dihydropyridin-4-yl)oxy]-1-methylcyclohexyl} acetamide

To a solution of Example 32b (0.1 g, 0.284 mmol) and triethylamine (0.119 mL, 0.853 mmol) in dichloromethane (3.79 mL) was added dropwise acetyl chloride (0.025 g, 0.313 mmol). The mixture was stirred at ambient temperature under nitrogen for 2 hours and partitioned with water. The organic layer was dried over anhydrous sodium sulfate, filtered, and concentrated. Purification of the residue by trituration in 9:1 heptanes/ethyl acetate afforded the title compound as a white solid (0.065 g, 58% yield).

### Example 32d

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)-1-methylcyclohexyl]acetamide

Example 3c (0.087 g, 0.218 mmol), Example 32c (0.065 g, 0.182 mmol), tris(dibenzylideneacetone)dipalladium(0) (5.00 mg, 5.46 µmol), 1,3,5,7-tetramethyl-6-phenyl-2,4,8-trioxa-6-phosphaadamantane (5.32 mg, 0.018 mmol) and sodium carbonate (0.077 g, 0.728 mmol) were combined and sparged with argon for 15 minutes. Meanwhile a solution of 4:1 tetrahydrofuran/water (1.2 mL) was sparged with nitrogen for 15 minutes and transferred by syringe into the reaction vessel under argon. The mixture was stirred for 6 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated to afford the title compound as a white powder (0.067 g, 64% yield). ¹H NMR (400 MHz, DMSO-*d*₆) δ 7.55 (s, 1H), 7.24 (dq, J = 4.4, 2.4 Hz, 2H), 7.16 (s, 1H), 6.95 (d, J = 9.1 Hz, 2H), 6.16 (d, J = 9.2 Hz, 1H), 5.89 (s, 1H), 4.89 (s, 1H), 4.29 (dt, J = 9.9, 5.5 Hz, 1H), 3.36 (s, 3H), 2.08 (d, J = 12.7 Hz, 2H), 1.99 (s, 6H), 1.73 (s, 3H), 1.72 - 1.62 (m, 2H), 1.42-1.1.23 (m, 4H), 1.37 s, 6H), 1.17 (s, 3H). MS (ESI-) m/z 549 (M-H)⁺.

### Example 33

### 6-ethyl-5-[2-(4-fluoro-2,6-dimethylphenoxy)-5-(2-hydroxypropan-2-yl)phenyl]-1-methyl-4-{[trans-4-(2-oxopyrrolidin-1-yl)cyclohexyl] oxy} pyridin-2(1H)-one

### Example 33a

### 5-bromo-6-ethyl-1-methyl-4-{[trans-4-(2-oxopyrrolidin-1-yl)cyclohexyl]oxy}pyridin-2(1H)-one

To a solution of Example 24a (0.32 g, 0.867 mmol) in tetrahydrofuran (8.67 mL) at - 78 °C under nitrogen was added dropwise 1.0 M lithium bis(trimethylsilyl)amide (1.907 mL, 1.907 mmol). The mixture was stirred at -78 °C under nitrogen for 2 hours and treated with iodomethane (0.163 mL, 2.60 mmol). The reaction mixture was allowed to warm to ambient temperature, stirred for 1 hour and quenched with saturated ammonium chloride / ethyl acetate. The organic layer was dried over anhydrous sodium sulfate, filtered, and concentrated to afford a complex mixture. Purification of the residue by chromatography (reverse phase C18, 10-90% acetonitrile in water) afforded the title compound (0.018 g, 5% yield).

### Example 33b

### 6-ethyl-5-[2-(4-fluoro-2,6-dimethylphenoxy)-5-(2-hydroxypropan-2-yl)phenyl]-1-methyl-4-{[trans-4-(2-oxopyrrolidin-1-yl)cyclohexyl] oxy} pyridin-2(1H)-one

Example 3c (0.021 g, 0.051 mmol), Example 33a (0.017 g, 0.043 mmol), tris(dibenzylideneacetone)dipalladium(0) (1.175 mg, 1.284 µmol), 1,3,5,7-tetramethyl-6-phenyl-2,4,8-trioxa-6-phosphaadamantane (1.251 mg, 4.28 µmol) and sodium carbonate (0.018 g, 0.171 mmol) were combined and sparged with argon for 15 minutes. Meanwhile a solution of 4:1 tetrahydrofuran/water (0.5 mL) was sparged with nitrogen for 15 minutes and transferred by syringe into the reaction vessel under argon. The mixture was stirred for 18 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1.5-7% methanol in dichloromethane) afforded the title compound as a white powder (0.0106 g, 40% yield). ¹H NMR (400 MHz, DMSO-*d*₆) δ 7.26 (dd, J = 8.6, 2.4 Hz, 1H), 7.14 (d, J = 2.4 Hz, 1H), 6.95 (d, J = 9.1 Hz, 2H), 6.15 (d, J = 8.6 Hz, 1H), 5.88 (s, 1H), 4.88 (s, 1H), 4.23 (td, J = 10.6, 5.2 Hz, 1H), 3.60 (tt, J = 11.6, 4.1 Hz, 1H), 3.43 (s, 3H), 3.22 (t, J = 7.0 Hz, 2H), 2.57 (dt, J = 14.4, 7.2 Hz, 1H), 2.40 (dt, J = 15.0, 7.5 Hz, 1H), 2.14 (dd, J = 8.6, 7.5 Hz, 2H), 2.10-1.92 (m, 2H), 1.96 (s, 6H), 1.88 - 1.78 (m, 2H), 1.69 - 1.41 (m, 4H), 1.36 (d, J = 3.1 Hz, 6H), 1.14 - 1.04 (m, 2H), 1.01 (t, J = 7.4 Hz, 3H). ). MS (ESI+) m/z 591 (M+H)⁺.

### Example 34

### 5-[2-(4-fluoro-2,6-dimethylphenoxy)-5-(2-hydroxypropan-2-yl)phenyl] -1-methyl-4-{ [trans-4-methyl-4-(2-oxopyrrolidin- 1 -yl)cyclohexyl] oxy} pyridin-2(1H)-one

### Example 34a

### 5-bromo-l-methyl-4-{[trans-4-methyl-4-(2-oxopyrrolidin-1-yl)cyclohexyl]oxy}pyridin-2(1H)-one

To a solution of Example 32b (0.2 g, 0.569 mmol) and triethylamine (0.238 mL, 1.706 mmol) in dichloromethane (5.69 mL) was added dropwise 4-chlorobutyryl chloride (0.088 g, 0.626 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (5.69 mL), treated with sodium hydride (0.086 g, 3.41 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by trituration in 9:1 heptane/ethyl acetate afforded the title compound as a white solid (0.14 g, 58% yield).

### Example 34b

### 5-[2-(4-fluoro-2,6-dimethylphenoxy)-5-(2-hydroxypropan-2-yl)phenyl] -1-methyl-4- {[trans-4-methyl-4-(2-oxopyrrolidin-1 -yl)cyclohexyl] oxy}pyridin-2(1H)-one

Example 3c (0.075 g, 0.188 mmol), Example 34a (0.06 g, 0.157 mmol), tris(dibenzylideneacetone)dipalladium(0) (4.30 mg, 4.70 µmol), 1,3,5,7-tetramethyl-6-phenyl-2,4,8-trioxa-6-phosphaadamantane (4.58 mg, 0.016 mmol) and sodium carbonate (0.066 g, 0.626 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (1.5-7% methanol in dichloromethane) afforded the title compound as a white powder (0.058 g, 62% yield). ¹H NMR (400 MHz, DMSO-*d*₆) δ 7.58 (s, 1H), 7.26 (s, 2H), 6.98 (d, J = 9.1 Hz, 2H), 6.18 (d, J = 8.3 Hz, 1H), 5.94 (s, 1H), 4.91 (s, 1H), 4.40 (m, 1H), 3.38 (s, 3H), 3.18 (t, J = 7.0 Hz, 2H), 2.28 (d, J = 12.4 Hz, 2H), 2.15 (t, J = 8.1 Hz, 2H), 1.98 (s, 6H), 1.77 (p, J = 7.5 Hz, 4H), 1.42-1.25 (m,4H), 1.38 (s, 6H), 1.12 (s, 3H). MS (ESI-) m/z 575 (M-H)⁺.

### Example 35

### 5-[2-(4-fluoro-2,6-dimethylphenoxy)-5-(2-hydroxypropan-2-yl)phenyl] -1-methyl-4-{ [trans-4-(2-oxoimidazolidin-1 -yl)cyclohexyl] oxy}pyridin-2(1H)-one

### Example 35a

### 5-bromo-1-methyl-4-{[trans-4-(2-oxoimidazolidin-1-yl)cyclohexyl]oxy}pyridin-2(1H)-one

To a solution of Example 1e (0.3 g, 0.889 mmol) and triethylamine (0.310 mL, 2.221 mmol) in dichloromethane (8.89 mL) was added dropwise 1-chloro-2-isocyanatoethane (0.094 g, 0.889 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (8.89 mL), treated with sodium hydride (0.135 g, 5.33 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by chromatography (2-7% methanol in dichloromethane) afforded the title compound (0.036 g, 10% yield).

### Example 35b

### 5-[2-(4-fluoro-2,6-dimethylphenoxy)-5-(2-hydroxypropan-2-yl)phenyl]-1-methyl-4-{[trans-4-(2-oxoimidazolidin-1 -yl)cyclohexyl] oxy} pyridin-2(1H)-one

Example 3c (0.045 g, 0.113 mmol), Example 35a (0.035 g, 0.095 mmol), tris(dibenzylideneacetone)dipalladium(0) (2.60 mg, 2.84 µmol), 1,3,5,7-tetramethyl-6-phenyl-2,4,8-trioxa-6-phosphaadamantane (2.76 mg, 9.45 µmol) and sodium carbonate (0.050 g, 0.473 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (1.5-7% methanol in dichloromethane) afforded the title compound as a white powder (0.037 g, 65% yield). ¹H NMR (501 MHz, DMSO-*d*₆) δ 7.58 (s, 1H), 7.30 - 7.22 (m, 2H), 7.01 - 6.93 (m, 2H), 6.19 (d, J = 8.4 Hz, 2H), 5.96 (s, 1H), 4.91 (s, 1H), 4.31 (ddt, J = 10.7, 8.2, 4.2 Hz, 1H), 3.47 - 3.40 (m, 1H), 3.38 (s, 3H), 3.27 - 3.13 (m, 4H), 2.03 (m, 2H), 2.01 (s, 6H), 1.66 - 1.49 (m, 4H), 1.39 (s, 6H), 1.25 (qd, J = 12.3, 4.2 Hz, 2H). MS (ESI-) m/z 562 (M-H)⁺.

### Example 36

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]hex-5-enamide

### Step A

To a solution of Example 1e (0.3 g, 0.889 mmol) and triethylamine (0.310 mL, 2.221 mmol) in dichloromethane (8.89 mL) was added dropwise 6-chlorohexanoyl chloride (0.150 g, 0.889 mmol). The mixture was stirred at ambient temperature under nitrogen for 2 hours and partitioned with water. The organic layer was dried over anhydrous sodium sulfate, filtered, and concentrated. The crude residue was dissolved in tetrahydrofuran (8.89 mL), treated with sodium hydride (0.135 g, 5.33 mmol) and heated at 70 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by chromatography (silica gel, 1.5-6% methanol in dichloromethane) afforded a white solid (0.184 g, 52% yield).

### Step B

Example 3c (0.097 g, 0.242 mmol), the product from Step A (0.08 g, 0.201 mmol), tris(dibenzylideneacetone)dipalladium(0) (5.53 mg, 6.04 µmol), 1,3,5,7-tetramethyl-6-phenyl-2,4,8-trioxa-6-phosphaadamantane (5.89 mg, 0.020 mmol) and sodium carbonate (0.085 g, 0.805 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (30-100% of 3:1 ethyl acetate/ethanol in heptanes the title compound as the second eluting peak (0.020 g, 16% yield).¹H NMR (400 MHz, DMSO-*d*₆) δ 7.64 (dd, J = 22.8, 7.5 Hz, 1H), 7.56 (s, 1H), 7.28 - 7.22 (m, 2H), 6.95 (d, J = 9.1 Hz, 2H), 6.17 (d, J = 8.4 Hz, 1H), 5.95 (d, J = 1.5 Hz, 1H), 5.72 (m, 1H), 5.00 - 4.91 (m, 1H), 4.89 (s, 1H), 4.32 (m, 1H), 3.43 (m, 1H), 3.36 (s, 3H), 1.97 (m, 11H), 1.74 - 1.59 (m, 2H), 1.58 - 1.41 (m, 4H), 1.37 (s, 6H), 1.32 - 1.16 (m, 4H). ). MS (ESI-) m/z 589 (M-H)⁺.

### Example 37

### 1-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] azepan-2-one

Purification of the residue from Step B of Example 36 by chromatography (silica gel, 30-100% of 3:1 ethyl acetate/ethanol in heptanes afforded the title compound as the first eluting peak. ¹H NMR (400 MHz, DMSO-*d*₆) δ 7.56 (s, 1H), 7.28 - 7.19 (m, 2H), 6.95 (d, J = 9.2 Hz, 2H), 6.20 - 6.13 (m, 1H), 5.94 (s, 1H), 4.89 (s, 1H), 4.33 (ddd, J = 11.0, 8.7, 3.3 Hz, 1H), 4.21 (tt, J = 12.1, 4.0 Hz, 1H), 3.37 (s, 3H), 3.26 - 3.17 (m, 2H), 2.41 - 2.31 (m, 2H), 2.02 (m, 2H), 1.99 (s, 6H), 1.61 (d, J = 14.2 Hz, 4H), 1.47 (d, J = 8.8 Hz, 6H), 1.37 (s, 6H), 1.29 - 1.16 (m, 2H). MS (ESI-) m/z 589 (M-H)⁺.

### Example 38

### 5-[2-(4-fluoro-2,6-dimethylphenoxy)-5-(2-hydroxypropan-2-yl)phenyl]-1-methyl-4-{[trans-4-(2-oxo-1,3-oxazolidin-3-yl)cyclohexyl]oxy}pyridin-2(1H)-one

### Example 38a

### 5-bromo-l-methyl-4-{[trans-4-(2-oxo-1,3-oxazolidin-3-yl)cyclohexyl]oxylpyridin-2(1H)-one

Example 1e (0.2506 g, 0.832 mmol) and triethylamine (0.290 mL, 2.080 mmol) in dichloromethane (8.32 mL) were treated with 2-chloroethyl chloroformate (0.090 mL, 0.874 mmol) dropwise. The reaction mixture was stirred at ambient temperature overnight. The reaction mixture was washed with water, dried over anhydrous magnesium sulfate, filtered, and concentrated. The residue was taken up into tetrahydrofuran (8.3 mL). Sodium hydride (0.200 g, 4.99 mmol) was added. The reaction mixture was stirred at about 70 °C overnight. The reaction mixture was poured onto water and extracted 3 x with ethyl acetate. The combined organic layers were washed with saturated aqueous sodium chloride, dried over anhydrous magnesium sulfate, filtered, and concentrated to provide the title compound as a white solid (0.296 g, 0.797 mmol, 96% yield).

### Example 38b

### 5-[2-(4-fluoro-2,6-dimethylphenoxy)-5-(2-hydroxypropan-2-yl)phenyl] -1-methyl-4-{ [trans-4-(2-oxo-1,3-oxazolidin-3-yl)cyclohexyl]oxy}pyridin-2(1H)-one

Example 38b was prepared according to the procedure used for the preparation of Example 1k, substituting Example 38a for Example 1f, and substituting Example 3c for Example 1j to provide the title compound as a white solid. (0.0507 g, 0.090 mmol, 61% yield).

### Example 39

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,3-oxazinan-2-one

### Example 39a

### 3-{trans-4-[(5-bromo-1-methyl-2-oxo-1,2-dihydropyridin-4-yl)oxy]cyclohexyl}-1,3-oxazinan-2-one

Example 39a was prepared according to the procedure used for the preparation of Example 38a, substituting 3-chloropropyl chloroformate for 2-chloroethyl chloroformate, to provide the title compound as a white solid (0.265 g, 0.688 mmol, 82% yield).

### Example 39b

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,3-oxazinan-2-one

Example 39b was prepared according to the procedure used for the preparation of Example 1k, substituting Example 39a for Example 1f, and substituting Example 3c for Example 1j to provide the title compound as a white solid. (0.0439 g, 0.076 mmol, 53% yield). ¹H NMR (400 MHz, DMSO-*d*₆) δ 7.56 (s, 1H), 7.28 - 7.19 (m, 2H), 6.99 - 6.91 (m, 2H), 6.17 (d, J = 8.4 Hz, 1H), 5.94 (s, 1H), 4.89 (s, 1H), 4.31 (td, J = 10.6, 5.2 Hz, 1H), 4.11 - 4.03 (m, 2H), 3.82 (tt, J = 11.9, 4.0 Hz, 1H), 3.37 (s, 3H), 3.13 (t, J = 6.1 Hz, 2H), 2.03 (d, J = 12.8 Hz, 2H), 1.99 (s, 6H), 1.91 - 1.80 (m, 2H), 1.76 - 1.52 (m, 4H), 1.37 (s, 6H), 1.22 (qd, J = 11.8, 3.0 Hz, 2H). MS (ESI-) m/z 577.1 (M-H)⁻.

### Example 40

### 4-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]morpholin-3-one

### Example 40a

### 4-{trans-4-[(5-bromo-1-methyl-2-oxo-1,2-dihydropyridin-4-yl)oxy]cyclohexyl}morpholin-3-one

Example 40a was prepared according to the procedure used for the preparation of Example 38a, substituting 2-(2-chloroethoxy)acetyl chloride for 2-chloroethyl chloroformate, to provide the title compound as a white solid (0.200 g, 0.519 mmol, 80% yield).

### Example 40b

### 4-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]morpholin-3-one

Example 40b was prepared according to the procedure used for the preparation of Example 1k, substituting Example 40a for Example 1f, and substituting Example 3c for Example 1j, to provide the title compound as a white solid. (0.0391 g, 0.068 mmol, 50% yield). ¹H NMR (400 MHz, DMSO-*d*₆) δ 7.58 (s, 1H), 7.30 - 7.21 (m, 2H), 6.97 (d, J = 9.1 Hz, 2H), 6.19 (d, J = 8.4 Hz, 1H), 5.96 (s, 1H), 4.91 (s, 1H), 4.34 (td, J = 10.8, 10.3, 4.8 Hz, 1H), 4.23 - 4.11 (m, 1H), 3.98 (s, 2H), 3.78 (dd, J = 5.9, 4.1 Hz, 2H), 3.38 (s, 3H), 3.21 (t, J = 5.1 Hz, 2H), 2.10 - 2.01 (m, 2H), 2.01 (s, 6H), 1.76 - 1.62 (m, 2H), 1.55 (t, J = 7.5 Hz, 2H), 1.39 (s, 6H), 1.34 - 1.20 (m, 2H). MS (ESI-) m/z 577.1 (M-H)⁻.

### Example 41

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methyltetrahydropyrimidin-2(1H)-one

### Example 41a

### 1-{trans-4-[(5 -bromo-1 -methyl-2-oxo-1,2-dihydropyridin-4-yl)oxy]cyclohexyl} -3-methyltetrahydropyrimidin-2(1H)-one

To a solution of Example 31a (0.11 g, 0.286 mmol) in dimethylformamide (3.82 mL) at ambient temperature under nitrogen was added portionwise sodium hydride (0.029 g, 1.145 mmol). The mixture was stirred at ambient temperature under nitrogen for 20 minutes and treated with iodomethane (0.107 mL, 1.72 mmol). The mixture was sealed in the microwave tube and heated at 40 °C for 16 hours, cooled to ambient temperature, and partitioned between ethyl acetate and water. The organic layer was dried over anhydrous sodium sulfate, filtered, and concentrated. Purification of the residue by chromatography (silica gel 2-7% methanol in dichloromethane) afforded the title compound as a white solid (0.1 g, 84% yield).

### Example 41b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyltetrahydropyrimidin-2(1H)-one

Example 3c (0.060 g, 0.15 mmol), Example 41a (0.05 g, 0.13 mmol), tris(dibenzylideneacetone)dipalladium(0) (3.45 mg, 3.77 µmol), 1,3,5,7-tetramethyl-6-phenyl-2,4,8-trioxa-6-phosphaadamantane (3.67 mg, 0.013 mmol) and sodium carbonate (0.053 g, 0.50 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-100% of 3:1 ethyl acetate/ethanol in heptanes) afforded the title compound as a white powder (0.063 g, 81% yield). ¹H NMR (400 MHz, DMSO-*d*₆) δ 7.56 (s, 1H), 7.28 - 7.20 (m, 2H), 6.98 - 6.90 (m, 2H), 6.17 (dd, J = 8.2, 0.7 Hz, 1H), 5.93 (s, 1H), 4.89 (s, 1H), 4.35 - 4.23 (m, 1H), 4.04 (ddd, J = 12.0, 8.1, 3.9 Hz, 1H), 3.36 (s, 3H), 3.11 (t, J = 6.0 Hz, 2H), 3.06 (t, J = 5.8 Hz, 2H), 2.72 (s, 3H), 2.02 (m, 2H), 1.99 (s, 6H), 1.82 - 1.72 (m, 2H), 1.59 (td, J = 13.8, 13.4, 10.2 Hz, 2H), 1.49 - 1.40 (m, 2H), 1.37 (s, 6H), 1.27 - 1.16 (m, 2H). MS (ESI-) m/z 590 (M-H)⁺.

### Example 42

### 5-[2-(4-fluoro-2,6-dimethylphenoxy)-5-(2-hydroxypropan-2-yl)phenyl] -1-methyl-4-{ [trans-4-(2-methyl-5-oxopyrrolidin-1-yl)cyclohexyl]oxy}pyridin-2(1H)-one

### Example 42a

### 5-bromo-1-methyl-4-{[trans-4-(2-methyl-5-oxopyrrolidin-1 -yl)cyclohexyl]oxy} pyridin-2(1H)-one

To a solution of Example 1e (0.545 g, 1.61 mmol) and triethylamine (0.562 mL, 4.03 mmol) in dichloromethane (16 mL) was added dropwise 4-chloro-pentanoyl chloride (0.25 g, 1.6 mmol). The mixture was stirred at ambient temperature under nitrogen for 2 hours and partitioned with water. The organic layer was dried over anhydrous sodium sulfate, filtered, and concentrated. The residue was dissolved in tetrahydrofuran (16 mL), treated with sodium hydride (0.244 g, 9.68 mmol) and heated at 65 °C for 18 hours. The mixture was cooled to ambient temperature and partitioned between ethyl acetate and water. The aqueous layer was extracted twice more with ethyl acetate. The organic layers were combined, dried over anhydrous sodium sulfate, filtered, and concentrated. Purification of the residue by trituration in 9:1 heptanes/ethyl acetate afforded the title compound as an off-white powder (0.355 g, 55% yield).

### Example 42b

### 5-[2-(4-fluoro-2,6-dimethylphenoxy)-5-(2-hydroxypropan-2-yl)phenyl] -1-methyl-4-{ [trans-4-(2-methyl-5-oxopyrrolidin-1-yl)cyclohexyl]oxy}pyridin-2(1H)-one

Example 3c (0.094 g, 0.235 mmol), Example 42a (0.075 g, 0.196 mmol), tris(dibenzylideneacetone)dipalladium(0) (5.38 mg, 5.87 µmol), 1,3,5,7-tetramethyl-6-phenyl-2,4,8-trioxa-6-phosphaadamantane (5.72 mg, 0.020 mmol) and sodium carbonate (0.083 g, 0.783 mmol) were combined and sparged with argon for 15 minutes. Meanwhile a solution of 4:1 tetrahydrofuran/water (2.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-100% of 3:1 ethyl acetate/ethanol in heptanes) afforded the title compound as a white powder (0.084 g, 72%). ¹H NMR (501 MHz, DMSO-*d*₆) δ 7.58 (s, 1H), 7.31 - 7.21 (m, 2H), 7.01 - 6.94 (m, 2H), 6.19 (d, J = 8.4 Hz, 1H), 6.01 (s, 1H), 4.91 (s, 1H), 4.43 - 4.34 (m, 1H), 3.76 - 3.67 (m, 1H), 3.50 (tt, J = 12.0, 4.0 Hz, 1H), 3.39 (s, 3H), 2.37 - 2.23 (m, 1H), 2.12 - 2.02 (m, 3H), 2.01 (s, 6H), 1.92 (qd, J = 12.6, 3.3 Hz, 1H), 1.81 - 1.44 (m, 5H), 1.39 (s, 6H), 1.32 - 1.19 (m, 2H), 1.18 (d, J = 6.3 Hz, 3H). MS (ESI-) m/z 575 (M-H)⁺.

### Example 43

### 2-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-1H-isoindole-1,3(2H)-dione

### Example 43a

### 2-{trans-4-[(5-bromo-1-methyl-2-oxo-1,2-dihydropyridin-4-yl)oxy]cyclohexyl}-1H isoindole-1,3(2H)-dione

A solution of 2-(*trans*-4-hydroxycyclohexyl)-1*H*-isoindole-1,3(2*H*)-dione (0.119 g, 0.485 mmol) in tetrahydrofuran (4.85 mL) at 0 °C under nitrogen was treated portionwise with sodium hydride (0.049 g, 1.94 mmol). The mixture was stirred for 20 minutes under nitrogen at 0-5 °C, followed by portionwise addition of Example 1c (0.1 g, 0.485 mmol). The mixture was stirred for 24 hours at ambient temperature and then partitioned between ethyl acetate and water. The water layer was carefully acidified to pH1 with 1M hydrochloric acid. The aqueous layer was extracted once more with ethyl acetate. The organic extracts were combined and washed with saturated aqueous sodium chloride, dried over anhydrous sodium sulfate, filtered, and concentrated. Purification of the residue by chromatography (reverse phase C18, 10-90% acetonitrile in water (0.1% trifluoroacetic acid) afforded the title compound (0.026 g, 12% yield).

### Example 43b

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1H-isoindole-1,3(2H)-dione

Example 3c (0.029 g, 0.072 mmol), Example 43a (0.026 g, 0.060 mmol), tris(dibenzylideneacetone)dipalladium(0) (1.656 mg, 1.809 µmol), 1,3,5,7-tetramethyl-6-phenyl-2,4,8-trioxa-6-phosphaadamantane (1.762 mg, 6.03 µmol) and sodium carbonate (0.032 g, 0.301 mmol) were combined and sparged with argon for 15 minutes. Meanwhile a solution of 4:1 tetrahydrofuran/water (0.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water, adjusting the pH to 1. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-70% of 3:1 ethyl acetate/ethanol in heptanes) afforded the title compound as a white powder (0.011 g, 29% yield). ¹H NMR (400 MHz, DMSO-*d*₆) δ 7.84 - 7.76 (m, 4H), 7.57 (s, 1H), 7.30 - 7.22 (m, 2H), 6.96 (d, J = 9.1 Hz, 2H), 6.19 (d, J = 8.5 Hz, 1H), 6.01 (s, 1H), 4.90 (s, 1H), 4.40 (dt, J= 11.0, 6.4 Hz, 1H), 4.00-3.75 (m, 1H), 3.38 (s, 3H), 2.34 - 2.18 (m, 2H), 2.11 (d, J = 12.4 Hz, 2H), 2.01 (s, 6H), 1.71 (d, J = 12.3 Hz, 2H), 1.38 (s, 6H), 1.27 (dt, J = 23.7, 12.5 Hz, 2H). MS (ESI+) m/z 625 (M+H)⁺.

### Example 44

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]pyrrolidine-2,5-dione

### Example 44a

### 1-{trans-4-[(5-bromo-1-methyl-2-oxo-1,2-dihydropyridin-4-yl)oxy]cyclohexyl}pyrrolidine-2,5-dione

A mixture of Example 1e (0.25 g, 0.740 mmol), succinic anhydride (0.089 g, 0.889 mmol) and triethylamine (0.227 mL, 1.629 mmol) in xylene (4.0 mL) was heated at 135 °C for 4 hours and concentrated. The crude intermediate was treated with sodium acetate (0.067 g, 0.814 mmol) in acetic anhydride (4.00 mL) and heated at 130 °C for 24 hours, cooled to ambient temperature, and concentrated. The residue was partitioned between ethyl acetate and water adjusting the pH to 8 with 5% aqueous sodium bicarbonate. The organic layer was washed with saturated aqueous sodium chloride, dried over anhydrous sodium sulfate, filtered, and concentrated. Purification of the residue by chromatography (silica gel 30-70% of 3:1 ethyl acetate/ethanol in heptanes) afforded the title compound as a tan foam (0.19 g, 67% yield).

### Example 44b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]pyrrolidine-2,5-dione

Example 3c (0.063 g, 0.157 mmol), Example 44a (0.05 g, 0.130 mmol), tris(dibenzylideneacetone)dipalladium(0) (3.58 mg, 3.91 µmol), 1,3,5,7-tetramethyl-6-phenyl-2,4,8-trioxa-6-phosphaadamantane (3.81 mg, 0.013 mmol) and sodium carbonate (0.069 g, 0.652 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water, adjusting the pH to 1. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-70% of 3:1 ethyl acetate/ethanol in heptanes) afforded the title compound as a white powder (0.061 g, 78% yield). ¹H NMR (400 MHz, DMSO-*d*₆) δ 7.56 (s, 1H), 7.29 - 7.19 (m, 2H), 6.95 (d, J = 9.1 Hz, 2H), 6.17 (d, J = 8.5 Hz, 1H), 5.97 (s, 1H), 4.89 (s, 1H), 4.33 (dt, J = 11.4, 6.4 Hz, 1H), 3.75 (tt, J = 12.2, 3.6 Hz, 1H), 3.37 (s, 3H), 2.53 (s, 4H), 2.30 - 2.14 (m, 2H), 2.11 - 2.02 (m, 2H), 1.99 (s, 6H), 1.51 (d, J = 12.3 Hz, 2H), 1.37 (s, 6H), 1.22 (dd, J = 14.0, 10.6 Hz, 2H). MS (ESI+) m/z 577 (M+H)⁺.

### Example 45

### 2- {[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl}benzoic acid

### Example 45a

### 2-({trans-4-[(5-bromo-1-methyl-2-oxo-1,2-dihydropyridin-4-yl)oxy]cyclohexyl}carbamoyl)benzoic acid

A solution of 2-(*trans*-4-hydroxycyclohexyl)-1*H*-isoindole-1,3(2*H*)-dione (0.119 g, 0.485 mmol) and Example 1c (0.1 g, 0.485 mmol) in tetrahydrofuran (3.24 mL) at 0 °C under nitrogen was treated dropwise with 1M potassium *tert*-butoxide (0.558 mL, 0.558 mmol) in tetrahydrofuran. The mixture was stirred for 60 minutes under nitrogen at 0-5 °C and then partitioned between ethyl acetate and water. The mixture was carefully acidified to pH1 with 1M hydrochloric acid. The aqueous layer was extracted once more with ethyl acetate. The organic extracts were combined and washed with saturated aqueous sodium chloride, dried over anhydrous sodium sulfate, filtered, and concentrated. The crude solid was triturated in 2:1 heptane/ethyl acetate to afford a white solid (0.11 g, 42% yield).

### Example 45b

### 2- {[trans-4-( {5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}benzoic acid

Example 3c (0.064 g, 0.160 mmol), Example 45a (0.06 g, 0.134 mmol), tris(dibenzylideneacetone)dipalladium(0) (3.67 mg, 4.01 µmol), 1,3,5,7-tetramethyl-6-phenyl-2,4,8-trioxa-6-phosphaadamantane (3.90 mg, 0.013 mmol) and sodium carbonate (0.071g, 0.668 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water, adjusting the pH to 1 by the addition of 1M HCl. The organic layer was washed with saturated aqueous sodium chloride, dried over anhydrous sodium sulfate, treated with 3-mercaptopropyl functionalized silica gel, filtered, and concentrated. Purification of the residue by reverse phase chromatography (C-18, 10-90% acetonitrile in water (0.1% trifluoroacetic acid)) to afford the title compound (0.036 g, 39% yield). ¹H NMR (400 MHz, DMSO-*d*₆) δ 10.79 (d, J = 7.1 Hz, 1H), 7.66 (dd, J = 7.8, 1.4 Hz, 1H), 7.60 (s, 1H), 7.41 - 7.18 (m, 5H), 6.97 (d, J = 9.1 Hz, 2H), 6.21 (d, J = 8.5 Hz, 1H), 5.96 (s, 1H), 4.46 (m, 1H), 3.65(m, 1H), 3.39 (s, 3H), 2.02 (s, 6H), 1.97 (m, 2H), 1.69 (m, 2H), 1.40 (m, 10H). MS (ESI+) m/z 641 (M+H)⁺.

### Example 46

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]methanesulfonamide

### Example 46a

### N- {trans-4-[(5-bromo-1-methyl-2-oxo-1,2-dihydropyridin-4-yl)oxy]cyclohexyl} methanesulfonamide

To a solution of Example 1e (0.1 g, 0.296 mmol) and triethylamine (0.124 mL, 0.889 mmol) in dichloromethane (5.92 mL) was added dropwise methanesulfonyl chloride (0.048 mL, 0.622 mmol). The mixture was stirred at ambient temperature under nitrogen for 2 hours and partitioned with water. The organic layer was dried over anhydrous sodium sulfate, filtered, and concentrated. Purification of the residue by trituration in 9:1 heptane/ethyl acetate afforded the title compound as a white solid (0.1 g, 81% yield).

### Example 46b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]methanesulfonamide

Example 3c (0.063 g, 0.158 mmol), Example 46a (0.05 g, 0.132 mmol), tris(dibenzylideneacetone)dipalladium(0) (3.62 mg, 3.95 µmol), 1,3,5,7-tetramethyl-6-phenyl-2,4,8-trioxa-6-phosphaadamantane (3.85 mg, 0.013 mmol) and sodium carbonate (0.070 g, 0.659 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-70% of 3:1 ethyl acetate/ethanol in heptanes) afforded the title compound as a white powder (0.05 g, 63% yield). ¹H NMR (400 MHz, DMSO-*d*₆) δ 7.58 (s, 1H), 7.29 - 7.23 (m, 2H), 7.02 (d, J = 6.7 Hz, 1H), 6.96 (d, J = 9.1 Hz, 2H), 6.19 (d, J = 8.4 Hz, 1H), 5.95 (s, 1H), 4.91 (s, 1H), 4.31 (m, 1H), 3.38 (s, 3H), 3.09 (m, 1H), 2.86 (s, 3H), 2.00 (s, 6H), 1.97 (m, 2H), 1.77 (m, 2H), 1.38 (s, 6H), 1.37 - 1.21 (m, 4H). ). MS (ESI+) m/z 573 (M+H)⁺.

### Example 47

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-2,2-dimethylbutanamide

### Example 47a

### N- {trans-4-[(5-bromo-1-methyl-2-oxo-1,2-dihydropyridin-4-yl)oxy]cyclohexyl} -4-hydroxy-2,2-dimethylbutanamide

Example 1e (338 mg, 1.00 mmol), 3,3-dimethyldihydrofuran-2(3H)-one (228 mg, 2.00 mmol), *N,N*-diisopropylethylamine (1.05 mL, 6.00 mmol) and 2M trimethylaluminum in toluene (0.500 mL, 1.00 mmol) were combined in tetrahydrofuran (4 mL). The reaction mixture was stirred at ambient temperature for 64 hours. To this mixture was added 3,3-dimethyldihydrofuran-2(3H)-one (228 mg, 2.00 mmol), N,N-diisopropylethylamine (1.05 mL, 6.00 mmol) and 2M trimethylaluminum in toluene (0.500 mL, 1.00 mmol) again. The reaction mixture was stirred at ambient temperature for another 48 hours, saturated aqueous sodium bicarbonate added slowly, and extracted with ethyl acetate. The organic layer was washed with saturated aqueous sodium chloride, dried with anhydrous sodium sulfate, filtered, and concentrated. The residue was purified by flash chromatography (silica gel, 20-40% 3:1 ethyl acetate/ethanol in heptanes) to provide the title compound (285 mg, 69% yield).

### Example 47b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-2,2-dimethylbutanamide

Example 47a (42 mg, 0.10 mmol), Example 3c (40 mg, 0.10 mmol), sodium carbonate (37 mg, 0.35 mmol), tris(dibenzylideneacetone)dipalladium (2.8 mg, 3.0 µmol) and 1,3,5,7-tetramethyl-6-phenyl-2,4,8-trioxa-6-phosphaadamantane (2.6 mg, 9.0 µmol) were combined in a microwave tube. The reaction mixture was purged with nitrogen for 15 minutes. The mixture of tetrahydrofuran (2 mL)/water (0.5 mL) was purged with nitrogen for 15 minutes and transferred to the reaction vessel. The reaction mixture was heated at 60 °C for 3 hours, cooled to ambient temperature, and partitioned with ethyl acetate and water. The organic layer was washed with saturated aqueous sodium chloride, dried with anhydrous sodium sulfate, treated with 3-mercaptopropyl functionalized silica gel, filtered, and concentrated. The residue was purified by flash chromatography (silica gel, 20-80% 3:1 ethyl acetate/ethanol in heptanes) to provide the title compound (46 mg, 76% yield). ¹H NMR (400 MHz, DMSO-*d*₆) δ 7.59 (s, 1H), 7.29 - 7.23 (m, 2H), 7.16 (d, *J=* 7.7 Hz, 1H), 6.97 (d, *J=* 9.1 Hz, 2H), 6.19 (d, *J=* 8.3 Hz, 1H), 5.94 (s, 1H), 4.91 (s, 1H), 4.36 - 4.26 (m, 2H), 3.52 - 3.43 (m, 1H), 3.38 (s, 3H), 3.32 - 3.26 (m, 2H), 2.04 - 1.93 (m, 8H), 1.70 - 1.56 (m, 4H), 1.42 - 1.23 (m, 10H), 1.03 (s, 6H). (ESI-) m/z 607 (M-H)⁺.

### Example 48

### 4- {[trans-4-(3,3-dimethyl-2-oxopyrrolidin-1-yl)cyclohexyl] oxy} -5-[2-(4-fluoro-2,6-dimethylphenoxy)-5-(2-hydroxypropan-2-yl)phenyl]-1-methylpyridin-2(1H)-one

### Example 48a

### 5-bromo-4-{[trans-4-(3,3-dimethyl-2-oxopyrrolidin-1-yl)cyclohexyl]oxy}-1-methylpyridin-2(1H)-one

To a solution of Example 47a (83 mg, 0.20 mmol) and triphenylphosphine (105 mg, 0.400 mmol) in tetrahydrofuran (1 mL) was added diisopropyl azodicarboxylate (0.058 mL, 0.30 mmol) dropwise. The reaction mixture was stirred at ambient temperature for 24 hours, saturated aqueous sodium bicarbonate was added, and the mixture was extracted with ethyl acetate. The organic layer was washed with saturated aqueous sodium chloride, dried with anhydrous sodium sulfate, filtered, and concentrated. The residue was purified by flash chromatography (silica gel, 20-60% 3:1 ethyl acetate/ethanol in heptanes) to provide the title compound (59 mg, 74% yield).

### Example 48b

### 4-1{[trans-4-(3,3-dimethyl-2-oxopyrrolidin-1-yl)cyclohexyl]oxy)-5-[2-(4-fluoro-2,6-dimethylphenoxy)-5-(2-hydroxypropan-2-yl)phenyl]-1-methylpyridin-2(1H)-one

Example 48b was prepared according to the procedure used for the preparation of Example 47b, substituting Example 48a for Example 47a, to provide the title compound (41 mg, 69% yield). ¹H NMR (400 MHz, DMSO-*d*₆) δ 7.58 (s, 1H), 7.28 - 7.23 (m, 2H), 6.97 (d, *J*= 9.1 Hz, 2H), 6.19 *(d, J=* 8.5 Hz, 1H), 5.92 (s, 1H), 4.90 (s, 1H), 4.41 - 4.35 (m, 1H), 4.09 (t, *J*= 6.8 Hz, 2H), 3.38 (s, 3H), 3.35 - 3.31 (m, 1H), 2.00 (s, 6H), 1.96 - 1.91 (m, 2H), 1.87 *(t, J=* 6.9 Hz, 2H), 1.58 - 1.50 (m, 2H), 1.38 (s, 6H), 1.35 - 1.22 (m, 4H), 1.07 (s, 6H). (ESI+) m/z 591 (M+H)⁺.

### Example 49

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-hydroxypropanamide

### Example 49a

### N- {trans-4-[(5-bromo-1-methyl-2-oxo-1,2-dihydropyridin-4-yl)oxy]cyclohexyl} -3-hydroxypropanamide

A mixture of Example 1e (0.1 g, 0.296 mmol), triethylamine (0.124 mL, 0.889 mmol), and 3-hydroxypropionic acid (0.133 g, 0.444 mmol) in tetrahydrofuran (4.0 mL)/dimethylformamide (0.3 mL) was treated portionwise with 2-(3H-[1,2,3]triazolo[4,5-b]pyridin-3-yl)-1,1,3,3-tetramethylisouronium hexafluorophosphate(V) (0.169 g, 0.444 mmol). The mixture was stirred at ambient temperature under nitrogen for 2 hours and partitioned with water and ethyl acetate, adjusting the pH to 8 with 5% aqueous sodium bicarbonate. The aqueous layer was extracted 3 x 30 mL with ethyl acetate. The organics were combined, dried over anhydrous sodium sulfate, filtered, and concentrated. Purification of the residue by chromatography (silica gel, 2-10% methanol in dichloromethane) afforded the title compound as a white solid (0.087 g, 68% yield).

### Example 49b

### N-[trans-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-hydroxypropanamide

Example 3c (0.051 g, 0.129 mmol), Example 49a (0.04 g, 0.107 mmol), tris(dibenzylideneacetone)dipalladium(0) (2.94 mg, 3.22 µmol), 1,3,5,7-tetramethyl-6-phenyl-2,4,8-trioxa-6-phosphaadamantane (3.13 mg, 10.72 µmol) and sodium carbonate (0.057 g, 0.536 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by trituration in 4:1 heptane/ethyl acetate afforded the title compound as an off-white solid (0.031 g, 47% yield). ¹H NMR (400 MHz, DMSO-*d*₆) δ 7.70 (d, J = 7.4 Hz, 1H), 7.58 (s, 1H), 7.31 - 7.21 (m, 2H), 6.97 (d, J = 9.1 Hz, 2H), 6.19 (d, J = 8.4 Hz, 1H), 5.97 (s, 1H), 4.91 (s, 1H), 4.49 (t, J = 5.2 Hz, 1H), 4.37 (m, 1H), 3.56 (td, J = 6.5, 5.1 Hz, 2H), 3.47 (m, 1H), 3.38 (s, 3H), 2.19 (t, J = 6.6 Hz, 2H), 2.00 (s, 6H), 1.96 (m, 2H), 1.68 (m, 2H), 1.39 (s, 6H), 1.34 - 1.21 (m, 4H). MS (ESI-) m/z 565 (M-H)⁺.

### Example 50

### N-[trans-4-(15-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] cyclopropanecarboxamide

### Example 50a N- {trans-4-[(5-bromo-1-methyl-2-oxo-1,2-dihydropyridin-4-yl)oxy]cyclohexyl} cyclopropanecarboxamide

To a solution of Example 1c (0.10 g, 0.30 mmol) and triethylamine (0.083 mL, 0.59 mmol) in dichloromethane (3 mL) was added dropwise cyclopropanecarbonyl chloride (0.031 g, 0.30 mmol). The mixture was stirred at ambient temperature under nitrogen for 1 hour and partitioned with water. The organic layer was dried over anhydrous sodium sulfate, filtered, and concentrated. Trituration in 9:1 heptane/ethyl acetate afforded the title compound as a cream colored solid (0.1 g, 86% yield).

### Example 50b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] cyclopropanecarboxamide

Example 3c (0.065 g, 0.162 mmol), Example 50a (0.05 g, 0.135 mmol), tris(dibenzylideneacetone)dipalladium(0) (3.72 mg, 4.06 µmol), 1,3,5,7-tetramethyl-6-phenyl-2,4,8-trioxa-6-phosphaadamantane (3.96 mg, 0.014 mmol) and sodium carbonate (0.057 g, 0.542 mmol) were combined and sparged with argon for 15 minutes. Meanwhile a solution of 4:1 tetrahydrofuran/water (1.5 mL) was sparged with nitrogen for 15 minutes and then transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-70% of 3:1 ethyl acetate/ethanol in heptanes) afforded the title compound as a white powder (0.047 g, 59% yield). ¹H NMR (501 MHz, DMSO-*d*₆) δ 7.97 (d, J = 7.5 Hz, 1H), 7.60 (s, 1H), 7.31 - 7.23 (m, 2H), 6.98 (d, J = 9.1 Hz, 2H), 6.20 (d, J = 8.4 Hz, 1H), 5.99 (s, 1H), 4.93 (s, 1H), 4.40 (m, 1H), 3.48 (m, 1H), 3.39 (s, 3H), 2.01 (s, 6H), 1.99 - 1.92 (m, 2H), 1.70 (m, 2H), 1.53 (tt, J = 7.6, 4.9 Hz, 1H), 1.39 (s, 6H), 1.36 - 1.20 (m, 4H), 0.60 (dtd, J = 10.0, 7.9, 5.0 Hz, 4H). MS (ESI-) m/z 561 (M-H)⁺.

### Example 51

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-methylcyclopropane-1-carboxamide

### Example 51a N- {trans-4-[(5-bromo-1 -methyl-2-oxo-1,2-dihydropyridin-4-yl)oxy]cyclohexyl} -1 - methylcyclopropane-1-carboxamide

To a solution of Example 1e (0.10 g, 0.30 mmol) and triethylamine (0.083 mL, 0.59 mmol) in dichloromethane (3 mL) was added dropwise 1-methylcyclopropanecarbonyl chloride (0.035 g, 0.30 mmol). The mixture was stirred at ambient temperature under nitrogen for 1 hour and partitioned with water. The organic layer was dried over anhydrous sodium sulfate, filtered, and concentrated. Trituration in 9:1 heptane/ethyl acetate afforded the title compound as a cream colored solid (0.10 g, 83% yield).

### Example 51b

### N-[trans-4-(15-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-methylcyclopropane-1-carboxamide

Example 3c (0.063 g, 0.16 mmol), Example 51a (0.050 g, 0.13 mmol), tris(dibenzylideneacetone)dipalladium(0) (3.58 mg, 3.91 µmol), 1,3,5,7-tetramethyl-6-phenyl-2,4,8-trioxa-6-phosphaadamantane (3.81 mg, 0.013 mmol) and sodium carbonate (0.055 g, 0.52 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a white solid (0.032 g, 40% yield). ¹H NMR (400 MHz, DMSO-*d*₆) δ 7.58 (s, 1H), 7.31 - 7.20 (m, 2H), 7.14 (d, J = 7.6 Hz, 1H), 6.97 (d, J = 9.1 Hz, 2H), 6.19 (d, J = 8.3 Hz, 1H), 5.94 (s, 1H), 4.91 (s, 1H), 4.29 (dt, J = 10.4, 5.5 Hz, 1H), 3.56 - 3.42 (m, 1H), 3.38 (s, 3H), 2.00 (s, 6H), 1.97 (m, 2H), 1.72 - 1.61 (m, 2H), 1.40 (m, 2H), 1.39 (s, 6H), 1.25 (m, 2H), 1.21 (s, 3H), 0.89 (q, J = 3.4 Hz, 2H), 0.44 (q, J = 3.6 Hz, 2H). MS (ESI-) m/z 575 (M-H)⁺.

### Example 52

### 2-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -1 lambda^{6,2}-thiazolidine-1,1 -dione

### Example 52a

### N- {trans-4-[(5-bromo-1-methyl-2-oxo-1,2-dihydropyridin-4-yl)oxy]cyclohexyl} -3-chloropropane-1-sulfonamide

To a solution of Example 1e (0.20 g, 0.59 mmol) and triethylamine (0.330 mL, 2.37 mmol) in dichloromethane (11.9 mL) was added dropwise 3-chloropropanesulfonyl chloride (0.210 g, 1.19 mmol). The mixture was stirred at ambient temperature under nitrogen for 3 hours and partitioned with water. The organic layer was dried over anhydrous sodium sulfate, filtered, and concentrated. Trituration in 9:1 heptane/ethyl acetate afforded the title compound as a cream colored solid (0.20 g, 72% yield).

### Example 52b

### 2- {trans-4-[(5 -bromo-1-methyl-2-oxo-1,2-dihydropyridin-4-yl)oxy]cyclohexyl} -11⁶,2-thiazolidine-1,1-dione

In a microwave tube, a solution of Example 52a (0.060 g, 0.14 mmol) in tetrahydrofuran (1.4 mL) at 23 °C under nitrogen was treated with sodium hydride (0.021 g, 0.815 mmol, 95% yield). The tube was sealed and the mixture was heated at 70 °C for 24 hours, cooled to ambient temperature, and partitioned between ethyl acetate and water. The aqueous layer was extracted once more with ethyl acetate. The organic extracts were combined and washed with saturated aqueous sodium chloride, dried over anhydrous sodium sulfate, filtered, and concentrated. Purification of the residue by chromatography (silica gel, 30-100% of 3:1 ethyl acetate/ethanol in heptanes) afforded the title compound (0.020 g, 36% yield).

### Example 52c

### 2-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -1lambda^{6,2}-thiazolidine-1,1 -dione

Example 3c (0.022 g, 0.054 mmol), Example 52b (0.02 g, 0.049 mmol), tris(dibenzylideneacetone)dipalladium(0) (1.36 mg, 1.48 µmol), 1,3,5,7-tetramethyl-6-phenyl-2,4,8-trioxa-6-phosphaadamantane (1.442 mg, 4.93 µmol) and sodium carbonate (0.031 g, 0.296 mmol) were combined and sparged with argon for 15 minutes. Meanwhile a solution of 4:1 tetrahydrofuran/water (0.7 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-6% methanol in dichloromethane) afforded the title compound as a white foam (0.020 g, 66% yield). ¹H NMR (400 MHz, DMSO-*d*₆) δ 7.56 (s, 1H), 7.28 - 7.19 (m, 2H), 6.95 (d, J = 9.1 Hz, 2H), 6.17 (d, J = 8.4 Hz, 1H), 5.94 (s, 1H), 4.89 (s, 1H), 4,33 (m, 1H), 3.36 (s, 3H), 3.18 - 3.08 (m, 5H), 2.21 - 2.11 (m, 2H), 1.98 (m, 8H), 1.76 - 1.58 (m, 4H), 1.37 (s, 6H), 1.33 - 1.19 (m, 2H). MS (ESI-) m/z 597 (M-H)⁺.

### Example 53

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2-methoxyacetamide

### Example 53a

### 4-[(trans-4-aminocyclohexyl)oxy]-5-[2-(4-fluoro-2,6-dimethylphenoxy)-5-(2-hydroxypropan-2-yl)phenyl] -1 -methylpyridin-2(1H)-one

Example 3c (0.249 g, 0.622 mmol), Example 1e (0.2 g, 0.592 mmol), tris(dibenzylideneacetone)dipalladium(0) (0.016 g, 0.018 mmol), 1,3,5,7-tetramethyl-6-phenyl-2,4,8-trioxa-6-phosphaadamantane (0.017 g, 0.059 mmol) and sodium carbonate (0.377 g, 3.55 mmol) were combined and sparged with argon for 15 minutes. Meanwhile a solution of 4:1 tetrahydrofuran/water (6.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by trituration (95/5 heptane/dichloromethane) afforded the title compound as a powder (0.13 g, 40% yield).

### Example 53b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methoxyacetamide

To a solution of Example 53a (0.04 g, 0.081 mmol) and triethylamine (0.034 mL, 0.243 mmol) in dichloromethane (1.617 mL) was added dropwise methoxyacetyl chloride (9.22 mg, 0.085 mmol). The mixture was stirred at ambient temperature under nitrogen for 2 hours and partitioned with water and additional dichloromethane. The organic layer was dried over anhydrous sodium sulfate, filtered, and concentrated. Purification of the residue by chromatography (silica gel, 1-6% methanol in dichloromethane) afforded the title compound as a foam (0.022 g, 46% yield). ¹H NMR (400 MHz, DMSO-*d*₆) δ 7.60 (s, 1H), 7.58 (d, J = 7.9 Hz, 1H), 7.29 - 7.24 (m, 2H), 6.98 (dt, J = 9.2, 0.7 Hz, 2H), 6.20 (d, J = 8.4 Hz, 1H), 5.95 (s, 1H), 4.93 (s, 1H), 4.33 (tt, J = 9.2, 3.9 Hz, 1H), 3.75 (s, 2H), 3.54 (m, 1H), 3.39 (s, 3H), 3.27 (s, 3H), 2.01 (s, 6H), 1.97 (m, 2H), 1.69 (dd, J = 13.2, 4.0 Hz, 2H), 1.39 (m, 8H), 1.33 - 1.23 (m, 2H). MS (ESI+) m/z 567 (M+H)⁺.

### Example 54

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N-(2-hydroxyethyl)acetamide

### Example 54a

### 5-bromo-4-({trans-4-[(2-{ [tert-butyl(dimethyl)silyl]oxy} ethyl)amino]cyclohexyl} oxy)-1-methylpyridin-2(1H)-one

A mixture of Example 1e (0.30 g, 0.89 mmol), (2-bromoethoxy)-tert-butyldimethylsilane (0.255 g, 1.07 mmol), and potassium carbonate (0.491 g, 3.55 mmol) in dimethyl sulfoxide (4.44 mL) was stirred for 18 hours at 70 °C, cooled to ambient temperature, and partitioned between ethyl acetate and water. The organic layer was washed twice with saturated aqueous sodium chloride, dried over anhydrous sodium sulfate, filtered, and concentrated. Purification of the residue by trituration in 9:1 heptane/ethyl acetate afforded the crude title compound as a beige colored powder (0.275 g, 67% yield).

### Example 54b

### N- {trans-4-[(5-bromo-1-methyl-2-oxo-1,2-dihydropyridin-4-yl)oxy]cyclohexyl} -N-(2-{[tert-butyl(dimethyl)silyl] oxy} ethyl)acetamide

To a solution of Example 54a (0.15 g, 0.33 mmol) and trimethylamine (0.137 mL, 0.979 mmol) in dichloromethane (6.5 mL) was added dropwise acetyl chloride (0.028 mL, 0.39 mmol). The mixture was stirred at ambient temperature under nitrogen for 2 hours and partitioned with water and additional dichloromethane. The organic layer was dried over anhydrous sodium sulfate, filtered, and concentrated. Purification of the residue by chromatography (silica gel, 20-60% of (3:1 ethyl acetate/ethanol)/heptanes) afforded the title compound (0.073 g, 45% yield).

### Example 54c

### N-(2-{[tert-butyl(dimethyl)silyl]oxy}ethyl)-N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]acetamide

Example 3c (0.061 g, 0.154 mmol), Example 54b (0.07 g, 0.140 mmol), tris(dibenzylideneacetone)dipalladium(0) (3.83 mg, 4.19 µmol), 1,3,5,7-tetramethyl-6-phenyl-2,4,8-trioxa-6-phosphaadamantane (4.08 mg, 0.014 mmol) and sodium carbonate (0.059 g, 0.558 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 20-60% of 3:1 ethyl acetate/ethanol in heptanes) afforded the title compound as a white foam (0.08 g, 80% yield).

### Example 54d

### N-[trans-4-({15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N-(2-hydroxyethyl)acetamide

To a solution of Example 54c (0.08 g, 0.115 mmol) in tetrahydrofuran (3.0 mL) under nitrogen was added dropwise tetrabutylammonium fluoride (0.173 mL, 0.173 mmol). The mixture was stirred at ambient temperature under nitrogen for 1 hour and partitioned with 5% aqueous ammonium chloride and ethyl acetate. The organic layer was dried over anhydrous sodium sulfate, filtered, and concentrated. Purification of the residue by chromatography (silica gel, 1-8% methanol in dichloromethane) afforded the title compound as a foam (0.056 g, 79% yield). ¹H NMR (400 MHz, DMSO-*d*₆) δ 7.44 (s, 1H), 7.30 (d, J = 2.4 Hz, 1H), 7.25 (dd, J = 8.6, 2.4 Hz, 1H), 6.87 (d, J = 9.2 Hz, 2H), 6.23 (d, J = 8.6 Hz, 1H), 5.92 (s, 1H), 4.28 (m, 3H), 3.76 (m, 1H), 3.46 (q, J = 6.3 Hz, 2H), 3.39 (s, 3H), 3.25 (t, J = 6.6 Hz, 2H), 2.05 (m, 2H), 2.02 (s, 6H), 1.98 (s, 3H), 1.78 - 1.58 (m, 4H), 1.43 (s, 6H), 1.31 (m, 2H). MS (APCI+) m/z 581 (M+H)⁺.

### Example 55

### ethyl 3-{[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]amino}-3-oxopropanoate

### Example 55a

### ethyl 3-({trans-4-[(5-bromo-1-methyl-2-oxo-1,2-dihydropyridin-4-yl)oxy]cyclohexyl} amino)-3 -oxopropanoate

A mixture of 2-(3*H*-[1 ,2,3]triazolo[4,5-b]pyridin-3-yl)-1,1 ,3,3-tetramethylisouronium hexafluorophosphate(V) (0.338 g, 0.889 mmol) and ethyl hydrogen malonate (0.157 g, 1.19 mmol) in tetrahydrofuran (5.33 mL)/ *N,N*-dimethylformamide (0.592 mL) at ambient temperature under nitrogen was treated with Example 1e (0.20 g, 0.59 mmol), and then treated with triethylamine (0.248 mL, 1.78 mmol). The mixture was stirred at ambient temperature for 2 hours and partitioned with water and ethyl acetate adjusting the pH to 8 with 5% aqueous sodium bicarbonate. The organic layer was washed with aqueous saturated sodium chloride, dried over anhydrous sodium sulfate, filtered, and concentrated. Purification of the residue by chromatography (silica gel, 25-60% of 3:1 ethyl acetate/ethanol in heptanes) afforded the title compound as a white solid (0.014 g, 57% yield).

### Example 55b

### ethyl 3-{[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]amino)-3-oxopropanoate

Example 3c (0.148 g, 0.371 mmol), Example 55a (0.14 g, 0.337 mmol), tris(dibenzylideneacetone)dipalladium(0) (9.26 mg, 10.11 µmol), 1,3,5,7-tetramethyl-6-phenyl-2,4,8-trioxa-6-phosphaadamantane (9.85 mg, 0.034 mmol) and sodium carbonate (0.143 g, 1.348 mmol) were combined and sparged with argon for 15 minutes. Meanwhile a solution of 4:1 tetrahydrofuran/water (3.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a light yellow foam (0.16 g, 75% yield). ¹H NMR (500 MHz, DMSO-*d*₆) δ 8.03 (d, J = 7.5 Hz, 1H), 7.61 (s, 1H), 7.32 - 7.25 (m, 2H), 7.03 - 6.96 (m, 2H), 6.21 (dd, J = 8.4, 0.5 Hz, 1H), 6.01 (s, 1H), 4.95 (s, 1H), 4.42 (m, 1H), 4.06 (q, J = 7.1 Hz, 2H), 3.49 (m, 1H), 3.41 (s, 3H), 3.18 (s, 2H), 2.03 (s, 6H), 1.98 (m, 2H), 1.71 (m, 2H), 1.41 (s, 6H), 1.33 (m, 4H), 1.18 (t, J = 7.1, 3H). MS (ESI+) m/z 609 (M+H)⁺.

### Example 56

### N-[trans-4-(15-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -2-oxopropanamide

### Example 56a

### N- {trans-4-[(5 -bromo-1 -methyl-2-oxo-1,2-dihydropyridin-4-yl)oxy]cyclohexyl} -2-oxopropanamide

A mixture of pyruvic acid (0.104 g, 1.185 mmol) and 2-(3*H*-[1,2,3]triazolo[4,5-b]pyridin-3-yl)-1,1,3,3-tetramethylisouronium hexafluorophosphate(V) (0.338 g, 0.889 mmol) in tetrahydrofuran (4.0 mL)/dimethylformamide (0.3 mL) was treated with Example 1e (0.20 g, 0.59 mmol) followed by triethylamine (0.248 mL, 1.777 mmol). The mixture was stirred at ambient temperature under nitrogen for 2 hours and partitioned with water and ethyl acetate adjusting the pH to 8 with 5% aqueous sodium bicarbonate. The organic layer was washed with aqueous saturated sodium chloride, dried over anhydrous sodium sulfate, filtered, and concentrated. Purification of the residue by chromatography (silica gel, 25-60% of 3:1 ethyl acetate/ethanol in heptanes) afforded the title compound as a white solid (0.123 g, 56% yield).

### Example 56b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -2-oxopropanamide

Example 3c (0.059 g, 0.148 mmol), Example 56a (0.05 g, 0.135 mmol), tris(dibenzylideneacetone)dipalladium(0) (3.70 mg, 4.04 µmol), 1,3,5,7-tetramethyl-6-phenyl-2,4,8-trioxa-6-phosphaadamantane (3.94 mg, 0.013 mmol) and sodium carbonate (0.057 g, 0.539 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-8% methanol in dichloromethane) afforded the title compound (0.011 g, 13% yield). ¹H NMR (500 MHz, DMSO-*d*₆) δ 8.39 (d, J = 8.0 Hz, 1H), 7.58 (s, 1H), 7.30 - 7.20 (m, 2H), 6.97 (d, J = 9.1 Hz, 2H), 6.19 (d, J = 8.5 Hz, 1H), 5.94 (s, 1H), 4.91 (s, 1H), 4.32 (tt, J = 9.7, 4.0 Hz, 1H), 3.55 - 3.43 (m, 1H), 3.38 (s, 3H), 2.30 (s, 3H), 2.00 (s, 6H), 1.97 (m, 2H), 1.74 - 1.65 (m, 2H), 1.53 - 1.41 (m, 2H), 1.39 (s, 6H), 1.33 - 1.21 (m, 2H). MS (ESI+) m/z 565 (M+H)⁺.

### Example 57

### N-[trans-4-(15-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2,2-dimethylpropanamide

### Example 57a

### N-{trans-4-[(5-bromo-1-methyl-2-oxo-1,2-dihydropyridin-4-yl)oxy]cyclohexyl}-2,2-dimethylpropanamide

To a solution of Example 1e (0.3 g, 0.889 mmol) and triethylamine (0.248 mL, 1.777 mmol) in dichloromethane (8.89 mL) was added dropwise pivaloyl chloride (0.107 g, 0.889 mmol). The mixture was stirred at ambient temperature under nitrogen for 1 hour and partitioned with water. The organic layer was dried over anhydrous sodium sulfate, filtered, and concentrated. Trituration in 9:1 heptane/ethyl acetate afforded the title compound as a white solid (0.294 g, 82% yield).

### Example 57b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2,2-dimethylpropanamide

Example 3c (0.057 g, 0.143 mmol), Example 57a (0.05 g, 0.130 mmol), tris(dibenzylideneacetone)dipalladium(0) (3.56 mg, 3.89 µmol), 1,3,5,7-tetramethyl-6-phenyl-2,4,8-trioxa-6-phosphaadamantane (3.79 mg, 0.013 mmol) and sodium carbonate (0.055 g, 0.519 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a white foam (0.061 g, 77% yield). ¹H NMR (400 MHz, DMSO-*d*₆) δ 7.57 (s, 1H), 7.27 - 7.21 (m, 2H), 7.09 (d, J = 7.6 Hz, 1H), 6.98 - 6.92 (m, 2H), 6.20 - 6.14 (m, 1H), 5.92 (s, 1H), 4.89 (s, 1H), 4.30 (dq, J = 9.9, 5.4, 4.7 Hz, 1H), 3.45 (m, 1H), 3.37 (s, 3H), 1.99 (s, 6H), 1.96 (m, 2H), 1.70 - 1.61 (m, 2H), 1.37 (s, 6H), 1.35 - 1.16 (m, 4H), 1.03 (s, 9H). MS (ESI-) m/z 577 (M-H)⁺.

### Example 58

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-N, 1-dimethylcyclopropane-1-carboxamide

### Example 58a

### N-{trans-4-[(5-bromo-1-methyl-2-oxo-1,2-dihydropyridin-4-yl)oxy]cyclohexyl}-N,1-dimethylcyclopropane-1-carboxamide

To a solution of Example 16a (0.080 g, 0.23 mmol) and triethylamine (0.095 mL, 0.68 mmol) in dichloromethane (2.28 mL) was added dropwise 1-methylcyclopropanecarbonyl chloride (0.027 g, 0.23 mmol). The mixture was stirred at ambient temperature under nitrogen for 2 hours, diluted with water, and stirred for 10 minutes. The organic layer was dried over anhydrous sodium sulfate, filtered, and concentrated. Purification of the residue by trituration in a minimal volume of 9:1 heptane/ethyl acetate afforded the title compound as a powder (0.050 g, 53% yield).

### Example 58b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-N, 1-dimethylcyclopropane-1-carboxamide

Example 3c (0.055 g, 0.138 mmol), Example 58a (0.05 g, 0.126 mmol), tris(dibenzylideneacetone)dipalladium(0) (3.46 mg, 3.78 µmol), 1,3,5,7-tetramethyl-6-phenyl-2,4,8-trioxa-6-phosphaadamantane (3.68 mg, 0.013 mmol) and sodium carbonate (0.053 g, 0.503 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a white foam (0.057 g, 72% yield). ¹H NMR (500 MHz, DMSO-*d*₆) δ 7.60 (s, 1H), 7.32 - 7.24 (m, 2H), 6.98 (d, J = 9.0 Hz, 2H), 6.20 (d, J = 8.5 Hz, 1H), 6.00 (s, 1H), 4.93 (s, 1H), 4.39 (m, 1H), 4.11 - 4.02 (m, 1H), 3.40 (s, 3H), 2.75 (s, 3H), 2.09 (d, J = 11.4 Hz, 2H), 2.02 (s, 6H), 1.85 - 1.66 (m, 2H), 1.54 (m, 2H), 1.41 (s, 6H), 1.29 (m, 2H), 1.17 (s, 3H), 0.75 (q, J = 4.2 Hz, 2H), 0.51 (q, J = 4.4 Hz, 2H). MS (ESI-) m/z 589 (M-H)⁺.

### Example 59

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N,2,2-trimethylpropanamide

### Example 59a

### N-{trans-4-[(5-bromo-1-methyl-2-oxo-1,2-dihydropyridin-4-yl)oxy]cyclohexyl}-N,2,2-trimethylpropanamide

To a solution of Example 16a (0.08 g, 0.227 mmol) and triethylamine (0.095 mL, 0.682 mmol) in dichloromethane (2.275 mL) was added dropwise pivaloyl chloride (0.027 g, 0.227 mmol). The mixture was stirred at ambient temperature under nitrogen for 1 hour, diluted with water, and stirred for 10 minutes. The organic layer was dried over anhydrous sodium sulfate, filtered, and concentrated. Purification of the residue by trituration in a minimal volume of 9:1 heptane/ethyl acetate afforded the title compound as a powder (0.038 g, 40% yield).

### Example 59b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N,2,2-trimethylpropanamide

Example 3c (0.039 g, 0.096 mmol), Example 59a (0.035 g, 0.088 mmol), tris(dibenzylideneacetone)dipalladium(0) (2.408 mg, 2.63 µmol), 1,3,5,7-tetramethyl-6-phenyl-2,4,8-trioxa-6-phosphaadamantane (2.56 mg, 8.76 µmol) and sodium carbonate (0.037 g, 0.351 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a white foam (0.037 g, 68% yield). ¹H NMR (400 MHz, DMSO-*d*₆) δ 7.56 (s, 1H), 7.28 - 7.21 (m, 2H), 6.95 (d, J = 9.1 Hz, 2H), 6.17 (d, J = 8.5 Hz, 1H), 5.96 (s, 1H), 4.89 (s, 1H), 4.35 (m, 1H), 3.94 (m, 1H), 3.37 (s, 3H), 2.70 (s, 3H), 2.04 (d, J = 11.7 Hz, 2H), 1.99 (s, 6H), 1.74 (m, 2H), 1.49 (d, J = 12.4 Hz, 2H), 1.37 (s, 6H), 1.33 - 1.18 (m, 2H), 1.13 (s, 9H). MS (ESI-) m/z 591 (M-H)⁺.

### Example 60

### 2'-(4-fluoro-2,6-dimethylphenoxy)-5'-(2-hydroxypropan-2-yl)-1-methyl-4-([trans-4-(2-oxopyrrolidin-1-yl)cyclohexyl]oxy}[3,3'-bipyridin]-6(1H)-one

Example 24a (37 mg, 0.10 mmol), Example 1j (40 mg, 0.10 mmol), sodium carbonate (37 mg, 0.35 mmol), tris(dibenzylideneacetone)dipalladium (2.8 mg, 3.0 µmol) and 1,3,5,7-tetramethyl-6-phenyl-2,4,8-trioxa-6-phosphaadamantane (2.6 mg, 9.00 µmol) were combined in a microwave tube. The reaction mixture was purged with nitrogen for 15 minutes. The mixture of tetrahydrofuran (2 mL)/water (0.5 mL) was purged with nitrogen for 15 minutes also and transferred to the reaction vessel. The reaction mixture was heated at 60 °C for 3 hours, cooled to ambient temperature, and partitioned with ethyl acetate and water. The organic layer was washed with saturated aqueous sodium chloride, dried with anhydrous sodium sulfate, treated with 3-mercaptopropyl functionalized silica gel, filtered, and concentrated. The residue was purified by flash chromatography (silica gel, 4-8% methanol in dichloromethane) to provide the title compound (37 mg, 66% yield). ¹H NMR (400 MHz, DMSO-*d*₆) δ 8.03 (d, *J=* 2.5 Hz, 1H), 7.72 (d, *J=* 2.5 Hz, 1H), 7.69 (s, 1H), 6.91 (d, *J=* 9.1 Hz, 2H), 6.00 (s, 1H), 5.10 (s, 1H), 4.41 -4.31 (m, 1H), 3.73 - 3.64 (m, 1H), 3.39 (s, 3H), 3.25 (t, *J=* 6.9 Hz, 2H), 2.17 (t, *J=* 8.0 Hz, 2H), 2.09 - 2.01 (m, 2H), 1.97 (s, 6H), 1.93 - 1.83 (m, 2H), 1.70 - 1.50 (m, 4H), 1.42 (s, 6H), 1.33 - 1.19 (m, 2H). (ESI+) m/z 564 (M+H)⁺.

### Example 61

### trans-4-(15-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxylic acid

### Example 61a

### ethyl trans-4-((5-bromo-1-methyl-2-oxo-1,2-dihydropyridin-4-yl)oxy)cyclohexane-1-carboxylate

A mixture of Example 1c (0.824 g, 4 mmol) and trans-ethyl 4-hydroxycyclohexanecarboxylate (0.758 g, 4.40 mmol) in tetrahydrofuran (20 mL) was cooled to 0 °C. To this solution was added potassium 2-methylpropan-2-olate (5.20 mL, 5.20 mmol). The reaction mixture was stirred at ambient temperature for 2 hours. The reaction mixture was quenched with saturated aqueous ammonium chloride and partitioned between water and ethyl acetate. The aqueous layer was extracted with additional ethyl acetate three times. The combined organic layers were washed with saturated aqueous sodium chloride, dried over anhydrous magnesium sulfate, and filtered. The residue was purified by column chromatography on silica gel eluting with 100% ethyl acetate to give the title compound as the first-eluting isomer (0.71 g, 1.982 mmol, 49.5 % yield).

### Example 61b

### ethyl trans-4-((5-(2-(4-fluoro-2,6-dimethylphenoxy)-5-(2-hydroxypropan-2-yl)phenyl)-1-methyl-2-oxo-1,2-dihydropyridin-4-yl)oxy)cyclohexane-1-carboxylate

A mixture of Example 3c (0.156 g, 0.390 mmol), Example 61a (0.107 g, 0.3 mmol), tetrakis(triphenylphosphine)palladium(0) (0.017 g, 0.015 mmol), and cesium fluoride (0.137 g, 0.900 mmol) in dimethoxyethane (2 mL) and methanol (1 mL) was heated at 120 °C for 40 minutes under microwave heating conditions. The reaction mixture was loaded onto a 15 g silica gel cartridge, and dried. It was then mounted onto a 12 g silica gel column, eluted with 15:85 methanol: ethyl acetate to give crude product, which was then purified by reverse phase Preparative HPLC (C18, acetonitrile/water (0.1% trifluoroacetic acid), 20-80% gradient) to give the title compound (0.14 g, 0.254 mmol, 85 % yield).

### Example 61c

### trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexane-1 -carboxylic acid

A mixture of Example 61b (0.14 g, 0.25 mmol) and sodium hydroxide (0.508 mL, 1.02 mmol) in dioxane (3 mL) was heated at 50 °C overnight. The solvent was partially evaporated, and the residue was then purified by reverse phase Preparative HPLC (C18, acetonitrile/Water (0.1% trifluoroacetic acid), 20-80% gradient) to give the title compound (0.11 g, 0.210 mmol, 83 % yield) ¹H NMR (400 MHz, DMSO-*d*₆) δ 7.59 (s, 1H), 7.30 - 7.21 (m, 2H), 6.96 (d, J = 9.1 Hz, 2H), 6.19 (d, J = 9.1 Hz, 1H), 5.96 (s, 1H), 4.37 (dt, J = 9.9, 5.5 Hz, 1H), 3.38 (s, 3H), 2.16 - 1.89 (m, 9H), 1.84 - 1.75 (m, 2H), 1.56 - 1.41 (m, 2H), 1.38 (s, 6H), 1.25 (dt, J = 11.8, 8.6 Hz, 2H). ). MS (ESI+) m/z 524.1 (M+H)⁺.

### Example 62

### 5-[2-(4-fluoro-2,6-dimethylphenoxy)-5-(2-hydroxypropan-2-yl)phenyl]-1-methyl-4-{[trans-4-(pyrrolidine-1-carbonyl)cyclohexyl]oxy}pyridin-2(1H)-one

A mixture of Example 61c (0.03 g, 0.057 mmol), pyrrolidine (8.15 mg, 0.115 mmol), and 2-(3*H*-[1,2,3]triazolo[4,5-b]pyridin-3-yl)-1,1,3,3-tetramethylisouronium hexafluorophosphate(V) (0.033 g, 0.086 mmol) in *N,N*-dimethylformamide (1 mL) was stirred at ambient temperature for 6 hours. The reaction mixture was purified by reverse phase Preparative HPLC (C18, acetonitrile/water (0.1% trifluoroacetic acid), 20-80% gradient) to give the title compound (0.019 g, 0.033 mmol, 57.5 % yield) ¹H NMR (400 MHz, DMSO-*d*₆) δ 7.57 (s, 1H), 7.28 - 7.19 (m, 2H), 6.95 (d, J = 9.1 Hz, 2H), 6.17 (d, J = 8.5 Hz, 1H), 5.96 (s, 1H), 4.33 (tt, J = 10.3, 4.1 Hz, 1H), 3.36-3.39 (m, 5H), 3.21 (t, J = 6.8 Hz, 2H), 2.29 (tt, J = 11.2, 3.5 Hz, 1H), 2.06 - 1.99 (m, 2H), 1.99 (s, 6H), 1.87 - 1.61 (m, 6H), 1.55 - 1.40 (m, 2H), 1.37 (s, 6H), 1.28 - 1.13 (m, 2H). MS (ESI+) m/z 577.0 (M+H)⁺.

### Example 63

### trans-N-ethyl-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxamide

A mixture of Example 61c (0.03 g, 0.057 mmol), 2.0 N ethanamine in tetrahydrofuran (0.143 mL, 0.286 mmol), and 2-(3*H*-[1,2,3]triazolo[4,5-b]pyridin-3-yl)-1,1,3,3-tetramethylisouronium hexafluorophosphate(V) (0.065 g, 0.172 mmol) in *N,N-*dimethylformamide (1 mL) was stirred at ambient temperature overnight. The reaction mixture was purified by reverse phase Preparative HPLC (C18, acetonitrile/water (0.1% trifluoroacetic acid), 20-80% gradient) to give the title compound (0.016 g, 0.029 mmol, 50.7 % yield). ¹H NMR (400 MHz, DMSO-*d*₆) δ 7.65 (t, J = 5.5 Hz, 1H), 7.56 (s, 1H), 7.28 - 7.20 (m, 2H), 6.95 (d, J = 9.1 Hz, 2H), 6.17 (d, J = 8.2 Hz, 1H), 5.93 (s, 1H), 4.31 (tt, J = 10.2, 4.1 Hz, 1H), 3.37 (s, 3H), 2.99 (qd, J = 7.2, 5.4 Hz, 2H), 1.94-2.01 (m, 9H), 1.70 - 1.61 (m, 2H), 1.55 - 1.40 (m, 2H), 1.37 (s, 6H), 1.15 (tdd, J = 14.2, 12.6, 11.5, 4.1 Hz, 2H), 0.94 (t, J = 7.2 Hz, 3H). MS (ESI+) m/z 551.3 (M+H)⁺.

### Example 64

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N-(2-methoxyethyl)-1-methylcyclopropane-1-carboxamide

### Example 64a

### 5-bromo-4-({trans-4-[(2-methoxyethyl)amino]cyclohexyl}oxy)-1-methylpyridin-2(1H)-one

A mixture of 1-bromo-2-methoxyethane (0.082 g, 0.59 mmol), Example 1e (0.20 g, 0.59 mmol) and potassium carbonate (0.327 g, 2.37 mmol) in dimethyl sulfoxide (1.97 mL) was stirred for 18 hours at 70 °C, cooled to ambient temperature, and partitioned between ethyl acetate and water. The organic layer was washed twice with saturated aqueous sodium chloride, dried over anhydrous sodium sulfate, filtered, and concentrated to afford the crude title compound (0.156 g, 44% yield) which was used without purification.

### Example 64b

### N- {trans-4-[(5-bromo-1-methyl-2-oxo-1,2-dihydropyridin-4-yl)oxy]cyclohexyl} -N-(2-methoxyethyl)-1-methylcyclopropane-1-carboxamide

To a solution of Example 64a (0.20 g, 0.56 mmol) and triethylamine (0.155 mL, 1.11 mmol) in dichloromethane (5.57 mL) was added dropwise 1-methylcyclopropanecarbonyl chloride (0.079 g, 0.668 mmol). The mixture was stirred at ambient temperature under nitrogen for 1 hour and concentrated. Purification of the residue by chromatography (silica gel, 1-6% methanol in dichloromethane) afforded the title compound as a foam (0.075 g, 27% yield).

### Example 64c

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N-(2-methoxyethyl)-1-methylcyclopropane-1-carboxamide

Example 3c (0.035 g, 0.087 mmol), Example 64b (0.035 g, 0.079 mmol), tris(dibenzylideneacetone)dipalladium(0) (2.179 mg, 2.379 µmol), 1,3,5,7-tetramethyl-6-phenyl-2,4,8-trioxa-6-phosphaadamantane (2.318 mg, 7.93 µmol) and sodium carbonate (0.034 g, 0.317 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a foam (0.037 g, 71% yield). ¹H NMR (400 MHz, DMSO-*d*₆) δ 7.56 (s, 1H), 7.28 - 7.20 (m, 2H), 6.98 - 6.93 (m, 2H), 6.17 (d, J = 8.5 Hz, 1H), 5.98 (s, 1H), 4.89 (s, 1H), 4.42 (m, 1H), 4.00 (m, 1H), 3.37 (s, 3H), 3.27 - 3.24 (m, 2H), 3.20 (s, 3H), 2.06 (d, J = 12.0 Hz, 2H), 1.99 (s, 6H), 1.77 (q, J = 12.4 Hz, 2H), 1.56 (d, J = 12.1 Hz, 2H), 1.38 (s, 6H), 1.35 - 1.17 (m, 4H), 1.14 (s, 3H), 0.73 (q, J = 4.2 Hz, 2H), 0.51 - 0.42 (m, 2H). MS (ESI-) m/z 633 (M-H)⁺.

### Example 65

### N[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -N-(3-methoxypropyl)-1-methylcyclopropane-1-carboxamide

### Example 65a

### 5-bromo-4-({trans-4-[(3 -methoxypropyl) amino] cyclohexyl} oxy)-1 -methylpyridin-2(1H)-one

A mixture of 1-bromo-3-methoxypropane (0.091 g, 0.59 mmol), Example 1e (0.20 g, 0.59 mmol) and potassium carbonate (0.327 g, 2.369 mmol) in dimethyl sulfoxide (1.97 mL) was stirred for 18 hours at 70 °C, cooled to ambient temperature, and partitioned between ethyl acetate and water. The organic layer was washed twice with saturated aqueous sodium chloride, dried over anhydrous sodium sulfate, filtered, and concentrated to afford crude title compound (0.16 g, 43% yield) which was used without purification.

### Example 65b

### N-{trans-4-[(5-bromo-1-methyl-2-oxo-1,2-dihydropyridin-4-yl)oxy]cyclohexyl}-N(3-methoxypropyl)-1-methylcyclopropane-1-carboxamide

To a solution of Example 65a (0.20 g, 0.54 mmol) and triethylamine (0.149 mL, 1.072 mmol) in dichloromethane (5.36 mL) was added dropwise 1-methylcyclopropanecarbonyl chloride (0.076 g, 0.64 mmol). The mixture was stirred at ambient temperature under nitrogen for 1 hour and concentrated. Purification of the residue by chromatography (silica gel, 1-6% methanol in dichloromethane) afforded the title compound as a foam (0.066 g, 27% yield).

### Example 65c

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -N-(3-methoxypropyl)-1-methylcyclopropane-1-carboxamide

Example 3c (0.064 g, 0.159 mmol), Example 65b (0.066 g, 0.145 mmol), tris(dibenzylideneacetone)dipalladium(0) (3.98 mg, 4.35 µmol), 1,3,5,7-tetramethyl-6-phenyl-2,4,8-trioxa-6-phosphaadamantane (4.24 mg, 0.014 mmol) and sodium carbonate (0.061 g, 0.580 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a foam (0.059 g, 59% yield). ¹H NMR (400 MHz, DMSO-*d*₆) δ 7.56 (s, 1H), 7.28 - 7.21 (m, 2H), 6.98 - 6.93 (m, 2H), 6.17 (d, J = 8.4 Hz, 1H), 6.00 (s, 1H), 4.89 (s, 1H), 4.42 (m, 1H), 4.00 (m, 1H), 3.37 (s, 3H), 3.26 (d, J = 6.2 Hz, 2H), 3.18 (s, 3H), 2.07 (d, J = 11.5 Hz, 2H), 1.99 (s, 6H), 1.76 (q, J = 12.5 Hz, 2H), 1.60 (m, 4H), 1.38 (s, 6H), 1.34 - 1.19 (m, 4H), 1.14 (s, 3H), 0.72 (q, J = 4.2 Hz, 2H), 0.50 - 0.43 (m, 2H). MS (ESI-) m/z 647 (M-H)⁺.

### Example 66

### ethyl N-[trans-4-({{5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-N-(1-methylcyclopropane-1-carbonyl)glycinate

### Example 66a

### ethyl N- {trans-4-[(5-bromo-1-methyl-2-oxo-1,2-dihydropyridin-4-yl)oxy]cyclohexyl}glycinate

A mixture of ethyl bromoacetate (0.099 g, 0.59 mmol), Example 1e (0.20 g, 0.59 mmol) and potassium carbonate (0.327 g, 2.369 mmol) in dimethyl sulfoxide (1.97 mL) was stirred for 18 hours at 70 °C, cooled to ambient temperature, and partitioned between ethyl acetate and water. The organic layer was washed twice with saturated aqueous sodium chloride, dried over anhydrous sodium sulfate, filtered, and concentrated to afford crude title compound (0.19 g, 33% yield) which was used without purification.

### Example 66b

### ethyl N- {trans-4-[(5-bromo-1 -methyl-2-oxo-1,2-dihydropyridin-4-yl)oxy]cyclohexyl} -N-(1 - methylcyclopropane-1-carbonyl)glycinate

To a solution of Example 66a (0.2 g, 0.516 mmol) and triethylamine (0.144 mL, 1.033 mmol) in dichloromethane (5.16 mL) was added dropwise 1-methylcyclopropanecarbonyl chloride (0.073 g, 0.620 mmol). The mixture was stirred at ambient temperature under nitrogen for 1 hour and concentrated. Purification of the residue by chromatography (silica gel, 1-6% methanol in dichloromethane) afforded the title compound as a foam (0.113 g, 45% yield).

### Example 66c

### ethyl N-[trans-4-({{5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-N-(1-methylcyclopropane-1-carbonyl)glycinate

Example 3c (0.106 g, 0.265 mmol), Example 66b (0.113 g, 0.241 mmol), tris(dibenzylideneacetone)dipalladium(0) (6.61 mg, 7.22 µmol), 1,3,5,7-tetramethyl-6-phenyl-2,4,8-trioxa-6-phosphaadamantane (7.04 mg, 0.024 mmol) and sodium carbonate (0.102 g, 0.963 mmol) were combined and sparged with argon for 15 minutes. Meanwhile a solution of 4:1 tetrahydrofuran/water (2.2 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a white foam (0.123 g, 74% yield). ¹H NMR (501 MHz, DMSO-*d*₆) δ 7.58 (s, 1H), 7.30 - 7.22 (m, 2H), 6.97 (d, J = 9.1 Hz, 2H), 6.19 (d, J = 8.5 Hz, 1H), 5.92 (s, 1H), 4.91 (s, 1H), 4.37 (m, 1H), 4.13 (m, 1H), 4.05 - 3.96 (m, 2H), 3.80 (s, 2H), 3.38 (s, 3H), 2.06 (d, J = 11.7 Hz, 2H), 2.01 (s, 6H), 1.68 (s, 4H), 1.39 (s, 6H), 1.32 (s, 2H), 1.19 (s, 3H), 1.14 (t, J = 7.2 Hz), 0.74 (q, J = 4.2 Hz, 2H), 0.56 - 0.49 (m, 2H). MS (ESI-) m/z 661 (M-H)⁺.

### Example 67

### 5-[2-(4-fluoro-2,6-dimethylphenoxy)-5-(2-hydroxypropan-2-yl)phenyl]-4-{[trans-4-(2-hydroxypropan-2-yl)cyclohexyl] oxy} -1 -methylpyridin-2(1H)-one

### Example 67a

### 5-bromo-4- {[trans-4-(2-hydroxypropan-2-yl)cyclohexyl]oxy)-1-methylpyridin-2(1H)-one

Example 61a (0.08 g, 0.223 mmol) in tetrahydrofuran (1 mL) was treated with 3.0 M methylmagnesium bromide in tetrahydrofuran (0.372 mL, 1.12 mmol). The reaction mixture was stirred at ambient temperature for 6 hours. The reaction mixture was quenched with saturated aqueous ammonium chloride, and extracted with ethyl acetate. The aqueous layer was extracted with additional ethyl acetate three times. The combined organic layers were washed with saturated aqueous sodium chloride, dried over anhydrous magnesium sulfate, filtered, and concentrated. The crude product was then purified by flash column chromatography on silica gel (9:1 ethyl acetate/heptanes) to give the title compound (0.070 g, 0.203 mmol, 91 % yield).

### Example 67b

### 5-[2-(4-fluoro-2,6-dimethylphenoxy)-5-(2-hydroxypropan-2-yl)phenyl]-4-{[trans-4-(2-hydroxypropan-2-yl)cyclohexyl] oxy} -1 -methylpyridin-2(1H)-one

Example 67b was prepared according to the procedure used for the preparation of Example 61b, substituting Example 67a for Example 61a, to provide the title compound. ¹H NMR (501 MHz, DMSO-*d*₆) δ 7.57 (s, 1H), 7.29 - 7.21 (m, 2H), 6.96 (d, J = 9.1 Hz, 2H), 6.17 (d, J = 8.5 Hz, 1H), 5.91 (s, 1H), 4.27 - 4.22 (m, 1H), 3.37 (s, 3H), 2.00-2.05 (m, 9H), 1.76 (d, J = 8.6 Hz, 2H), 1.38 (s, 6H), 1.12 (d, J = 7.3 Hz, 6H), 0.99 (s, 6H). (ESI+) m/z 538.3 (M+H)⁺.

### Example 68

### cis-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carbonitrile

### Example 68a

### 4-((5-bromo-1-methyl-2-oxo-1,2-dihydropyridin-4-yl)oxy)cyclohexane-1-carbonitrile

Example 68a was prepared according to the procedure used for the preparation of Example 61a, substituting 4-hydroxycyclohexanecarbonitrile for trans-ethyl 4-hydroxycyclohexanecarboxylate, to provide the title compound.

### Example 68b

### cis-4-({5-[2-(4-fluoro-2,6-dimethylphenoxy)-5 -(2-hydroxypropan-2-yl)phenyl]-1-methyl-2-oxo-1,2-dihydropyridin-4-yl} oxy)cyclohexane-1-carbonitrile

Example 68b was prepared according to the procedure used for the preparation of Example 61b, substituting Example 68a for Example 61a. The title compound was isolated by reverse phase Preparative HPLC (C18, acetonitrile/water (0.1% trifluoroacetic acid), 20-80% gradient) as the first isomer to elute. ¹H NMR (501 MHz, DMSO-*d*₆) δ 7.61 (s, 1H), 7.27-7.29 (m, 2H), 6.97 (d, J = 9.1 Hz, 2H), 6.21 (dt, J = 8.5, 1.0 Hz, 1H), 5.96 (s, 1H), 4.52 (dd, J = 6.2, 3.3 Hz, 1H), 3.38 (s, 3H), 2.88 - 2.81 (m, 1H), 2.01 (s, 6H), 1.75 - 1.58 (m, 6H), 1.51 (dd, J = 11.7, 7.7 Hz, 2H), 1.39 (s, 6H). (ESI+) m/z 505.1 (M+H)⁺.

### Example 69

### trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carbonitrile

The title compound was isolated as the second eluting isomer from the preparation of Example 68b. ¹H NMR (501 MHz, DMSO-*d*₆) δ 7.60 (s, 1H), 7.30 - 7.21 (m, 2H), 6.96 (d, J = 9.1 Hz, 2H), 6.19 (d, J = 8.6 Hz, 1H), 5.98 (s, 1H), 4.51 (tt, J = 7.5, 3.3 Hz, 1H), 3.38 (s, 3H), 2.73 (tt, J = 8.2, 3.9 Hz, 1H), 1.99 (s, 6H), 1.84 (dq, J = 12.2, 3.7 Hz, 2H), 1.75 (dq, J = 12.7, 3.7 Hz, 2H), 1.60 (dtd, J = 12.5, 8.5, 3.5 Hz, 2H), 1.44 (dt, J = 13.4, 8.0 Hz, 2H), 1.37 (s, 6H). (ESI+) m/z 505.2 (M+H)⁺.

### Example 70

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-1-(methoxymethyl)cyclopropane-1-carboxamide

### Example 70a

### N- {trans-4-[(5-bromo-1 -methyl-2-oxo-1,2-dihydropyridin-4-yl)oxy]cyclohexyl} -1 - (methoxymethyl)cyclopropane-1-carboxamide

Example 1e (0.07 g, 0.187 mmol), 1-(methoxymethyl)cyclopropanecarboxylic acid (0.027 g, 0.205 mmol), 2-(3*H*-[1,2,3]triazolo[4,5-b]pyridin-3-yl)-1,1,3,3-tetramethylisouronium hexafluorophosphate(V) (0.078 g, 0.205 mmol), dimethyl sulfoxide (1 mL) and *N*-ethyl-*N*-isopropylpropan-2-amine (0.1 mL, 0.573 mmol) were combined and stirred at ambient temperature for 3.25 hours. The reaction mixture was partitioned between ethyl acetate and water, washed with saturated aqueous sodium chloride, dried over anhydrous magnesium sulfate, filtered, and concentrated. The residue was purified by flash chromatography (silica gel, 30 to 100% of a 3:1 mixture of ethyl acetate/ethanol in heptanes) to provide 0.077 g (100% yield) of the title compound.

### Example 70b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-(methoxymethyl)cyclopropane-1-carboxamide

Example 70b was prepared according to the procedure used for the preparation of Example 2h substituting Example 70a for Example 2d and substituting Example 3c for Example 2g. The compound was purified by flash chromatography (amine-functionalized silica gel, 0 to 50% of a 3:1 mixture of ethyl acetate/ethanol in heptanes) to provide the title compound. ¹H NMR (501 MHz, DMSO-*d*₆) δ 7.60 (s, 1H), 7.29 (d, J = 2.3 Hz, 1H), 7.27 (dd, J = 8.5, 2.4 Hz, 1H), 7.14 (d, J = 7.6 Hz, 1H), 6.98 (d, J = 9.1 Hz, 2H), 6.21 (d, J = 8.5 Hz, 1H), 5.96 (s, 1H), 4.93 (s, 1H), 4.39 (dq, J = 12.7, 3.9 Hz, 1H), 3.53 (m, 1H), 3.44 (s, 2H), 3.40 (s, 3H), 3.27 (s, 3H), 2.02 (s, 6H), 1.95 (m, 2H), 1.70 (m, 2H), 1.40 (s, 6H), 1.33 (m, 4H), 0.93 (q, J = 3.8 Hz, 2H), 0.61 (q, J = 3.8 Hz, 2H). MS (ESI+) m/z 629.2 (M+Na)⁺.

### Example 71

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methoxy-2,2-dimethylpropanamide

### Example 71a

### N-{trans-4-[(5-bromo-1-methyl-2-oxo-1,2-dihydropyridin-4-yl)oxy]cyclohexyl}-3-methoxy-2,2-dimethy lpropanamide

Example 71a was prepared according to the procedure used for the preparation of Example 70a, substituting 3-methoxy-2,2-dimethylpropanoic acid for 1-(methoxymethyl)cyclopropanecarboxylic acid to provide the title compound.

### Example 71b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methoxy-2,2-dimethylpropanamide

Example 71b was prepared according to the procedure used for the preparation of Example 2h, substituting Example 71a for Example 2d and substituting Example 3c for Example 2g. The compound was purified by flash chromatography (amine-functionalized silica gel, 0 to 50% of a 3:1 mixture of ethyl acetate/ethanol in heptanes) to provide the title compound. ¹H NMR (500 MHz, DMSO-*d*₆) δ 7.60 (s, 1H), 7.28 (m, 2H), 7.10 (d, J = 7.6 Hz, 1H), 6.99 (d, J = 9.1 Hz, 2H), 6.21 (d, J = 8.4 Hz, 1H), 5.96 (s, 1H), 4.93 (s, 1H), 4.35 (tt, J = 9.0, 3.7 Hz, 1H), 3.50 (m, 2H), 3.40 (s, 3H), 3.28 (s, 2H), 3.23 (s, 3H), 2.02 (s, 6H), 1.98 (m, 2H), 1.68 (m, 2H), 1.41 (s, 6H), 1.37 (m, 2H), 1.27 (m, 2H), 1.02 (s, 6H). MS (ESI+) m/z 631.2 (M+Na)⁺.

### Example 72

### tert-butyl (3-{[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl}bicyclo[1.1.1 ]pentan-1-yl)carbamate

### Example 72a

### tert-butyl [3-({trans-4-[(5-bromo-1-methyl-2-oxo-1,2-dihydropyridin-4-yl)oxy]cyclohexyl} carbamoyl)bicyclo [1.1.1]pentan-1-yl] carbamate

Example 72a was prepared according to the procedure used for the preparation of Example 70a, substituting 3-((tert-butoxycarbonyl)amino)bicyclo[1.1.1]pentane-1-carboxylic acid for 1-(methoxymethyl)cyclopropanecarboxylic acid. After completion of the reaction, the reaction mixture was suspended between ethyl acetate and water with no significant dissolution of the solid in ethyl acetate. The layers were separated with the solid product remaining with the organic layer. The organic layer was washed a second time with water and the layers were separated. The organic layer containing the solid product was concentrated and then triturated with ethyl acetate and filtered. The solid was dried in a vacuum oven at 60°C to provide the title compound.

### Example 72b

### tert-butyl (3-{[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl}bicyclo[1.1.1]pentan-1-yl)carbamate

Example 72b was prepared according to the procedure used for the preparation of Example 2h, substituting Example 72a for Example 2d and substituting Example 3c for Example 2g, to provide the title compound. ¹H NMR (500 MHz, DMSO-*d*₆) δ 7.60 (s, 1H), 7.52 (d, J = 7.6 Hz, 1H), 7.48 (s, 1H), 7.28 (m, 2H), 6.98 (d, J = 9.1 Hz, 2H), 6.21 (d, J = 8.4 Hz, 1H), 5.97 (s, 1H), 4.93 (s, 1H), 4.33 (ddd, J = 12.9, 9.1, 3.6 Hz, 1H), 3.46 (m, 1H), 3.40 (s, 3H), 2.022 (s, 6H), 2.017 (s, 6H), 1.98 (d, J = 5.3 Hz, 2H), 1.68 (m, 2H), 1.40 (s, 6H), 1.37 (s, 9H), 1.32 (m, 2H), 1.25 (ddd, J = 23.2, 12.6, 3.1 Hz, 2H). MS (ESI+) m/z 704.3 (M+H)⁺.

### Example 73

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methyloxetane-3-carboxamide

### Example 73a

### N- {trans-4-[(5 -bromo-1-methyl-2-oxo-1,2-dihydropyridin-4-yl)oxy]cyclohexyl} -3-methyloxetane-3 -carboxamide

Example 73a was prepared according to the procedure used for the preparation of Example 70a, substituting 3-methyloxetane-3-carboxylic acid for 1-(methoxymethyl)cyclopropanecarboxylic acid, to provide the title compound

### Example 73b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methyloxetane-3-carboxamide

Example 73b was prepared according to the procedure used for the preparation of Example 2h, substituting Example 73a for Example 2d and substituting Example 3c for Example 2g. The compound was purified by flash chromatography (amine-functionalized silica gel, 0 to 60% of a 3:1 mixture of ethyl acetate/ethanol in heptanes) to provide the title compound. ¹H NMR (400 MHz, DMSO-*d*₆) δ 7.66 (d, J = 7.4 Hz, 1H), 7.60 (s, 1H), 7.28 (m, 2H), 6.99 (d, J = 9.1 Hz, 2H), 6.21 (d, J = 8.3 Hz, 1H), 5.99 (s, 1H), 4.93 (s, 1H), 4.67 (d, J = 5.9 Hz, 2H), 4.37 (m, 1H), 4.22 (d, J = 5.9 Hz, 2H), 3.51 (m, 1H), 3.40 (s, 3H), 2.02 (s, 6H), 1.99 (m, 2H), 1.72 (m, 2H), 1.45 (s, 3H), 1.41 (s, 6H), 1.32 (m, 4H). MS (ESI+)

### Example 74

### 5-[2-(4-fluoro-2,6-dimethylphenoxy)-5-(2-hydroxypropan-2-yl)phenyl]-4-{[cis-4-(2-hydroxypropan-2-yl)cyclohexyl]oxy} -1 -methylpyridin-2(1H)-one

### Example 74a

### ethyl cis-4-[(5-bromo-1-methyl-2-oxo-1,2-dihydropyridin-4-yl)oxy]cyclohexane-1-carboxylate

Example 74a was isolated as the second eluting isomer in the preparation of Example 61a.

### Example 74b

### ethyl cis-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxylate

Example 74b was prepared according to the procedure used for the preparation of Example 61b, substituting Example 74a for Example 61a, to provide the title compound.

### Example 74c

### 5-[2-(4-fluoro-2,6-dimethylphenoxy)-5-(2-hydroxypropan-2-yl)phenyl]-4-{[cis-4-(2-hydroxypropan-2-yl)cyclohexyl]oxy} -1 -methylpyridin-2(1H)-one

Example 74c was prepared according to the procedure used for the preparation of Example 67a, substituting Example 74b for Example 61a, to provide the title compound. ¹H NMR (501 MHz, DMSO-*d*₆) δ 7.58 (s, 1H), 7.32 (d, J = 2.4 Hz, 1H), 7.22 (dd, J = 8.7, 2.4 Hz, 1H), 6.97 (d, J = 9.1 Hz, 2H), 6.18 (d, J = 8.6 Hz, 1H), 5.89 (s, 1H), 4.65 (s, 1H), 3.38 (s, 3H), 2.00 (s, 6H), 1.90 (d, J = 13.8 Hz, 2H), 1.35-1.41 (m, 8H), 1.11 (dd, J = 13.5, 10.9 Hz, 1H), 0.92 - 0.80 (m, 2H), 0.72 (s, 6H). (ESI+) m/z 538.3 (M+H)⁺.

### Example 75

### cis-4-({5-[2-(4-fluoro-2,6-dimethylphenoxy)-5 -(2-hydroxypropan-2-yl)phenyl] -1-methyl-2-oxo-1 ,2-dihydropyridin-4-yl} oxy)cyclohexane-1-carboxylic acid

Example 75 was prepared according to the procedure used for the preparation of Example 61c, substituting Example 74b for Example 61b, to provide the title compound. ¹H NMR (400 MHz, DMSO-*d*₆) δ 7.57 (s, 1H), 7.22-7.25 (m, 2H), 6.94 (d, J = 9.0 Hz, 2H), 6.21 - 6.14 (m, 1H), 5.90 (s, 1H), 4.53 (dd, J = 6.7, 3.6 Hz, 1H), 3.36 (s, 3H), 2.21 (tt, J = 9.7, 3.6 Hz, 1H), 1.97 (s, 6H), 1.69 (dq, J = 15.0, 5.4, 4.9 Hz, 2H), 1.54 (tq, J = 12.8, 4.4, 3.9 Hz, 4H), 1.44 - 1.35 (m, 2H), 1.35 (s, 6H). ). (ESI+) m/z 524.2 (M+H)⁺.

### Example 76

### cis-N-ethyl-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxamide

Example 76 was prepared according to the procedure used for the preparation of Example 63, substituting Example 75 for Example 61c, to provide the title compound. ¹H NMR (501 MHz, DMSO-*d*₆) δ 7.61-7.64 (m, 2H), 7.33 (d, J = 2.3 Hz, 1H), 7.22 (dd, J = 8.6, 2.4 Hz, 1H), 6.95 (d, J = 9.1 Hz, 2H), 6.20 (d, J = 8.6 Hz, 1H), 5.89 (s, 1H), 4.53 (dd, J = 6.7, 3.6 Hz, 1H), 3.37 (s, 3H), 2.97 (qd, J = 7.2, 5.4 Hz, 2H), 2.05 (p, J = 7.3 Hz, 1H), 1.99 (s, 6H), 1.79 (dd, J = 13.8, 4.1 Hz, 2H), 1.51 - 1.44 (m, 2H), 1.34-1.39 (m, 8H), 0.93 (t, J = 7.2 Hz, 3H). (ESI+) m/z 551.2 (M+H)⁺.

### Example 77

### 5-[2-(4-fluoro-2,6-dimethylphenoxy)-5-(2-hydroxypropan-2-yl)phenyl]-1-methyl-4-{[cis-4-(pyrrolidine-1-carbonyl)cyclohexyl]oxy}pyridin-2(1H)-one

Example 77 was prepared according to the procedure used for the preparation of Example 62, substituting Example 75 for Example 61c, to provide the title compound. ¹H NMR (501 MHz, DMSO-*d*₆) δ 7.62 (s, 1H), 7.35 (d, J = 2.4 Hz, 1H), 7.21 (dd, J = 8.7, 2.4 Hz, 1H), 6.95 (d, J = 9.1 Hz, 2H), 6.20 (d, J = 8.6 Hz, 1H), 5.89 (s, 1H), 4.60 (t, J = 3.2 Hz, 1H), 3.37-3.40 (m, 5H), 3.17 (t, J = 6.9 Hz, 2H), 2.42 - 2.34 (m, 1H), 1.99 (s, 6H), 1.88 - 1.76 (m, 4H), 1.70 (p, J = 6.8 Hz, 2H), 1.56 - 1.49 (m, 2H), 1.32-1.37 (m, 8H). (ESI+) m/z 551.2 (M+H)⁺.

### Example 78

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-methylimidazolidine-2,4-dione

### Example 78a

### tert-butyl [2-({trans-4-[(5-bromo-1-methyl-2-oxo-1,2-dihydropyridin-4-yl)oxy]cyclohexyl}amino) -2-oxoethyl]methylcarbamate

A mixture of *N*-(*tert*-butoxycarbonyl)-*N*-methylglycine(0.146 g, 0.770 mmol), 1H-benzo[*d*][1,2,3]triazol-1-olhydrate (0.118 g, 0.770 mmol), and *N*¹-((ethylimino)methylene)-*N*³,*N*³-dimethylpropane-1,3-diamine hydrochloride (0.148 g, 0.770 mmol) in tetrahydrofuran (4.0 mL)/dimethylformamide (0.3 mL) was treated with Example 1e (0.20 g, 0.592 mmol) and then with triethylamine (0.248 mL, 1.777 mmol) . The mixture was stirred at ambient temperature for 18 hours and partitioned with water and ethyl acetate. The organic layer was washed with aqueous saturated sodium chloride, dried over anhydrous sodium sulfate, filtered, and concentrated to afford crude title compound (0.24 g, 86% yield) which was used without purification.

### Example 78b

### N- {trans-4-[(5-bromo-1-methyl-2-oxo-1,2-dihydropyridin-4-yl)oxy]cyclohexyl} -N²-methylglycinamide trifluoroacetate salt

A solution of Example 78a (0.24 g, 0.508 mmol) in dichloromethane (20 mL) was treated with trifluoroacetic acid (5.00 mL), stirred for 1 hour, and concentrated. The residue was azeotroped with toluene (3 x 20 mL) affording the title compound as a trifluoroacetic acid salt (0.25 g, quant. yield). The crude material was used without purification.

### Example 78c

### 3- { trans-4-[(5 -bromo-1 -methyl-2-oxo-1,2-dihydropyridin-4-yl)oxy]cyclohexyl} -1-methylimidazolidine-2,4-dione

A mixture of Example 78b (0.247 g, 0.508 mmol), triethylamine (0.283 mL, 2.032 mmol), and di(1*H*-imidazol-1-yl)methanone(0.165 g, 1.016 mmol) in acetonitrile (5.08 mL) was treated with *N,N-*dimethylpyridin-4-amine (0.124 g, 1.016 mmol) and the mixture was heated at 70 °C for 24 hours. The reaction mixture was cooled to ambient temperature and concentrated. The residue was partitioned between ethyl acetate and water. The organic layer was washed with 1M aqueous hydrochloric acid, washed with saturated aqueous sodium chloride, dried over anhydrous sodium sulfate, filtered, and concentrated to a foam (0.14 g, 63% yield). The material was used without purification.

### Example 78d

### 3-[trans-4-({5-[2-(4-fluoro-2,6-dimethylphenoxy)-5-(2-hydroxypropan-2-yl)phenyl]-1 - methyl-2-oxo-1,2-dihydropyridin-4-yl} oxy)cyclohexyl]-1-methylimidazolidine-2,4-dione

Example 3c (0.066 g, 0.166 mmol), Example 78c (0.06 g, 0.151 mmol), tris(dibenzylideneacetone)dipalladium(0) (4.14 mg, 4.52 µmol), 1,3,5,7-tetramethyl-6-phenyl-2,4,8-trioxa-6-phosphaadamantane (4.40 mg, 0.015 mmol) and sodium carbonate (0.064 g, 0.603 mmol) were combined and sparged with argon for 15 minutes. Meanwhile a solution of 4:1 tetrahydrofuran/water (1.5 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-5 % methanol in dichloromethane) afforded the title compound as a foam (0.062 g, 68% yield). ¹H NMR (400 MHz, DMSO-*d*₆) δ 7.58 (s, 1H), 7.33 - 7.19 (m, 2H), 6.97 (d, J = 9.1 Hz, 2H), 6.19 (d, J = 8.4 Hz, 1H), 5.98 (s, 1H), 4.91 (s, 1H), 4.35 (m, 1H), 3.87 (s, 2H), 3.69 (m, 1H), 3.38 (s, 3H), 2.80 (s, 3H), 2.21 (q, J = 11.9 Hz, 2H), 2.08 (d, J = 12.0 Hz, 2H), 2.01 (s, 6H), 1.58 (d, J = 12.4 Hz, 2H), 1.39 (s, 6H), 1.23 (q, J = 11.1 Hz, 2H). MS (ESI+) m/z 592 (M+H)⁺.

### Example 79

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-hydroxy-2,2-dimethylpentanamide

### Example 79a

### N-{trans-4-[(5-bromo-1-methyl-2-oxo-1,2-dihydropyridin-4-yl)oxy]cyclohexyl}-5-hydroxy-2,2-dimethylpentanamide

Example 1e (169 mg, 0.500 mmol), 3,3-dimethyltetrahydro-2H-pyran-2-one (256 mg, 2.00 mmol), *N,N-*diisopropylethylamine (0.873 mL, 5.00 mmol) and 2M trimethylaluminum in toluene (0.500 mL, 1.00 mmol) were combined in tetrahydrofuran (2 mL). The reaction mixture was stirred at ambient temperature for 72 hours, saturated aqueous sodium bicarbonate was added slowly, and the mixture was extracted with ethyl acetate. The organic layer was washed with saturated aqueous sodium chloride, dried with anhydrous sodium sulfate, filtered, and concentrated. The residue was purified by flash chromatography (silica gel, 20-40% 3:1 ethyl acetate/ethanol in heptanes) to provide the title compound (115 mg, 54% yield).

### Example 79b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-hydroxy-2,2-dimethylpentanamide

Example 79b was prepared according to the procedure used for the preparation of Example 60, substituting Example 79a for Example 24a and substituting Example 3c for Example 1j, to provide the title compound (33 mg, 66% yield). ¹H NMR (400 MHz, DMSO-*d₆*) δ 7.57 (s, 1H), 7.27 - 7.21 (m, 2H), 7.06 (d, *J=* 7.7 Hz, 1H), 6.95 (d, *J=* 9.1 Hz, 2H), 6.17 (d, *J=* 8.4 Hz, 1H), 5.92 (s, 1H), 4.89 (s, 1H), 4.33 - 4.25 (m, 2H), 3.52 - 3.43 (m, 1H), 3.37 (s, 3H), 3.30 - 3.25 (m, 2H), 2.02 - 1.93 (m, 8H), 1.68 - 1.60 (m, 2H), 1.43 - 1.29 (m, 10H), 1.28 - 1.17 (m, 4H), 0.99 (s, 6H). (ESI+) m/z 623 (M+H)⁺.

### Example 80

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]piperidine-2,6-dione

### Example 80a

### 1-{trans-4-[(5-bromo-1-methyl-2-oxo-1,2-dihydropyridin-4-yl)oxy]cyclohexyl}piperidine-2,6-dione

Example 1e (169 mg, 0.500 mmol), glutaric anhydride (68.5 mg, 0.600 mmol), and *N*,*N*-diisopropylethylamine (0.192 mL, 1.10 mmol) were combined in xylene (4 mL). The reaction mixture was heated at 135 °C for 2 hours, cooled to ambient temperature, and concentrated. The residue was treated with sodium acetate (45.1 mg, 0.550 mmol) in acetic anhydride (4 mL). The reaction mixture was heated at 135 °C for 28 hours, cooled to ambient temperature, concentrated, and partitioned with ethyl acetate and saturated aqueous sodium bicarbonate. The organic layer was washed with saturated aqueous sodium chloride, dried with anhydrous sodium sulfate, filtered, and concentrated. The residue was purified by flash chromatography (silica gel, 20-60% 3:1 ethyl acetate/ethanol in heptanes) to provide the title compound (60 mg, 30% yield).

### Example 80b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]piperidine-2,6-dione

Example 80b was prepared according to the procedure used for the preparation of Example 60, substituting Example 80a for Example 24a and substituting Example 3c for Example 1j , to provide the title compound (35 mg, 59% yield). ¹H NMR (400 MHz, DMSO-*d₆*) δ 7.58 (s, 1H), 7.29 - 7.23 (m, 2H), 6.97 (d, *J=* 9.1 Hz, 2H), 6.19 (d, *J=* 8.4 Hz, 1H), 5.95 (s, 1H), 4.91 (s, 1H), 4.42 - 4.26 (m, 2H), 3.38 (s, 3H), 2.54 (t, J= 6.4 Hz, 4H), 2.37 - 2.25 (m, 2H), 2.10 - 2.03 (m, 2H), 2.01 (s, 6H), 1.80 - 1.70 (m, 2H), 1.51 -1.43 (m, 2H), 1.39 (s, 6H), 1.27 - 1.14 (m, 2H). (ESI+) m/z 591 (M+H)⁺.

### Example 81

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione

### Example 81a

### tert-butyl [(2R)-1-({trans-4-[(5-bromo-1-methyl-2-oxo-1,2-dihydropyridin-4-yl)oxy]cyclohexyl} amino)-1-oxopropan-2-yl]methylcarbamate

Example 81a was prepared according to the procedure used for the preparation of Example 78a, substituting *N*-(*tert*-butoxycarbonyl)-N-methyl-D-alanine for *N-*(*tert-*butoxycarbonyl)-*N*-methylglycine to provide the title compound as a white solid. (0.270 g, 0.555 mmol, 94% yield).

### Example 81b

### N-{trans-4-[(5-bromo-1-methyl-2-oxo-1,2-dihydropyridin-4-yl)oxy]cyclohexyl}-N²-methyl-D-alaninamide trifluoroacetate salt

Example 81b was prepared according to the procedure used for the preparation of Example 78b, substituting Example 81a for Example 78a to provide the title compound as a colorless glass. (0.270 g, 0.540 mmol, 97% yield).

### Example 81c

### (5R)-3-{trans-4-[(5-bromo-1-methyl-2-oxo-1,2-dihydropyridin-4-yl)oxy]cyclohexyl} -1,5-dimethylimidazolidine-2,4-dione

Example 81c was prepared according to the procedure used for the preparation of Example 78c, substituting Example 81b for Example 78b to provide the title compound as a white solid. (0.190 g, 0.392 mmol, 73% yield).

### Example 81d

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione

Example 81d was prepared according to the procedure used for the preparation of Example 1k, substituting Example 81c for Example 1f, and substituting Example 3c for Example 1j, to provide the title compound as a white solid. (0.0391 g, 0.068 mmol, 50% yield). Enantiomeric excess (Chiral SFC) = 74%. ¹H NMR (400 MHz, DMSO-*d*₆) δ 7.58 (s, 1H), 7.30 - 7.21 (m, 2H), 6.97 (d, J = 9.1 Hz, 2H), 6.19 (d, J = 8.4 Hz, 1H), 5.98 (s, 1H), 4.91 (s, 1H), 4.43 - 4.28 (m, 1H), 3.94 (q, J = 6.9 Hz, 1H), 3.71 (ddt, J = 12.2, 8.4, 3.8 Hz, 1H), 3.38 (s, 3H), 2.78 (s, 3H), 2.24 - 2.14 (m, 1H), 2.08 (d, J = 11.7 Hz, 2H), 2.01 (s, 6H), 1.59 (d, J = 12.1 Hz, 2H), 1.39 (s, 6H), 1.30 - 1.20 (m, 6H). MS (ESI+) m/z 606.3 (M+H)⁺.

### Example 82

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,3-oxazolidine-2,4-dione

### Example 82a

### N- {trans-4-[(5 -bromo-1 -methyl-2-oxo-1,2-dihydropyridin-4-yl)oxy]cyclohexyl} -2-hydroxyacetamide

Example 82a was prepared according to the procedure used for the preparation of Example 78a, substituting 2-hydroxyacetic acid for *N*-(*tert*-butoxycarbonyl)-*N*-methylglycine to provide the title compound as a white solid. (0.170 g, 0.473 mmol, 57% yield).

### Example 82b

### 3-{trans-4-[(5-bromo-1-methyl-2-oxo-1,2-dihydropyridin-4-yl)oxy]cyclohexyl}-1,3-oxazolidine-2,4-dione

Example 82b was prepared according to the procedure used for the preparation of Example 78c, substituting Example 82a for Example 78b, to provide the title compound as a white solid. (0.146 g, 0.356 mmol, 75% yield).

### Example 82c

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,3-oxazolidine-2,4-dione

Example 82c was prepared according to the procedure used for the preparation of Example 1k, substituting Example 82b for Example 1f, and substituting Example 3c for Example 1j, to provide the title compound as a white solid. (0.0391 g, 0.068 mmol, 50% yield). ¹H NMR (400 MHz, DMSO-*d*₆) δ 7.56 (s, 1H), 7.29 - 7.20 (m, 2H), 6.95 (d, J = 9.1 Hz, 2H), 6.17 (d, J = 8.4 Hz, 1H), 5.99 (s, 1H), 4.89 (s, 1H), 4.71 (s, 2H), 4.36 (td, J = 10.9, 5.3 Hz, 1H), 3.69 (ddt, J = 12.1, 7.3, 3.8 Hz, 1H), 3.37 (s, 3H), 2.12 (ddd, J = 22.1, 14.2, 10.9 Hz, 4H), 1.99 (s, 6H), 1.68 (d, J = 11.8 Hz, 2H), 1.38 (s, 6H), 1.25 (ddd, J = 23.2, 11.2, 6.4 Hz, 2H). MS (ESI+) m/z 579.1 (M+H)⁺.

### Example 83

### (5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione

### Example 83a

### tert-butyl [(2S)-1-({trans-4-[(5-bromo-1-methyl-2-oxo-1,2-dihydropyridin-4-yl)oxy]cyclohexyl}amino)-1-oxopropan-2-yl]methylcarbamate

Example 83a was prepared according to the procedure used for the preparation of Example 78a, substituting *N*-(*tert*-butoxycarbonyl)-*N*-methyl-L-alanine for *N-(tert-*butoxycarbonyl)-*N*-methylglycine to provide the title compound as a white solid. (0.240 g, 0.493 mmol, 83% yield).

### Example 83b

### N-{trans-4-[(5-bromo-1-methyl-2-oxo-1,2-dihydropyridin-4-yl)oxy]cyclohexyl}-N²-methyl-L-alaninamide trifluoroacetate salt

Example 83b was prepared according to the procedure used for the preparation of Example 78b, substituting Example 83a for Example 78a to provide the title compound as a colorless glass. (0.300 g, 0.600 mmol, 100% yield).

### Example 83c

### (5S)-3-{trans-4-[(5-bromo-1-methyl-2-oxo-1,2-dihydropyridin-4-yl)oxy]cyclohexyl}-1,5-dimethylimidazolidine-2,4-dione

Example 83c was prepared according to the procedure used for the preparation of Example 78c, substituting Example 83b for Example 78b, to provide the title compound as a white solid. (0.190 g, 0.433 mmol, 87% yield).

### Example 83d

### (5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione

Example 83d was prepared according to the procedure used for the preparation of Example 1k, substituting Example 83c for Example 1f, and substituting Example 3c for Example 1j, to provide the title compound as a white solid. (0.0391 g, 0.068 mmol, 50% yield). Enantiomeric excess (Chiral SFC) = 77%. ¹H NMR (501 MHz, DMSO-*d*₆) δ 7.58 (s, 1H), 7.30 - 7.22 (m, 2H), 6.97 (d, J = 9.0 Hz, 2H), 6.19 (d, J = 8.5 Hz, 1H), 5.98 (s, 1H), 4.91 (s, 1H), 4.35 (td, J = 10.7, 5.1 Hz, 1H), 3.94 (q, J = 7.0 Hz, 1H), 3.70 (tt, J = 12.2, 3.9 Hz, 1H), 3.38 (s, 3H), 2.78 (s, 3H), 2.19 (tt, J = 12.8, 9.6 Hz, 1H), 2.07 (d, J = 10.1 Hz, 2H), 2.01 (s, 6H), 1.59 (d, J = 12.6 Hz, 2H), 1.39 (s, 6H), 1.23 (t, J = 7.1 Hz, 6H). MS (ESI+) m/z 606.1 (M+H)⁺.

### Example 84

### N-[trans-4-(15-[2-(4-fluoro-2,6-dimethylphenoxy)-5-(2-hydroxypropan-2-yl)phenyl]-1-methyl-2-oxo-1,2-dihydropyridin-4-yl}oxy)-4-methylcyclohexyl]acetamide

### Example 84a

### tert-butyl {trans-4-[(5-bromo-1-methyl-2-oxo-1,2-dihydropyridin-4-yl)oxy]-4-methylcyclohexyl} carbamate

A mixture of Example 1c (0.515 g, 2.5 mmol), tert-butyl (*trans*-4-hydroxy-4-methylcyclohexyl)carbamate (0.631 g, 2.75 mmol), and potassium 2-methylpropan-2-olate (3.25 mL, 3.25 mmol) in tetrahydrofuran (15 mL) was stirred at ambient temperature for 2 hours. The reaction mixture was quenched with saturated aqueous ammonium chloride and partitioned between water and ethyl acetate. The aqueous layer was extracted with additional ethyl acetate three times. The combined organic layers were washed with saturated aqueous sodium chloride, dried over anhydrous magnesium sulfate, and filtered. The residue was purified by reverse phase Preparative HPLC (C18, acetonitrile/water (0.1% trifluoroacetic acid), 20-80% gradient) to give the title compound (0.14 g, 0.337 mmol, 13.48 % yield).

### Example 84b

### 4-[(trans-4-amino-1 -methylcyclohexyl)oxy] -5 -bromo-1-methylpyridin-2(1H)-one hydrochloride

A mixture of Example 84a (0.13 g, 0.313 mmol) and 4.0 N HCl (1.565 mL, 6.26 mmol) in dioxane was stirred at ambient temperature for 6 hours. Filtration of the solid that formed afforded the title compound (0.12 g, 0.271 mmol, 87 % yield).

### Example 84c

### N-{trans-4-[(5-bromo-1-methyl-2-oxo-1,2-dihydropyridin-4-yl)oxy]-4-methylcyclohexyl} acetamide

A mixture of Example 84b (0.04 g, 0.114 mmol), acetyl chloride (0.013 g, 0.171 mmol), and triethylamine (0.058 g, 0.569 mmol) in dichloromethane (2 mL) was stirred at ambient temperature for 2 hours. The reaction mixture was partitioned between water and ethyl acetate. The aqueous layer was extracted with additional ethyl acetate three times. The combined organic layers were washed with saturated aqueous sodium chloride, dried over anhydrous magnesium sulfate, and filtered. The residue was purified by flash chromatography on silica gel to afford the title compound (0.035 g, 0.098 mmol, 86 % yield).

### Example 84d

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-4-methylcyclohexyl]acetamide

Example 84d was prepared according to the procedure used for the preparation of Example 61b, substituting Example 84c for Example 61a, to provide the title compound. ¹H NMR (501 MHz, DMSO-*d*₆) δ 7.68 (d, J = 7.6 Hz, 1H), 7.61 (s, 1H), 7.30 - 7.22 (m, 2H), 6.97 (d, J = 9.0 Hz, 2H), 6.17 (d, J = 8.5 Hz, 1H), 6.00 (s, 1H), 3.58 (dt, J = 8.5, 4.3 Hz, 1H), 3.38 (s, 3H), 2.01 (s, 6H), 1.71-1.75 (m, 5H), 1.71 (s, 1H), 1.69 - 1.59 (m, 2H), 1.44 (s, 3H), 1.37 (s, 6H), 1.29 - 1.13 (m, 4H). (ESI+) m/z 550.8 (M+H)⁺.

### Example 85

### 1-cyano-N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]cyclopropane-1-carboxamide

### Example 85a

### N-{trans-4-[(5-bromo-1-methyl-2-oxo-1,2-dihydropyridin-4-yl)oxy]cyclohexyl} -1 - cyanocyclopropane-1-carboxamide

Example 85a was prepared according to the procedure used for the preparation of Example 70a, substituting 1-cyanocyclopropanecarboxylic acid for 1-(methoxymethyl)cyclopropanecarboxylic acid to provide the title compound.

### Example 85b

### 1-cyano-N-[trans-4-({5-[2-(4-fluoro-2,6-dimethylphenoxy)-5-(2-hydroxypropan-2-yl)phenyl] -1-methyl-2-oxo-1,2-dihydropyridin-4-yl}oxy)cyclohexyl]cyclopropane-1-carboxamide

Example 85b was prepared according to the procedure used for the preparation of Example 2h, substituting Example 85a for Example 2d and substituting Example 3c for Example 2g, to provide the title compound. ¹H NMR (400 MHz, DMSO-*d*₆) δ 7.92 (d, J = 7.6 Hz, 1H), 7.60 (s, 1H), 7.27 (m, 2H), 6.98 (d, J = 9.1 Hz, 2H), 6.21 (d, J = 8.3 Hz, 1H), 5.97 (s, 1H), 4.93 (s, 1H), 4.32 (ddd, J = 13.6, 9.2, 3.5 Hz, 1H), 3.53 (dtt, J = 14.4, 7.6, 4.0 Hz, 1H), 3.40 (s, 3H), 2.02 (s, 6H), 2.01 (m, 2H), 1.69 (m, 2H), 1.48 (m, 6H), 1.40 (s, 6H), 1.26 (m, 2H). MS (ESI+) m/z 588.2 (M+H)⁺.

### Example 86

### N-[trans-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide

### Example 86a

### N-{trans-4-[(5-bromo-1-methyl-2-oxo-1,2-dihydropyridin-4-yl)oxy]cyclohexyl}bicyclo[1.1.1]pentane-1-carboxamide

Example 86a was prepared according to the procedure used for the preparation of Example 70a substituting bicyclo[1.1.1]pentane-1-carboxylic acid for 1-(methoxymethyl)cyclopropanecarboxylic acid, to provide the title compound.

### Example 86b

### N-[trans-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide

Example 86b was prepared according to the procedure used for the preparation of Example 2h, substituting Example 86a for Example 2d and substituting Example 3c for Example 2g, to provide the title compound. ¹H NMR (400 MHz, DMSO-*d*₆) δ 7.60 (s, 1H), 7.48 (d, J = 7.6 Hz, 1H), 7.28 (m, 2H), 6.98 (d, J = 9.1 Hz, 2H), 6.21 (d, J = 8.3 Hz, 1H), 5.97 (s, 1H), 4.93 (s, 1H), 4.33 (ddd, J = 13.1, 8.9, 3.7 Hz, 1H), 3.45 (m, 1H), 3.40 (s, 3H), 2.37 (s, 1H), 2.02 (s, 6H), 1.98 (m, 2H), 1.91 (s, 6H), 1.68 (m, 2H), 1.40 (s, 6H), 1.27 (m, 4H). MS (ESI+) m/z 588.9 (M+H)⁺.

### Example 87

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-1 -methyl-3 -oxocyclobutane-1-carboxamide

### Example 87a

### N-{trans-4-[(5-bromo-1-methyl-2-oxo-1,2-dihydropyridin-4-yl)oxy]cyclohexyl}-1-methyl-3-oxocyclobutane-1-carboxamide

Example 87a was prepared according to the procedure used for the preparation of Example 70a, substituting 1-methyl-3-oxocyclobutanecarboxylic acid for 1-(methoxymethyl)cyclopropanecarboxylic acid to provide the title compound.

### Example 87b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl]-1 -methyl-3-oxocyclobutane-1-carboxamide

Example 87b was prepared according to the procedure used for the preparation of Example 2h, substituting Example 87a for Example 2d and substituting Example 3c for Example 2g, to provide the title compound. ¹H NMR (400 MHz, DMSO-*d*₆) δ 7.70 (d, J = 7.5 Hz, 1H), 7.61 (s, 1H), 7.28 (m, 2H), 6.99 (d, J = 9.1 Hz, 2H), 6.21 (d, J = 8.4 Hz, 1H), 5.99 (s, 1H), 4.94 (s, 1H), 4.37 (ddd, J = 13.6, 9.2, 4.0 Hz, 1H), 3.53 (m, 1H), 3.40 (s, 3H), 3.36 (m, 2H), 2.79 (m, 2H), 2.02 (s, 6H), 2.01 (m, 2H), 1.73 (m, 2H), 1.47 (s, 3H), 1.41 (s, 6H), 1.35 (m, 4H). MS (ESI+) m/z 605.1 (M+H)⁺.

### Example 88

### 1-cyano-N-[trans-4-({5-[2-(4-fluoro-2,6-dimethylphenoxy)-5-(2-hydroxypropan-2-yl)phenyl] -1 -methyl-2-oxo-1 ,2-dihydropyridin-4-yl} oxy)cyclohexyl]cyclobutane-1-carboxamide

### Example 88a

### N-{trans-4-[(5-bromo-1 -methyl-2-oxo-1,2-dihydropyridin-4-yl)oxy]cyclohexyl}-1 - cyanocyclobutane-1-carboxamide

Example 88a was prepared according to the procedure used for the preparation of Example 70a, substituting 1-cyanocyclobutanecarboxylic acid for 1-(methoxymethyl)cyclopropanecarboxylic acid, to provide the title compound.

### Example 88b

### 1-cyano-N-[trans-4-({5-[2-(4-fluoro-2,6-dimethylphenoxy)-5-(2-hydroxypropan-2-yl)phenyl] -1 -methyl-2-oxo-1 ,2-dihydropyridin-4-yl}oxy)cyclohexyl]cyclobutane-1-carboxamide

Example 88b was prepared according to the procedure used for the preparation of Example 2h, substituting Example 88a for Example 2d and substituting Example 3c for Example 2g, to provide the title compound. ¹H NMR (400 MHz, DMSO-*d*₆) δ 8.20 (d, J = 7.4 Hz, 1H), 7.61 (s, 1H), 7.28 (m, 2H), 6.99 (d, J = 9.1 Hz, 2H), 6.21 (d, J = 8.4 Hz, 1H), 6.00 (s, 1H), 4.94 (s, 1H), 4.38 (ddd, J = 13.8, 9.1, 3.9 Hz, 1H), 3.52 (m, 1H), 3.40 (s, 3H), 2.51 (m, 4H), 2.07 (m, 1H), 2.02 (s, 6H), 2.00 (m, 2H), 1.86 (m, 1H), 1.73 (m, 2H), 1.41 (s, 6H), 1.35 (m, 4H). MS (ESI+) m/z 602.2 (M+H)⁺.

### Example 89

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpiperidine-2,6-dione

### Example 89a

### 1-{trans-4-[(5-bromo-1-methyl-2-oxo-1,2-dihydropyridin-4-yl)oxy]cyclohexyl}-3,3-dimethylpiperidine-2,6-dione

Example 89a was prepared according to the procedure used for the preparation of Example 80a, substituting 2,2-dimethylglutaric anhydride for glutaric anhydride. Purification of the residue by flash chromatography (silica gel, 0-4% methanol in dichloromethane) provided the title compound (81 mg, 38% yield).

### Example 89b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpiperidine-2,6-dione

Example 89b was prepared according to the procedure used for the preparation of Example 60, substituting Example 89a for Example 24a and Example substituting 3c for Example 1j. Purification of the residue by flash chromatography (silica gel, 4-6 % methanol in dichloromethane) provided the title compound (51 mg, 82 % yield). ¹H NMR (400 MHz, DMSO-*d*₆) δ 7.59 (s, 1H), 7.29 - 7.23 (m, 2H), 6.98 (d, *J=* 9.1 Hz, 2H), 6.18 (d, *J=* 8.4 Hz, 1H), 5.94 (s, 1H), 4.93 (s, 1H), 4.40 - 4.26 (m, 2H), 3.38 (s, 3H), 2.61 (t, *J=* 6.7 Hz, 2H), 2.37 - 2.25 (m, 2H), 2.11 - 2.02 (m, 2H), 2.01 (s, 6H), 1.69 (t, *J=* 6.7 Hz, 2H), 1.50 - 1.40 (m, 2H), 1.39 (s, 6H), 1.27 - 1.15 (m, 2H), 1.13 (s, 6H). (ESI+) m/z 619 (M+H)⁺.

### Example 90

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpyrrolidine-2,5-dione

### Example 90a

### 1-{trans-4-[(5-bromo-1-methyl-2-oxo-1,2-dihydropyridin-4-yl)oxy]cyclohexyl}-3,3-dimethylpyrrolidine-2,5-dione

Example 90a was prepared according to the procedure used for the preparation of Example 80a, substituting 2,2-dimethylsuccinic anhydride for glutaric anhydride. Purification of the residue by flash chromatography (silica gel, 0-4% methanol in dichloromethane) provided the title compound (180 mg, 88% yield).

### Example 90b

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpyrrolidine-2,5-dione

Example 90b was prepared according to the procedure used for the preparation of Example 60, substituting Example 90a for Example 24a and substituting Example 3c for Example 1j. Purification of the residue by flash chromatography (silica gel, 4-6% methanol in dichloromethane) provided the title compound (44 mg, 73% yield). ¹H NMR (400 MHz, DMSO-*d*₆) δ 7.59 (s, 1H), 7.29 - 7.23 (m, 2H), 6.97 (d, *J=* 9.1 Hz, 2H), 6.19 (d, *J*= 8.4 Hz, 1H), 5.99 (s, 1H), 4.93 (s, 1H), 4.40 - 4.31 (m, 1H), 3.80 - 3.71 (m, 1H), 3.38 (s, 3H), 2.50 (s, 2H), 2.27 - 2.14 (m, 2H), 2.13 - 2.05 (m, 2H), 2.01 (s, 6H), 1.58 - 1.50 (m, 2H), 1.39 (s, 6H), 1.32 - 1.18 (m, 2H), 1.15 (s, 6H). (ESI+) m/z 605 (M+H)⁺.

### Example 91

### (7aS)-2-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]tetrahydro-1H-pyrrolo[1,2-c] imidazole-1 ,3(2H)-dione

### Example 91a

### tert-butyl (2S)-2-({trans-4-[(5-bromo-1-methyl-2-oxo-1,2-dihydropyridin-4-yl)oxy]cyclohexyl}carbamoyl)pyrrolidine-1-carboxylate

Example 91a was prepared according to the procedure used for the preparation of Example 78a, substituting 1-(*tert*-butoxycarbonyl)-L-proline for *N*-(*tert*-butoxycarbonyl)-*N-*methylglycine to provide the title compound as a white solid. (0.3223 g, 0.647 mmol, 100% yield).

### Example 91b

### N-{trans-4-[(5-bromo-1 -methyl-2-oxo-1,2-dihydropyridin-4-yl)oxy]cyclohexyl} -L-prolinamide trifluoroacetate salt

Example 91b was prepared according to the procedure used for the preparation of Example 78b, substituting Example 91a for Example 78a to provide the title compound as a colorless glass. (0.352 g, 0.600 mmol, 100 % yield).

### Example 91c

### (7aS)-2-{trans-4-[(5-bromo-1-methyl-2-oxo-1,2-dihydropyridin-4-yl)oxy]cyclohexyl)tetrahydro-1H-pyrrolo[1,2-c]imidazole-1,3(2H)-dione

Example 91c was prepared according to the procedure used for the preparation of Example 78c, substituting Example 91b for Example 78b to provide the title compound as a white solid. (0.1783 g, 0.420 mmol, 65% yield).

### Example 91d

### (7aS)-2-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]tetrahydro-1H-pyrrolo[1,2-c]imidazole-1,3(2H)-dione

Example 91d was prepared according to the procedure used for the preparation of Example 1k, substituting Example 91c for Example 1f, and substituting Example 3c for Example 1j, to provide the title compound as a white solid. (0.0705 g, 0.114 mmol, 74% yield). Enantiomeric excess (Chiral SFC) = 94%. ¹H NMR (501 MHz, DMSO-*d*₆) δ 7.58 (s, 1H), 7.29 - 7.22 (m, 2H), 6.97 (d, J = 9.1 Hz, 2H), 6.18 (d, J = 8.5 Hz, 1H), 5.98 (s, 1H), 4.92 (s, 1H), 4.34 (dq, J = 10.4, 5.3, 4.1 Hz, 1H), 4.08 (dd, J = 9.1, 7.5 Hz, 1H), 3.65 (tt, J = 12.2, 3.9 Hz, 1H), 3.44 (dt, J = 10.8, 7.7 Hz, 1H), 3.38 (s, 3H), 3.09 (ddd, J = 10.8, 8.0, 4.7 Hz, 1H), 2.21 - 2.13 (m, 1H), 2.07 (dd, J = 11.7, 4.0 Hz, 2H), 2.00 (s, 6H), 1.94 (ddd, J = 16.8, 8.2, 5.5 Hz, 2H), 1.63 - 1.49 (m, 4H), 1.39 (s, 6H), 1.22 (s, 3H). MS (ESI+) m/z 618.2 (M+H)⁺.

### Example 92

### (5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methyl-1,3-oxazolidine-2,4-dione

### Example 92a

### (2S)-N-{trans-4-[(5-bromo-1-methyl-2-oxo-1,2-dihydropyridin-4-yl)oxy]cyclohexyl)-2-hydroxypropanamide

Example 92a was prepared according to the procedure used for the preparation of Example 78a, substituting (2*S*)-2-hydroxypropanoic acid for *N*-(*tert*-butoxycarbonyl)-*N-*methylglycine to provide the title compound as a white solid. (0.0927 g, 0.248 mmol, 41% yield).

### Example 92b

### (5S)-3-{trans-4-[(5-bromo-1-methyl-2-oxo-1,2-dihydropyridin-4-yl)oxy]cyclohexyl}-5-methyl-1,3-oxazolidine-2,4-dione

Example 92b was prepared according to the procedure used for the preparation of Example 78c, substituting Example 92a for Example 78b to provide the title compound as a white solid. (0.0283 g, 0.071 mmol, 28% yield).

### Example 92c

### 3(5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methyl-1,3-oxazolidine-2,4-dione

Example 92c was prepared according to the procedure used for the preparation of Example 1k, substituting Example 92b for Example 1f and substituting Example 3c for Example 1j, to provide the title compound as a white solid. (0.0391 g, 0.068 mmol, 50 % yield). Enantiomeric excess (Chiral SFC) > 99%. ¹H NMR (400 MHz, DMSO-*d*₆) δ 7.56 (s, 1H), 7.29 - 7.20 (m, 2H), 6.95 (d, J = 9.1 Hz, 2H), 6.17 (d, J = 8.5 Hz, 1H), 6.00 (s, 1H), 4.96 (q, J = 7.0 Hz, 1H), 4.89 (s, 1H), 4.40 - 4.30 (m, 1H), 3.68 (td, J = 12.2, 6.2 Hz, 1H), 3.37 (s, 3H), 2.09 (dq, J = 13.0, 7.0, 4.0 Hz, 4H), 1.99 (s, 6H), 1.70 (d, J = 11.9 Hz, 3H), 1.38 (s, 6H), 1.32 - 1.17 (m, 4H). MS (ESI+) m/z 593.1 (M+H)⁺.

### Example 93

### 3-amino-N-[trans-4-({5-[2-(4-fluoro-2,6-dimethylphenoxy)-5-(2-hydroxypropan-2-yl)phenyl] -1 -methyl-2-oxo-1 ,2-dihydropyridin-4-yl} oxy)cyclohexyl]bicyclo[1.1.1]pentane-1-carboxamide

### Example 93a

### 3-amino-N-{trans-4-[(5-bromo-1-methyl-2-oxo-1,2-dihydropyridin-4-yl)oxy]cyclohexyl}bicyclo[1.1.1]pentane-1-carboxamide, hydrochloric acid

Hydrogen chloride solution (4 M in 1,4-dioxane) (1 mL, 4 mmol) was added to a mixture of Example 72a (0.0726 g, 0.142 mmol) in methanol (1 mL). The resulting mixture was stirred at 35 °C for 5 hours. The reaction mixture was concentrated and dried in a vacuum oven at 60 °C to provide 0.073 g (quantitative yield) of the title compound.

### Example 93b

### 3-amino-N-[trans-4-({5-[2-(4-fluoro-2,6-dimethylphenoxy)-5-(2-hydroxypropan-2-yl)phenyl] -1 -methyl-2-oxo-1 ,2-dihydropyridin-4-yl}oxy)cyclohexyl]bicyclo[1.1.1 ]pentane-1-carboxamide

Example 93b was prepared according to the procedure used for the preparation of Example 2h, substituting Example 93a for Example 2d and substituting Example 3c for Example 2g. The compound was purified by reverse phase HPLC (C18, acetonitrile/water (0.1% trifluoroacetic acid), 10-90%) to provide the title compound. ¹H NMR (400 MHz, DMSO-*d*₆) δ 8.55 (s, 3H), 7.72 (d, J = 7.5 Hz, 1H), 7.60 (s, 1H), 7.27 (m, 2H), 6.98 (d, J = 9.1 Hz, 2H), 6.21 (d, J = 8.4 Hz, 1H), 5.99 (s, 1H), 4.89 (m, 1H), 4.35 (tt, J = 8.4, 3.6 Hz, 1H), 3.40 (s, 3H), 2.14 (s, 6H), 2.02 (s, 6H), 2.01 (m, 2H), 1.69 (m, 2H), 1.40 (s, 6H), 1.29 (m, 4H). LCMS (APCI+) m/z 586.3 (M+H)⁺.

### Example 94

### 5-[2-(4-fluoro-2,6-dimethylphenoxy)-5-(2-hydroxypropan-2-yl)phenyl]-4-{[cis-3-(2-hydroxypropan-2-yl)cyclobutyl] oxy}-1-methylpyridin-2(1H)-one

### Example 94a

### methyl cis-3-[(5-bromo-1-methyl-2-oxo-1,2-dihydropyridin-4-yl)oxy]cyclobutane-1-carboxylate

Example 94a was prepared according to the procedure used for the preparation of Example 61a, substituting methyl trans-3-hydroxycyclobutane-1-carboxylate for trans-ethyl 4-hydroxycyclohexanecarboxylate, to provide the title compound. This material was the second eluting isomer during the chromatographic purification.

### Example 94b

### methyl cis-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1 -carboxylate

Example 94b was prepared according to the procedure used for the preparation of Example 61b, substituting Example 94a for Example 61a, to provide the title compound.

### Example 94c

### 5-[2-(4-fluoro-2,6-dimethylphenoxy)-5-(2-hydroxypropan-2-yl)phenyl]-4-{[cis-3-(2-hydroxypropan-2-yl)cyclobutyl] oxy} -1-methylpyridin-2(1H)-one

Example 94c was prepared according to the procedure used for the preparation of Example 67a, substituting Example 94b for Example 61a, to provide the title compound. ¹H NMR (400 MHz, DMSO-*d*₆) δ 7.55 (s, 1H), 7.31 - 7.19 (m, 2H), 6.94 (d, J = 9.1 Hz, 2H), 6.13 (d, J = 8.5 Hz, 1H), 5.71 (s, 1H), 4.49 - 4.39 (m, 1H), 3.36 (s, 3H), 2.17-2.33 (m, 2H), 1.97 (s, 6H), 1.88 - 1.71 (m, 3H), 1.38 (s, 6H), 0.91 (s, 6H). (ESI+) m/z 510.2 (M+H)⁺.

### Example 95

### cis-N-ethyl-3-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclobutane-1-carboxamide

### Example 95a

### cis-3-({5-[2-(4-fluoro-2,6-dimethylphenoxy)-5-(2-hydroxypropan-2-yl)phenyl]-1-methyl-2-oxo-1 ,2-dihydropyridin-4-yl}oxy)cyclobutane-1-carboxylic acid

Example 95a was prepared according to the procedure used for the preparation of Example 61c, substituting Example 94b for Example 61b, to provide the title compound.

### Example 95b

### cis-N-ethyl-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxamide

Example 95b was prepared according to the procedure used for the preparation of Example 63, substituting Example 95a for Example 61c, to provide the title compound. ¹H NMR (400 MHz, DMSO-*d*₆) δ 7.77 (t, J = 5.5 Hz, 1H), 7.57 (s, 1H), 7.31 - 7.20 (m, 2H), 6.95 (d, J = 9.1 Hz, 2H), 6.14 (d, J = 8.5 Hz, 1H), 5.71 (s, 1H), 4.61 (p, J = 7.3 Hz, 1H), 3.37 (s, 3H), 2.99 (qd, J = 7.2, 5.4 Hz, 2H), 2.60 - 2.47 (m, 2H), 1.95-2.05 (m, 9H), 1.38 (s, 6H), 0.94 (t, J = 7.2 Hz, 3H). (ESI+) m/z 523.0 (M+H)⁺.

### Example 96

### 5-[2-(4-fluoro-2,6-dimethylphenoxy)-5-(2-hydroxypropan-2-yl)phenyl]-1-methyl-4-{[cis-3-(pyrrolidine-1-carbonyl)cyclobutyl]oxy}pyridin-2(1H)-one

Example 96 was prepared according to the procedure used for the preparation of Example 62, substituting Example 95a for Example 61c, to provide the title compound. ¹H NMR (501 MHz, DMSO-*d*₆) δ 7.59 (s, 1H), 7.32 - 7.22 (m, 2H), 6.97 (d, J = 9.1 Hz, 2H), 6.15 (d, J = 8.6 Hz, 1H), 5.72 (s, 1H), 4.64 (p, J = 7.4 Hz, 1H), 3.38 (s, 3H), 3.30 (t, J = 6.7 Hz, 2H), 3.21 (t, J = 6.9 Hz, 2H), 2.89 - 2.78 (m, 1H), 2.58 (ddd, J = 11.9, 9.5, 7.3 Hz, 2H), 2.08 - 1.98 (m, 2H), 1.98 (s, 6H), 1.81 (q, J = 6.7 Hz, 2H), 1.71 (p, J = 6.7 Hz, 2H), 1.40 (s, 6H). (ESI+) m/z 571.3 (M+Na)⁺.

### Example 97

### cis-3-({5-[2-(4-fluoro-2,6-dimethylphenoxy)-5-(2-hydroxypropan-2-yl)phenyl]-1-methyl-2-oxo-1 ,2-dihydropyridin-4-yl}oxy)cyclobutane-1-carboxylic acid

Example 97 was prepared according to the procedure used for the preparation of Example 61c, substituting Example 94b for Example 61b, to provide the title compound. ¹H NMR (400 MHz, DMSO-*d*₆) δ 7.57 (s, 1H), 7.31 - 7.21 (m, 2H), 6.94 (d, J = 9.1 Hz, 2H), 6.15 (d, J = 8.5 Hz, 1H), 5.71 (s, 1H), 4.91 (s, 1H), 4.62 (p, J = 7.4 Hz, 1H), 3.53 (s, 1H), 3.37 (s, 3H), 2.74 - 2.55 (m, 3H), 1.95-2.08 (m, 9H), 1.38 (s, 6H). (ESI+) m/z 496.2 (M+H)⁺.

### Example 98

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl] acetohydrazide

### Example 98a

### 5-bromo-4-[(1,4-dioxaspiro[4.5]decan-8-yl)oxy]-1-methylpyridin-2(1H)-one

A solution of 1,4-dioxaspiro[4.5]decan-8-ol (0.806 g, 5.10 mmol) and Example 1c (1.0 g, 4.85 mmol) in tetrahydrofuran (32.4 mL) at 0 °C under nitrogen was treated dropwise with 1M potassium tert-butoxide (5.58 mL, 5.58 mmol) in tetrahydrofuran. The mixture was stirred for 60 minutes under nitrogen at 0-5 °C and then partitioned between ethyl acetate and water. The aqueous layer was extracted once more with ethyl acetate. The organic extracts were combined and washed with saturated aqueous sodium chloride, dried over anhydrous sodium sulfate, filtered, and concentrated to give an off-white solid. Purification of the residue by trituration in a minimal volume of 9:1 heptane/ethyl acetate afforded the title compound as a white powder (1.5 g, 90% yield).

### Example 98b

### 5-bromo-1-methyl-4-[(4-oxocyclohexyl)oxy]pyridin-2(1H)-one

A solution of Example 98a (1.388 g, 4.03 mmol) in 1M hydrochloric acid (26.9 mL) and acetone (13.4 mL) was stirred at ambient temperature for 3 hours and concentrated. The residue was partitioned between ethyl acetate and water, adjusting the pH to 7 with aqueous 1N sodium hydroxide. The organic layer was dried over anhydrous sodium sulfate, filtered, and concentrated. Purification of the residue by chromatography (silica gel, 10-70% of 3:1 ethyl acetate/ethanol in heptanes) afforded the title compound as a white powder (0.87 g, 68% yield).

### Example 98c

### tert-butyl 2-{trans-4-[(5-bromo-1-methyl-2-oxo-1 ,2-dihydropyridin-4-yl)oxy]cyclohexyl} hydrazine-1-carboxylate

A solution of Example 98b (0.82 g, 2.73 mmol) and *tert*-butyl carbazate (0.542 g, 4.10 mmol) in dichloromethane (31.0 mL) and acetic acid (3.10 mL) was stirred at ambient temperature for 1 hour, cooled to 5 °C and treated portionwise with sodium triacetoxyhydroborate (1.737 g, 8.20 mmol).The mixture was then stirred at ambient temperature for 4 hours and partitioned with 5% aqueous sodium bicarbonate. The organic layer was washed with saturated aqueous sodium chloride, dried over anhydrous sodium sulfate, filtered, and concentrated. LCMS shows two distinct peaks for the cis and trans isomers. Purification of the residue by chromatography (silica gel, 1-6% methanol in dichloromethane, 120 g silica cartridge) afforded separation of the two peaks. Peak A (Example 107a) eluted at 4% methanol (0.656 g, 58%). The title compound, Peak B, was eluted at 5.5% methanol (0.406 g 36%).

### Example 98d

### tert-butyl 2-acetyl-2-{trans-4-[(5-bromo-1-methyl-2-oxo-1,2-dihydropyridin-4-yl)oxy]cyclohexyl}hydrazine-1-carboxylate

To a solution of Example 98c (0.1 g, 0.240 mmol) and triethylamine (0.067 mL, 0.480 mmol) in dichloromethane (4.80 mL) was added dropwise acetyl chloride (0.019 mL, 0.264 mmol). The mixture was stirred at ambient temperature under nitrogen for 1 hour and concentrated to afford the title compound (0.11 g, 100% yield).

### Example 98e

### N-{trans-4-[(5-bromo-1-methyl-2-oxo-1,2-dihydropyridin-4-yl)oxy]cyclohexyl}acetohydrazide hydrochloride

To Example 98d (0.110 g, 0.240 mmol) was added 4M hydrogen chloride in dioxane (5.0 mL, 20 mmol). The mixture was stirred at ambient temperature for 1 hour, concentrated and azeotroped twice with toluene to afford the title compound (0.095 g, 100% yield).

### Example 98f

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] acetohydrazide

Example 3c (0.116 g, 0.289 mmol), Example 98e (0.095 g, 0.241 mmol), tris(dibenzylideneacetone)dipalladium(0) (6.61 mg, 7.22 µmol), 1,3,5,7-tetramethyl-6-phenyl-2,4,8-trioxa-6-phosphaadamantane (7.04 mg, 0.024 mmol) and sodium carbonate (0.179 g, 1.685 mmol) were combined and sparged with argon for 15 minutes. Meanwhile a solution of 4:1 tetrahydrofuran/water (5.0 mL) was sparged with nitrogen for 15 minutes and transferred by syringe into the reaction vessel under argon. The mixture was stirred for 6 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-8% methanol in dichloromethane) afforded the title compound as a foam (0.092 g, 65 % yield). ¹H NMR (501 MHz, DMSO-*d*₆) δ 7.58 (d, J = 3.9 Hz, 1H), 7.28 - 7.22 (m, 2H), 6.97 (d, J = 9.1 Hz, 2H), 6.18 (d, J = 8.5 Hz, 1H), 5.90 (s, 1H), 4.92 (s, 1H), 4.43 (s, 2H), 4.25 (td, J = 10.8, 5.2 Hz, 1H), 4.15 (m, 1H), 3.38 (s, 3H), 2.04 (d, J = 11.5 Hz, 2H), 2.00 (s, 9H), 1.71 (q, J = 12.8, 12.2 Hz, 2H), 1.44 (d, J = 12.5 Hz, 2H), 1.39 (s, 6H), 1.27 (dq, J = 24.4, 13.2, 12.0 Hz, 2H). MS (ESI+) m/z 552 (M+H)⁺.

### Example 99

### N'-[cis-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]acetohydrazide

### Example 99a

### N'-{cis-4-[(5-bromo-1-methyl-2-oxo-1,2-dihydropyridin-4-yl)oxy]cyclohexyl} acetohydrazide

A solution of Example 98b (0.2 g, 0.666 mmol) and acetohydrazide (0.074 g, 1.000 mmol) in dichloromethane (7.57 mL) and acetic acid (0.757 mL) was stirred at ambient temperature for 1 hour, cooled to 5 °C and treated portionwise with sodium triacetoxyhydroborate (0.424 g, 1.999 mmol).The mixture was then stirred at ambient temperature for 16 hours and partitioned with 5% aqueous sodium bicarbonate. The organic layer was washed with saturated aqueous sodium chloride, dried over anhydrous sodium sulfate, filtered, and concentrated. LCMS shows two distinct peaks for the cis and trans isomers. Purification of the residue by silica chromatography (1-10% methanol in dichloromethane, 24 g silica cartridge) afforded separation of the two peaks. Peak A, the title compound was eluted at 8% methanol (0.128 g, 54% yield). Peak B (Example 105a) was eluted at 10% methanol (0.051 g, 21%). ¹H NMR (400 MHz, DMSO-*d*₆) δ 9.19 (d, *J* = 6.5 Hz, 1H), 7.98 (s, 1H), 5.91 (s, 1H), 4.64 (dd, *J* = 6.6, 3.3 Hz, 1H), 4.56 (p, *J* = 4.0, 3.5 Hz, 1H), 3.30 (s, 3H), 2.73 (dt, *J*= 8.7, 5.2 Hz, 1H), 1.89 - 1.81 (m, 2H), 1.74 (s, 3H), 1.60 - 1.36 (m, 6H).

### Example 99b

### N'-[cis-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]acetohydrazide

Example 3c (0.061 g, 0.154 mmol), Example 99a (0.05 g, 0.140 mmol), tris(dibenzylideneacetone)dipalladium(0) (3.83 mg, 4.19 µmol), 1,3,5,7-tetramethyl-6-phenyl-2,4,8-trioxa-6-phosphaadamantane (4.08 mg, 0.014 mmol) and sodium carbonate (0.074 g, 0.698 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-8 % methanol in dichloromethane) afforded the title compound (0.025 g, 30% yield). ¹H NMR (501 MHz, DMSO-*d*₆) δ 9.18 (s, 1H), 7.59 (s, 1H), 7.30 (d, J = 2.3 Hz, 1H), 7.25 (dd, J = 8.6, 2.4 Hz, 1H), 6.96 (d, J = 9.1 Hz, 2H), 6.20 (d, J = 8.6 Hz, 1H), 5.89 (s, 1H), 4.88 (s, 1H), 4.48 (s, 2H), 3.37 (s, 3H), 2.62 (m, 1H), 2.00 (s, 6H), 1.84 (s, 3H), 1.80 - 1.73 (m, 2H), 1.46 (td, J = 16.1, 14.9, 7.6 Hz, 4H), 1.39 (s, 6H), 1.18 (m, 2H). MS (ESI+) m/z 552 (M+H)⁺.

### Example 100

### N-[trans-3-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclobutyl] acetamide

### Example 100a

### tert-butyl {trans-3-[(5-bromo-1-methyl-2-oxo-1,2-dihydropyridin-4-yl)oxy]cyclobutyl}carbamate

Example 100a was prepared according to the procedure used for the preparation of Example 61a, substituting tert-butyl (trans-3-hydroxycyclobutyl)carbamate for trans-ethyl 4-hydroxycyclohexanecarboxylate.

### Example 100b

### 4-[(trans-3-aminocyclobutyl)oxy]-5 -bromo-1-methylpyridin-2(1H)-one

The hydrochloride salt of Example 100b was prepared according to the procedure used for the preparation of Example 84b, substituting Example 100a for Example 84a.

### Example 100c

### N-{trans-3-[(5-bromo-1-methyl-2-oxo-1,2-dihydropyridin-4-yl)oxy]cyclobutyl)acetamide

Example 100c was prepared according to the procedure used for the preparation of Example 84c, substituting the hydrochloride salt of Example 100b for Example 84b.

### Example 100d

### N-[trans-3-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclobutyl] acetamide

Example 100d was prepared according to the procedure used for the preparation of Example 61b, substituting Example 100c for Example 61a. ¹H NMR (400 MHz, DMSO-*d*₆) δ 8.17 (d, J = 7.0 Hz, 1H), 7.59 (s, 1H), 7.33 - 7.21 (m, 2H), 6.95 (d, J = 9.1 Hz, 2H), 6.16 (d, J = 8.6 Hz, 1H), 5.63 (s, 1H), 4.86 - 4.75 (m, 1H), 4.10 (tq, J = 8.0, 4.1, 2.7 Hz, 2H), 3.37 (s, 3H), 2.04-2.32 (m, 4H), 1.98 (s, 6H), 1.74 (s, 3H), 1.38 (s, 6H). (ESI+) m/z 509.1 (M+H)⁺.

### Example 101

### trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)-N-methylcyclohexane-1-carboxamide

### Example 101a

### trans-4-[(5-bromo-1-methyl-2-oxo-1,2-dihydropyridin-4-yl)oxy]cyclohexane-1-carboxylic acid

Example 101a was prepared according to the procedure used for the preparation of Example 61c, substituting Example 61a for Example 61b.

### Example 101b

### trans-4-[(5-bromo-1-methyl-2-oxo-1,2-dihydropyridin-4-yl)oxy]-N-methylcyclohexane-1-carboxamide

A mixture of Example 101a (0.08 g, 0.242 mmol), oxalyl chloride (0.218 mL, 0.436 mmol), and *N,N*-dimethylformamide (1.69 µl, 0.022 mmol) in dichloromethane (2 mL) was stirred at ambient temperature for 2 hours. The solvent was removed, and the residue was treated with tetrahydrofuran (1 mL) and dimethylformamide (1 mL) to give a clear solution. To this solution was added 2.0 N methanamine in tetrahydrofuran (1.091 mL, 2.182 mmol). The reaction mixture was stirred at ambient temperature overnight. The reaction mixture was partitioned between water and ethyl acetate. The aqueous layer was extracted with additional ethyl acetate three times. The combined organic layers were washed with saturated aqueous sodium chloride, dried over anhydrous magnesium sulfate, and filtered to afford the title compound (0.045 g, 0.131 mmol, 60.1 % yield).

### Example 101c

### trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-N-methylcyclohexane-1-carboxamide

Example 101c was prepared according to the procedure used for the preparation of Example 61b, substituting Example 101b for Example 61a, to provide the title compound. ¹H NMR (501 MHz, DMSO-*d*₆) δ 7.64 (q, J = 4.6 Hz, 1H), 7.58 (s, 1H), 7.29 - 7.22 (m, 2H), 6.96 (d, J = 9.1 Hz, 2H), 6.18 (d, J = 8.4 Hz, 1H), 5.95 (s, 1H), 4.32 (tt, J = 10.2, 4.1 Hz, 1H), 3.38 (s, 3H), 2.51 (d, J = 4.6 Hz, 3H), 1.96-2.02 (m, 8H), 1.67 (dd, J = 14.0, 3.8 Hz, 2H), 1.49 (qd, J = 13.2, 3.3 Hz, 2H), 1.38 (s, 6H), 1.16 (qd, J = 12.8, 3.6 Hz, 2H). (ESI+) m/z 537.1 (M+H)⁺.

### Example 102

### trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexane-1-carboxamide

### Example 102a

### trans-4-[(5-bromo-1-methyl-2-oxo-1,2-dihydropyridin-4-yl)oxy]cyclohexane-1-carboxamide

Example 102a was prepared according to the procedure used for the preparation of Example 101b, substituting concentrated ammonium hydroxide for 2.0 N methanamine in tetrahydrofuran.

### Example 102b

### trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide

Example 102b was prepared according to the procedure used for the preparation of Example 61b, substituting Example 102a for Example 61a. ¹H NMR (400 MHz, DMSO-*d*₆) δ 7.57 (s, 1H), 7.28 -7.20 (m, 2H), 7.14 (s, 1H), 6.95 (d, J = 9.1 Hz, 2H), 6.64 (s, 1H), 6.17 (d, J = 8.4 Hz, 1H), 5.93 (s, 1H), 4.31 (td, J = 10.1, 5.1 Hz, 1H), 3.37 (s, 3H), 1.95-2.04 (m, 8H), 1.69 (dd, J = 14.1, 3.8 Hz, 2H), 1.53 - 1.39 (m, 2H), 1.37 (s, 6H), 1.23 - 1.08 (m, 2H). (ESI+) m/z 523.1 (M+H)⁺.

### Example 103

### tert-butyl (4-{[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl}bicyclo[2.1.1]hexan-1-yl)carbamate

### Example 103a

### tert-butyl [4-({trans-4-[(5-bromo-1-methyl-2-oxo-1,2-dihydropyridin-4-yl)oxy]cyclohexyl}carbamoyl)bicyclo[2.1.1]hexan-1-yl]carbamate

Example 103a was prepared according to the procedure used for the preparation of Example 70a, substituting 4-((tert-butoxycarbonyl)amino)bicyclo[2.1.1]hexane-1-carboxylic acid for 1-(methoxymethyl)cyclopropanecarboxylic acid.

### Example 103b

### tert-butyl (4-{[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[2.1.1]hexan-1-yl)carbamate

Example 103b was prepared according to the procedure used for the preparation of Example 2h, substituting Example 103a for Example 2d and substituting Example 3c for Example 2g. The compound was purified by flash chromatography (amine-functionalized silica gel, 0 to 55% of a 3:1 mixture of ethyl acetate/ethanol in heptanes) to provide the title compound. ¹H NMR (400 MHz, DMSO-*d*₆) δ 7.60 (s, 1H), 7.32 (d, J = 7.8 Hz, 1H), 7.30 - 7.24 (m, 2H), 6.98 (d, J = 9.1 Hz, 2H), 6.21 (d, J = 8.3 Hz, 1H), 5.97 (s, 1H), 4.93 (s, 1H), 4.33 (dq, J = 13.6, 3.9 Hz, 1H), 3.50 (dtd, J = 10.5, 7.7, 4.4 Hz, 1H), 3.40 (s, 3H), 2.02 (s, 6H), 1.96 (m, 3H), 1.69 (s, 6H), 1.68 (m, 2H), 1.49 (s, 2H), 1.41 (s, 6H), 1.37 (s, 9H), 1.26 (m, 4H). MS (ESI+) m/z 718.1 (M+H)⁺.

### Example 104

### tert-butyl [(1-{[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}cyclopropyl)methyl]carbamate

### Example 104a

### tert-butyl {[1-({trans-4-[(5-bromo-1-methyl-2-oxo-1,2-dihydropyridin-4-yl)oxy]cyclohexyl} carbamoyl)cyclopropyl]methyl} carbamate

Example 104a was prepared according to the procedure used for the preparation of Example 70a, substituting 1-(((tert-butoxycarbonyl)amino)methyl)cyclopropanecarboxylic acid for 1-(methoxymethyl)cyclopropanecarboxylic acid. After the reaction was complete, the mixture was diluted with ethyl acetate and water which further induced precipitation of the product. The solid was collected by filtration, washed with water and dried by pulling air through it to provide the title compound.

### Example 104b

### tert-butyl [(1-{[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]carbamoyl} cyclopropyl)methyl]carbamate

Example 104b was prepared according to the procedure used for the preparation of Example 2h, substituting Example 104a for Example 2d and substituting Example 3c for Example 2g. ¹H NMR (501 MHz, DMSO-*d*₆) δ 7.67 (d, J = 7.1 Hz, 1H), 7.61 (s, 1H), 7.28 (m, 3H), 6.98 (d, J = 9.0 Hz, 2H), 6.21 (d, J = 8.4 Hz, 1H), 5.96 (s, 1H), 4.93 (s, 1H), 4.37 (m, 1H), 3.50 (m, 1H), 3.40 (s, 3H), 3.16 (d, J = 6.5 Hz, 2H), 2.02 (s, 6H), 1.98 (m, 2H), 1.70 (m, 2H), 1.41 (s, 9H), 1.40 (s, 6H), 1.31 (m, 4H), 0.89 (q, J = 3.5 Hz, 2H), 0.67 (q, J = 3.7 Hz, 2H). MS (ESI+) m/z 692 (M+H)⁺.

### Example 105

### N'-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] acetohydrazide

### Example 105a

### N'-{trans-4-[(5-bromo-1-methyl-2-oxo-1,2-dihydropyridin-4-yl)oxy]cyclohexyl} acetohydrazide

The title compound was isolated as Peak B from the chromatography described in Example 99a. ¹H NMR (400 MHz, DMSO-*d*₆) δ 9.21 (d, *J=* 6.6 Hz, 1H), 7.97 (s, 1H), 5.96 (s, 1H), 4.68 (dd, *J* = 6.6, 3.6 Hz, 1H), 4.41 (tt, *J* = 8.7, 3.7 Hz, 1H), 3.29 (s, 6H), 2.69 (dq, *J* = 9.2, 4.4, 3.7 Hz, 1H), 2.01 - 1.95 (m, 2H), 1.81 - 1.76 (m, 1H), 1.74 (s, 3H), 1.42 - 1.19 (m, 4H).

### Example 105b

### N'-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] acetohydrazide

Example 3c (0.061 g, 0.154 mmol), Example 105a (0.05 g, 0.140 mmol), tris(dibenzylideneacetone)dipalladium(0) (3.83 mg, 4.19 µmol), 1,3,5,7-tetramethyl-6-phenyl-2,4,8-trioxa-6-phosphaadamantane (4.08 mg, 0.014 mmol) and sodium carbonate (0.074 g, 0.698 mmol) were combined and sparged with argon for 15 minutes. Meanwhile a solution of 4:1 tetrahydrofuran/water (1.3 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-10 % methanol in dichloromethane) afforded the title compound as a white powder (0.027 g, 33% yield). ¹H NMR (501 MHz, DMSO-*d*₆) δ 9.17 (d, J = 6.5 Hz, 1H), 7.56 (s, 1H), 7.28 - 7.20 (m, 2H), 6.97 - 6.92 (m, 2H), 6.17 (d, J = 8.4 Hz, 1H), 5.91 (s, 1H), 4.88 (s, 1H), 4.63 (dd, J = 6.6, 3.5 Hz, 1H), 4.36 (m, 1H), 3.36 (s, 3H), 2.57 (m, 1H), 1.97 (s, 6H), 1.92 (s, 2H), 1.72 (s, 3H), 1.61 (d, J = 9.9 Hz, 2H), 1.36 (s, 6H), 1.27 - 1.16 (m, 4H). MS (ESI+) m/z 552 (M+H)⁺.

### Example 106

### tert-butyl 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate

Example 3c (0.042 g, 0.106 mmol), Example 98c (0.04 g, 0.096 mmol), tris(dibenzylideneacetone)dipalladium(0) (2.64 mg, 2.88 µmol), 1,3,5,7-tetramethyl-6-phenyl-2,4,8-trioxa-6-phosphaadamantane (2.81 mg, 9.61 µmol) and sodium carbonate (0.051 g, 0.480 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a foam (0.049 g, 79% yield). ¹H NMR (501 MHz, DMSO-*d*₆) δ 8.17 (s, 1H), 7.60 (s, 1H), 7.32 - 7.24 (m, 2H), 6.99 (d, J = 9.1 Hz, 2H), 6.21 (d, J = 8.4 Hz, 1H), 5.95 (s, 1H), 4.93 (s, 1H), 4.41 (s, 1H), 4.23 (d, J = 4.3 Hz, 1H), 3.40 (s, 3H), 2.64 (s, 1H), 2.01 (s, 6H), 1.95 (s, 2H), 1.60 (s, 2H), 1.40 (s, 6H), 1.37 (s, 9H), 1.31 - 1.18 (m, 4H).). MS (ESI+) m/z 610 (M+H)⁺.

### Example 107

### tert-butyl 2-[cis-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate

### Example 107a

### tert-butyl 2- {cis-4-[(5-bromo-1-methyl-2-oxo-1,2-dihydropyridin-4-yl)oxy]cyclohexyl} hydrazine-1-carboxylate

The title compound was isolated as Peak A from the chromatography described in Example 98c.

### Example 107b

### tert-butyl 2-[cis-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate

Example 3c (0.042 g, 0.106 mmol), Example 107a (0.04 g, 0.096 mmol), tris(dibenzylideneacetone)dipalladium(0) (2.64 mg, 2.88 µmol), 1,3,5,7-tetramethyl-6-phenyl-2,4,8-trioxa-6-phosphaadamantane (2.81 mg, 9.61 µmol) and sodium carbonate (0.051 g, 0.480 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 30-60% of 3:1 ethyl acetate/ethanol in heptanes) afforded the title compound as a foam (0.051 g, 82% yield). ¹H NMR (400 MHz, DMSO-*d*₆) δ 8.08 (s, 1H), 7.56 (s, 1H), 7.27 (d, J = 2.4 Hz, 1H), 7.23 (dd, J = 8.6, 2.4 Hz, 1H), 6.93 (dd, J = 9.3, 1.1 Hz, 2H), 6.18 (d, J = 8.7 Hz, 1H), 5.86 (s, 1H), 4.84 (s, 1H), 4.43 (m, 1H), 4.00 - 3.92 (m, 1H), 3.36 (s, 3H), 2.68 (s, 1H), 1.99 (s, 6H), 1.72 (m, 2H), 1.47 (m, 2H), 1.37 (s, 6H), 1.32 (s, 9H), 1.26 - 1.15 (m, 4H). MS (ESI+) m/z 610 (M+H)⁺.

### Example 108

### trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)-N,N-dimethylcyclohexane-1-carboxamide

### Example 108a

### trans-4-[(5-bromo-1-methyl-2-oxo-1,2-dihydropyridin-4-yl)oxy]-N,N-dimethylcyclohexane-1-carboxamide

Example 108a was prepared according to the procedure used for the preparation of Example 101b, substituting 2.0 N *N,N*-dimethyl amine for 2.0 N methanamine in tetrahydrofuran.

### Example 108b

### trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)-N,N-dimethylcyclohexane-1-carboxamide

Example 108b was prepared according to the procedure used for the preparation of Example 61b, substituting Example 108a for Example 61a. ¹H NMR (501 MHz, DMSO-*d*₆) δ 7.58 (s, 1H), 7.29 - 7.21 (m, 2H), 6.96 (d, J = 9.1 Hz, 2H), 6.18 (d, J = 8.5 Hz, 1H), 5.97 (s, 1H), 4.34 (tt, J = 10.5, 4.2 Hz, 1H), 3.38 (s, 3H), 2.95 (s, 3H), 2.76 (s, 3H), 1.99-2.02 (m, 8H), 1.68 - 1.60 (m, 2H), 1.48 (dt, J = 13.3, 10.3 Hz, 2H), 1.38 (s, 6H), 1.30 - 1.19 (m, 2H). (ESI+) m/z 551.0 (M+H)⁺.

### Example 109

### 5-[2-(4-fluoro-2,6-dimethylphenoxy)-5-(2-hydroxypropan-2-yl)phenyl] -1-methyl-4-{[trans-4-(5-oxopyrazolidin-1 -yl)cyclohexyl] oxy}pyridin-2(1H)-one

### Example 109a

### tert-butyl 2- { trans-4-[(5 -bromo-1 -methyl-2-oxo-1,2-dihydropyridin-4-yl)oxy]cyclohexyl}-3-oxopyrazolidine-1-carboxylate

To a solution of Example 98c (0.25 g, 0.601 mmol) and potassium carbonate (0.207 g, 1.501 mmol) in *N,N-*dimethylformamide (6.01 mL) was added dropwise 3-chloropropionyl chloride (0.080 g, 0.631 mmol). The mixture was stirred in a sealed microwave tube at 40 °C temperature for 18 hours. The reaction mixture was cooled to ambient temperature and partitioned between ethyl acetate and water. The organic layer was washed repeatedly with saturated aqueous sodium chloride, dried over anhydrous sodium sulfate, filtered, and concentrated. Purification of the residue by chromatography (silica gel, 2-6 % methanol in dichloromethane) afforded the title compound (0.12 g, 41% yield).

### Example 109b

### 5-bromo-1-methyl-4-{[trans-4-(5-oxopyrazolidin-1-yl)cyclohexyl]oxy}pyridin-2(1H)-one hydrochloride

A solution of Example 109a (0.12 g, 0.255 mmol) in hydrochloric acid 4M in dioxane (5.0 mL, 20.00 mmol) was stirred at ambient temperature for 1 hour, concentrated, and azeotroped twice with toluene to afford the title compound (0.102 g, 98% yield).

### Example 109c

### 5-[2-(4-fluoro-2,6-dimethylphenoxy)-5-(2-hydroxypropan-2-yl)phenyl]-1-methyl-4-{[trans-4-(5-oxopyrazolidin-1 -yl)cyclohexyl] oxy}pyridin-2(1H)-one

Example 3c (0.043 g, 0.108 mmol), Example 109b (0.04 g, 0.098 mmol), tris(dibenzylideneacetone)dipalladium(0) (2.70 mg, 2.95 µmol), 1,3,5,7-tetramethyl-6-phenyl-2,4,8-trioxa-6-phosphaadamantane (2.87 mg, 9.84 µmol) and sodium carbonate (0.063 g, 0.590 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (reverse phase C-18, 40 minute elution of 20-80% acetonitrile in water) afforded the title compound (0.0131 g, 22% yield). ¹H NMR (400 MHz, DMSO-*d*₆) δ 7.58 (s, 1H), 7.29 - 7.21 (m, 2H), 6.97 (d, J = 9.1 Hz, 2H), 6.19 (d, J = 8.4 Hz, 1H), 5.96 (s, 1H), 5.39 (t, J = 8.2 Hz, 1H), 4.90 (s, 1H), 4.30 (m, 1H), 3.67 (m, 1H), 3.37 (s, 3H), 3.08 (m, 2H), 2.30 (t, J = 7.3 Hz, 2H), 2.05 (m, 2H), 2.00 (s, 6H), 1.69 (q, J = 12.9 Hz, 2H), 1.52 (d, J = 11.9 Hz, 2H), 1.39 (s, 6H), 1.25 (q, J = 10.1, 8.3 Hz, 2H). MS (ESI+) m/z 564 (M+H)⁺.

### Example 110

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -4-(hydroxymethyl)bicyclo[2.2.2]octane-1-carboxamide

### Example 110a

### N-{trans-4-[(5 -bromo-1 -methyl-2-oxo-1,2-dihydropyridin-4-yl)oxy]cyclohexyl} -4-(hydroxymethyl)bicyclo[2.2.2]octane-1-carboxamide

Example 110a was prepared according to the procedure used for the preparation of Example 70a, substituting 4-(hydroxymethyl)bicyclo[2.2.2]octane-1-carboxylic acid for 1-(methoxymethyl)cyclopropanecarboxylic acid. The compound was purified by flash chromatography (amine-functionalized silica gel, 0 to 70% of a 3:1 mixture of ethyl acetate/ethanol in heptanes) to provide the title compound.

### Example 110b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -4-(hydroxymethyl)bicyclo[2.2.2] octane-1-carboxamide

Example 110b was prepared according to the procedure used for the preparation of Example 2h, substituting Example 110a for Example 2d and substituting Example 3c for Example 2g. The compound was purified by flash chromatography (amine-functionalized silica gel, 0 to 100% of a 3:1 mixture of ethyl acetate/ethanol in heptanes) to provide the title compound. ¹H NMR (400 MHz, DMSO-*d*₆) δ 7.60 (s, 1H), 7.27 (m, 2H), 7.01 (d, J = 7.6 Hz, 1H), 6.98 (d, J = 9.2 Hz, 2H), 6.20 (m, 1H), 5.95 (s, 1H), 4.93 (s, 1H), 4.34 (m, 1H), 4.30 (t, J = 5.4 Hz, 1H), 3.48 (m, 1H), 3.40 (s, 3H), 3.02 (d, J = 5.5 Hz, 2H), 2.01 (s, 6H), 1.98 (m, 2H), 1.59 (m, 8H), 1.40 (s, 6H), 1.29 (m, 10H). MS (ESI+) m/z 683.2 (M+Na)⁺.

### Example 111

### N¹-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl] cyclopropane-1 ,1 -dicarboxamide

### Example 111a

### N¹-{trans-4-[(5-bromo-1-methyl-2-oxo-1,2-dihydropyridin-4-yl)oxy]cyclohexyl}cyclopropane-1,1-dicarboxamide

Example 111a was prepared according to the procedure used for the preparation of Example 70a, substituting 1-carbamoylcyclopropanecarboxylic acid for 1-(methoxymethyl)cyclopropanecarboxylic acid. The compound was purified by flash chromatography (amine-functionalized silica gel, 0 to 70% of a 3:1 mixture of ethyl acetate/ethanol in heptanes) to provide the title compound.

### Example 111b

### N¹-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl] cyclopropane-1 ,1-dicarboxamide

Example 111b was prepared according to the procedure used for the preparation of Example 2h, substituting Example 111a for Example 2d and substituting Example 3c for Example 2g. The compound was purified by flash chromatography (amine-functionalized silica gel, 0 to 100% of a 3:1 mixture of ethyl acetate/ethanol in heptanes) to provide the title compound. ¹H NMR (501 MHz, DMSO-*d*₆) δ 8.84 (d, J = 7.6 Hz, 1H), 7.60 (s, 1H), 7.30 (s, 1H), 7.29 (d, J = 2.3 Hz, 1H), 7.27 (dd, J = 8.5, 2.4 Hz, 1H), 7.19 (s, 1H), 6.98 (d, J = 9.1 Hz, 2H), 6.21 (d, J = 8.4 Hz, 1H), 5.98 (s, 1H), 4.94 (s, 1H), 4.41 (m, 1H), 3.53 (m, 1H), 3.40 (s, 3H), 2.01 (s, 6H), 1.94 (d, J = 10.0 Hz, 2H), 1.72 (d, J = 11.2 Hz, 2H), 1.40 (s, 6H), 1.34 (m, 4H), 1.25 (m, 4H). MS (ESI+) m/z 606.0 (M+H)⁺.

### Example 112

### 4-{[trans-4-(2-acetyl-5-oxopyrazolidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

### Example 112a

### 4-{[trans-4-(2-acetyl-5-oxopyrazolidin-1-yl)cyclohexyl]oxy}-5-bromo-1-methylpyridin-2(1H)-one

A solution of Example 109b (0.060 g, 0.148 mmol) and triethylamine (0.062 mL, 0.443 mmol) in dichloromethane (2.95 mL) was treated with acetyl chloride (0.013 mL, 0.177 mmol) and stirred at ambient temperature for 1 hour. The reaction mixture was partitioned between ethyl acetate and water. The organic layer was washed with 5% aqueous sodium bicarbonate, saturated aqueous sodium chloride, dried over anhydrous sodium sulfate, filtered, and concentrated to afford the title compound as a foam (0.050 g, 76% yield).

### Example 112b

### 4-{[trans-4-(2-acetyl-5-oxopyrazolidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

Example 3c (0.049 g, 0.123 mmol), Example 112a (0.046 g, 0.112 mmol), tris(dibenzylideneacetone)dipalladium(0) (3.07 mg, 3.35 µmol), 1,3,5,7-tetramethyl-6-phenyl-2,4,8-trioxa-6-phosphaadamantane (3.26 mg, 0.011 mmol) and sodium carbonate (0.059 g, 0.558 mmol) were combined and sparged with argon for 15 minutes. Meanwhile a solution of 4:1 tetrahydrofuran/water (1.2 mL) was sparged with nitrogen for 15 minutes and transferred by syringe into the reaction vessel under argon. The mixture was stirred for 3 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-7% methanol in dichloromethane) afforded the title compound as a foam (0.044 g, 62% yield). ¹H NMR (400 MHz, DMSO-*d*₆) δ 7.57 (s, 1H), 7.28 - 7.22 (m, 2H), 6.96 (d, J = 9.1 Hz, 2H), 6.18 (d, J = 8.4 Hz, 1H), 5.94 (s, 1H), 4.90 (s, 1H), 4.33 - 4.22 (m, 1H), 4.00 - 3.92 (m, 2H), 3.54 (m, 1H), 3.38 (s, 3H), 2.52 (m, 2H), 2.07 (s, 3H), 2.03-1.90 (m, 4H), 1.96 (s, 6H), 1.80 (d, J = 12.3 Hz, 2H), 1.38 (s, 6H), 1.22 (d, J = 10.2 Hz, 2H). MS (ESI+) m/z 606 (M+H)⁺.

### Example 113

### N-[trans-4-({5-[2-(2,6-dimethylphenoxy)-5-{[methyl(methylcarbamoyl)amino]methyl} phenyl] -1-methyl-2-oxo-1,2-dihydropyridin-4-yl} oxy)cyclohexyl] -1-methylcyclopropane-1 -carboxamide

### Example 113a

### 1-methyl-N-(trans-4-{[1-methyl-2-oxo-5-(4,4,5,5-tetramethyl-1,3,2-dioxaborolan-2-yl)-1,2-dihydropyridin-4-yl] oxy} cyclohexyl)cyclopropane-1 -carboxamide

Example 51a (422 mg, 1.10 mmol), 4,4,4',4',5,5,5',5'-octamethyl-2,2'-bi(1,3,2-dioxaborolane) (838 mg, 3.30 mmol), 2-(dicyclohexylphosphino)-2',4',6'-triisopropylbiphenyl (21 mg, 0.044 mmol),), chloro(2-dicyclohexylphosphino-2',4',6'-triisopropyl-1,1'-biphenyl)[2-(2'-amino-1,1'-biphenyl)]palladium(II) (35 mg, 0.044 mmol) and potassium acetate (324 mg, 3.30 mmol) were combined in a microwave tube. The mixture was purged with nitrogen for 15 minutes. Degased 2-methyl tetrahydrofuran (8 mL) was transferred to the reaction vessel and purged with nitrogen for another 5 minutes. The reaction mixture was heated at 78 °C for 16 hours, cooled to ambient temperature, diatomaceous earth was added, and the mixture was concentrated. The residue was purified by flash chromatography (silica gel, 2-4% methanol in dichloromethane) to provide the title compound (316 mg, 67% yield).

### Example 113b

### 3-bromo-4-(2,6-dimethylphenoxy)benzaldehyde

3-Bromo-4-fluorobenzaldehyde (11.8 g, 58.0 mmol), 2,6-dimethylphenol (7.08 g, 58.0 mmol) and cesium carbonate (18.9 g, 58.0 mmol) were combined in dimethyl sulfoxide (100 mL). The reaction mixture was stirred at ambient temperature overnight, and then partitioned with methyl tert-butyl ether and water. The aqueous layer was extracted with methyl tert-butyl ether again. The combined organic layers were washed with saturated aqueous sodium chloride, water, dried with anhydrous magnesium sulfate, filtered, and concentrated. The residue was purified by flash chromatography (silica gel, 0-10% ethyl acetate in heptanes) to provide the title compound (16.7 mg, 95% yield).

### Example 113c

### 1-(3-bromo-4-(2,6-dimethylphenoxy)phenyl)-N-methylmethanamine

Example 113b (2.76 g, 9.04 mmol), 33% methanamine in ethanol (2.30 mL, 18.5 mmol), and zinc(II) chloride (1.23 g, 9.04 mmol) were combined in methanol (140 mL). The reaction mixture was stirred at ambient temperature for 40 minutes, treated with sodium cyanoborohydride (1.23 g, 19.6 mmol), stirred at ambient temperature for 3 days, and concentrated. The residue was partitioned with dichloromethane and a mixture of saturated aqueous sodium chloride and saturated aqueous sodium carbonate. The organic layer was dried with anhydrous sodium sulfate, filtered, and concentrated. The residue was purified by flash chromatography (silica gel, 0-10% ammonia saturated methanol in dichloromethane) to provide the title compound (1.03 g, 36% yield).

### Example 113d

### 1-(3-bromo-4-(2,6-dimethylphenoxy)benzyl)-1,3-dimethylurea

Example 113c (0.515 g, 1.61 mmol), 2,5-dioxopyrrolidin-1-yl methylcarbamate (0.324 g, 1.88 mmol) and triethylamine (1.00 mL, 7.17 mmol) were combined in dichloromethane (10 mL). The reaction mixture was stirred at ambient temperature for 72 hours, and then partitioned with dichloromethane and water. The organic layer was dried with anhydrous sodium sulfate, filtered, and concentrated. The residue was purified by flash chromatography (silica gel, 0-10% ammonia saturated methanol in dichloromethane) to provide the title compound (407 mg, 67% yield).

### Example 113e

### N-[trans-4-({5-[2-(2,6-dimethylphenoxy)-5-{[methyl(methylcarbamoyl)amino]methyl} phenyl] -1-methyl-2-oxo-1,2-dihydropyridin-4-yl} oxy)cyclohexyl] -1 -methylcyclopropane-1 -carboxamide

Example 113a (26 mg, 0.060 mmol), Example 113d (19 mg, 0.050 mmol), tetrakis(triphenylphosphine)palladium(0) (2.9 mg, 2.5 µmol) and cesium fluoride (38 mg, 0.25 mmol) were combined in the mixture of dimethoxyethane (0.8 mL)/methanol (0.4 mL) in a microwave tube. The reaction mixture was microwaved at 100 °C for 40 minutes, cooled to ambient temperature, and partitioned with ethyl acetate and water. The organic layer was washed with saturated aqueous sodium chloride, dried with anhydrous sodium sulfate, treated with 3-mercaptopropyl functionalized silica gel, filtered, and concentrated. The residue was purified by flash chromatography (silica gel, 4-8% methanol in dichloromethane) to provide the title compound (16 mg, 53% yield). ¹H NMR (400 MHz, DMSO-*d*₆) δ 7.60 (s, 1H), 7.17 - 6.97 (m, 6H), 6.28 (q, *J=* 4.3 Hz, 1H), 6.20 (d, *J=* 8.4 Hz, 1H), 5.94 (s, 1H), 4.35 - 4.25 (m, 3H), 3.55 - 3.47 (m, 1H), 3.38 (s, 3H), 2.70 (s, 3H), 2.56 (d, *J=* 4.3 Hz, 3H), 2.04 - 1.92 (m, 8H), 1.72 - 1.63 (m, 2H), 1.47 - 1.33 (m, 2H), 1.29 - 1.17 (m, 5H), 0.89 (q, *J=* 3.4 Hz, 2H), 0.44 (q, *J* = 3.6 Hz, 2H). (ESI+) m/z 601 (M+H)⁺.

### Example 114

### 5-[2-(4-fluoro-2,6-dimethylphenoxy)-5-(2-hydroxypropan-2-yl)phenyl]-4-{[trans-4-(5-hydroxy-1H-pyrazol-1 -yl)cyclohexyl] oxy} -1 -methylpyridin-2(1H)-one

Example 3c (0.043 g, 0.108 mmol), Example 109b (0.04 g, 0.098 mmol), tris(dibenzylideneacetone)dipalladium(0) (2.70 mg, 2.95 µmol), 1,3,5,7-tetramethyl-6-phenyl-2,4,8-trioxa-6-phosphaadamantane (2.87 mg, 9.84 µmol) and sodium carbonate (0.063 g, 0.590 mmol) were combined and sparged with argon for 15 minutes. Meanwhile a solution of 4:1 tetrahydrofuran/water (1.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (reverse phase C-18, 40 minute elution of 20-80% acetonitrile in water) afforded the title compound as the second eluting peak (0.0056 g, 10% yield). ¹H NMR (501 MHz, DMSO-*d*₆) δ 10.74 (bs, 1H), 7.59 (s, 1H), 7.32 - 7.23 (m, 2H), 7.07 (d, J = 1.9 Hz, 1H), 6.97 (d, J = 9.1 Hz, 2H), 6.19 (d, J = 8.5 Hz, 1H), 6.00 (s, 1H), 5.27 (d, J = 1.9 Hz, 1H), 4.92 (s, 1H), 4.44 (m, 1H), 3.99 - 3.92 (m, 1H), 3.38 (s, 3H), 2.13 - 2.06 (m, 2H), 2.01 (s, 6H), 1.95 - 1.86 (m, 2H), 1.80 - 1.71 (m, 2H), 1.39 (s, 6H), 1.34 (m, 2H). MS (ESI-) m/z 560 (M-H)⁺.

### Example 115

### N-[trans-4-({5-[5-{[carbamoyl(methyl)amino]methyl}-2-(2,6-dimethylphenoxy)phenyl]-1-methyl-2-oxo-1,2-dihydropyridin-4-yl}oxy)cyclohexyl]-1-methylcyclopropane-1-carboxamide

### Example 115a

### 1-(3-bromo-4-(2,6-dimethylphenoxy)benzyl)-1-methylurea

Example 113c (0.515 g, 1.61 mmol) and isocyanatotrimethylsilane (0.700 mL, 4.40 mmol) were combined in dichloromethane (10 mL). The reaction mixture was stirred at ambient temperature for 72 hours, and then partitioned with dichloromethane and water. The aqueous layer was extracted with dichloromethane again. The combined organic layers were dried with anhydrous sodium sulfate, filtered, and concentrated. The residue was purified by flash chromatography (silica gel, 0-10% ammonia saturated methanol in dichloromethane) to provide the title compound (296 mg, 51% yield).

### Example 115b

### N-[trans-4-({5-[5-{[carbamoyl(methyl)amino]methyl}-2-(2,6-dimethylphenoxy)phenyl]-1-methyl-2-oxo-1,2-dihydropyridin-4-yl}oxy)cyclohexyl]-1-methylcyclopropane-1-carboxamide

Example 115b was prepared according to the procedure used for the preparation of Example 113e, substituting Example 115a for Example 113d, to provide the title compound (30 mg, 64% yield). ¹H NMR (400 MHz, DMSO-*d*₆) δ 7.58 (s, 1H), 7.15 - 6.96 (m, 6H), 6.19 (d, *J* = 8.3 Hz, 1H), 5.92 (s, 1H), 5.86 (s, 2H), 4.32 - 4.23 (m, 3H), 3.55 - 3.45 (m, 1H), 3.36 (s, 3H), 2.69 (s, 3H), 2.02 - 1.92 (m, 8H), 1.70 - 1.61 (m, 2H), 1.46 - 1.32 (m, 2H), 1.28 - 1.16 (m, 5H), 0.87 (q, *J=* 3.4 Hz, 2H), 0.42 (q, *J=* 3.6 Hz, 2H). (ESI+) m/z 587 (M+H)⁺.

### Example 116

### N-{trans-4-[(5-{5-[(1R)-1-{acetyl[(1S)-1-phenylethyl]amino)ethyl]-2-(2,6-dimethylphenoxy)phenyl}-1-methyl-2-oxo-1,2-dihydropyridin-4-yl)oxy]cyclohexyl}-1-methylcyclopropane-1-carboxamide

### Example 116a

### 1-(3-bromo-4-(2,6-dimethylphenoxy)phenyl)ethanone

1-(3-Bromo-4-fluorophenyl)ethanone (10.9 g, 50.1 mmol), 2,6-dimethylphenol (6.12 g, 50.1 mmol) and cesium carbonate (19.6 g, 60.2 mmol) were combined in dimethyl sulfoxide (63 mL). The reaction mixture was heated at 100 °C for 45 minutes, cooled to ambient temperature, and partitioned with ethyl acetate and water. The organic layer was dried with anhydrous magnesium sulfate, filtered, and concentrated. The residue was purified by flash chromatography (silica gel, 0-15% methyl tert-butyl ether in heptanes) to provide the title compound (15.6 g, 98% yield).

### Example 116b

### (1R)-1-[3-bromo-4-(2,6-dimethylphenoxy)phenyl]-N-[(1S)-1-phenylethyl]ethan-1-amine

To a solution of Example 116a (0.896 g, 2.81 mmol) and (S)-1-phenylethanamine (0.684 g, 5.64 mmol) in dichloromethane (28 mL) at 0 °C under nitrogen was added 1M titanium(IV) chloride in dichloromethane (2.81 mL, 2.81 mmol) dropwise. The reaction mixture was stirred at ambient temperature for 90 minutes, treated with sodium cyanoborohydride (0.367 g, 5.84 mmol), and stirred at ambient temperature for 20 hours. The reaction mixture was carefully quenched with cold water, and extracted with dichloromethane. The organic layer was washed with saturated aqueous sodium chloride, dried with anhydrous sodium sulfate, filtered, and concentrated. The residue was purified by flash chromatography (silica gel, 0-100% 3:1 ethyl acetate/ethanol in heptanes) to give two diastereomers. The first diastereomer to elute was the title compound (232 mg, 19% yield).

### Example 116c

### N-{(1R)-1-[3 -bromo-4-(2,6-dimethylphenoxy)phenyl] ethyl} -N-[(1S)-1-phenylethyl] acetamide

Example 116b (232 mg, 0.547 mmol), acetyl chloride (0.078 mL, 1.1 mmol) and triethylamine (0.229 mL, 1.64 mmol) were combined in dichloromethane (7 mL). The reaction mixture was stirred at ambient temperature for 72 hours, partitioned with dichloromethane and saturated aqueous sodium bicarbonate, and the organic layer was dried with anhydrous magnesium sulfate, filtered, and concentrated. The residue was purified by flash chromatography (silica gel, 0-10% methanol in dichloromethane) to provide the title compound (231 mg, 91% yield).

### Example 116d

### N-{trans-4-[(5-{5-[(1R)-1-{acetyl[(1S)-1-phenylethyl]amino}ethyl]-2-(2,6-dimethylphenoxy)phenyl}-1-methyl-2-oxo-1,2-dihydropyridin-4-yl)oxy]cyclohexyl}-1-methylcyclopropane-1-carboxamide

Example 116d was prepared according to the procedure used for the preparation of Example 113e, substituting Example 116c for Example 113d, to provide the title compound (29 mg, 53% yield). ¹H NMR (400 MHz, DMSO-*d*₆) δ 7.56 (s, 1H), 7.39 - 7.01 (m, 11H), 6.28 - 6.17 (m, 1H), 5.93 (s, 1H), 5.01 (s, br, 1H), 4.81 (s, br, 1H), 4.31 - 4.22 (m, 1H), 3.50 - 3.44 (m, 1H), 3.37 (s, 3H), 2.04 - 1.91 (m, 9H), 1.84 - 1.60 (m, 4H), 1.45 - 1.31 (m, 5H), 1.25 (d, *J=* 6.9 Hz, 3H), 1.24 -1.12 (m, 5H), 0.88 (q, *J=* 3.5 Hz, 2H), 0.42 (q, *J=* 3.4 Hz, 2H). (ESI+) m/z 690 (M+H)⁺.

### Example 117

### 2,2,2-trifluoro-N-[trans-4-({5-[2-(4-fluoro-2,6-dimethylphenoxy)-5-(2-hydroxypropan-2-yl)phenyl] -1-methyl-2-oxo-1,2-dihydropyridin-4-yl} oxy)cyclohexyl]acetamide

### Example 117a

### N-{trans-4-[(5-bromo-1-methyl-2-oxo-1,2-dihydropyridin-4-yl)oxy]cyclohexyl}-2,2,2-trifluoroacetamide

A mixture of Example 1e (0.07 g, 0.207 mmol), trifluoroacetic anhydride (0.044 mL, 0.311 mmol), and triethylamine (0.126 g, 1.244 mmol) in dichloromethane (2 mL) was stirred at ambient temperature for 2 hours. The reaction mixture was partitioned between water and ethyl acetate. The aqueous layer was extracted with additional ethyl acetate three times. The combined organic layers were washed with saturated aqueous sodium chloride, dried over anhydrous magnesium sulfate, filtered, and concentrated to afford the title compound (0.07 g, 0.176 mmol, 85 % yield).

### Example 117b

### 2,2,2-trifluoro-N-[trans-4-({5-[2-(4-fluoro-2,6-dimethylphenoxy)-5-(2-hydroxypropan-2-yl)phenyl] -1 -methyl-2-oxo-1 ,2-dihydropyridin-4-yl} oxy)cyclohexyl]acetamide

Example 117b was prepared according to the procedure used for the preparation of Example 61b, substituting Example 117a for Example 61a.
¹H NMR (400 MHz, DMSO-*d*₆) δ 9.29 (d, J = 7.5 Hz, 1H), 7.57 (s, 1H), 7.28 - 7.20 (m, 2H), 6.95 (d, J = 9.2 Hz, 1H), 6.17 (d, J = 8.6 Hz, 1H), 5.98 (s, 1H), 4.33 (tt, J = 9.7, 4.0 Hz, 1H), 3.54 (tdt, J = 11.1, 7.7, 4.0 Hz, 4H), 2.06 - 1.99 (m, 2H), 1.98 (s, 8H), 1.78 - 1.68 (m, 2H), 1.53 - 1.39 (m, 2H), 1.37 (s, 9H), 1.33 - 1.18 (m, 4H). (ESI+) m/z 591.3 (M+H)⁺.

### Example 118

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl}oxy)cyclohexyl]-4-hydroxy-3,3-dimethylbutanamide

### Example 118a

### N-{trans-4-[(5-bromo-1-methyl-2-oxo-1,2-dihydropyridin-4-yl)oxy]cyclohexyl} -4-hydroxy-3,3-dimethylbutanamide

Example 1e (338 mg, 1.00 mmol), 4,4-dimethyldihydrofuran-2(3*H*)-one (457 mg, 4.00 mmol), and *N,N*-diisopropylethylamine (1.75 mL, 10.0 mmol) were combined in tetrahydrofuran (4 mL). To this reaction mixture was added 2M trimethylaluminum in toluene (1.00 mL, 2.00 mmol) dropwise. The reaction mixture was stirred at ambient temperature for 72 hours. To this mixture was then added saturated aqueous sodium bicarbonate slowly, and the mixture was extracted with ethyl acetate. The organic layer was washed with saturated aqueous sodium chloride, dried with anhydrous sodium sulfate, filtered, and concentrated. The residue was purified by flash chromatography (silica gel, 2-6% methanol in dichloromethane) to provide the title compound (292 mg, 70% yield).

### Example 118b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl}oxy)cyclohexyl]-4-hydroxy-3,3-dimethylbutanamide

Example 118b was prepared according to the procedure used for the preparation of Example 60, substituting Example 118a for Example 24a and substituting Example 3c for Example 1j, to provide the title compound (50 mg, 82% yield). ¹H NMR (400 MHz, DMSO-*d₆*) δ 7.66 (d, *J=* 7.6 Hz, 1H), 7.57 (s, 1H), 7.28 - 7.20 (m, 2H), 6.95 (d, *J=* 9.1 Hz, 2H), 6.17 (d, *J=* 8.5 Hz, 1H), 5.95 (s, 1H), 4.90 (s, 1H), 4.59 (t, *J=* 5.6 Hz, 1H), 4.42 - 4.32 (m, 1H), 3.51 - 3.42 (m, 1H), 3.36 (s, 3H), 3.10 *(d, J=* 5.5 Hz, 2H), 2.03 - 1.86 (m, 10H), 1.71 - 1.60 (m, 2H), 1.37 (s, 6H), 1.32 - 1.20 (m, 4H), 0.82 (s, 6H). (ESI-) m/z 607 (M-H)⁺.

### Example 119

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-hydroxy-4-methylpentanamide

### Example 119a

### N- {trans-4-[(5-methyl-2-oxo-1,2-dihydropyridin-4-yl)oxy]cyclohexyl} -4-hydroxy-4-methylpentanamide

Example 119a was prepared according to the procedure used for the preparation of Example 118a, substituting 5,5-dimethyldihydrofaran-2(3*H*)-one for 4,4-dimethyldihydrofuran-2(3H)-one, to provide the title compound (257 mg, 62% yield).

### Example 119b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-hydroxy-4-methylpentanamide

Example 119b was prepared according to the procedure used for the preparation of Example 60, substituting Example 119a for Example 24a and substituting Example 3c for Example 1j, to provide the title compound (44 mg, 72% yield). ¹H NMR (400 MHz, DMSO-*d₆*) δ 7.68 (d, *J* = 7.5 Hz, 1H), 7.57 (s, 1H), 7.27 - 7.21 (m, 2H), 6.95 (d, *J* = 9.2 Hz, 2H), 6.17 (d, *J=* 8.5 Hz, 1H), 5.95 (s, 1H), 4.90 (s, 1H), 4.40 - 4.30 (m, 1H), 4.16 (s, 1H), 3.47 - 3.40 (m, 1H), 3.36 (s, 3H), 2.09 - 2.03 (m, 2H), 1.98 (s, 6H), 1.97 - 1.88 (m, 2H), 1.71 - 1.60 (m, 2H), 1.55 - 1.49 (m, 2H), 1.37 (s, 6H), 1.33 - 1.18 (m, 4H), 1.01 (s, 6H). (ESI-) m/z 607 (M-H)⁺.

### Example 120

### 4-{[trans-4-(4,4-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

### Example 120a

### 5-bromo-4-{[trans-4-(4,4-dimethyl-2-oxopyrrolidin-1-yl)cyclohexyl]oxy}-1-methylpyridin-2(1H)-one

Example 120a was prepared according to the procedure used for the preparation of Example 48a, substituting Example 118a for Example 47a, to provide the title compound (75 mg, 94% yield).

### Example 120b

### 4-{[trans-4-(4,4-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1H)-one

Example 120b was prepared according to the procedure used for the preparation of Example 60, substituting Example 120a for Example 24a and substituting Example 3c for Example 1j, to provide the title compound (37 mg, 63% yield). ¹H NMR (400 MHz, DMSO-*d₆*) δ 7.56 (s, 1H), 7.27 - 7.21 (m, 2H), 6.95 (d, *J=* 9.1 Hz, 2H), 6.17 (d, *J=* 8.3 Hz, 1H), 5.91 (s, 1H), 4.90 (s, 1H), 4.38 - 4.31 (m, 1H), 3.79 (s, 2H), 3.43 - 3.33 (m, 4H), 2.18 (s, 2H), 1.98 (s, 6H), 1.96 - 1.89 (m, 2H), 1.60 - 1.52 (m, 2H), 1.36 (s, 6H), 1.33 - 1.21 (m, 4H), 1.00 (s, 6H). (ESI+) m/z 591 (M+H)⁺.

### Example 121

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]pyrazolidine-3,5-dione

### Example 121a

### ethyl 3-(2-{trans-4-[(5-bromo-1-methyl-2-oxo-1,2-dihydropyridin-4-yl)oxy]cyclohexyl}hydrazinyl)-3-oxopropanoate

A solution of Example 98b (1.0 g, 3.33 mmol) and ethyl 3-hydrazinyl-3-oxopropanoate (0.730 g, 5.00 mmol) in dichloromethane (30.3 mL) and acetic acid (3.03 mL) was stirred at ambient temperature for 1 hour, cooled to 5 °C and treated portionwise with sodium triacetoxyhydroborate (2.118 g, 10.00 mmol). The mixture was then stirred at ambient temperature for 16 hours and partitioned with 5% aqueous sodium bicarbonate. The organic layer was washed with saturated aqueous sodium chloride, dried over anhydrous sodium sulfate, filtered, and concentrated. LCMS shows two distinct peaks for the presumed cis and trans isomers. Purification of the residue by silica chromatography (1-9% methanol in dichloromethane, 80 g silica cartridge) afforded separation of the cis isomer as the first eluting isomer (0.7 g, 49% yield), ¹H NMR (501 MHz, DMSO-*d*₆) δ 9.41 (d, *J* = 6.3 Hz, 1H), 8.00 (s, 1H), 5.93 (s, 1H), 4.78 (dd, *J=* 6.4, 3.5 Hz, 1H), 4.58 (s, 1H), 4.10 - 4.01 (m, 2H), 3.31 (s, 3H), 3.15 (s, 2H), 1.85 (m, 2H), 1.65 - 1.37 (m, 6H), 1.18 - 1.12 (m, 3H) and the title compound (later eluting, 0.19 g, 13% yield), ¹H NMR (400 MHz, DMSO-*d*₆) δ 9.41 (d, *J* = 6.4 Hz, 1H), 7.97 (s, 1H), 5.96 (s, 1H), 4.81 (dd, *J=* 6.4, 3.7 Hz, 1H), 4.41 (dt, *J=* 9.3, 5.1 Hz, 1H), 4.03 (qd, J= 7.4, 3.1 Hz, 2H), 3.30 (s, 3H), 3.14 (s, 2H), 2.74 (m, 1H), 1.96 (m 2H), 1.78 (d, *J=* 12.1 Hz, 2H), 1.41 - 1.20 (m, 4H), 1.15 (t, *J=* 7.1, 3H).

### Example 121b

### 1-{trans-4-[(5-bromo-1-methyl-2-oxo-1,2-dihydropyridin-4-yl)oxy]cyclohexyl}pyrazolidine-3,5-dione

A solution of Example 112a (0.19 g, 0.442 mmol) in ethanol (5.89 mL) at ambient temperature under nitrogen was treated portionwise with sodium ethoxide (0.063 g, 0.927 mmol). The resulting yellow solution was stirred at ambient temperature for 1 hour and partitioned between ethyl acetate and water, adjusting the pH to 7 with dilute hydrochloric acid. The aqueous layer was extracted twice more with ethyl acetate. The organics were combined and washed with saturated aqueous sodium chloride, dried over anhydrous sodium sulfate, filtered, and concentrated. Purification of the residue by trituration in 9:1 heptane/ethyl acetate afforded the title compound as a tan powder (0.14 g, 75% yield).

### Example 121c

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]pyrazolidine-3,5-dione

Example 3c (0.046 g, 0.115 mmol), Example 121b (0.04 g, 0.104 mmol), tris(dibenzylideneacetone)dipalladium(0) (2.86 mg, 3.12 µmol), 1,3,5,7-tetramethyl-6-phenyl-2,4,8-trioxa-6-phosphaadamantane (3.04 mg, 10.41 µmol) and sodium carbonate (0.055 g, 0.521 mmol) were combined and sparged with argon for 15 minutes. Meanwhile a solution of 4:1 tetrahydrofuran/water (1.2 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (reverse phase C18, acetonitrile and 10 mM ammonium acetate in water) afforded the title compound as a white powder (0.0068 g, 11% yield). ¹H NMR (400 MHz, DMSO-*d*₆) δ 7.57 (s, 1H), 7.28 7.21 (m, 2H), 6.95 (d, J = 9.1 Hz, 2H), 6.17 (d, J = 8.4 Hz, 1H), 5.91 (s, 1H), 4.89 (s, 1H), 4.23 (dt, J = 11.0, 6.3 Hz, 1H), 3.88 (s, 1H), 3.37 (s, 3H), 2.04 (d, J = 12.2 Hz, 1H), 1.99 (s, 4H), 1.74 (s, 2H), 1.62 (s, 2H), 1.37 (s, 4H), 1.26 (q, J = 12.8, 12.4 Hz, 2H). MS (ESI-) m/z 576 (M-H)⁺.

### Example 122

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]morpholine-4-carboxamide

### Example 122a

### N-{trans-4-[(5-bromo-1-methyl-2-oxo-1,2-dihydropyridin-4-yl)oxy]cyclohexyl}morpholine-4-carboxamide

A solution of 4-morpholinecarbonyl chloride (0.011 mL, 0.096 mmol) in dichloromethane (1 mL) was added to a vial charged with Example 1e (0.03 g, 0.08 mmol). Triethylamine (0.05 mL, 0.359 mmol) was then added and the mixture was stirred at ambient temperature for 5 hours. *N,N*-dimethylpyridin-4-amine (1.7 mg, 0.014 mmol) was added and stirring was continued at 30 °C overnight. The solid was collected by filtration, rinsed with a heptanes/dichloromethane mixture (4 mL, 1:1) and then dried in a vacuum oven at 60 °C to give 0.0191 g (50.9% yield) of the title compound.

### Example 122b

### N-[trans-4-({5-[2-(4-fluoro-2,6-dimethylphenoxy)-5-(2-hydroxypropan-2-yl)pheny1]-1-methyl-2-oxo-1,2-dihydropyridin-4-yl} oxy)cyclohexyl]morpholine-4-carboxamide

Example 122b was prepared according to the procedure used for the preparation of Example 2h, substituting Example 122a for Example 2d and substituting Example 3c for Example 2g. The compound was purified by flash chromatography (amine-functionalized silica gel, 0 to 60% of a 3:1 mixture of ethyl acetate/ethanol in heptanes). The material was purified further by reverse phase HPLC (C18, acetonitrile/water (0.1% trifluoroacetic acid), 10-100%). The relevant fractions were neutralized with aqueous saturated sodium bicarbonate solution and extracted with ethyl acetate (4 x 5 mL). The combined extracts were dried over anhydrous magnesium sulfate, filtered, concentrated, and dried in a vacuum oven at 60 °C to provide the title compound. ¹H NMR (501 MHz, DMSO-*d*₆) δ 7.60 (s, 1H), 7.27 (m, 2H), 6.99 (d, J = 9.1 Hz, 2H), 6.21 (m, 2H), 5.96 (s, 1H), 4.94 (s, 1H), 4.33 (ddd, J = 13.8, 9.2, 3.8 Hz, 1H), 3.51 (m, 4H), 3.40 (s, 3H), 3.37 (m, 1H), 3.23 (m, 4H), 2.02 (s, 6H), 1.99 (m, 2H), 1.73 (m, 2H), 1.40 (s, 6H), 1.33 (m, 2H), 1.26 (m, 2H). MS (ESI-) m/z 605.8 (M-H)⁻.

### Example 123

### N-[trans-4-({5-[2-(4-fluoro-2,6-dimethylphenoxy)-5-(2-hydroxypropan-2-yl)pheny1]-1-methyl-2-oxo-1,2-dihydropyridin-4-yl}oxy)cyclohexyl]-2-oxoimidazolidine-1-carboxamide

### Example 123a

### N- {trans-4-[(5-bromo-1 -methyl-2-oxo-1,2-dihydropyridin-4-yl)oxy]cyclohexyl}-2-oxoimidazolidine-1-carboxamide

Example 123a was prepared according to the procedure used for the preparation of Example 122a, substituting 2-oxoimidazolidine-1-carbonyl chloride for 4-morpholinecarbonyl chloride.

### Example 123b

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-oxoimidazolidine-1-carboxamide

Example 123b was prepared according to the procedure used for the preparation of Example 2h, substituting Example 123a for Example 2d and substituting Example 3c for Example 2g. The compound was purified by flash chromatography (amine-functionalized silica gel, 0 to 70% of a 3:1 mixture of ethyl acetate/ethanol in heptanes). The material was further purified by reverse phase HPLC (C18, acetonitrile/water (0.1% trifluoroacetic acid), 10-100%). The relevant fractions were neutralized with aqueous saturated sodium bicarbonate solution and extracted with ethyl acetate (4 x 5 mL). The combined extracts were dried over anhydrous magnesium sulfate, filtered, concentrated, and dried in a vacuum oven at 60 °C to provide the title compound. ¹H NMR (500 MHz, DMSO-*d*₆) δ 8.13 (d, J = 7.6 Hz, 1H), 7.61 (s, 1H), 7.55 (s, 1H), 7.30 (d, J = 2.3 Hz, 1H), 7.27 (dd, J = 8.5, 2.4 Hz, 1H), 6.98 (d, J = 9.1 Hz, 2H), 6.21 (d, J = 8.5 Hz, 1H), 5.97 (s, 1H), 4.94 (s, 1H), 4.45 (m, 1H), 3.70 (dd, J = 9.2, 7.3 Hz, 2H), 3.49 (m, 1H), 3.40 (s, 3H), 3.29 (m, 2H), 2.02 (s, 6H), 1.89 (m, 2H), 1.76 (m, 2H), 1.40 (s, 6H), 1.36 (m, 4H). MS (ESI+) m/z 607.1 (M+H)⁺.

### Example 124

### (5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methylimidazolidine-2,4-dione

### Example 124a

### tert-butyl [(2S)-1-({trans-4-[(5-bromo-1-methyl-2-oxo-1,2-dihydropyridin-4-yl)oxy]cyclohexyl}amino)-1-oxopropan-2-yl]carbamate

Example 124a was prepared according to the procedure used for the preparation of Example 78a, substituting *N*-(*tert*-butoxycarbonyl)-L-alanine for *N*-(*tert*-butoxycarbonyl)-*N-*methylglycine, to provide the title compound as a white solid. (0.258 g, 0.547 mmol, 88% yield).

### Example 124b

### N- {trans-4-[(5-bromo-1 -methyl-2-oxo-1,2-dihydropyridin-4-yl)oxy]cyclohexyl} -L-alaninamide trifluoroacetate salt

Example 124b was prepared according to the procedure used for the preparation of Example 78b, substituting Example 124a for Example 78a, to provide the title compound as a colorless glass. (0.2942 g, 0.600 mmol, 100% yield).

### Example 124c

### (5S)-3-{trans-4-[(5-bromo-1-methyl-2-oxo-1,2-dihydropyridin-4-yl)oxy]cyclohexyl}-5-methylimidazolidine-2,4-dione

Example 124c was prepared according to the procedure used for the preparation of Example 78c, substituting Example 124b for Example 78b, to provide the title compound as a white solid. (0.095 g, 0.240 mmol, 44% yield).

### Example 124d

### (5S)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-5-methylimidazolidine-2,4-dione

Example 124d was prepared according to the procedure used for the preparation of Example 1k, substituting Example 124c for Example 1f and substituting Example 3c for Example 1j, to provide the title compound as a white solid. (0.0318 g, 0.054 mmol, 60% yield). Enantiomeric excess (Chiral SFC) = 94%. ¹H NMR (501 MHz, DMSO-*d*₆) δ 8.13 (s, 1H), 7.58 (s, 1H), 7.29 - 7.22 (m, 2H), 6.97 (d, J = 9.1 Hz, 2H), 6.18 (d, J = 8.5 Hz, 1H), 5.97 (s, 1H), 4.93 (s, 1H), 4.38 - 4.29 (m, 1H), 4.02 - 3.93 (m, 1H), 3.73 - 3.63 (m, 1H), 3.38 (s, 3H), 2.20 (s, 2H), 2.07 (d, J = 10.4 Hz, 2H), 2.00 (s, 6H), 1.62 - 1.55 (m, 2H), 1.39 (s, 6H), 1.28 - 1.16 (m, 5H). MS (ESI+) m/z 592.1 (M+H)⁺.

### Example 125

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]imidazolidine-2,4-dione

### Example 125a

### tert-butyl [2-({trans-4-[(5-bromo-1-methyl-2-oxo-1,2-dihydropyridin-4-yl)oxy]cyclohexyl} amino)-2-oxoethyl]carb amate

Example 125a was prepared according to the procedure used for the preparation of Example 78a, substituting *N*-(*tert*-butoxycarbonyl)glycine for *N*-(*tert*-butoxycarbonyl)-*N-*methylglycine, to provide the title compound as a white solid. (0.2714 g, 0.592 mmol, 99% yield).

### Example 125b

### N-{trans-4-[(5-bromo-1-methyl-2-oxo-1,2-dihydropyridin-4-yl)oxy]cyclohexyl} glycinamide trifluoroacetate salt

Example 125b was prepared according to the procedure used for the preparation of Example 78b, substituting Example 125a for Example 78a, to provide the title compound as a colorless glass (0.360 g, 0.592 mmol, 100% yield).

### Example 125c

### 3-{trans-4-[(5-bromo-1-methyl-2-oxo-1,2-dihydropyridin-4-yl)oxy]cyclohexyl} imidazolidine-2,4-dione

Example 125c was prepared according to the procedure used for the preparation of Example 78c, substituting Example 125b for Example 78b, to provide the title compound as a white solid (0.0393 g, 0.102 mmol, 17% yield).

### Example 125d

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]imidazolidine-2,4-dione

Example 125d was prepared according to the procedure used for the preparation of Example 1k, substituting Example 125c for Example 1f, and substituting Example 3c for Example 1j to provide the title compound as a white solid (0.0203 g, 0.035 mmol, 34% yield). ¹H NMR (400 MHz, DMSO-*d*₆) δ 7.95 (s, 1H), 7.56 (s, 1H), 7.28 - 7.19 (m, 2H), 6.95 (d, J = 9.1 Hz, 2H), 6.17 (d, J = 8.4 Hz, 1H), 5.96 (s, 1H), 4.91 (s, 1H), 4.32 (dt, J = 10.8, 6.5 Hz, 1H), 3.79 (d, J = 1.1 Hz, 2H), 3.75 - 3.61 (m, 1H), 3.37 (s, 3H), 2.21 (q, J = 13.2 Hz, 2H), 2.06 (d, J = 12.5 Hz, 2H), 1.99 (s, 6H), 1.61 - 1.52 (m, 2H), 1.37 (s, 6H), 1.28 - 1.17 (m, 2H). MS (ESI+) m/z 578.1 (M+H)⁺.

### Example 126

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1H-imidazo[5,1-c][1,4]oxazine-1,3(2H)-dione

### Example 126a

### tert-butyl (3S)-3-({trans-4-[(5-bromo-1-methyl-2-oxo-1,2-dihydropyridin-4-yl)oxy]cyclohexyl} carbamoyl)morpholine-4-carboxylate

Example 126a was prepared according to the procedure used for the preparation of Example 78a, substituting (3*S*)-4-(*tert*-butoxycarbonyl)morpholine-3-carboxylic acid for *N-*(tert-butoxycarbonyl)-N-methylglycine to provide the title compound as a white solid (0.292 g, 0.568 mmol, 95% yield).

### Example 126b

### (3S)-N-{trans-4-[(5-bromo-1-methyl-2-oxo-1,2-dihydropyridin-4-yl)oxy]cyclohexyl}morpholine-3-carboxamide trifluoroacetate salt

Example 126b was prepared according to the procedure used for the preparation of Example 78b, substituting Example 126a for Example 78a, to provide the title compound as a colorless glass (0.287 g, 0.543 mmol, 96% yield).

### Example 126c

### 2-{trans-4-[(5-bromo-1-methyl-2-oxo-1,2-dihydropyridin-4-yl)oxy]cyclohexyl}tetrahydro-1H-imidazo[5,1-c][1,4]oxazine-1,3(2H)-dione

Example 126c was prepared according to the procedure used for the preparation of Example 78c, substituting Example 126b for Example 78b, to provide the title compound as a white solid (0.1146 g, 0.260 mmol, 48% yield).

### Example 126d

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1H-imidazo[5,1-c][1,4]oxazine-1,3(2H)-dione

Example 126d was prepared according to the procedure used for the preparation of Example 1k, substituting Example 126c for Example 1f and substituting Example 3c for Example 1j to provide the title compound as a white solid (0.0365 g, 0.058 mmol, 50% yield). ¹H NMR (400 MHz, DMSO-*d*₆) δ 7.56 (s, 1H), 7.28 - 7.19 (m, 2H), 6.95 (d, J = 9.1 Hz, 2H), 6.17 (d, J = 8.5 Hz, 1H), 5.97 (s, 1H), 4.91 (s, 1H), 4.42 - 4.26 (m, 1H), 4.14 (dd, J = 10.8, 5.0 Hz, 1H), 4.01 (dt, J = 9.6, 4.8 Hz, 1H), 3.82 - 3.65 (m, 3H), 3.36 (s, 3H), 3.31 - 3.19 (m, 2H), 3.09 - 2.96 (m, 1H), 2.17 (td, J = 13.6, 13.0, 10.4 Hz, 2H), 2.06 (d, J = 12.4 Hz, 2H), 1.99 (s, 6H), 1.61 (d, J = 11.8 Hz, 2H), 1.37 (s, 6H), 1.31 - 1.09 (m, 2H). MS (ESI+) m/z 634.2 (M+H)⁺.

### Example 127

### 1-ethyl-3-[trans-4-({5-[2-(4-fluoro-2,6-dimethylphenoxy)-5-(2-hydroxypropan-2-yl)phenyl] 1-methyl-2-oxo-1,2-dihydropyridin-4-yl} oxy)cyclohexyl]imidazolidine-2,4-dione

### Example 127a

### tert-butyl [2-({trans-4-[(5-bromo-1-methyl-2-oxo-1,2-dihydropyridin-4-yl)oxy]cyclohexyl} amino)-2-oxoethyl]ethylcarbamate

A mixture of *tert*-butoxycarbonyl *N*-ethylglycinate (0.181 g, 0.889 mmol), 1H-benzo[*d*][1,2,3]triazol-1-ol hydrate (0.147 g, 0.963 mmol) and *N*¹-((ethylimino)methylene)-*N*³,*N*³-dimethylpropane-1,3-diamine hydrochloride (0.185 g, 0.963 mmol) in tetrahydrofuran (4.0 mL)/*N,N*-dimethylformamide (0.3 mL) was treated with Example 1e (0.310 mL, 2.22 mmol) at 5 °C. The mixture was stirred at ambient temperature for 18 hours and partitioned with water and ethyl acetate. The organic layer was washed with aqueous saturated sodium chloride, dried over anhydrous sodium sulfate, filtered, and concentrated. Purification of the residue by chromatography (silica gel, 25-60% of 3:1 ethyl acetate/ethanol in heptanes) afforded the title compound as a white foam (0.29 g, 77% yield).

### Example 127b

### N-{trans-4-[(5-bromo-1-methyl-2-oxo-1,2-dihydropyridin-4-yl)oxy]cyclohexyl} -N²- ethylglycinamide trifluoroacetate salt

A solution of Example 127a (0.29 g, 0.596 mmol) in dichloromethane (9.54 mL) was treated with trifluoroacetic acid (2.385 mL), stirred for 1 hour and concentrated. The residue was azeotroped 3 x 20 mL with toluene affording the title compound (0.27 g, 97% yield). The crude material was used without purification.

### Example 127c

### 3- { trans-4-[(5 -bromo-1 -methyl-2-oxo-1,2-dihydropyridin-4-yl)oxy]cyclohexyl}-1-ethylimidazolidine-2,4-dione

A mixture of Example 127b (0.29 g, 0.580 mmol), triethylamine (0.323 mL, 2.319 mmol), *N*,*N*-dimethylpyridin-4-amine (0.142 g, 1.159 mmol) and di(1*H*-imidazol-1-yl)methanone (0.188 g, 1.159 mmol) in *N*-methyl-2-pyrrolidinone (1.932 mL) was placed in a sealed tube and heated at 105 °C for 24 hours. The reaction mixture was cooled to ambient temperature and partitioned between ethyl acetate and water. The organic layer was washed with 5% aqueous sodium bicarbonate, saturated aqueous sodium chloride, dried over anhydrous sodium sulfate, filtered, and concentrated. Purification of the residue by chromatography (reverse phase C18, 10-90% acetonitrile in water (0.1% trifluoroacetic acid) afforded the title compound. (0.048 g, 14% yield).

### Example 127d

### 1-ethyl-3-[trans-4-({5-[2-(4-fluoro-2,6-dimethylphenoxy)-5-(2-hydroxypropan-2-yl)phenyl] 1-methyl-2-oxo-1,2-dihydropyridin-4-yl} oxy)cyclohexyl]imidazolidine-2,4-dione

Example 3c (0.040 g, 0.100 mmol), Example 127c (0.048 g, 0.091 mmol), tris(dibenzylideneacetone)dipalladium(0) (2.500 mg, 2.73 µmol), 1,3,5,7-tetramethyl-6-phenyl-2,4,8-trioxa-6-phosphaadamantane (2.66 mg, 9.10 µmol) and sodium carbonate (0.048 g, 0.455 mmol) were combined and sparged with argon for 15 minutes. Meanwhile a solution of 4:1 tetrahydrofuran/water (1.2 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-6% methanol in dichloromethane) afforded the title compound as a foam (0.030 g, 52% yield). ¹H NMR (400 MHz, DMSO-*d*₆) δ 7.59 (s, 1H), 7.30 - 7.22 (m, 2H), 6.97 (d, J = 9.1 Hz, 2H), 6.18 (d, J = 8.4 Hz, 1H), 5.98 (s, 1H), 4.93 (s, 1H), 4.35 (m, 1H), 3.89 (s, 2H), 3.70 (m, 1H), 3.38 (s, 3H), 3.26 (q, J = 7.2 Hz, 2H), 2.21 (q, J = 12.8, 12.1 Hz, 2H), 2.07 (d, J = 12.0 Hz, 2H), 2.01 (s, 6H), 1.59 (d, J = 12.3 Hz, 2H), 1.39 (s, 6H), 1.30 - 1.18 (m, 2H), 1.04 (t, J = 7.2 Hz, 3H). MS (ESI+) m/z 606 (M+H)⁺.

### Example 128

### N-[trans-4-(15-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -N'-methylurea

### Example 128a

### N-{trans-4-[(5-bromo-1-methyl-2-oxo-1,2-dihydropyridin-4-yl)oxy]cyclohexyl} -N'-methylurea

Example 1e (0.05 g, 0.149 mmol) and 2,5-dioxopyrrolidin-1-yl methylcarbamate (0.031 g, 0.179 mmol) were combined with dichloromethane (1 mL) and stirred at ambient temperature for 10 minutes. 2-Methyl tetrahydrofuran (0.4 mL) was added and stirring was continued for 6 hours. Triethylamine (0.05 mL, 0.359 mmol), *N*-methyl-2-pyrrolidinone (0.4 mL), dimethyl sulfoxide (1.3 mL) and additional 2,5-dioxopyrrolidin-1-yl methylcarbamate (0.031 g, 0.179 mmol) were added sequentially and stirring was continued overnight at ambient temperature. The reaction mixture was partitioned between ethyl acetate and water, washed with saturated aqueous sodium chloride, dried over anhydrous magnesium sulfate, filtered, and concentrated to provide 0.0386 g (54.6% yield, 75% purity) of the title compound as a mixture with a succinimide by-product.

### Example 128b

### N-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -N'-methylurea

Example 128b was prepared according to the procedure used for the preparation of Example 2h, substituting Example 128a for Example 2d and substituting Example 3c for Example 2g. The compound was purified by flash chromatography (amine-functionalized silica gel, 0 to 60% of a 3:1 mixture of ethyl acetate/ethanol in heptanes) to provide the title compound as a mixture. The material was further purified by reverse phase HPLC (C18, acetonitrile/water (0.1% trifluoroacetic acid), 10-100%). The relevant fractions were neutralized with aqueous saturated sodium bicarbonate solution and extracted with ethyl acetate (4 x 5 mL). The combined extracts were dried over anhydrous magnesium sulfate, filtered, concentrated, and dried in a vacuum oven at 60 °C to provide the title compound. ¹H NMR (400 MHz, DMSO-*d*₆) δ 7.47 (s, 1H), 7.33 (d, J = 2.4 Hz, 1H), 7.28 (dd, J = 8.6, 2.4 Hz, 1H), 6.91 (d, J = 9.1 Hz, 2H), 6.27 (d, J = 8.6 Hz, 1H), 5.91 (s, 1H), 5.44 (d, J = 7.4 Hz, 1H), 5.36 (dd, J = 9.7, 5.0 Hz, 1H), 4.39 (m, 1H), 4.35 (tt, J = 8.8, 3.9 Hz, 1H), 3.42 (s, 3H), 3.36 (m, 1H), 2.56 (d, J = 4.7 Hz, 3H), 2.05 (s, 6H), 1.95 (m, 2H), 1.77 (m, 2H), 1.46 (s, 6H), 1.32 (m, 4H). LCMS (APCI+) m/z 534.3 (M+H)⁺.

### Example 129

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-azabicyclo[3.1.0]hexane-2,4-dione

### Example 129a

### 3- {trans-4-[(5-bromo-1-methyl-2-oxo-1,2-dihydropyridin-4-yl)oxy]cyclohexyl}-3-azabicyclo[3.1.0]hexane-2,4-dione

A mixture of Example 1e (0.25 g, 0.740 mmol), 3-oxabicyclo[3.1.0]hexane-2,4-dione (0.083 g, 0.740 mmol), and triethylamine (0.227 mL, 1.63 mmol) in xylene (4.0 mL) was heated at 135 °C for 3 hours and concentrated. The crude intermediate was treated with sodium acetate (0.067 g, 0.814 mmol) in acetic anhydride (4.00 mL) and heated at 130 °C for 18 hours, cooled to ambient temperature, and concentrated. The residue was treated with 5 mL of water, adjusting the pH to 7 with 5% aqueous sodium bicarbonate. The mixture was sonicated for 10 minutes to give a fine solid dispersion which was collected by filtration, washed with water, and dried to constant mass affording the title compound as a tan solid (0.22 g, 71% yield).

### Example 129b

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-azabicyclo[3.1.0]hexane-2,4-dione

Example 3c (0.056 g, 0.139 mmol), Example 129a (0.05 g, 0.127 mmol), tris(dibenzylideneacetone)dipalladium(0) (3.48 mg, 3.80 µmol), 1,3,5,7-tetramethyl-6-phenyl-2,4,8-trioxa-6-phosphaadamantane (3.70 mg, 0.013 mmol) and sodium carbonate (0.067 g, 0.633 mmol) were combined and sparged with argon for 15 minutes. Meanwhile a solution of 4:1 tetrahydrofuran/water (1.2 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 2-6% methanol in dichloromethane) afforded the title compound as a foam (0.0586 g, 75% yield). ¹H NMR (400 MHz, DMSO-*d*₆) δ 7.57 (s, 1H), 7.28 - 7.23 (m, 2H), 6.98 - 6.94 (m, 2H), 6.18 (d, J = 8.5 Hz, 1H), 5.97 (s, 1H), 4.90 (s, 1H), 4.32 (td, J = 11.0, 5.3 Hz, 1H), 3.55 (m, 1H), 3.38 (s, 3H), 2.48 (m, 2H), 2.14 - 2.01 (m, 4H), 2.00 (s, 6H), 1.50 (dd, J = 10.4, 4.5 Hz, 2H), 1.45 (dq, J = 8.0, 4.1 Hz, 1H), 1.39 (s, 6H), 1.36 (m, 1H), 1.19 (m, 2H). MS (ESI+) m/z 589 (M+H)⁺.

### Example 130

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-6-methylpyridazin-3(2H)-one

### Example 130a

### 5-bromo-4-((4-hydroxycyclohexyl)oxy)-1-methylpyridin-2(1H)-one

A mixture of Example 98b (0.6 g, 1.999 mmol) and sodium tetrahydroborate (0.151 g, 4.00 mmol) in tetrahydrofuran (15 mL) was heated at 60 °C 2 hours. The reaction mixture was quenched with methanol. The excess solvents were removed under reduced pressure. The residue was partitioned between water and ethyl acetate. The aqueous layer was extracted with additional ethyl acetate three times. The combined organic layers were washed with saturated aqueous sodium chloride, dried over anhydrous magnesium sulfate, filtered, and concentrated to give the title compound (0.574 g, 1.900 mmol, 95 % yield).

### Example 130b

### 2-{trans-4-[(5-bromo-1-methyl-2-oxo-1,2-dihydropyridin-4-yl)oxy]cyclohexyl}-6-methylpyridazin-3(2H)-one

A mixture of Example 130a (0.12 g, 0.397 mmol), 6-methylpyridazin-3(2*H*)-one (0.066 g, 0.596 mmol), and triphenylphosphine (0.260 g, 0.993 mmol) in tetrahydrofuran (5 mL) was cooled to 0 °C. To this solution was added (*E*)-di-tert-butyl diazene-1,2-dicarboxylate (0.229 g, 0.993 mmol). The reaction mixture was stirred at ambient temperature overnight. Additional 6-methylpyridazin-3(2*H*)-one (0.066 g, 0.596 mmol), triphenylphosphine (0.260 g, 0.993 mmol), and (*E*)-di-tert-butyl diazene-1,2-dicarboxylate (0.229 g, 0.993 mmol) were added sequentially. The reaction mixture was stirred at 50 °C overnight. The solvent was removed, and the residue was loaded onto a 15 g silica gel cartridge and blow-dry. The cartridge was then mounted onto a 24 g of silica gel column and eluted with 15% methanol in ethyl acetate to give the title compound (0.049 g, 0.124 mmol, 31.3 % yield).

### Example 130c

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-6-methylpyridazin-3(2H)-one

Example 130c was prepared according to the procedure used for the preparation of Example 61b, substituting Example 130b for Example 61a. ¹H NMR (400 MHz, DMSO-*d*₆) δ 7.59 (d, J = 2.7 Hz, 1H), 7.33 - 7.22 (m, 4H), 6.99 - 6.92 (m, 2H), 6.87 (dd, J = 9.4, 1.7 Hz, 1H), 6.19 (dd, J = 8.5, 2.6 Hz, 1H), 6.02 (d, J = 2.5 Hz, 1H), 4.94 (d, J = 2.8 Hz, 1H), 4.66 (s, 1H), 4.44 (s, 1H), 3.38 (s, 3H), 3.31 (s, 3H), 2.15 -2.07 (m, 2H), 2.02 (s, 6H), 1.93 - 1.78 (m, 2H), 1.74 (d, J = 10.1 Hz, 2H), 1.38 (s, 6H), 1.38 - 1.28 (m, 2H). (ESI+) m/z 588.0 (M+H)⁺.

### Example 131

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-(propan-2-yl)imidazolidine-2,4-dione

### Example 131a

### tert-butyl [(2R)-1-({trans-4-[(5-bromo-1-methyl-2-oxo-1,2-dihydropyridin-4-yl)oxy] cyclohexyl} amino) -3 -methyl-1 -oxobutan-2-yl] carbamate

Example 131a was prepared according to the procedure used for the preparation of Example 78a, substituting *N*-(tert-butoxycarbonyl)-D-valine for *N*-(*tert*-butoxycarbonyl)-*N-*methylglycine, to provide the title compound as a white solid. (0.3511 g, 0.702 mmol, 100% yield).

### Example 131b

### N-{trans-4-[(5-bromo-1-methyl-2-oxo-1,2-dihydropyridin-4-yl)oxy]cyclohexyl} -D-valinamide trifluoroacetate salt

Example 131b was prepared according to the procedure used for the preparation of Example 78b, substituting Example 131a for Example 78a, to provide the title compound as a colorless glass. (0.419 g, 0.702 mmol, 100% yield).

### Example 131c

### (5R)-3-{trans-4-[(5-bromo-1-methyl-2-oxo-1,2-dihydropyridin-4-yl)oxy]cyclohexyl} -5-(propan-2-yl)imidazolidine-2,4-dione

Example 131c was prepared according to the procedure used for the preparation of Example 78c, substituting Example 131b for Example 78b, to provide the title compound as a white solid. (0.208 g, 0.488 mmol, 70% yield).

### Example 131d

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-(propan-2-yl)imidazolidine-2,4-dione

Example 131d was prepared according to the procedure used for the preparation of Example 1k, substituting Example 131c for Example 1f and substituting Example 3c for Example 1j, to provide the title compound as a white solid. (0.0399 g, 0.064 mmol, 72% yield). Enantiomeric excess (Chiral SFC) = 94.6%. ¹H NMR (500 MHz, DMSO-*d*₆) δ 8.21 (d, J = 1.4 Hz, 1H), 7.61 (s, 1H), 7.32 - 7.24 (m, 2H), 7.03 - 6.97 (m, 2H), 6.21 (d, J = 8.4 Hz, 1H), 6.00 (s, 1H), 4.95 (s, 1H), 4.36 (td, J = 10.5, 5.2 Hz, 1H), 3.88 (dd, J = 3.4, 1.3 Hz, 1H), 3.72 (tt, J = 12.2, 3.9 Hz, 1H), 3.41 (s, 3H), 2.25 (d, J = 12.7 Hz, 2H), 2.10 (d, J = 11.7 Hz, 2H), 2.05 - 2.00 (m, 6H), 1.57 (s, 2H), 1.41 (s, 6H), 1.27 (s, 3H), 0.93 (d, J = 7.0 Hz, 3H), 0.75 (d, J = 6.7 Hz, 3H). MS (ESI+) m/z 620.0 (M+H)⁺.

### Example 132

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methylimidazolidine-2,4-dione

### Example 132a

### tert-butyl [(2R)-1-({trans-4-[(5-bromo-1-methyl-2-oxo-1,2-dihydropyridin-4-yl)oxy]cyclohexyl}amino)-1-oxopropan-2-yl]carbamate

Example 132a was prepared according to the procedure used for the preparation of Example 78a, substituting *N*-(*tert*-butoxycarbonyl)-D-alanine for *N*-(*tert*-butoxycarbonyl)-*N-*methylglycine, to provide the title compound as a white solid (0.2679 g, 0.567 mmol, 96% yield).

### Example 132b

### N-{trans-4-[(5-bromo-1-methyl-2-oxo-1,2-dihydropyridin-4-yl)oxy]cyclohexyl}-D-alaninamide trifluoroacetate salt

Example 132b was prepared according to the procedure used for the preparation of Example 78b, substituting Example 132a for Example 78a, to provide the title compound as a colorless glass (0.3467 g, 0.567 mmol, 100% yield).

### Example 132c

### (5R)-3-{trans-4-[(5-bromo-1-methyl-2-oxo-1,2-dihydropyridin-4-yl)oxy]cyclohexyl} -5-methylimidazolidine-2,4-dione

Example 132c was prepared according to the procedure used for the preparation of Example 78c, substituting Example 132b for Example 78b, to provide the title compound as a white solid (0.161 g, 0.404 mmol, 71% yield).

### Example 132d

### (5R)-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-5-methylimidazolidine-2,4-dione

Example 132d was prepared according to the procedure used for the preparation of Example 1k, substituting Example 132c for Example 1f, and substituting Example 3c for Example 1j, to provide the title compound as a white solid. (0.0315 g, 0.053 mmol, 59% yield). Enantiomeric excess (Chiral SFC) = 86%. ¹H NMR (500 MHz, DMSO-*d*₆) δ 8.16 (d, J = 1.3 Hz, 1H), 7.61 (s, 1H), 7.32 - 7.25 (m, 2H), 7.00 (d, J = 9.1 Hz, 2H), 6.21 (d, J = 8.5 Hz, 1H), 6.00 (s, 1H), 4.94 (s, 1H), 4.36 (dt, J = 11.0, 6.5 Hz, 1H), 4.00 (qd, J = 6.9, 1.2 Hz, 1H), 3.70 (tt, J = 12.2, 3.8 Hz, 1H), 3.41 (s, 3H), 2.30 - 2.16 (m, 2H), 2.10 (d, J = 11.8 Hz, 2H), 2.03 (s, 6H), 1.60 (d, J = 12.2 Hz, 2H), 1.41 (s, 6H), 1.31 - 1.22 (m, 2H), 1.21 (d, J = 6.9 Hz, 3H). MS (ESI+) m/z 592.2 (M+H)⁺.

### Example 133

### 5-ethylidene-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione

### Example 133a

### tert-butyl [(2S,3R)-1-({trans-4-[(5-bromo-1-methyl-2-oxo-1,2-dihydropyridin-4-yl)oxy]cyclohexyl}amino)-3-hydroxy-1-oxobutan-2-yl]carbamate

Example 133a was prepared according to the procedure used for the preparation of Example 78a, substituting *N*-(*tert*-butoxycarbonyl)-L-threonine for *N*-(*tert*-butoxycarbonyl)-*N*-methylglycine, to provide the title compound as a white solid (0.2504 g, 0.498 mmol, 80% yield).

### Example 133b

### N-{trans-4-[(5-bromo-1-methyl-2-oxo-1,2-dihydropyridin-4-yl)oxy]cyclohexyl}-L-threoninamide trifluoroacetate salt

Example 133b was prepared according to the procedure used for the preparation of Example 78b, substituting Example 132a for Example 78a, to provide the title compound as a colorless glass (0.3109 g, 0.498 mmol, 100% yield).

### Example 133c

### 3-{trans-4-[(5-bromo-1-methyl-2-oxo-1,2-dihydropyridin-4-yl)oxy]cyclohexyl}-5-ethylideneimidazolidine-2,4-dione

Example 133c was prepared according to the procedure used for the preparation of Example 78c, substituting Example 133b for Example 78b, to provide the title compound as a white solid (0.0244 g, 0.059 mmol, 12% yield).

### Example 133d

### 5-ethylidene-3-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl] imidazolidine-2,4-dione

Example 133d was prepared according to the procedure used for the preparation of Example 1k, substituting Example 133c for Example 1f and substituting Example 3c for Example 1j, to provide the title compound as a white solid, in a 3:1 mix of *Z*- to *E*-isomers. (0.0206 g, 0.034 mmol, 57% yield). ¹H NMR (500 MHz, DMSO-*d*₆) δ 10.42 (s, 1H), 10.16 (s, 0H), 7.61 (s, 1H), 7.32 - 7.25 (m, 2H), 7.00 (d, J = 9.1 Hz, 2H), 6.21 (d, J = 8.5 Hz, 1H), 6.01 (d, J = 2.3 Hz, 1H), 5.64 (q, J = 7.4 Hz, 1H), 5.54 (q, J = 7.6 Hz, 0H), 4.95 (s, 1H), 4.43 - 4.33 (m, 0H), 3.84 - 3.73 (m, 1H), 3.41 (s, 3H), 2.28 - 2.18 (m, 2H), 2.08 (dd, J = 23.1, 10.1 Hz, 3H), 2.03 (s, 6H), 1.76 (d, J = 7.5 Hz, 2H), 1.64 (d, J = 12.1 Hz, 2H), 1.41 (s, 6H), 1.30 - 1.22 (m, 4H).. MS (ESI+) m/z 604.2 (M+H)⁺.

### Example 134

### 5-[2-(4-fluoro-2,6-dimethylphenoxy)-5-(2-hydroxypropan-2-yl)phenyl]-1-methyl-4-{[trans-4-(2,3,4,4-tetramethyl-5-oxoimidazolidin-1-yl)cyclohexyl]oxylpyridin-2(1H)-one

¹H NMR (400 MHz, DMSO-*d*₆) δ 7.58 (s, 1H), 7.29 - 7.22 (m, 2H), 6.97 (d, *J=* 9.1 Hz, 2H), 6.18 (d, *J=* 8.4 Hz, 1H), 6.01 (s, 1H), 4.92 (s, 1H), 4.43 - 4.33 (m, 1H), 3.89 (q, *J=* 5.3 Hz, 1H), 3.49 - 3.35 (m, 4H), 2.18 (s, 3H), 2.10 - 1.88 (m, 10H), 1.63 - 1.53 (m, 2H), 1.39 (s, 6H), 1.28 - 1.18 (m, 5H), 1.04 (s, 3H), 0.87 (s, 3H). (ESI+) m/z 620 (M+H)⁺.

### Example 135

### 3-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -1 -(2-methoxyethyl)imidazolidine-2,4-dione

### Example 135a

### 3-{trans-4-[(5-bromo-1-methyl-2-oxo-1,2-dihydropyridin-4-yl)oxy]cyclohexyl}-1-(2-methoxyethyl)imidazolidine-2,4-dione

A mixture of Example 125c (0.05 g, 0.130 mmol), potassium carbonate (0.054 g, 0.390 mmol), and 2-bromoethyl methyl ether (0.045 g, 0.325 mmol) in *N,N-*dimethylformamide (0.434 mL) under nitrogen was stirred at 100 °C for 24 hours, cooled to ambient temperature, and partitioned between ethyl acetate and water. The organic layer was washed with saturated aqueous sodium chloride, dried over anhydrous sodium sulfate, filtered, and concentrated to afford the title compound (0.048 g, 58% yield). The crude material was used without additional purification.

### Example 135b

### 3-[trans-4-({5-[2-(4-fluoro-2,6-dimethylphenoxy)-5-(2-hydroxypropan-2-yl)phenyl]-1-methyl-2-oxo-1 ,2-dihydropyridin-4-yl} oxy)cyclohexyl] -1 -(2-methoxyethyl)imidazolidine-2,4-dione

Example 3c (0.040 g, 0.099 mmol), Example 135a (0.04 g, 0.090 mmol), tris(dibenzylideneacetone)dipalladium(0) (2.484 mg, 2.71 µmol), 1,3,5,7-tetramethyl-6-phenyl-2,4,8-trioxa-6-phosphaadamantane (2.64 mg, 9.04 µmol) and sodium carbonate (0.048 g, 0.452 mmol) were combined and sparged with argon for 15 minutes. Meanwhile a solution of 4:1 tetrahydrofuran/water (2.0 mL) was sparged with nitrogen for 15 minutes and transferred by syringe into the reaction vessel under argon. The mixture was stirred for 16 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1-6% methanol in dichloromethane) afforded the title compound as a foam (0.0172 g, 28% yield). ¹H NMR (400 MHz, DMSO-*d*₆) δ 7.58 (s, 1H), 7.32 - 7.20 (m, 2H), 6.97 (d, J = 9.1 Hz, 2H), 6.19 (d, J = 8.4 Hz, 1H), 5.98 (s, 1H), 4.91 (s, 1H), 4.34 (p, J = 6.4 Hz, 1H), 3.91 (s, 2H), 3.78 - 3.64 (m, 1H), 3.42 (dq, J = 7.6, 4.0, 3.4 Hz, 4H), 3.38 (s, 3H), 3.22 (s, 3H), 2.21 (q, J = 12.8, 11.8 Hz, 2H), 2.08 (d, J = 12.3 Hz, 2H), 2.01 (s, 6H), 1.59 (d, J = 12.2 Hz, 2H), 1.39 (s, 6H), 1.23 (q, J = 11.1 Hz, 2H). MS (ESI+) m/z 636 (M+H)⁺.

### Example 136

### 6-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4,6-diazaspiro[2.4]heptane-5,7-dione

### Example 136a

### tert-butyl[1-({trans-4-[(5-bromo-1-methyl-2-oxo-1,2-dihydropyridin-4-yl)oxy]cyclohexyl}carbamoyl)cyclopropyl]carbamate

A mixture of 1-[(*tert*-butoxycarbonyl)amino]cyclopropane-1-carboxylic acid (0.286 g, 1.422 mmol), 1*H*-benzo[*d*][1,2,3]triazol-1-ol hydrate (0.218 g, 1.422 mmol) and *N*¹-((ethylimino)methylene)-*N*³,*N*³-dimethylpropane-1,3-diamine hydrochloride (0.273 g, 1.422 mmol) in ethyl acetate (6.97 mL) was treated with Example 1e (0.4 g, 1.185 mmol) and then with triethylamine (0.495 mL, 3.55 mmol) at ambient temperature.The mixture was stirred at ambient temperature for 3 hours and then partitioned with water and ethyl acetate. The organic layer was washed with aqueous saturated sodium chloride, dried over anhydrous sodium sulfate, filtered, and concentrated. Purification of the residue by chromatography (silica gel, 1-5% methanol in dichloromethane) afforded the title compound as a white powder (0.43 g, 73% yield).

### Example 136b

### 1-amino-N-{trans-4-[(5-bromo-1-methyl-2-oxo-1,2-dihydropyridin-4-yl)oxy]cyclohexyl}cyclopropane-1-carboxamide hydrochloride

Example 136a (0.43 g, 0.888 mmol) in 4M hydrochloric acid in dioxane (6 mL, 24.00 mmol) was stirred for 2 hour and concentrated. The residue was azeotroped 3 x 20 mL with toluene to afford the title compound (0.37 g, 100% yield).The crude material was used without purification.

### Example 136c

### 6-{trans-4-[(5-bromo-1-methyl-2-oxo-1,2-dihydropyridin-4-yl)oxy]cyclohexyl}-4,6-diazaspiro[2.4]heptane-5,7-dione

A mixture of Example 136b (0.37 g, 0.879 mmol), triethylamine (1.226 mL, 8.79 mmol), N,N-dimethylpyridin-4-amine (0.322 g, 2.64 mmol) and di(1*H*-imidazol-1-yl)methanone (0.428 g, 2.64 mmol) in acetonitrile (6.0 mL) was stirred at 100 °C in a sealed microwave tube for 24 hours, cooled to ambient temperature, and concentrated. The resulting solid was triturated in 10 mL of water for 15 minutes, collected by filtration, washed with a minimal volume of water, and dried to constant mass affording the title compound (0.305 g, 83% yield).

### Example 136d

### 6-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4,6-diazaspiro[2.4]heptane-5,7-dione

Example 136c (0.05 g, 0.122 mmol), Example 3c (0.054 g, 0.134 mmol), tris(dibenzylideneacetone)dipalladium(0) (3.35 mg, 3.66 µmol), 1,3,5,7-tetramethyl-6-phenyl-2,4,8-trioxa-6-phosphaadamantane (3.56 mg, 0.012 mmol) and sodium carbonate (0.065 g, 0.609 mmol) were combined and sparged with argon for 15 minutes. Meanwhile a solution of 4:1 tetrahydrofuran/water (2.0 mL) was sparged with nitrogen for 15 minutes and transferred by syringe into the reaction vessel under argon. The mixture was stirred for 6 hours under argon at 60 °C, cooled to ambient temperature, and partitioned between ethyl acetate and water. The organic layer was washed with saturated aqueous sodium chloride, dried over anhydrous sodium sulfate, treated with 3-mercaptopropyl functionalized silica gel, filtered, and concentrated. Purification of the residue by chromatography (silica gel, 1.5-8% methanol in dichloromethane) afforded the title compound as a foam (0.041, 54% yield). ¹H NMR (400 MHz, DMSO-*d*₆) δ 8.30 (s, 1H), 7.58 (s, 1H), 7.30 - 7.22 (m, 2H), 6.97 (d, J = 9.1 Hz, 2H), 6.19 (d, J = 8.4 Hz, 1H), 5.98 (s, 1H), 4.91 (s, 1H), 4.26 (m, 1H), 3.75 (m, 1H), 3.38 (s, 3H), 2.23 (q, J = 12.7 Hz, 2H), 2.08 (d, J = 12.0 Hz, 2H), 2.01 (s, 6H), 1.62 (d, J = 12.0 Hz, 2H), 1.39 (s, 6H), 1.32 - 1.20 (m, 2H), 1.18 (ddd, J = 8.2, 6.0, 4.0 Hz, 4H). MS (ESI-) m/z 602 (M-H)⁺.

### Example 137

### 4-acetamido-N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[2.1.1]hexane-1-carboxamide

### Example 137a

### 4-amino-N-{trans-4-[(5-bromo-1-methyl-2-oxo-1,2-dihydropyridin-4-yl)oxy]cyclohexyl}bicyclo[2.1.1]hexane-1-carboxamide hydrochloride

Example 137a was prepared according to the procedure used for the preparation of Example 93a, substituting Example 103a for Example 72a.

### Example 137b

### 4-acetamido-N-{trans-4-[(5-bromo-1-methyl-2-oxo-1,2-dihydropyridin-4-yl)oxy]cyclohexyl}bicyclo[2.1.1]hexane-1-carboxamide

Example 137b was prepared according to the procedure used for the preparation of Example 10c, substituting Example 137a for Example 10b.

### Example 137c

### 4-acetamido-N-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[2.1.1]hexane-1-carboxamide

Example 137c was prepared according to the procedure used for the preparation of Example 2h, substituting Example 137b for Example 2d, and substituting Example 3c for Example 2g. The compound was purified by flash chromatography (amine-functionalized silica gel, 0 to 80% of a 3:1 mixture of ethyl acetate/ethanol in heptanes). The relevant fractions were concentrated and then further purified by reverse phase HPLC (C18, acetonitrile/water (0.1% trifluoroacetic acid, 10-100%). The relevant fractions were neutralized with saturated aqueous sodium bicarbonate solution and extracted with ethyl acetate (4 x 5 mL). The organic extracts were combined, dried over anhydrous magnesium sulfate, filtered, concentrated and dried in a vacuum oven at 60 °C to provide the title compound. ¹H NMR (500 MHz, DMSO-*d*₆) δ 7.70 (s, 1H), 7.46 (s, 1H), 7.31 (d, J = 2.4 Hz, 1H), 7.26 (dd, J = 8.6, 2.4 Hz, 1H), 6.89 (d, J = 9.1 Hz, 2H), 6.75 (m, 1H), 6.24 (d, J = 8.6 Hz, 1H), 5.88 (s, 1H), 4.40 (m, 1H), 4.29 (tt, J = 8.9, 4.0 Hz, 1H), 3.52 (m, 1H), 3.39 (s, 3H), 2.02 (s, 6H), 1.99 (m, 2H), 1.94 (m, 2H), 1.78 (m, 2H), 1.76 (s, 3H), 1.72 (m, 4H), 1.59 (m, J = 3.9, 1.9 Hz, 2H), 1.43 (s, 6H), 1.34 (m, 4H). LCMS (ESI+) m/z 660.4 (M+H)⁺.

### Example 138

### (7aR)-2-[trans-4-(15-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1H-pyrrolo[1,2-c]imidazole-1,3(2H)-dione

### Example 138a

### tert-butyl (2R)-2-({trans-4-[(5-bromo-1-methyl-2-oxo-1,2-dihydropyridin-4-yl)oxy]cyclohexyl}carbamoyl)pyrrolidine-1-carboxylate

A mixture of 1-(*tert*-butoxycarbonyl)-D-proline (0.281 g, 1.303 mmol), 1*H-*benzo[*d*][1,2,3]triazol-1-ol hydrate (0.218 g, 1.422 mmol) and *N*¹-((ethylimino)methylene)-*N*³,*N*³-dimethylpropane-1,3-diamine hydrochloride (0.273 g, 1.422 mmol) in ethyl acetate (6.97 mL) at 5 °C was treated with Example 1e (0.4 g, 1.185 mmol) and then with triethylamine (0.495 mL, 3.55 mmol).The mixture was stirred at ambient temperature for 3 hours and partitioned with water and ethyl acetate. The organic layer was washed with aqueous saturated sodium chloride, dried over anhydrous sodium sulfate, filtered, and concentrated. Purification of the residue by trituration (9:1 heptane/ethyl acetate) afforded the title compound as a white powder (0.49 g, 78%).

### Example 138b

### N-{trans-4-[(5-bromo-1-methyl-2-oxo-1,2-dihydropyridin-4-yl)oxy]cyclohexyl}-D-prolinamide hydrochloride

Example 138a (0.49 g, 0.983 mmol) in 4M hydrochloric acid in dioxane (10 mL, 40.0 mmol) was stirred for 2 hour and concentrated. The residue was azeotroped 3 x 20 mL with toluene to afford the title compound (0.42 g, 98%).The crude material was used without purification.

### Example 138c

### (7aR)-2-{trans-4-[(5-bromo-1-methyl-2-oxo-1,2-dihydropyridin-4-yl)oxy]cyclohexyl}tetrahydro-1H-pyrrolo[1,2-c]imidazole-1,3(2H)-dione

A mixture of Example 138b (0.2 g, 0.459 mmol), triethylamine (0.640 mL, 4.59 mmol), *N,N*-dimethylpyridin-4-amine (0.168 g, 1.377 mmol) and di(1*H*-imidazol-1-yl)methanone (0.223 g, 1.377 mmol) in acetonitrile (3 mL) was stirred at 100 °C in a sealed microwave tube for 24 hours, cooled, and concentrated. The resulting solid was triturated in 10 mL of water for 15 minutes, collected by filtration, washed with a minimal volume of water, and dried to constant mass affording the title compound (0.155 g, 76%).

### Example 138d

### (7aR)-2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1H-pyrrolo[1,2-c] imidazole-1 ,3 (2H)-dione

Example 138c (0.05 g, 0.118 mmol), Example 3c (0.052 g, 0.130 mmol), tris(dibenzylideneacetone)dipalladium(0) (3.24 mg, 3.54 µmol), 1,3,5,7-tetramethyl-6-phenyl-2,4,8-trioxa-6-phosphaadamantane (3.44 mg, 0.012 mmol) and sodium carbonate (0.062 g, 0.589 mmol) were combined and sparged with argon for 15 minutes. Meanwhile a solution of 4:1 tetrahydrofuran/water (2.0 mL) was sparged with nitrogen for 15 minutes and transferred by syringe into the reaction vessel under argon. The mixture was stirred for 16 hours under argon at 60 °C, cooled, and partitioned between ethyl acetate and water. The organic layer was washed with saturated aqueous sodium chloride, dried over anhydrous sodium sulfate, treated with 3-mercaptopropyl functionalized silica gel, filtered and concentrated. Purification of the residue by chromatography (silica, 2-6% methanol in dichloromethane) afforded the title compound as a foam (0.051, 67%). Enantiomeric excess (Chiral SFC) = 89%. ¹H NMR (501 MHz, DMSO-*d*₆) δ 7.59 (s, 1H), 7.30 - 7.24 (m, 2H), 6.98 (d, J = 9.1 Hz, 2H), 6.20 (d, J = 8.5 Hz, 1H), 6.00 (s, 1H), 4.92 (s, 1H), 4.35 (td, J = 10.6, 5.2 Hz, 1H), 4.09 (dd, J = 9.1, 7.5 Hz, 1H), 3.67 (tt, J = 12.2, 3.9 Hz, 1H), 3.46 (dt, J = 10.8, 7.7 Hz, 1H), 3.40 (s, 3H), 3.11 (ddd, J = 10.8, 7.9, 4.6 Hz, 1H), 2.25 - 2.14 (m, 2H), 2.08 (dp, J = 11.3, 3.5 Hz, 3H), 2.02 (s, 6H), 1.98 - 1.86 (m, 2H), 1.66 - 1.52 (m, 3H), 1.40 (s, 6H), 1.24 (q, J = 10.8 Hz, 2H). MS (ESI+) m/z 618 (M+H)⁺.

### Example 139

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2-methylalaninamide

### Example 139a

### tert-butyl[1-({trans-4-[(5-bromo-1-methyl-2-oxo-1,2-dihydropyridin-4-yl)oxy]cyclohexyl}amino)-2-methyl-1-oxopropan-2-yl]carbamate

2-((*Tert*-butoxycarbonyl)amino)-2-methylpropanoic acid (320 mg, 1.58 mmol), 1-hydroxybenzotriazole hydrate (241 mg, 1.58 mmol) and 1-(3-dimethylaminopropyl)-3-ethylcarbodiimide hydrochloride (302 mg, 1.58 mmol) were combined in the mixture of tetrahydrofuran (8 mL)/N,N-dimethylformamide (2 mL). The reaction mixture was treated with Example 1e (506 mg, 1.50 mmol) and triethylamine (0.627 mL, 4.50 mmol), stirred at ambient temperature for 16 hours, and partitioned with ethyl acetate and water. The organic layer was washed with saturated aqueous sodium chloride, dried with anhydrous sodium sulfate, filtered, and concentrated to provide the title compound (730 mg, 100%).

### Example 139b

### N-{trans-4-[(5-bromo-1-methyl-2-oxo-1,2-dihydropyridin-4-yl)oxy]cyclohexyl}-2-methylalaninamide hydrochloride

To a slurry of Example 139a (0.730 g, 1.50 mmol) in dioxane (8 mL) was added 4M hydrogen chloride in dioxane (8.0 mL, 32 mmol). The reaction mixture was stirred at ambient temperature for 2 hours and concentrated. The residue was azeotroped with toluene three times to provide the title compound (634 mg, 100%).

### Example 139c

### N-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2-methylalaninamide

Example 139c was prepared according to the procedure used for the preparation of Example 60, substituting Example 139b for Example 24a and substituting Example 3c for Example 1j. Purification of the residue by flash chromatography (silica gel, 4-10% methanol in dichloromethane) provided the title compound (7 mg, 15%). ¹H NMR (500 MHz, DMSO-*d*₆) δ 7.64 (d, *J=* 7.9 Hz, 1H), 7.59 (s, 1H), 7.28 - 7.24 (m, 2H), 6.97 (d, *J=* 9.2 Hz, 2H), 6.19 (d, *J=* 8.4 Hz, 1H), 5.94 (s, 1H), 4.92 (s, 1H), 4.40 - 4.34 (m, 1H), 3.45 - 3.37 (m, 4H), 2.00 (s, 6H), 1.97 - 1.91 (m, 2H), 1.72 - 1.65 (m, 2H), 1.39 (s, 6H), 1.38 - 1.24 (m, 4H), 1.12 (s, 6H). (ESI-) m/z 578 (M-H)⁺.

### Example 140

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5,5-dimethylimidazolidine-2,4-dione

### Example 140a

### 3-{trans-4-[(5-bromo-1-methyl-2-oxo-1,2-dihydropyridin-4-yl)oxy]cyclohexyl}-5,5-dimethylimidazolidine-2,4-dione

Example 139b (338 mg, 0.800 mmol), *N,N'*-carbonyldiimidazole (519 mg, 3.20 mmol), 4-dimethylaminopyridine (391 mg, 3.20 mmol) and triethylamine (1.12 mL, 8.00 mmol) were combined in acetonitrile (8 mL). The reaction mixture was heated at 60 °C for 16 hours, cooled and concentrated. The residue was diluted with water, stirred for 5 minutes, filtered, washed with water, and dried to provide the title compound (275 mg, 83%)

### Example 140b

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5,5-dimethylimidazolidine-2,4-dione

Example 140b was prepared according to the procedure used for the preparation of Example 60, substituting Example 140a for Example 24a and substituting Example 3c for Example 1j, to provide the title compound (43 mg, 71%). ¹H NMR (500 MHz, DMSO-*d*₆) δ 8.21 (s, 1H), 7.58 (s, 1H), 7.29 - 7.23 (m, 2H), 6.97 (d, *J* = 9.1 Hz, 2H), 6.19 (d, *J* = 8.5 Hz, 1H), 5.98 (s, 1H), 4.91 (s, 1H), 4.38 - 4.31 (m, 1H), 3.72 - 3.64 (m, 1H), 3.38 (s, 3H), 2.25 - 2.15 (m, 2H), 2.11 - 2.05 (m, 2H), 2.01 (s, 6H), 1.62 - 1.56 (m, 2H), 1.39 (s, 6H), 1.28 - 1.19 (m, 8H). (ESI+) m/z 606 (M+H)⁺.

### Example 141

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyridazin-3(2H)-one

### Example 141a

### 2-{trans-4-[(5-bromo-1-methyl-2-oxo-1,2-dihydropyridin-4-yl)oxy]cyclohexyl}pyridazin-3 (2H)-one

Example 141a was prepared according to the procedure used for the preparation of Example 130b, substituting pyridazin-3(2*H*)-one for 6-methylpyridazin-3(2H)-one.

### Example 141b

### 2-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyridazin-3(2H)-one

Example 141b was prepared according to the procedure used for the preparation of Example 61b, substituting Example 141a for Example 61a. ¹H NMR (400 MHz, DMSO-*d*₆) δ 7.91 (dd, J = 3.8, 1.7 Hz, 1H), 7.58 (s, 1H), 7.20 - 7.38 (m, 3H), 6.92 - 6.99 (m, 2H), 6.87 (dd, J = 9.4, 1.7 Hz, 1H), 6.18 (d, J = 8.5 Hz, 1H), 6.00 (s, 1H), 4.91 (s, 1H), 4.69 (tt, J = 11.3, 4.0 Hz, 1H), 4.42 (dq, J = 10.2, 5.2, 4.0 Hz, 1H), 3.37 (s, 3H), 2.06 - 2.14 (m, 2H), 2.00 (s, 6H), 1.93 - 1.78 (m, 2H), 1.74 (d, J = 10.1 Hz, 2H), 1.38 (s, 6H), 1.28 - 1.38 (m, 2H). (ESI+) m/z 574.0 (M+H)⁺.

### Example 142

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5,5-trimethylimidazolidine-2,4-dione

### Example 142a

### 3-{trans-4-[(5-bromo-1-methyl-2-oxo-1,2-dihydropyridin-4-yl)oxy]cyclohexyl}-1,5,5-trimethylimidazo lidine-2,4-dione

To a slurry of Example 140a (61.8 mg, 0.150 mmol) in *N*,*N-*dimethylformamide (1 mL) was added 60 % sodium hydride (18 mg, 0.45 mmol). The reaction mixture was stirred at ambient temperature for 5 minutes, and treated with iodomethane (0.028 mL, 0.45 mmol). The reaction mixture was stirred at ambient temperature for 2 hours, and partitioned with ethyl acetate and water. The organic layer was washed with saturated aqueous sodium chloride, dried with anhydrous sodium sulfate, filtered, and concentrated to provide the title compound (63 mg, 99%).

### Example 142b

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl)oxy)cyclohexyl]-1,5,5-trimethylimidazolidine-2,4-dione

Example 142b was prepared according to the procedure used for the preparation of Example 60, substituting Example 142a for Example 24a and substituting Example 3c for Example 1j, to provide the title compound (59 mg, 95%). ¹H NMR (400 MHz, DMSO-*d*₆) δ 7.58 (s, 1H), 7.30 - 7.23 (m, 2H), 6.97 (d, *J=* 9.1 Hz, 2H), 6.19 (d, *J=* 8.5 Hz, 1H), 5.98 (s, 1H), 4.91 (s, 1H), 4.39 - 4.31 (m, 1H), 3.76 - 3.67 (m, 1H), 3.38 (s, 3H), 2.75 (s, 3H), 2.26 - 2.11 (m, 2H), 2.13 - 2.03 (m, 2H), 2.01 (s, 6H), 1.64 - 1.55 (m, 2H), 1.39 (s, 6H), 1.31 - 1.17 (m, 8H). (ESI+) m/z 620 (M+H)⁺.

### Example 143

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-2(1H)-one

### Example 143a

### 1,4-dioxaspiro[4.5]decan-8-yl 4-methylbenzenesulfonate

A mixture of 1,4-dioxaspiro[4.5]decan-8-ol (5.5 g, 34.8 mmol) and 4-methylbenzene-1-sulfonyl chloride (4.50 mL, 34.8 mmol) in pyridine (30 mL) was stirred at ambient temperature for 1 hour. The majority of the pyridine was removed under reduced pressure. The reaction mixture was partitioned between aqueous 0.1% HCl and ethyl acetate. The aqueous layer was extracted with ethyl acetate several times. The combined organic layers were washed with 0.1% HCl, saturated aqueous sodium chloride, filtered, and concentrated. The residue was purified by flash column chromatography (silica gel, 20% ethyl acetate in heptanes to give the title compound (9.85 g, 31.5 mmol, 91 % yield).

### Example 143b

### 1-(1,4-dioxaspiro[4.5]decan-8-yl)pyrimidin-2(1H)-one

A mixture of Example 143a (0.5 g, 1.601 mmol), pyrimidin-2(1*H*)-one, hydrochloric acid (0.424 g, 3.20 mmol), and cesium carbonate (2.086 g, 6.40 mmol) in *N,N-*dimethylformamide (5 mL) was heated at 80 °C overnight. The reaction mixture was partitioned between water and ethyl acetate. The organic layer was extracted with additional ethyl acetate several times. The combined organic layers were washed with saturated aqueous sodium chloride, filtered, and concentrated. The residue was purified by flash column chromatography (silica gel, 40% ethyl acetate in heptanes) to give the title compound (0.270 g, 1.14 mmol, 71.4 % yield).

### Example 143c

### 1-(4-oxocyclohexyl)pyrimidin-2(1H)-one

Example 143b (0.42 g, 1.778 mmol) in tetrahydrofuran (15 mL) was treated with 5% HCl (5 mL). The reaction mixture was heated at 60 °C for 2 hours. After cooling, the solvent was evaporated under reduced pressure. The reaction mixture was partitioned between water and ethyl acetate. The organic layer was extracted with additional ethyl acetate several times. The combined organic layers were washed with saturated aqueous sodium chloride, filtered, and concentrated. The residue was purified by flash column chromatography (silica gel, 40% ethyl acetate in heptanes) to give the title compound (0.32 g, 1.665 mmol, 94 % yield).

### Example 143d

### 1-(trans-4-hydroxycyclohexyl)pyrimidin-2(1H)-one

A mixture of Example 143c (0.3 g, 1.561 mmol) and sodium tetrahydroborate (0.118 g, 3.12 mmol) in tetrahydrofuran (5 mL) was heated at 60 °C for two hours. The reaction mixture was quenched with methanol. The excess solvents were removed under reduced pressure. The reaction mixture was partitioned between water and ethyl acetate. The organic layer was extracted with additional ethyl acetate several times. The combined organic layers were washed with saturated aqueous sodium chloride, filtered, and concentrated. The residue was purified by flash column chromatography (silica gel, 40% ethyl acetate in heptanes) to give the crude product, which was further purified by reverse phase Preparative HPLC (C18, acetonitrile/water (0.1% trifluoroacetic acid, 10-100%) to give the title compound (0.12 g, 0.618 mmol, 39.6 % yield).

### Example 143e

### 1-{trans-4-[(5-bromo-1-methyl-2-oxo-1,2-dihydropyridin-4-yl)oxy]cyclohexyl}pyrimidin-2(1H)-one

Example 143e was prepared according to the procedure used for the preparation of Example 61a, substituting Example 143d for *trans*-ethyl 4-hydroxycyclohexanecarboxylate.

### Example 143f

### 1-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-2(1H)-one

Example 143f was prepared according to the procedure used for the preparation of Example 61b, substituting Example 143e for Example 61a. ¹H NMR (501 MHz, DMSO-*d*₆) δ 8.53 (d, J = 4.8 Hz, 2H), 7.60 (s, 1H), 7.25 - 7.32 (m, 2H), 7.05 (t, J = 4.8 Hz, 1H), 6.96 (d, J = 9.1 Hz, 2H), 6.21 (d, J = 8.5 Hz, 1H), 5.97 (s, 1H), 4.94 (m, 1H), 4.87 (s, 1H), 4.58 (m, 1H), 3.39 (s, 3H), 2.02 (s, 6H), 1.68 - 1.78 (m, 4H), 1.72 (s, 6H), 1.55 - 1.59 (m, 2H), 1.39 (s, 6H). (ESI+) m/z 574.0 (M+H)⁺.

### Example 144

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-4(3H)-one

### Example 144a

### 3-{trans-4-[(5-bromo-1-methyl-2-oxo-1,2-dihydropyridin-4-yl)oxy]cyclohexyl}pyrimidin-4(3H)-one

Example 144a was prepared according to the procedure used for the preparation of Example 130b, substituting pyrimidin-4(3*H*)-one for 6-methylpyridazin-3(2*H*)-one.

### Example 144b

### 3-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-4(3H)-one

Example 144b was prepared according to the procedure used for the preparation of Example 61b, substituting Example 144a for Example 61a. 1H NMR (400 MHz, DMSO-*d*₆) δ 8.70 (d, J = 1.3 Hz, 1H), 8.45 (d, J = 5.8 Hz, 1H), 7.60 (s, 1H), 7.21 - 7.32 (m, 2H), 6.95 (d, J = 9.1 Hz, 2H), 6.86 (dd, J = 5.8, 1.2 Hz, 1H), 6.19 (d, J = 8.6 Hz, 1H), 5.97 (s, 1H), 5.01 (tt, J = 7.4, 3.5 Hz, 1H), 4.93 (s, 1H), 4.55 (td, J = 8.2, 7.4, 3.8 Hz, 1H), 3.37 (s,3), 1.99 (s, 6H), 1.92 (m, 2H), 1.79 (dq, J = 12.8, 4.0 Hz, 2H), 1.60 (qd, J = 11.9, 10.0, 3.4 Hz, 2H), 1.48 (dp, J = 13.0, 4.8, 4.0 Hz, 2H), 1.38 (s, 6H). (ESI+) m/z 574.0 (M+H)⁺.

### Example 145

### 6-[trans-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-methyl-4,6-diazaspiro[2.4]heptane-5,7-dione

### Example 145a

### 6-{trans-4-[(5-bromo-1-methyl-2-oxo-1,2-dihydropyridin-4-yl)oxy]cyclohexyl}-4-methyl-4,6-diazaspiro[2.4]heptane-5,7-dione

A solution of Example 136c (0.075 g, 0.183 mmol) in *N*,*N-*dimethylformamide (0.914 mL) under nitrogen at ambient temperature was treated portionwise with 95% sodium hydride (9.24 mg, 0.366 mmol), stirred for twenty minutes, and treated with iodomethane (0.023 mL, 0.366 mmol). The mixture was stirred for 2 hours and partitioned between ethyl acetate and water. The organic layer was washed with saturated aqueous sodium chloride, dried over anhydrous sodium sulfate, filtered, and concentrated to afford the title compound (0.07 g, 87%). The crude material was used without additional purification.

### Example 145b

### 6-[trans-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-methyl-4,6-diazaspiro[2.4]heptane-5,7-dione

Example 145a (0.07 g, 0.165 mmol), Example 3c (0.073 g, 0.181 mmol), tris(dibenzylideneacetone)dipalladium(0) (4.53 mg, 4.95 µmol), 1,3,5,7-tetramethyl-6-phenyl-2,4,8-trioxa-6-phosphaadamantane (4.82 mg, 0.016 mmol) and sodium carbonate (0.087 g, 0.825 mmol) were combined and sparged with argon for 15 minutes. Meanwhile a solution of 4:1 tetrahydrofuran/water (2.5 mL) was sparged with nitrogen for 15 minutes and transferred by syringe into the reaction vessel under argon. The mixture was stirred for 16 hours under argon at 60 °C, cooled, and partitioned between ethyl acetate and water. The organic layer was washed with saturated aqueous sodium chloride, dried over anhydrous sodium sulfate, treated with 3-mercaptopropyl functionalized silica gel, filtered and concentrated. Purification of the residue by chromatography (silica, 2-6% methanol in dichloromethane) afforded the title compound as a foam (0.070, 67%). ¹H NMR (501 MHz, DMSO-*d*₆) δ 7.58 (s, 1H), 7.28 (d, J = 2.4 Hz, 1H), 7.25 (dd, J = 8.6, 2.4 Hz, 1H), 6.96 (d, J = 9.1 Hz, 2H), 6.19 (d, J = 8.5 Hz, 1H), 5.98 (s, 1H), 4.92 (s, 1H), 4.34 (tt, J = 10.7, 4.2 Hz, 1H), 3.80 (tt, J = 12.2, 3.9 Hz, 1H), 3.38 (s, 3H), 2.61 (s, 3H), 2.28 - 2.17 (m, 2H), 2.13 - 2.03 (m, 2H), 2.00 (s, 6H), 1.66 - 1.58 (m, 2H), 1.49 - 1.43 (m, 2H), 1.39 (s, 6H), 1.25 (dt, J = 13.6, 9.8 Hz, 2H), 1.14 - 1.07 (m, 2H). MS (ESI+) m/z 618 (M+H)⁺.

### g. Biological Examples

### Bromodomain domain binding assay

A time-resolved fluorescence resonance energy transfer (TR-FRET) assay was used to determine the affinities of compounds of the Examples listed in Table 1 for each bromodomain of BRD4. His-tagged first (BDI: amino acids K57-E168) and second (BDII: amino acids E352- M457) bromodomains of BRD4 were expressed and purified. An Alexa647-labeled BET-inhibitor was used as the fluorescent probe in the assay.

### Synthesis of Alexa647-labeled bromodomain inhibitor compound 2-((6S,Z)-4-(4-chlorophenyl)-2,3,9-trimethyl-6H-thieno[3,2-f][1,2,4]triazolo[4,3-a][1,4]diazepin-6-yl)acetic acid.

Methyl 2-((6S,Z)-4-(4-chlorophenyl)-2,3,9-trimethyl-6H-thieno[3,2-f][1,2,4]triazolo[4,3-a][1,4]diazepin-6-yl)acetate (see e.g., WO 2006129623)(100.95 mg, 0.243 mmol was suspended in 1 mL methanol to which was added a freshly prepared solution of lithium hydroxide monohydrate (0.973 mL, 0.5 M, 0.487 mmol) and shaken at ambient temperature for 3 hours. The methanol was evaporated and the pH adjusted with aqueous hydrochloric acid (1 M, 0.5 mL, 0.5 mmol) and extracted four times with ethyl acetate. The combined ethyl acetate layers were dried over magnesium sulfate and evaporated to afford 2-((6S,Z)-4-(4-chlorophenyl)-2,3,9-trimethyl-6H-thieno[3,2-f][1,2,4]triazolo[4,3-a][1,4]diazepin-6-yl)acetic acid (85.3 mg, 87.0%); ESI-MS m/z = 401.1 [(M+H)⁺] which was used directly in the next reaction.

### N-(2-(2-(2-aminoethoxy)ethoxy)ethyl)-2-((6S,Z)-4-(4-chlorophenyl)-2,3,9-trimethyl-6H-thieno[3,2-f][1,2,4]triazolo[4,3-a][1,4]diazepin-6-yl)acetamide bis(2,2,2-trifluoroacetate).

2-((6S,Z)-4-(4-chlorophenyl)-2,3,9-trimethyl-6H-thieno[3,2-f][1,2,4]triazolo[4,3-a][1,4]diazepin-6-yl)acetic acid)(85.3 mg, 0.213 mmol) was combined with 2,2'-(ethane-1,2-diylbis(oxy))diethanamine (Sigma-Aldrich, 0.315 mg, 2.13 mmol) were combined in 5 mL anhydrous dimethylformamide. (1*H*-benzo[d][1,2,3]triazol-1-yloxy)tripyrrolidin-1-ylphosphonium hexafluorophosphate(V) (PyBOB, CSBio, Menlo Park CA; 332 mg, 0.638 mmol) was added and the reaction shaken at ambient temperature for 16 hours. The reaction was diluted to 6 mL with dimethylsulfoxide:water (9:1, v:v) and purified in two injections with time collection Waters Deltapak C18 200 x 25 mm column eluted with a gradient of 0.1% trifluoroacetic acid (v/v) in water and acetonitrile. The fractions containing the two purified products were lyophilized to afford *N*-(2-(2-(2-aminoethoxy)ethoxy)ethyl)-2-((6*S*,Z)-4-(4-chlorophenyl)-2,3,9-trimethyl-6*H*-thieno[3,2-f][1,2,4]triazolo[4,3-a][1,4]diazepin-6-yl)acetamide bis(2,2,2-trifluoroacetate) (134.4 mg, 82.3%); ESI-MS m/z = 531.1 [(M+H)⁺]; 529.1 [(M-H)⁻] and (S,Z)-*N,N'*-(2,2'-(ethane-1,2-diylbis(oxy))bis(ethane-2,1-diyl))bis(2-((6*S*,Z)-4-(4-chlorophenyl)-2,3,9-trimethyl-6*H*-thieno[3,2-f][1,2,4]triazolo[4,3-a][1,4]diazepin-6-yl)acetamide) bis(2,2,2-trifluoroacetate) (3.0 mg, 1.5%); ESI-MS m/z = 913.2 [(M+H)⁺]; 911.0 [(M-H)⁻].

### N-(2-(2-(2-amido-(Alexa647)-ethoxy)ethoxy)ethyl)-2-((6S,Z)-4-(4-chlorophenyl)-2,3,9-trimethyl-6H-thieno[3,2-f][1,2,4]triazolo[4,3-a][1,4]diazepin-6-yl)acetamide(2,2,2-trifluoroacetate).

*N*-(2-(2-(2-aminoethoxy)ethoxy)ethyl)-2-((6*S*,Z)-4-(4-chlorophenyl)-2,3,9-trimethyl-6*H*-thieno[3,2-f][1,2,4]triazolo[4,3-a][1,4]diazepin-6-yl)acetamide bis(2,2,2-trifluoroacetate) (5.4 mg, 0.0071 mmol) was combined with Alexa Fluor® 647 carboxylic Acid, succinimidyl ester (Life Technologies, Grand Island, NY; 3 mg, 0.0024 mmol) were combined in 1 mL anhydrous dimethylsulfoxide containing diisopropylethylamine (1% v/v) and shaken at ambient temperature for 16 hours. The reaction was diluted to 3 mL with dimethylsulfoxide:water (9:1, v:v) and purified in one injection with time collection Waters Deltapak C18 200 x 25 mm column eluted with a gradient of 0.1% trifluoroacetic acid (v/v) in water and acetonitrile. The fractions containing the purified product were lyophilized to afford *N*-(2-(2-(2-amido-(Alexa647)-ethoxy)ethoxy)ethyl)-2-((6*S,Z*)-4-(4-chlorophenyl)-2,3,9-trimethyl-6*H*-thieno[3,2-f][1,2,4]triazolo[4,3-a][1,4]diazepin-6-yl)acetamide(2,2,2-trifluoroacetate) (1.8 mg); MALDI-MS m/z = 1371.1, 1373.1 [(M+H)⁺] as a dark blue powder.

### Assay

Compound dilution series were prepared in DMSO via an approximately 3-fold serial dilution. Compound dilutions were added directly into white, low-volume assay plates (Perkin Elmer Proxiplate 384 Plus# 6008280) using a Labcyte Echo in conjunction with Labcyte Access and Thermo Multidrop CombinL robotics. Compounds were then suspended in eight microliters (µL) of assay buffer (20 mM Sodium Phosphate, pH 6.0, 50 mM NaCl, 1 mM Ethylenediaminetetraacetic acid disodium salt dihydrate, 0.01% Triton X-100, 1 mM DL-Dithiothreitol) containing His-tagged bromodomain, Europium-conjugated anti-His antibody (Invitrogen PV5596) and Alexa-647-conjugated probe.

The final concentration of 1X assay mixture contained 2% DMSO, 12 nM His tagged BRD4 (BDI_K57-E168) and 100 nM probe or 4 nM His tagged BRD4 (BDII_E352-M457) and 30 nM probe, and 1 nM Europium-conjugated anti-His-tag antibody, and compound concentrations in the range of: 49.02 µM-0.61 nM or 0.98 µM - 0.15 nM.

After a one-hour equilibration at room temperature, TR-FRET ratios were determined using an Envision multilabel plate reader (Ex 340, Em 495/520).

TR-FRET data were normalized to the means of 24 no-compound controls ("high") and 8 controls containing 1 µM un-labeled probe ("low"). Percent inhibition was plotted as a function of compound concentration and the data were fit with the 4 parameter logistic equation to obtain IC50s. Inhibition constants (Kᵢ) were calculated from the IC₅₀s, probe K_{d} and probe concentration.

The mean Kᵢ values are reported in Table 1.

**Table 1**

| Example # | TR-FRET Binding Ki: BRD4 (BDI_K57-E168) (µM) | TR-FRET Binding Ki: BRD4 (BDII_E352-M457) (µM) |
|---|---|---|
| 1 | 1.71 | 0.0049 |
| 2 | 5.89 | 0.00613 |
| 3 | 1.02 | 0.00211 |
| 4 | 1.45 | 0.00244 |
| 5 | 1.96 | 0.00798 |
| 6 | 0.72 | 0.00762 |
| 7 | 0.906 | 0.00311 |
| 8 | 2.7 | 0.0177 |
| 9 | 3.68 | 0.0125 |
| 10 | 2.33 | 0.0057 |
| 11 | 1.27 | 0.00444 |
| 12 | 0.553 | 0.035 |
| 13 | >13 | 1.07 |
| 14 | 1.55 | 0.0919 |
| 15 | 6.02 | 0.00633 |
| 16 | 2.83 | 0.00243 |
| 17 | 1.17 | 0.00143 |
| 18 | 0.982 | 0.000878 |
| 19 | 2.89 | 0.00352 |
| 20 | 1.09 | 0.00106 |
| 21 | 3.02 | 0.00217 |
| 22 | 2.87 | 0.17 |
| 23 | 3.63 | 0.0329 |
| 24 | 2.86 | 0.00137 |
| 25 | 2.22 | 0.00265 |
| 26 | 8.85 | 0.30 |
| 27 | 1.9 | 0.000834 |
| 28 | 1.52 | 0.00168 |
| 29 | 2.22 | 0.00152 |
| 30 | 3.3 | 0.00193 |
| 31 | 2.41 | 0.00139 |
| 32 | 1.01 | 0.034 |
| 33 | >13 | 0.376 |
| 34 | 0.89 | 0.0231 |
| 35 | 1.81 | 0.000933 |
| 36 | 5.79 | 0.00331 |
| 37 | 2.51 | 0.00178 |
| 38 | 2.5 | 0.00238 |
| 39 | 2.25 | 0.0020 |
| 40 | 2.03 | 0.00259 |
| 41 | 2.48 | 0.0012 |
| 42 | 3.84 | 0.00177 |
| 43 | >13 | 0.00348 |
| 44 | 3.38 | 0.00263 |
| 45 | 4.17 | 0.011 |
| 46 | 5.45 | 0.0133 |
| 47 | 4.02 | 0.00618 |
| 48 | 1.79 | 0.00848 |
| 49 | 2.53 | 0.00483 |
| 50 | 2.83 | 0.00301 |
| 51 | >13 | 0.00233 |
| 52 | 3.48 | 0.00247 |
| 53 | 1.44 | 0.00295 |
| 54 | 2.26 | 0.00566 |
| 55 | 1.87 | 0.00278 |
| 56 | 2.43 | 0.0084 |
| 57 | 1.66 | 0.00183 |
| 58 | 0.783 | 0.00289 |
| 59 | 6.55 | 0.00309 |
| 60 | 4.66 | 0.00774 |
| 61 | 12.2 | 0.0247 |
| 62 | 8.37 | 0.0154 |
| 63 | 5.96 | 0.00551 |
| 64 | 1.95 | 0.00672 |
| 65 | 1.86 | 0.00884 |
| 66 | 1.32 | 0.00833 |
| 67 | 2.83 | 0.00204 |
| 68 | 1.95 | 0.0347 |
| 69 | 7.25 | 0.0122 |
| 70 | 4.1 | 0.00483 |
| 71 | 5.08 | 0.00555 |
| 72 | >13 | 0.00268 |
| 73 | 2.26 | 0.00522 |
| 74 | >13 | 1.04 |
| 75 | >13 | 0.786 |
| 76 | 7.69 | 0.148 |
| 77 | >13 | 0.407 |
| 78 | 4.65 | 0.00186 |
| 79 | 2.53 | 0.00171 |
| 80 | 9.88 | 0.00205 |
| 81 | 6.74 | 0.00219 |
| 82 | 1.51 | 0.00393 |
| 83 | 5.89 | 0.00297 |
| 84 | >13 | 0.368 |
| 85 | 2.27 | 0.00412 |
| 86 | 1.74 | 0.00193 |
| 87 | 2.43 | 0.00323 |
| 88 | 3.98 | 0.00382 |
| 89 | >13 | 0.00571 |
| 90 | 11.5 | 0.00464 |
| 91 | 5.86 | 0.00175 |
| 92 | >13 | 0.00408 |
| 93 | 1.19 | 0.00194 |
| 94 | 1.67 | 0.134 |
| 95 | 1.58 | 0.019 |
| 96 | 2.20 | 0.0108 |
| 97 | 9.53 | 0.0583 |
| 98 | 1.14 | 0.0205 |
| 99 | 10.4 | 0.326 |
| 100 | 0.719 | 0.00997 |
| 101 | 3.53 | 0.0101 |
| 102 | 4.00 | 0.0116 |
| 103 | 8.01 | 0.00897 |
| 104 | >13 | 0.00689 |
| 105 | 2.16 | 0.0683 |
| 106 | 3.41 | 0.0713 |
| 107 | 1.79 | 0.552 |
| 108 | 5.44 | 0.0401 |
| 109 | >13 | 0.00901 |
| 110 | 3.65 | 0.00443 |
| 111 | 1.19 | 0.00411 |
| 112 | 4.86 | 0.00711 |
| 113 | 6.06 | 0.0878 |
| 114 | 4.66 | 0.00607 |
| 115 | 2.92 | 0.00254 |
| 116 | >13 | 0.00237 |
| 117 | 3.57 | 0.00853 |
| 118 | 1.91 | 0.00583 |
| 119 | 2.81 | 0.00684 |
| 120 | 0.891 | 0.038 |
| 121 | >13 | 0.229 |
| 122 | 3.67 | 0.0131 |
| 123 | >13 | 0.00337 |
| 124 | >13 | 0.00261 |
| 125 | >13 | 0.0055 |
| 126 | >13 | 0.0102 |
| 127 | 8.85 | 0.00677 |
| 128 | 2.11 | 0.00601 |
| 129 | 0.772 | 0.00332 |
| 130 | 3.19 | 0.00152 |
| 131 | 6.32 | 0.00382 |
| 132 | 4.21 | 0.00276 |
| 133 | >13 | 0.00274 |
| 134 | 1.67 | 0.00918 |
| 135 | 3.68 | 0.0037 |
| 136 | 2.47 | 0.00346 |
| 137 | 1.8 | 0.00354 |
| 138 | 12.8 | 0.00359 |
| 139 | 0.645 | 0.00842 |
| 140 | 11.2 | 0.00577 |
| 141 | 4.09 | 0.00479 |
| 142 | 0.94 | 0.00967 |
| 143 | 5.24 | 0.0947 |
| 144 | >13 | 0.0374 |
| 145 | >13 | 0.00448 |

Compound dilution series for Compounds A-E were prepared in DMSO via an approximately 3-fold serial dilution from 0.47mM to 7.8nM.

Compound dilutions were added directly into white, low-volume assay plates (Perkin Elmer Proxiplate 384 Plus# 6008280) using a Labcyte Echo in conjunction with Labcyte Access and Thermo Multidrop CombinL robotics. Compounds were then suspended in eight microliters (µL) of assay buffer (20 mM Sodium Phosphate, pH 6.0, 50 mM NaCl, 1 mM Ethylenediaminetetraacetic acid disodium salt dihydrate, 0.01% Triton X-100, 1 mM DL-Dithiothreitol) containing His-tagged bromodomain, Europium-conjugated anti-His antibody (Invitrogen PV5596) and Alexa-647-conjugated probe.

The final concentration of 1X assay mixture contained 2% DMSO, 8 nM His-tagged bromodomain, 1 nM Europium-conjugated anti-His-tag antibody and 100 nM or 30 nM probe (for BDI or BDII, respectively) and compound concentration in the range of 9.19 µM - 150 pM.

After a one-hour equilibration at room temperature, TR-FRET ratios were determined using an Envision multilabel plate reader (Ex 340, Em 495/520).

TR-FRET data were normalized to the means of 24 no-compound controls ("high") and 8 controls containing 1 µM un-labeled probe ("low"). Percent inhibition was plotted as a function of compound concentration and the data were fit with the 4 parameter logistic equation to obtain IC₅₀s. Inhibition constants (Kᵢ) were calculated from the IC₅₀s, probe K_{d} and probe concentration. Typical Z' values were between 0.65 and 0.75. The minimum significant ratio was determined to evaluate assay reproducibility (Eastwood et al., (2006) J Biomol Screen, 11: 253-261). The MSR was determined to be 2.03 for BDI and 1.93 for BDII, and a moving MSR (last six run MSR overtime) for both BDI and BDII was typically < 3. The Kᵢ values are reported in Table 2.

**Table 2**

| | | |
|---|---|---|
| Compounds | TR-FRET Binding Ki: BRD4 (BDI K57-E168) (µM) | TR-FRET Binding Ki: BRD4 (BDII E352-M457) (µM) |
| A | 0.316 | 0.104 |
| B | 0.05 | 0.00915 |
| C | 0.437 | 0.0909 |
| D | 0.045 | 0.0181 |
| E | 0.0306 | 0.0214 |

Compound A is *tert*-butyl 4-[(5-{2-(2,4-difluorophenoxy)-5-[(ethylsulfonyl)amino]phenyl}-1-methyl-2-oxo-1,2-dihydropyridin-4-yl)oxy]piperidine-1-carboxylate
Compound B is *N*-[4-(2,4-difluorophenoxy)-3-(4-{[*trans*-4-(dimethylamino)cyclohexyl]oxy}-1-methyl-6-oxo-1,6-dihydropyridin-3-yl)phenyl] ethanesulfonamide
Compound C is *N*-{4-(2,4-difluorophenoxy)-3-[1-methyl-6-oxo-4-(piperidin-4-yloxy)-1,6-dihydropyridin-3-yl]phenyl}ethanesulfonamide
Compound D is *tert*-butyl 4-{[(5-{2-(2,4-difluorophenoxy)-5-[(ethylsulfonyl)amino]phenyl}-1-methyl-2-oxo-1,2-dihydropyridin-4-yl)oxy]methyl}piperidine-1-carboxylate
Compound E is *tert*-butyl 6-[(5-{2-(2,4-difluorophenoxy)-5-[(ethylsulfonyl)amino]phenyl}-1-methyl-2-oxo-1,2-dihydropyridin-4-yl)oxy]-2-azaspiro[3.3]heptane-2-carboxylate

All tested compounds were found to have selectivity for BRD4 BDII over BRD4 BDI in the TR-FRET assay described above, and are at least 3 fold selective for BRD4 BDII over BRD4 BDI. In one embodiment, the present compounds are about 50 to about 100 fold selective for BRD4 BDII over BRD4 BDI. In one embodiment, the present compounds are about 100 to about 200 fold selective for BRD4 BDII over BRD4 BDI. In one embodiment, the present compounds are at least about 200 fold selective for BRD4 BDII over BRD4 BDI.

### EMBODIMENTS

The present invention provides therefore, inter alia, the following embodiments:
1. A compound of formula (I) or a pharmaceutically acceptable salt thereof, 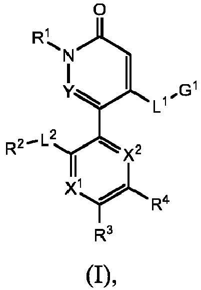 wherein
   R¹ is C₁-C₃ alkyl;
   Y is N or C(R^{Y}) wherein R^{Y} is hydrogen or C₁-C₃ alkyl;
   L¹ is O or N(R^{x}) wherein R^{x} is hydrogen or C₁-C₃ alkyl;
   G¹ is a 4-11 membered monocyclic, bicyclic, or polycyclic hydrocarbon ring with zero, one, or two double bonds, wherein one or two carbon ring atoms of G¹ are optionally replaced by heteroatoms selected from the group consisting of N, O, and S; the rings within the polycyclic and bicyclic are in a bridged, fused, or spiro orientation, or combinations thereof; each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl;
   R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)C(O)R^{b}, -N(R^{d})N(R^{c})C(O)R^{b}, -N(R^{a})C(O)OR^{b}, -N(R^{d})N(R^{c})C(O)OR^{b}, -N(R^{a})C(O)N(R^{b})₂, -N(R^{a})(C₁-C₃ alkylenyl)-C(O)R^{b}, -N(R^{a})(C₁-C₃ alkylenyl)-S(O)₂R^{b}, or C₁-C₆ alkyl substituted with an substituent selected from the group consisting of -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)OR^{b}, and -N(R^{a})C(O)N(R^{b})₂;
   R^{a}, at each occurrence, is independently hydrogen, C₁-C₆ alkyl, C₁-C₆ haloalkyl, C₂-C₆ alkenyl, -N(R^{j})₂, -(C₂-C₆ alkylenyl)-OR^{j}, or -(C₁-C₆ alkylenyl)-C(O)OR^{j};
   R^{b}, at each occurrence, is independently hydrogen, C₁-C₆ alkyl, C₂-C₆ alkenyl, C₁-C₆ haloalkyl, G^{1B}, -(C₁-C₆ alkylenyl)-OR^{j}, -(C₁-C₆ alkylenyl)-N(R^{j})₂, or -(C₁-C₆ alkylenyl)-C(O)OR^{j};
   R^{c}, at each occurrence, is independently hydrogen, C₁-C₆ alkyl, C₂-C₆ alkenyl, or C₁-C₆ haloalkyl;
   R^{d}, at each occurrence, is independently hydrogen, C₁-C₆ alkyl, C₂-C₆ alkenyl, C₁-C₆ haloalkyl, or -C(O)R^{b};
   G^{1A} is phenyl, C₃-C₁₁ cycloalkyl, 4-11 membered heterocycle, or 5-11 membered heteroaryl; wherein each G^{1A} is optionally substituted with 1, 2, 3, 4, or 5 independently selected R^{s} groups;
   G^{1B} is phenyl, C₃-C₁₁ cycloalkyl, 4-11 membered heterocycle, or 5-11 membered heteroaryl; wherein each G^{1B} is optionally substituted with 1, 2, 3, or 4 independently R^{t} groups;
   L² is O or N(R^{e}) wherein R^{e} is hydrogen or C₁-C₃ alkyl;
   R² is phenyl or monocyclic heteroaryl; each R² is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH;
   R³ is hydrogen, halogen, -CN, C₁-C₆ haloalkyl, or C₁-C₆ alkyl;
   R⁴ is 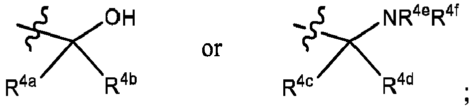 wherein
      R^{4a} is C₁-C₆ alkyl or C₁-C₆ haloalkyl, wherein the C₁-C₆ alkyl and the C₁-C₆ haloalkyl are each optionally substituted with one substituent selected from the group consisting of -OH and -CN;
      R^{4b} is C₁-C₆ alkyl or C₁-C₆ haloalkyl;
      R^{4c} and R^{4d} are each independently hydrogen, C₁-C₆ alkyl, or C₁-C₆ haloalkyl;
      R^{4e} is hydrogen, C₁-C₃ alkyl, C₂-C₄ alkenyl, C₁-C₃ haloalkyl, or -(C₁-C₃ alkylenyl)-G^{1C}; wherein G^{1C} is phenyl, monocyclic heteroaryl, monocyclic C₃-C₆ cycloalkyl, or 4-6 membered monocyclic heterocycle; wherein each G^{1C} is optionally substituted with 1, 2, 3, or 4 independently selected R^{u} groups;
      R^{4f} is C₁-C₃ alkyl, C₂-C₄ alkenyl, C₁-C₃ haloalkyl, -C(O)R^{4cc}, or -C(O)N(R^{4cd})(R^{4ce}); wherein R^{4cc} is C₁-C₃ alkyl, C₂-C₄ alkenyl, or C₁-C₃ haloalkyl; and R^{4cd} and R^{4ce} are each independently hydrogen, C₁-C₃ alkyl, C₂-C₄ alkenyl, or C₁-C₃ haloalkyl;
   X¹ and X² are C(R⁵) or
   one of X¹ and X² is N and the other is C(R⁵);
   R⁵, at each occurrence, is independently hydrogen or halogen;
   R^{s}, R^{t}, and R^{u}, at each occurrence, are each independently C₁-C₆ alkyl, C₂-C₆ alkenyl, C₂-C₆ alkynyl, halogen, C₁-C₆ haloalkyl, -CN, oxo, NO₂, -OR^{j}, -OC(O)R^{k}, -OC(O)N(R^{j})₂, -SR^{j}, -S(O)₂R^{j}, -S(O)₂N(R^{j})₂, -C(O)R^{j}, -C(O)OR^{j}, -C(O)N(R^{j})₂, -C(O)N(R^{j})S(O)₂R^{k}, -N(R^{j})₂, -N(R^{j})C(O)R^{k}, -N(R^{j})S(O)₂R^{k}, -N(R^{j})C(O)O(R^{k}), -N(R^{j})C(O)N(R^{j})₂, -(C₁-C₆ alkylenyl)-OR^{j}, -(C₁-C₆ alkylenyl)-OC(O)R^{k}, -(C₁-C₆ alkylenyl)-OC(O)N(R^{j})₂, -(C₁-C₆ alkylenyl)-SR^{j}, -(C₁-C₆ alkylenyl)-S(O)₂R^{j}, -(C₁-C₆ alkylenyl)-S(O)₂N(R^{j})₂, -(C₁-C₆ alkylenyl)-C(O)R^{j}, -(C₁-C₆ alkylenyl)-C(O)OR^{j}, -(C₁-C₆ alkylenyl)-C(O)N(R^{j})₂, -(C₁-C₆ alkylenyl)-C(O)N(R^{j})S(O)₂R^{k}, -(C₁-C₆ alkylenyl)-N(R^{j})₂, -(C₁-C₆ alkylenyl)-N(R^{j})C(O)R^{k}, -(C₁-C₆ alkylenyl)-N(R^{j})S(O)₂R^{k}, -(C₁-C₆ alkylenyl)-N(R^{j})C(O)O(R^{k}), -(C₁-C₆ alkylenyl)-N(R^{j})C(O)N(R^{j})₂, or -(C₁-C₆ alkylenyl)-CN;
   R^{j}, at each occurrence, is independently hydrogen, C₁-C₆ alkyl, or C₁-C₆ haloalkyl; and
   R^{k}, at each occurrence, is independently C₁-C₆ alkyl or C₁-C₆ haloalkyl.
2. The compound of embodiment 1 or a pharmaceutically acceptable salt thereof, wherein
   G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.
3. The compound of embodiment 1 or a pharmaceutically acceptable salt thereof, wherein
   G¹ is cyclobutyl, cyclopentyl, cyclohexyl, spiro[3.3]heptanyl, pyrrolidinyl, or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen; and
   R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)C(O)R^{b}, -N(R^{a})C(O)OR^{b}, or C₁-C₆ alkyl substituted with an substituent selected from the group consisting of -OR^{b}, -N(R^{a})C(O)R^{b}, and -N(R^{a})C(O)OR^{b}.
4. The compound of embodiment 3 or a pharmaceutically acceptable salt thereof, wherein
   R^{1g} is G^{1A}, -N(R^{a})C(O)R^{b}, or -N(R^{a})C(O)OR^{b}.
5. The compound of embodiment 1 or a pharmaceutically acceptable salt thereof, wherein
   Y is C(R^{Y});
   X¹ is N or C(R⁵); and
   X² is C(R⁵).
6. The compound of embodiment 1 or a pharmaceutically acceptable salt thereof, wherein
   L² is O and
   R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.
7. The compound of embodiment 1 of formula (I) or a pharmaceutically acceptable salt thereof, wherein R⁴ is 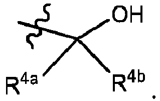
8. The compound of embodiment 1 of formula (I) or a pharmaceutically acceptable salt thereof, wherein R⁴ is 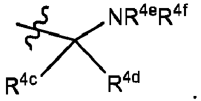
9. The compound of embodiment 1 of formula (I-a) or a pharmaceutically acceptable salt thereof, 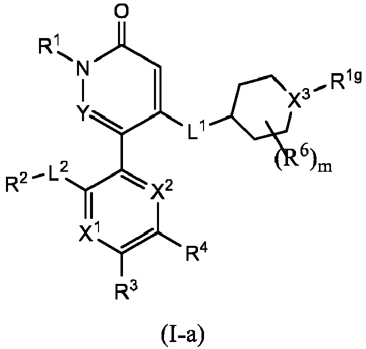 wherein
   X³ is N, C(H), or C(R⁶);
   each R⁶ is independently C₁-C₃ alkyl, C₁-C₃ haloalkyl, or halogen;
   m is 0, 1, or 2; and
   R¹, Y, L¹, R^{1g}, X¹, X², L², R², R³, and R⁴ are as set forth in embodiment 1.
10. The compound of embodiment 9 or a pharmaceutically acceptable salt thereof, wherein
   R^{1g} is -CN, G^{1A}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)OR^{b}, or C₁-C₆ alkyl substituted with an -OR^{b}.
11. The compound of embodiment 9 or a pharmaceutically acceptable salt thereof, wherein
   R^{1g} is G^{1A}, -N(R^{a})C(O)R^{b}, or -N(R^{a})C(O)OR^{b}.
12. The compound of embodiment 9 or a pharmaceutically acceptable salt thereof, wherein
   R⁴ is 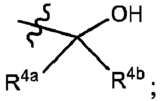 R^{4a} is C₁-C₆ alkyl or C₁-C₆ haloalkyl, wherein the C₁-C₆ alkyl and the C₁-C₆ haloalkyl are each optionally substituted with one -OH; and
   R^{4b} is C₁-C₆ alkyl or C₁-C₆ haloalkyl.
13. The compound of embodiment 9 or a pharmaceutically acceptable salt thereof, wherein
   R⁴ is 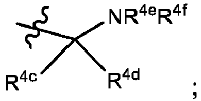 R^{4e} is hydrogen, C₁-C₃ alkyl, or -(C₁-C₃ alkylenyl)-G^{1C} wherein G^{1C} is optionally substituted phenyl; and
   R^{4f} is -C(O)R^{4cc} or -C(O)N(R^{4cd})(R^{4ce}).
14. The compound of embodiment 9 or a pharmaceutically acceptable salt thereof, wherein
   L² is O;
   X¹ is N or C(R⁵);
   X² is C(R⁵); and
   L¹ is O or N(R^{x}) wherein R^{x} is hydrogen.
15. The compound of embodiment 14 or a pharmaceutically acceptable salt thereof, wherein
   R^{1g} is G^{1A}, -N(R^{a})C(O)R^{b}, or -N(R^{a})C(O)OR^{b}.
16. The compound of embodiment 15 or a pharmaceutically acceptable salt thereof, wherein
   R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH.
17. The compound of embodiment 16 or a pharmaceutically acceptable salt thereof, wherein
   R⁴ is 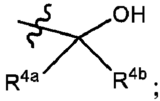 and
   R^{4a} and R^{4b} are each independently C₁-C₆ alkyl or C₁-C₆ haloalkyl.
18. The compound of embodiment 16 or a pharmaceutically acceptable salt thereof, wherein R⁴ is 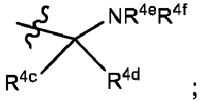 R^{4e} is hydrogen, C₁-C₃ alkyl, or -(C₁-C₃ alkylenyl)-G^{1C} wherein G^{1C} is optionally substituted phenyl; and
   R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.
19. The compound of embodiment 1 of formula (I-b) or a pharmaceutically acceptable salt thereof, 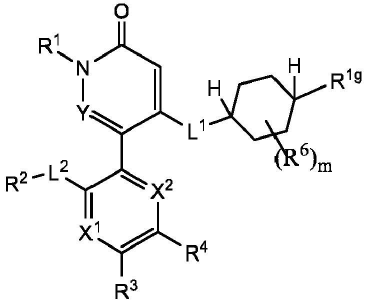 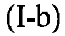 wherein
   each R⁶ is independently C₁-C₃ alkyl, C₁-C₃ haloalkyl, or halogen;
   m is 0, 1, or 2; and
   R¹, Y, L¹, R^{1g}, X¹, X², L², R², R³, and R⁴ are as set forth in embodiment 1_{.}
20. The compound of embodiment 19 or a pharmaceutically acceptable salt thereof, wherein
   R^{1g} is G^{1A}, -N(R^{a})C(O)R^{b}, or -N(R^{a})C(O)OR^{b}.
21. The compound of embodiment 19 or a pharmaceutically acceptable salt thereof, wherein
   R⁴ is 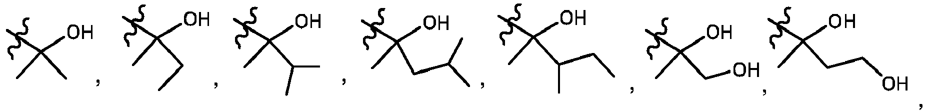 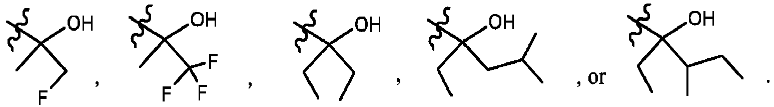
22. The compound of embodiment 19 or a pharmaceutically acceptable salt thereof, wherein
   L² is O;
   X¹ is N or C(R⁵);
   X² is C(R⁵); and
   L¹ is O or N(R^{x}) wherein R^{x} is hydrogen.
23. The compound of embodiment 22 or a pharmaceutically acceptable salt thereof, wherein
   R^{1g} is G^{1A}, -N(R^{a})C(O)R^{b}, or -N(R^{a})C(O)OR^{b}.
24. The compound of embodiment 23 or a pharmaceutically acceptable salt thereof, wherein
   R⁴ is 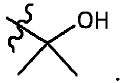
25. The compound of embodiment 23 or a pharmaceutically acceptable salt thereof, wherein
   R⁴ is 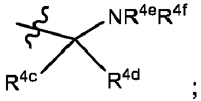 R^{4c} and R^{4d} are each independently hydrogen or C₁-C₆ alkyl;
   R^{4e} is hydrogen, C₁-C₃ alkyl, or -(C₁-C₃ alkylenyl)-G^{1C} wherein G^{1C} is optionally substituted phenyl; and
   R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.
26. The compound of any one of embodiments 1-25, or a pharmaceutically acceptable salt thereof, wherein
   Y is C(R^{Y}); and
   X¹ and X² are C(R⁵).
27. The compound of any one of embodiments 1-25, or a pharmaceutically acceptable salt thereof, wherein
   Y is C(R^{Y});
   X¹ is N; and
   X² is C(R⁵).
28. The compound of embodiment 1 or a pharmaceutically acceptable salt thereof, wherein the compound is selected from the group consisting of
   *N*-(*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy} cyclohexyl)acetamide;
   5-[2-(2,6-dimethylphenoxy)-5-(2-hydroxypropan-2-yl)phenyl]-4-{[1-(methanesulfonyl)piperidin-4-yl]amino}-1-methylpyridin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide;
   methyl [*trans*-4-({5-[2-(4-fluoro-2,6-dimethylphenoxy)-5-(2-hydroxypropan-2-yl)phenyl] -1 -methyl-2-oxo-1 ,2-dihydropyridin-4-yl} oxy)cyclohexyl]carbamate;
   methyl (*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)carbamate;
   *N*-{[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}acetamide;
   methyl {[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
   *tert*-butyl {[*cis*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
   5-[2-(4-fluoro-2,6-dimethylphenoxy)-5-(2-hydroxypropan-2-yl)phenyl]-4-1[1-(methanesulfonyl)piperidin-4-yl]amino}-1-methylpyridin-2(1*H*)-one;
   *N*-[6-({5-[2-(2,6-dimethylphenoxy)-5-(2-hydroxypropan-2-yl)phenyl]-1-methyl-2-oxo-1,2-dihydropyridin-4-yl}amino)spiro[3.3]heptan-2-yl]acetamide;
   tert-butyl 3-({5-[2-(2,6-dimethylphenoxy)-5-(2-hydroxypropan-2-yl)phenyl]-1-methyl-2-oxo-1,2-dihydropyridin-4-yl}amino)pyrrolidine-1-carboxylate;
   5-[2-(2,6-dimethylphenoxy)-5-(2-hydroxypropan-2-yl)phenyl]-4-{[1-(methanesulfonyl)pyrrolidin-3-yl]amino}-1-methylpyridin-2(1*H*)-one;
   *N*-{*trans*-4-[{5-[2-(4-fluoro-2,6-dimethylphenoxy)-5-(2-hydroxypropan-2-yl)phenyl]-1-methyl-2-oxo-1,2-dihydropyridin-4-yl}(methyl)amino]cyclohexyl}acetamide;
   4-[(1-acetylpyrrolidin-3-yl)amino]-5-[2-(2,6-dimethylphenoxy)-5-(2-hydroxypropan-2-yl)phenyl]-1-methylpyridin-2(1*H*)-one;
   *tert*-butyl [*trans*-4-({5-[2-(4-fluoro-2,6-dimethylphenoxy)-5-(2-hydroxypropan-2-yl)phenyl] -1 -methyl-2-oxo-1 ,2-dihydropyridin-4-yl} oxy)cyclohexyl]methylcarbamate;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-methylacetamide;
   *N*-[*trans*-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl} oxy)cyclohexyl]acetamide;
   *N*-{*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl} acetamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} amino)cyclohexyl]acetamide;
   methyl {*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl} carbamate;
   methyl [*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate;
   *N*-{[(1*R*,2*S*,3*S*)-3-({5-[2-(4-fluoro-2,6-dimethylphenoxy)-5-(2-hydroxypropan-2-yl)phenyl]-1-methyl-2-oxo-1,2-dihydropyridin-4-yl}oxy)-2-methylcyclopentyl]methyl} acetamide;
   5-[2-(4-fluoro-2,6-dimethylphenoxy)-5-(2-hydroxypropan-2-yl)phenyl]-4-[(*trans*-4-hydroxy-4-methylcyclohexyl)amino]-1-methylpyridin-2(1*H*)-one;
   5-[2-(4-fluoro-2,6-dimethylphenoxy)-5-(2-hydroxypropan-2-yl)phenyl]-1-methyl-4-{[*trans*-4-(2-oxopyrrolidin-1-yl)cyclohexyl]oxy}pyridin-2(1*H*)-one;
   methyl [*trans*-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]carbamate;
   2'-(4-fluoro-2,6-dimethylphenoxy)-5'-(2-hydroxypropan-2-yl)-4-{[1-(methoxyacetyl)piperidin-4-yl]oxy}-1-methyl[3,3'-bipyridin]-6(1*H*)-one;
   5-[2-(2,6-dimethylphenoxy)-5-(2-hydroxypropan-2-yl)phenyl]-1-methyl-4-{[*trans*-4-(2-oxopyrrolidin-1-yl)cyclohexyl]oxy}pyridin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]prop-2-enamide;
   5-[2-(4-fluoro-2,6-dimethylphenoxy)-5-(2-hydroxypropan-2-yl)phenyl]-1-methyl-4-{[*trans*-4-(2-oxopiperidin-1-yl)cyclohexyl]oxy}pyridin-2(1*H*)-one;
   4-{[*trans*-4-(3,3-dimethyl-2-oxoazetidin-1-yl)cyclohexyl]oxyl-5-[2-(4-fluoro-2,6-dimethylphenoxy)-5-(2-hydroxypropan-2-yl)phenyl]-1-methylpyridin-2(1*H*)-one;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydropyrimidin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-1-methylcyclohexyl]acetamide;
   6-ethyl-5-[2-(4-fluoro-2,6-dimethylphenoxy)-5-(2-hydroxypropan-2-yl)phenyl]-1-methyl-4-{[*trans*-4-(2-oxopyrrolidin-1-yl)cyclohexyl]oxy}pyridin-2(1*H*)-one;
   5-[2-(4-fluoro-2,6-dimethylphenoxy)-5-(2-hydroxypropan-2-yl)phenyl]-1-methyl-4-{[*trans*-4-methyl-4-(2-oxopyrrolidin-1-yl)cyclohexyl]oxy}pyridin-2(1*H*)-one;
   5-[2-(4-fluoro-2,6-dimethylphenoxy)-5-(2-hydroxypropan-2-yl)phenyl]-1-methyl-4-{[*trans*-4-(2-oxoimidazolidin-1-yl)cyclohexyl]oxy}pyridin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hex-5-enamide;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]azepan-2-one;
   5-[2-(4-fluoro-2,6-dimethylphenoxy)-5-(2-hydroxypropan-2-yl)phenyl]-1-methyl-4-{[*trans*-4-(2-oxo-1,3-oxazolidin-3-yl)cyclohexyl]oxy}pyridin-2(1*H*)-one;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,3-oxazinan-2-one;
   4-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]morpholin-3-one;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyltetrahydropyrimidin-2(1*H*)-one;
   5-[2-(4-fluoro-2,6-dimethylphenoxy)-5-(2-hydroxypropan-2-yl)phenyl]-1-methyl-4-{[*trans*-4-(2-methyl-5-oxopyrrolidin-1-yl)cyclohexyl]oxy}pyridin-2(1*H*)-one;
   2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1*H*-isoindole-1,3(2*H*)-dione;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrrolidine-2,5-dione;
   2-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}benzoic acid;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]methanesulfonamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-2,2-dimethylbutanamide;
   4-{[*trans*-4-(3,3-dimethyl-2-oxopyrrolidin-1-yl)cyclohexyl]oxyl-5-[2-(4-fluoro-2,6-dimethylphenoxy)-5-(2-hydroxypropan-2-yl)phenyl]-1-methylpyridin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-hydroxypropanamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]cyclopropanecarboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methylcyclopropane-1-carboxamide;
   2-[*trans*-4-({5-[2-(4-fluoro-2,6-dimethylphenoxy)-5-(2-hydroxypropan-2-yl)phenyl-1-methyl-2-oxo-1,2-dihydropyridin-4-yl}oxy)cyclohexyl]-1lambda^{6,2}-thiazolidine-1,1-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2-methoxyacetamide;
   *N*-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-hydroxyethyl)acetamide;
   ethyl 3-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]amino}-3-oxopropanoate;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-2-oxopropanamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2,2-dimethylpropanamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N,1-dimethylcyclopropane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*,2,2-trimethylpropanamide;
   2'-(4-fluoro-2,6-dimethylphenoxy)-5'-(2-hydroxypropan-2-yl)-1-methyl-4-{[*trans*-4-(2-oxopyrrolidin-1-yl)cyclohexyl]oxy}[3,3'-bipyridin]-6(1*H*)-one;
   *trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxylic acid;
   5-[2-(4-fluoro-2,6-dimethylphenoxy)-5-(2-hydroxypropan-2-yl)phenyl]-1-methyl-4-{[*trans*-4-(pyrrolidine-1-carbonyl)cyclohexyl]oxy}pyridin-2(1*H*)-one;
   *trans-N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-methoxyethyl)-1-methylcyclopropane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(3-methoxypropyl)-1-methylcyclopropane-1-carboxamide;
   ethyl *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(1-methylcyclopropane-1-carbonyl)glycinate;
   5-[2-(4-fluoro-2,6-dimethylphenoxy)-5-(2-hydroxypropan-2-yl)phenyl]-4-{[*trans*-4-(2-hydroxypropan-2-yl)cyclohexyl]oxy}-1-methylpyridin-2(1*H*)-one;
   *cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
   *trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-(methoxymethyl)cyclopropane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-3-methoxy-2,2-dimethylpropanamide;
   *tert*-butyl(3-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[1.1.1]pentan-1-yl)carbamate;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyloxetane-3-carboxamide;
   5-[2-(4-fluoro-2,6-dimethylphenoxy)-5-(2-hydroxypropan-2-yl)phenyl]-4-{[*cis*-4-(2-hydroxypropan-2-yl)cyclohexyl]oxy}-1-methylpyridin-2(1*H*)-one;
   *cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxylic acid;
   *cis*-*N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
   5-[2-(4-fluoro-2,6-dimethylphenoxy)-5-(2-hydroxypropan-2-yl)phenyl]-1-methyl-4-{[*cis*-4-(pyrrolidine-1-carbonyl)cyclohexyl]oxy}pyridin-2(1*H*)-one;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-methylimidazolidine-2,4-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-5-hydroxy-2,2-dimethylpentanamide;
   1-[*trans*-4-({5-[2-(4-fluoro-2,6-dimethylphenoxy)-5-(2-hydroxypropan-2-yl)phenyl-1-methyl-2-oxo-1,2-dihydropyridin-4-yl}oxy)cyclohexyl]piperidine-2,6-dione;
   (5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5-dimethylimidazolidine-2,4-dione;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,3-oxazolidine-2,4-dione;
   (5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,5-dimethylimidazolidine-2,4-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)-4-methylcyclohexyl]acetamide;
   1-cyano-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methyl-3-oxocyclobutane-1-carboxamide;
   1-cyano-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclobutane-1-carboxamide;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpiperidine-2,6-dione;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpyrrolidine-2,5-dione;
   (7a*S*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-c]imidazole-1,3(2*H*)-dione;
   (5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methyl-1,3-oxazolidine-2,4-dione;
   3-amino-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide;
   5-[2-(4-fluoro-2,6-dimethylphenoxy)-5-(2-hydroxypropan-2-yl)phenyl]-4-{[*cis*-3-(2-hydroxypropan-2-yl)cyclobutyl]oxy}-1-methylpyridin-2(1*H*)-one;
   *cis-N*-ethyl-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxamide;
   5-[2-(4-fluoro-2,6-dimethylphenoxy)-5-(2-hydroxypropan-2-yl)phenyl]-1-methyl-4-{[*cis*-3-(pyrrolidine-1-carbonyl)cyclobutyl]oxy}pyridin-2(1*H*)-one;
   *cis*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxylic acid;
   *N*-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
   *N'*-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
   *N*-[*trans*-3-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclobutyl]acetamide;
   *trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N*-methylcyclohexane-1-carboxamide;
   *trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
   *tert*-butyl (4-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[2.1.1]hexan-1-yl)carbamate;
   *tert*-butyl [(1-{[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}cyclopropyl)methyl]carbamate;
   *N'*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
   *tert*-butyl 2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
   *tert*-butyl 2-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
   *trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N*,*N*-dimethylcyclohexane-1-carboxamide;
   5-[2-(4-fluoro-2,6-dimethylphenoxy)-5-(2-hydroxypropan-2-yl)phenyl]-1-methyl-4-{[*trans*-4-(5-oxopyrazolidin-1-yl)cyclohexyl]oxy}pyridin-2(1*H*)-one;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-(hydroxymethyl)bicyclo[2.2.2]octane-1-carboxamide;
   *N*¹-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl] cyclopropane-1 ,1 -dicarboxamide;
   4-{[*trans*-4-(2-acetyl-5-oxopyrazolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
   *N*-[*trans*-4-({5-[2-(2,6-dimethylphenoxy)-5-{[methyl(methylcarbamoyl)amino]methyl}phenyl]-1-methyl-2-oxo-1,2-dihydropyridin-4-yl}oxy)cyclohexyl]-1-methylcyclopropane-1-carboxamide;
   5-[2-(4-fluoro-2,6-dimethylphenoxy)-5-(2-hydroxypropan-2-yl)phenyl]-4-{[*trans*-4-(5-hydroxy-1*H*-pyrazol-1-yl)cyclohexyl]oxy}-1-methylpyridin-2(1*H*)-one;
   *N*-[*trans*-4-({5-[5-{[carbamoyl(methyl)amino]methyl}-2-(2,6-dimethylphenoxy)phenyl]-1-methyl-2-oxo-1,2-dihydropyridin-4-yl}oxy)cyclohexyl]-1-methylcyclopropane-1-carboxamide;
   *N*-{*trans*-4-[(5-{5-[(1*R*)-1-{acetyl[(1*S*)-1-phenylethyl]amino}ethyl]-2-(2,6-dimethylphenoxy)phenyl}-1-methyl-2-oxo-1,2-dihydropyridin-4-yl)oxy]cyclohexyl}-1-methylcyclopropane-1-carboxamide;
   2,2,2-trifluoro-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-3,3-dimethylbutanamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-4-hydroxy-4-methylpentanamide;
   4-{[*trans*-4-(4,4-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrazolidine-3,5-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]morpholine-4-carboxamide;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-oxoimidazolidine-1-carboxamide;
   (5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-5-methylimidazolidine-2,4-dione;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
   2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H*-imidazo[5,1-*c*][1,4]oxazine-1,3(2*H*)-dione;
   1-ethyl-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-N'-methylurea;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-azabicyclo[3.1.0]hexane-2,4-dione;
   2-[*trans*-4-({5-[2-(4-fluoro-2,6-dimethylphenoxy)-5-(2-hydroxypropan-2-yl)phenyl-1-methyl-2-oxo-1,2-dihydropyridin-4-yl}oxy)cyclohexyl]-6-methylpyridazin-3(2*H*)-one;
   (5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-(propan-2-yl)imidazolidine-2,4-dione;
   (5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-5-methylimidazolidine-2,4-dione;
   5-ethylidene-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
   5-[2-(4-fluoro-2,6-dimethylphenoxy)-5-(2-hydroxypropan-2-yl)phenyl]-1-methyl-4-{[*trans*-4-(2,3,4,4-tetramethyl-5-oxoimidazolidin-1-yl)cyclohexyl]oxy}pyridin-2(1*H*)-one;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1-(2-methoxyethyl)imidazolidine-2,4-dione;
   6-[*trans*-4-({5-[2-(4-fluoro-2,6-dimethylphenoxy)-5-(2-hydroxypropan-2-yl)phenyl-1-methyl-2-oxo-1,2-dihydropyridin-4-yl}oxy)cyclohexyl]-4,6-diazaspiro[2.4]heptane-5,7-dione;
   4-acetamido-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[2.1.1]hexane-1-carboxamide;
   (7*aR*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-c]imidazole-1,3(2*H*)-dione;
   *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methylalaninamide;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5,5-dimethylimidazolidine-2,4-dione;
   2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyridazin-3(2*H*)-one;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]-1,5,5-trimethylimidazolidine-2,4-dione;
   1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-2(1*H*)-one;
   3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-4(3*H*)-one; and
   6-[*trans*-4-({5-[2-(4-fluoro-2,6-dimethylphenoxy)-5-(2-hydroxypropan-2-yl)phenyl-1-methyl-2-oxo-1,2-dihydropyridin-4-yl}oxy)cyclohexyl]-4-methyl-4,6-diazaspiro[2.4]heptane-5,7-dione.
29. A pharmaceutical composition comprising a therapeutically effective amount of a compound of formula (I) according to embodiment 1, or a pharmaceutically acceptable salt thereof, in combination with a pharmaceutically acceptable carrier.
30. A method for treating cancer in a subject comprising administering a therapeutically effective amount of a compound of formula (I) according to embodiment 1 or a pharmaceutically acceptable salt thereof, to a subject in need thereof.
31. The method of embodiment 30 wherein the cancer is selected from the group consisting of: acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin, and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenström's macroglobulinemia, testicular tumors, uterine cancer, and Wilms' tumor.
32. A method for treating a disease or condition in a subject comprising administering a therapeutically effective amount of a compound of formula (I) according to embodiment 1 or a pharmaceutically acceptable salt thereof, to a subject in need thereof, wherein said disease or condition is selected from the group consisting of Addison's disease, acute gout, ankylosing spondylitis, asthma, atherosclerosis, Behcet's disease, bullous skin diseases, chronic obstructive pulmonary disease (COPD), Crohn's disease, dermatitis, eczema, giant cell arteritis, glomerulonephritis, hepatitis, hypophysitis, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis.
33. A method for treating an acquired immunodeficiency syndrome (AIDS) in a subject comprising administering a therapeutically effective amount of a compound of formula (I) according to embodiment 1 or a pharmaceutically acceptable salt thereof, to a subject in need thereof.
34. A method for treating a disease or condition in a subject comprising administering a therapeutically effective amount of a compound of formula (I) according to embodiment 1 or a pharmaceutically acceptable salt thereof, to a subject in need thereof, wherein said disease or condition is selected from the group consisting of: obesity, dyslipidemia, hypercholesterolemia, Alzheimer's disease, metabolic syndrome, hepatic steatosis, type II diabetes, insulin resistance, diabetic retinopathy, and diabetic neuropathy.
35. A method of contraception in a male subject comprising administering a therapeutically effective amount of a compound of formula (I) according to embodiment 1 or a pharmaceutically acceptable acceptable salt thereof, to a subject in need thereof.
36. A method for treating an acute kidney disease or condition in a subject comprising administering a therapeutically effective amount of a compound of embodiment 1 or a pharmaceutically acceptable salt thereof, to a subject in need thereof, wherein said acute kidney disease or condition is selected from the group consisting of: ischemia-reperfusion induced kidney disease, cardiac and major surgery induced kidney disease, percutaneous coronary intervention induced kidney disease, radio-contrast agent induced kidney disease, sepsis induced kidney disease, pneumonia induced kidney disease, and drug toxicity induced kidney disease.
37. A method of treating a chronic kidney disease or condition in a subject comprising administering a therapeutically effective amount of a compound of embodiment 1 or a pharmaceutically acceptable salt thereof, to a subject in need thereof, wherein said disease or condition is selected from the group consisting of: diabetic nephropathy, hypertensive nephropathy, HIV-associated nephropathy, glomerulonephritis, lupus nephritis, IgA nephropathy, focal segmental glomerulosclerosis, membranous glomerulonephritis, minimal change disease, polycystic kidney disease and tubular interstitial nephritis.

It is understood that the foregoing detailed description and accompanying examples are merely illustrative and are not to be taken as limitations upon the scope of the invention, which is defined solely by the appended claims and their equivalents. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the spirit and scope thereof. All publications, patents, and patent applications cited herein are hereby incorporated by reference in their entirety for all purposes.

## Claims

1. A compound of formula (I) or a pharmaceutically acceptable salt thereof, 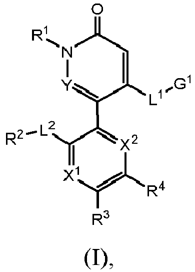 wherein
R¹ is C₁-C₃ alkyl;
Y is N or C(R^{Y}) wherein R^{Y} is hydrogen or C₁-C₃ alkyl;
L¹ is O or N(R^{x}) wherein R^{x} is hydrogen or C₁-C₃ alkyl;
G¹ is a 4-11 membered monocyclic, bicyclic, or polycyclic hydrocarbon ring with zero, one, or two double bonds, wherein one or two carbon ring atoms of G¹ are optionally replaced by heteroatoms selected from the group consisting of N, O, and S; the rings within the polycyclic and bicyclic are in a bridged, fused, or spiro orientation, or combinations thereof; each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl;
R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)C(O)R^{b}, -N(R^{d})N(R^{c})C(O)R^{b}, -N(R^{a})C(O)OR^{b}, -N(R^{d})N(R^{c})C(O)OR^{b}, -N(R^{a})C(O)N(R^{b})₂, -N(R^{a})(C₁-C₃ alkylenyl)-C(O)R^{b}, -N(R^{a})(C₁-C₃ alkylenyl)-S(O)₂R^{b}, or C₁-C₆ alkyl substituted with an substituent selected from the group consisting of -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)OR^{b}, and -N(R^{a})C(O)N(R^{b})₂;
R^{a}, at each occurrence, is independently hydrogen, C₁-C₆ alkyl, C₁-C₆ haloalkyl, C₂-C₆ alkenyl, -N(R^{j})₂, -(C₂-C₆ alkylenyl)-OR^{j}, or -(C₁-C₆ alkylenyl)-C(O)OR^{j};
R^{b}, at each occurrence, is independently hydrogen, C₁-C₆ alkyl, C₂-C₆ alkenyl, C₁-C₆ haloalkyl, G^{1B}, -(C₁-C₆ alkylenyl)-OR^{j}, -(C₁-C₆ alkylenyl)-N(R^{j})₂, or -(C₁-C₆ alkylenyl)-C(O)OR^{j};
R^{c}, at each occurrence, is independently hydrogen, C₁-C₆ alkyl, C₂-C₆ alkenyl, or C₁-C₆ haloalkyl;
R^{d}, at each occurrence, is independently hydrogen, C₁-C₆ alkyl, C₂-C₆ alkenyl, C₁-C₆ haloalkyl, or -C(O)R^{b};
G^{1A} is phenyl, C₃-C₁₁ cycloalkyl, 4-11 membered heterocycle, or 5-11 membered heteroaryl; wherein each G^{1A} is optionally substituted with 1, 2, 3, 4, or 5 independently selected R^{s} groups;
G^{1B} is phenyl, C₃-C₁₁ cycloalkyl, 4-11 membered heterocycle, or 5-11 membered heteroaryl; wherein each G^{1B} is optionally substituted with 1, 2, 3, or 4 independently R^{t} groups;
L² is O or N(R^{e}) wherein R^{e} is hydrogen or C₁-C₃ alkyl;
R² is phenyl or monocyclic heteroaryl; each R² is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH;
R³ is hydrogen, halogen, -CN, C₁-C₆ haloalkyl, or C₁-C₆ alkyl;
R⁴ is 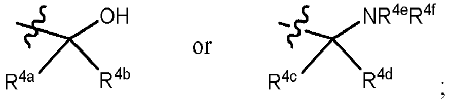 wherein
R^{4a} is C₁-C₆ alkyl or C₁-C₆ haloalkyl, wherein the C₁-C₆ alkyl and the C₁-C₆ haloalkyl are each optionally substituted with one substituent selected from the group consisting of -OH and -CN;
R^{4b} is C₁-C₆ alkyl or C₁-C₆ haloalkyl;
R^{4c} and R^{4d} are each independently hydrogen, C₁-C₆ alkyl, or C₁-C₆ haloalkyl;
R^{4e} is hydrogen, C₁-C₃ alkyl, C₂-C₄ alkenyl, C₁-C₃ haloalkyl, or -(C₁-C₃ alkylenyl)-G^{1C}; wherein G^{1C} is phenyl, monocyclic heteroaryl, monocyclic C₃-C₆ cycloalkyl, or 4-6 membered monocyclic heterocycle; wherein each G^{1C} is optionally substituted with 1, 2, 3, or 4 independently selected R^{u} groups;
R^{4f} is C₁-C₃ alkyl, C₂-C₄ alkenyl, C₁-C₃ haloalkyl, -C(O)R^{4cc}, or -C(O)N(R^{4cd})(R^{4ce}); wherein R^{4cc} is C₁-C₃ alkyl, C₂-C₄ alkenyl, or C₁-C₃ haloalkyl; and R^{4cd} and R^{4ce} are each independently hydrogen, C₁-C₃ alkyl, C₂-C₄ alkenyl, or C₁-C₃ haloalkyl;
X¹ and X² are C(R⁵) or
one of X¹ and X² is N and the other is C(R⁵);
R⁵, at each occurrence, is independently hydrogen or halogen;
R^{s} R^{t}, and R^{u}, at each occurrence, are each independently C₁-C₆ alkyl, C₂-C₆ alkenyl, C₂-C₆ alkynyl, halogen, C₁-C₆ haloalkyl, -CN, oxo, NO₂, -OR^{j}, -OC(O)R^{k}, -OC(O)N(R^{j})₂, -SR^{j}, -S(O)₂R^{j}, -S(O)₂N(R^{j})₂, -C(O)R^{j}, -C(O)OR^{j}, -C(O)N(R^{j})₂, -C(O)N(R^{j})S(O)₂R^{k}, -N(R^{j})₂, -N(R^{j})C(O)R^{k}, -N(R^{j})S(O)₂R^{k}, -N(R^{j})C(O)O(R^{k}), -N(R^{j})C(O)N(R^{j})₂, -(C₁-C₆ alkylenyl)-OR^{j}, -(C₁-C₆ alkylenyl)-OC(O)R^{k}, -(C₁-C₆ alkylenyl)-OC(O)N(R^{j})₂, -(C₁-C₆ alkylenyl)-SR^{j}, -(C₁-C₆ alkylenyl)-S(O)₂R^{j}, -(C₁-C₆ alkylenyl)-S(O)₂N(R^{j})₂, -(C₁-C₆ alkylenyl)-C(O)R^{j}, -(C₁-C₆ alkylenyl)-C(O)OR^{j}, -(C₁-C₆ alkylenyl)-C(O)N(R^{j})₂, -(C₁-C₆ alkylenyl)-C(O)N(R^{j})S(O)₂R^{k}, -(C₁-C₆ alkylenyl)-N(R^{j})₂, -(C₁-C₆ alkylenyl)-N(R^{j})C(O)R^{k}, -(C₁-C₆ alkylenyl)-N(R^{j})S(O)₂R^{k}, -(C₁-C₆ alkylenyl)-N(R^{j})C(O)O(R^{k}), -(C₁-C₆ alkylenyl)-N(R^{j})C(O)N(R^{j})₂, or -(C₁-C₆ alkylenyl)-CN;
R^{j}, at each occurrence, is independently hydrogen, C₁-C₆ alkyl, or C₁-C₆ haloalkyl; and
R^{k}, at each occurrence, is independently C₁-C₆ alkyl or C₁-C₆ haloalkyl.

2. The compound of claim 1 or a pharmaceutically acceptable salt thereof, wherein
G¹ is monocyclic C₃-C₆ cycloalkyl, spiro[3.3]heptanyl, or a 4-6 membered monocyclic heterocycle; and each G¹ is substituted with 1, 2, 3, or 4 substituents wherein one of the substituents is an R^{1g} group, and the optional substituents of G¹ are independently selected from the group consisting of C₁-C₃ alkyl, C₁-C₃ haloalkyl, halogen, -CN, -OR^{2g}, -N(R^{2g})₂, -C(O)R^{2g}, cyclopropyl, and cyclobutyl; wherein each R^{2g} is independently hydrogen, C₁-C₃ alkyl, or C₁-C₃ haloalkyl.

3. The compound of claim 1 or a pharmaceutically acceptable salt thereof, wherein
G¹ is cyclobutyl, cyclopentyl, cyclohexyl, spiro[3.3]heptanyl, pyrrolidinyl, or piperidinyl; and each G¹ is substituted with 1, 2, 3, or 4 substituents wherein one of the substituents is an R^{1g} group, and the optional substituents are independently selected from the group consisting of C₁-C₃ alkyl, C₁-C₃ haloalkyl, and halogen; and
R^{1g} is -CN, G^{1A}, -OR^{b}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)C(O)R^{b}, -N(R^{a})C(O)OR^{b}, or C₁-C₆ alkyl substituted with an substituent selected from the group consisting of -OR^{b}, -N(R^{a})C(O)R^{b}, and -N(R^{a})C(O)OR^{b}.

4. The compound of claim 3 or a pharmaceutically acceptable salt thereof, wherein
R^{1g} is G^{1A}, -N(R^{a})C(O)R^{b}, or -N(R^{a})C(O)OR^{b}.

5. The compound of claim 1 or a pharmaceutically acceptable salt thereof, wherein
Y is C(R^{Y});
X¹ is N or C(R⁵); and
X² is C(R⁵).

6. The compound of claim 1 or a pharmaceutically acceptable salt thereof, wherein
L² is O and
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen, -CN, C₁-C₆ alkyl, C₁-C₆ haloalkyl, -S(C₁-C₆ alkyl), -S(O)₂(C₁-C₆ alkyl), and -(C₂-C₆ alkylenyl)-OH.

7. The compound of claim 1 of formula (I) or a pharmaceutically acceptable salt thereof, wherein R⁴ is 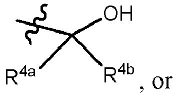 R4 is 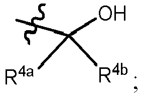 R^{4a} is C₁-C₆ alkyl or C₁-C₆ haloalkyl, wherein the C₁-C₆ alkyl and the C₁-C₆ haloalkyl are each optionally substituted with one -OH; and
R^{4b} is C₁-C₆ alkyl or C₁-C₆ haloalkyl.

8. The compound of claim 1 of formula (I) or a pharmaceutically acceptable salt thereof, wherein R⁴ is 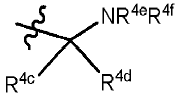 or
R4 is 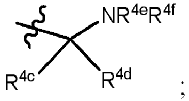 R^{4e} is hydrogen, C₁-C₃ alkyl, or -(C₁-C₃ alkylenyl)-G^{1C} wherein G^{1C} is optionally substituted phenyl; and
R^{4f} is -C(O)R^{4cc} or -C(O)N(R^{4cd})(R^{4ce}).

9. The compound of claim 1 of formula (I-a) or a pharmaceutically acceptable salt thereof, 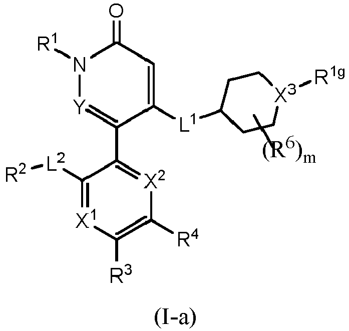 wherein
X³ is N, C(H), or C(R⁶);
each R⁶ is independently C₁-C₃ alkyl, C₁-C₃ haloalkyl, or halogen;
m is 0, 1, or 2; and
R¹, Y, L¹, R^{1g}, X¹, X², L², R², R³, and R⁴ are as set forth in claim 1.

10. The compound of claim 9 or a pharmaceutically acceptable salt thereof, wherein
R^{1g} is -CN, G^{1A}, -C(O)R^{b}, -C(O)OR^{c}, -C(O)N(R^{b})₂, -S(O)₂R^{b}, -N(R^{a})S(O)₂R^{b}, -N(R^{a})C(O)R^{b}, -N(R^{a})C(O)OR^{b}, or C₁-C₆ alkyl substituted with an -OR^{b}.

11. The compound of claim 9 or a pharmaceutically acceptable salt thereof, wherein
R^{1g} is G^{1A}, -N(R^{a})C(O)R^{b}, or -N(R^{a})C(O)OR^{b}.

12. The compound of claim 9 or a pharmaceutically acceptable salt thereof, wherein
L² is O;
X¹ is N or C(R⁵);
X² is C(R⁵); and
L¹ is O or N(R^{x}) wherein R^{x} is hydrogen.

13. The compound of claim 12 or a pharmaceutically acceptable salt thereof, wherein
R^{1g} is G^{1A}, -N(R^{a})C(O)R^{b}, or -N(R^{a})C(O)OR^{b}.

14. The compound of claim 13 or a pharmaceutically acceptable salt thereof, wherein
R² is phenyl which is substituted with 2, 3, or 4 substituents wherein two of the substituents are independently selected from the group consisting of halogen, C₁-C₆ alkyl, and C₁-C₆ haloalkyl, and the optional substituents are independently selected from the group consisting of halogen and -(C₂-C₆ alkylenyl)-OH.

15. The compound of claim 14 or a pharmaceutically acceptable salt thereof, wherein
R⁴ is 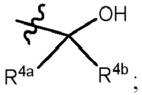 and
R^{4a} and R^{4b} are each independently C₁-C₆ alkyl or C₁-C₆ haloalkyl, or
R4 is 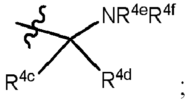 R^{4e} is hydrogen, C₁-C₃ alkyl, or -(C₁-C₃ alkylenyl)-G^{1C} wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

16. The compound of claim 1 of formula (I-b) or a pharmaceutically acceptable salt thereof, 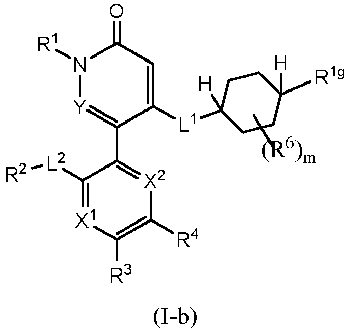 wherein
each R⁶ is independently C₁-C₃ alkyl, C₁-C₃ haloalkyl, or halogen;
m is 0, 1, or 2; and
R¹, Y, L¹, R^{1g}, X¹, X², L², R², R³, and R⁴ are as set forth in claim 1.

17. The compound of claim 16 or a pharmaceutically acceptable salt thereof, wherein R^{1g} is G^{1A}, -N(R^{a})C(O)R^{b}, or -N(R^{a})C(O)OR^{b}.

18. The compound of claim 16 or a pharmaceutically acceptable salt thereof, wherein R⁴ is

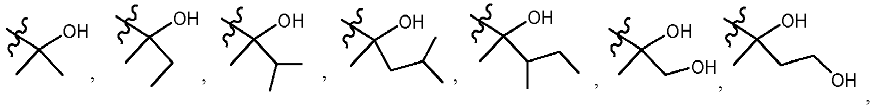

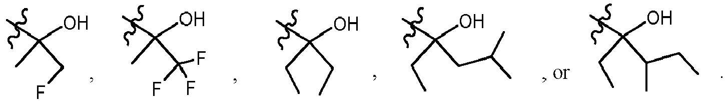

19. The compound of claim 19 or a pharmaceutically acceptable salt thereof, wherein
L² is O;
X¹ is N or C(R⁵);
X² is C(R⁵); and
L¹ is O or N(R^{x}) wherein R^{x} is hydrogen.

20. The compound of claim 19 or a pharmaceutically acceptable salt thereof, wherein R^{1g} is G^{1A}, -N(R^{a})C(O)R^{b}, or -N(R^{a})C(O)OR^{b}.

21. The compound of claim 19 or a pharmaceutically acceptable salt thereof, wherein
R4 is 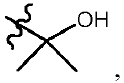 or
R4 is 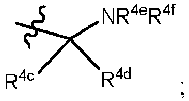 R^{4c} and R^{4d} are each independently hydrogen or C₁-C₆ alkyl;
R^{4e} is hydrogen, C₁-C₃ alkyl, or -(C₁-C₃ alkylenyl)-G^{1C} wherein G^{1C} is optionally substituted phenyl; and
R^{4f} is -C(O)R^{4cc} wherein R^{4cc} is C₁-C₃ alkyl; or R^{4f} is -C(O)N(R^{4cd})(R^{4ce}) wherein R^{4cd} and R^{4ce} are hydrogen.

22. The compound of any one of claims 1-21, or a pharmaceutically acceptable salt thereof, wherein
Y is C(R^{Y}); and
X¹ and X² are C(R⁵).

23. The compound of any one of claims 1-21, or a pharmaceutically acceptable salt thereof, wherein
Y is C(R^{Y});
X¹ is N; and
X² is C(R⁵).

24. The compound of claim 1 or a pharmaceutically acceptable salt thereof, wherein the compound is selected from the group consisting of
*N*-(*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)acetamide;
5-[2-(2,6-dimethylphenoxy)-5-(2-hydroxypropan-2-yl)phenyl]-4-{[1-(methanesulfonyl)piperidin-4-yl]amino}-1-methylpyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide;
methyl [*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamate;
methyl (*trans*-4-{[2'-(4-fluoro-2,6-dimethylphenoxy)-5'-(2-hydroxypropan-2-yl)-1-methyl-6-oxo[1,6-dihydro[3,3'-bipyridine]]-4-yl]oxy}cyclohexyl)carbamate;
*N*-{[trans-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}acetamide;
methyl {[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
*tert*-butyl {[*cis*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]methyl}carbamate;
5-[2-(4-fluoro-2,6-dimethylphenoxy)-5-(2-hydroxypropan-2-yl)phenyl]-4-{[1-(methanesulfonyl)piperidin-4-yl]amino}-1-methylpyridin-2(1*H*)-one;
*N*-[6-({5-[2-(2,6-dimethylphenoxy)-5-(2-hydroxypropan-2-yl)phenyl]-1-methyl-2-oxo-1,2-dihydropyridin-4-yl}amino)spiro[3.3]heptan-2-yl]acetamide;
*tert*-butyl 3-({5-[2-(2,6-dimethylphenoxy)-5-(2-hydroxypropan-2-yl)phenyl]-1-methyl-2-oxo-1,2-dihydropyridin-4-yl}amino)pyrrolidine-1-carboxylate;
5-[2-(2,6-dimethylphenoxy)-5-(2-hydroxypropan-2-yl)phenyl]-4-{[1-(methanesulfonyl)pyrrolidin-3-yl]amino}-1-methylpyridin-2(1*H*)-one;
*N*-{*trans*-4-[{5-[2-(4-fluoro-2,6-dimethylphenoxy)-5-(2-hydroxypropan-2-yl)phenyl]-1-methyl-2-oxo-1,2-dihydropyridin-4-yl}(methyl)amino]cyclohexyl}acetamide;
4-[(1-acetylpyrrolidin-3-yl)amino]-5-[2-(2,6-dimethylphenoxy)-5-(2-hydroxypropan-2-yl)phenyl]-1-methylpyridin-2(1*H*)-one;
*tert*-butyl [*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-methylacetamide;
*N*-[*trans*-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]acetamide;
*N*-{*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl} acetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}amino)cyclohexyl]acetamide;
methyl {*trans*-4-[(5-{5-(2-hydroxypropan-2-yl)-2-[4-(2-hydroxypropan-2-yl)-2,6-dimethylphenoxy]phenyl}-1-methyl-2-oxo-1,2-dihydropyridin-4-yl)oxy]cyclohexyl}carbamate;
methyl [*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methylcarbamate;
*N*-{[(1*R*,2*S*,3*S*)-3-({5-[2-(4-fluoro-2,6-dimethylphenoxy)-5-(2-hydroxypropan-2-yl)phenyl]-1-methyl-2-oxo-1,2-dihydropyridin-4-yl}oxy)-2-methylcyclopentyl]methyl}acetamide;
5-[2-(4-fluoro-2,6-dimethylphenoxy)-5-(2-hydroxypropan-2-yl)phenyl]-4-[(*trans*-4-hydroxy-4-methylcyclohexyl)amino]-1-methylpyridin-2(1*H*)-one;
5-[2-(4-fluoro-2,6-dimethylphenoxy)-5-(2-hydroxypropan-2-yl)phenyl]-1-methyl-4-{*trans*-4-(2-oxopyrrolidin-1-y1)cyclohexyl|oxy}pyridin-2(1*H*)-one:
methyl [*trans*-4-({5'-(2-hydroxypropan-2-yl)-2'-[4-(2-hydroxypropan-2-yl)-2,6-dimethylphenoxy]-1-methyl-6-oxo[1,6-dihydro[3,3'-bipyridine]]-4-yl}oxy)cyclohexyl]carbamate;
2'-(4-fluoro-2,6-dimethylphenoxy)-5'-(2-hydroxypropan-2-yl)-4-{[1-(methoxyacetyl)piperidin-4-yl]oxy}-1-methyl[3,3'-bipyridin]-6(1*H*)-one;
5-[2-(2,6-dimethylphenoxy)-5-(2-hydroxypropan-2-yl)phenyl]-1-methyl-4-{[*trans*-4-(2-oxopyrrolidin-1-yl)cyclohexyl]oxy}pyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]prop-2-enamide;
5-[2-(4-fluoro-2,6-dimethylphenoxy)-5-(2-hydroxypropan-2-yl)phenyl]-1-methyl-4-{[*trans*-4-(2-oxopiperidin-1-yl)cyclohexyl]oxy}pyridin-2(1*H*)-one;
4-{[*trans*-4-(3,3-dimethyl-2-oxoazetidin-1-yl)cyclohexyl]|oxy}-5-[2-(4-fluoro-2,6-dimethylphenoxy)-5-(2-hydroxypropan-2-yl)phenyl]-1-methylpyridin-2(1*H*)-one;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydropyrimidin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-1-methylcyclohexyl]acetamide;
6-ethyl-5-[2-(4-fluoro-2,6-dimethylphenoxy)-5-(2-hydroxypropan-2-yl)phenyl]-1-methyl-4-{[trans-4-(2-oxopyrrolidin-1-yl)cyclohexyl]oxy}pyridin-2(1*H*)-one;
5-[2-(4-fluoro-2,6-dimethylphenoxy)-5-(2-hydroxypropan-2-yl)phenyl]-1-methyl-4-{[trans-4-methyl-4-(2-oxopyrrolidin-1-yl)cyclohexyl]|oxy}pyridin-2(1*H*)-one:
5-[2-(4-fluoro-2,6-dimethylphenoxy)-5-(2-hydroxypropan-2-yl)phenyl]-1-methyl-4-{[*trans*-4-(2-oxoimidazolidin-1-yl)cyclohexyl]oxy}pyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hex-5-enamide;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]azepan-2-one;
5-[2-(4-fluoro-2,6-dimethylphenoxy)-5-(2-hydroxypropan-2-yl)phenyl]-1-methyl-4-{[*trans*-4-(2-oxo-1,3-oxazolidin-3-yl)cyclohexyl]oxy}pyridin-2(1H)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,3-oxazinan-2-one;
4-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]morpholin-3-one;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyltetrahydropyrimidin-2(1*H*)-one;
5-[2-(4-fluoro-2,6-dimethylphenoxy)-5-(2-hydroxypropan-2-yl)phenyl]-1-methyl-4-{[*trans*-4-(2-methyl-5-oxopyrrolidin-1-yl)cyclohexyl]oxy}pyridin-2(1*H*)-one;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1*H*-isoindole-1,3(2*H*)-dione;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrrolidine-2,5-dione;
2-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}benzoic acid;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]methanesulfonamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-4-hydroxy-2,2-dimethylbutanamide;
4-{[*trans*-4-(3.3-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
*N*-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-3-hydroxypropanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropanecarboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methylcyclopropane-1-carboxamide;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1lambda^{6,2}-thiazolidine-1,1-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methoxyacetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-hydroxyethyl)acetamide;
ethyl 3-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]amino}-3-oxopropanoate;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-oxopropanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2,2-dimethylpropanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N,1-dimethylcyclopropane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*,2,2-trimethylpropanamide;
2'-(4-fluoro-2,6-dimethylphenoxy)-5'-(2-hyydroxypropan-2-yl)-1-methyl-4-{[*trans*-4-(2-oxopyrrolidin-1-yl)cyclohexyl]oxy}[3,3'-bipyridin]-6(1*H*)-one;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxylic acid;
5-[2-(4-fluoro-2,6-dimethylphenoxy)-5-(2-hydroxypropan-2-yl)phenyl]-1-methyl-4-{[*trans*-4-(pyrrolidine-1-carbonyl)cyclohexyl]oxy}pyridin-2(1*H*)-one;
*trans*-*N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(2-methoxyethyl)-1-methylcyclopropane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(3-methoxypropyl)-1-methylcyclopropane-1-carboxamide;
ethyl *N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-*N*-(1-methylcyclopropane-1-carbonyl)glycinate;
5-[2-(4-fluoro-2,6-dimethylphenoxy)-5-(2-hydroxypropan-2-yl)phenyl]-4-{[*trans*-4-(2-hydroxypropan-2-yl)cyclohexyl]oxy}-1-methylpyridin-2(1*H*)-one;
*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carbonitrile;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-(methoxymethyl)cyclopropane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methoxy-2,2-dimethylpropanamide;
*tert-butyl* (3-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[1.1.1]pentan-1-yl)carbamate;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-methyloxetane-3-carboxamide;
5-[2*-*(4-fluoro-2,6-dimethylphenoxy)-5-(2-hydroxypropan-2-yl)phenyl]-4-{[*cis*-4-(2-hydroxypropan-2-yl)cyclohexyl]oxy}-1-methylpyridin-2(1*H*)-one;
*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxylic acid;
*cis*-*N*-ethyl-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
5-[2-(4-fluoro-2,6-dimethylphenoxy)-5-(2-hydroxypropan-2-yl)phenyl]-1-methyl-4-{[*cis*-4-(pyrrolidine-1-carbonyl)cyclohexyl]oxy}pyridin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methylimidazolidine-2,4-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-5-hydroxy-2,2-dimethylpentanamide;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]piperidine-2,6-dione;
(5R)-3-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-1,5-dimethylimidazolidine-2,4-dione;
3-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-1,3-oxazolidine-2,4-dione;
(5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-1,5-dimethylimidazolidine-2,4-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-4-methylcyclohexyl]acetamide;
1-cyano-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropane-1-carboxamide;
*N-*[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]bicyclo[1.1.1]pentane-1-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-methyl-3-oxocyclobutane-1-carboxamide;
1-cyano-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclobutane-1-carboxamide;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpiperidine-2,6-dione;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3,3-dimethylpyrrolidine-2,5-dione;
(7a*S*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-c]imidazole-1,3(2*H*)-dione;
(5*S*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-methyl-1,3-oxazolidine-2,4-dione;
3-amino-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[1.1.1]pentane-1-carboxamide;
5-[2-(4-fluoro-2,6-dimethylphenoxy)-5-(2-hydroxypropan-2-yl)phenyl]-4-{[*cis*-3-(2-hydroxypropan-2-yl)cyclobutyl]oxy}-1-methylpyridin-2(1*H*)-one;
*cis*-*N*-ethyl-3-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclobutane-1-carboxamide;
5-[2-(4-fluoro-2,6-dimethylphenoxy)-5-(2-hydroxypropan-2-yl)phenyl]-1-methyl-4-{[*cis*-3-(pyrrolidine-1-carbonyl)cyclobutyl]oxy}pyridin-2(1*H*)-one;
*cis*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutane-1-carboxylic acid;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
*N'*-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetohydrazide;
*N*-[*trans*-3-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclobutyl]acetamide;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N*-methylcyclohexane-1-carboxamide;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexane-1-carboxamide;
*tert*-butyl(4-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}bicyclo[2.1.1]hexan-1-yl)carbamate;
*tert-butyl* [(1-{[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]carbamoyl}cyclopropyl)methyl]carbamate;
*N'*-[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]acetohydrazide;
*tert*-butyl 2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
*tert*-butyl 2-[*cis*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]hydrazine-1-carboxylate;
*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)-*N*,*N*-dimethylcyclohexane-1-carboxamide;
5-[2-(4-fluoro-2,6-dimethylphenoxy)-5-(2-hydroxypropan-2-yl)phenyl]-1-methyl-4-{[*trans*-4-(5-oxopyrazolidin-1-yl)cyclohexyl]oxy}pyridin-2(1*H*)-one;
*N-*[*trans*-4-({5-[2-(4-fluoro-2,6-dimethylphenoxy)-5-(2-hydroxypropan-2-yl)phenyl]-1-methyl-2-oxo-1,2-dihydropyndin-4-yl}oxy)cyclohexyl]-4-(hydroxymethyl)bicyclo[2.2.2] octane-1-carboxamide;
*N*¹-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]cyclopropane-1,1-dicarboxamide;
4-{[*trans*-4-(2-acetyl-5-oxopyrazolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
*N-*[*trans*-4-({5-[2-(2,6-dimethylphenoxy)-5-{[methyl(methylcarbamoyl)amino]methyl}phenyl]-1-methyl-2-oxo-1,2-dihydropyridin-4-yl}oxy)cyclohexyl]-1-methylcyclopropane-1-carboxamide;
5-[2-(4-fluoro-2,6-dimethylphenoxy)-5-(2-hydroxypropan-2-yl)phenyl]-4-{[*trans*-4-(5-hydroxy-1*H*-pyrazol-1-yl)cyclohexyl]oxy}-1-methylpyridin-2(1*H*)-one;
*N-*[*trans*-4-({5-[5-{[carbamoyl(methyl)amino]methyl}-2-(2,6-dimethylphenoxy)phenyl]-1-methyl-2-oxo-1,2-dihydropyridin-4-yl}oxy)cyclohexyl]-1-methylcyclopropane-1-carboxamide;
*N*-{*trans*-4-[(5-{5-[(1*R*)-1-{acetyl[(1*S*)-1-phenylethyl]amino}ethyl]-2-(2,6-dimethylphenoxy)phenyl}-1-methyl-2-oxo-1,2-dihydropyridin-4-yl)oxy]cyclohexyl}-1-methylcyclopropane-1-carboxamide;
2,2,2-trifluoro-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]acetamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-3,3-dimethylbutanamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-hydroxy-4-methylpentanamide;
4-{[*trans*-4-(4,4-dimethyl-2-oxopyrrolidin-1-yl)cyclohexyl]oxy}-5-[2-(4-fluoro-2,6-dimethylphenoxy)-5-(2-hydroxypropan-2-yl)phenyl]-1-methylpyridin-2(1*H*)-one;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrazolidine-3,5-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl} oxy)cyclohexyl]morpholine-4-carboxamide;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl]oxy)cyclohexyl]-2-oxoimidazolidine-1-carboxamide;
(5*S*)-3-[*trans*-4-({5-[2-(4-fluoro-2,6-dimethylphenoxy)-5-(2-hydroxypropan-2-yl)phenyl]-1-methyl-2-oxo-1,2-dhydropyridin-4-yl}oxy)cyclohexyl]-5-methylimidazolidine-2,4-dione;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H*-imidazo[5,1-c] [1,4]oxazine-1,3(2*H*)-dione;
1-ethyl-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-N'-methylurea;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-3-azabicyclo[3.1.0]hexane-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-6-methylpyridazin-3(2*H*)-one;
(5*R*)-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5-(propan-2-yl)imidazolidine-2,4-dione;
(5*R*)-3-[*trans*-4-({5-[2-(4-fluoro-2,6-dimethylphenoxy)-5-(2-hydroxypropan-2-yl)phenyl]-1methyl-2-oxo-1,2-dₗhydropyridin-4-yl}oxy)cyclohexyl]-5-methylimidazolidine-2,4-dione;
5-ethylidene-3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]imidazolidine-2,4-dione;
5-[2-(4-fluoro-2,6-dimethylphenoxy)-5-(2-hydroxypropan-2-yl)phenyl]-1-methyl-4-{[*trans*-4-(2,3,4,4-tetramethyl-5-oxoimidazolidin-1-yl)cyclohexyl]oxy}pyridin-2(1*H*)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1-(2-methoxyethyl)imidazolidine-2,4-dione;
6-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4,6-diazaspiro[2.4]heptane-5,7-dione;
4-acetamido-*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]bicyclo[2.1.1]hexane-1-carboxamide;
(7*aR*)-2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]tetrahydro-1*H-*pyrrolo[1,2-*c*]imidazole-1,3 (2*H*)-dione;
*N*-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-2-methylalaninamide;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-5,5-dimethylimidazolidine-2,4-dione;
2-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyridazin-3(2H)-one;
3-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-1,5,5-trimethylimidazolidine-2,4-dione;
1-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-2(1*H*)-one;
3-[*trans-*4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]pyrimidin-4(3*H*)-one; and
6-[*trans*-4-({5-[2-(4-fluoro-2,6-dimethylphenoxy)-5-(2-hydroxypropan-2-yl)phenyl]-1-methyl-2-oxo-1,2-dihydropyridin-4-yl}oxy)cyclohexyl]-4-methyl-4,6-diazaspiro[2.4]heptane-5,7-dione.

25. A pharmaceutical composition comprising a therapeutically effective amount of a compound of formula (I) according to any of claims 1 to 24, or a pharmaceutically acceptable salt thereof, in combination with a pharmaceutically acceptable carrier.
